(19) **Europäisches Patentamt / European Patent Office / Office européen des brevets**

(11) **EP 3 874 274 B1**

(12) **EUROPEAN PATENT SPECIFICATION**

(45) Date of publication and mention of the grant of the patent:
**03.01.2024   Bulletin 2024/01**

(21) Application number: **19809947.5**

(22) Date of filing: **31.10.2019**

(51) International Patent Classification (IPC):
**G01N 33/574** *(2006.01)*

(52) Cooperative Patent Classification (CPC):
**G01N 33/57449**

(86) International application number:
**PCT/US2019/059218**

(87) International publication number:
**WO 2020/092808 (07.05.2020 Gazette 2020/19)**

(54) **METHODS FOR PREDICTING A RESPONSE TO BEVACIZUMAB OR PLATINUM-BASED CHEMOTHERAPY OR BOTH IN PATIENTS WITH OVARIAN CANCER**

VERFAHREN ZUR VORHERSAGE DES ANSPRECHENS AUF EINE BEVACIZUMAB- ODER PLATINBASIERTE CHEMOTHERAPIE ODER BEIDEN BEI PATIENTINNEN MIT EIERSTOCKKREBS

PROCÉDÉS DE PRÉDICTION D'UNE RÉPONSE À UNE CHIMIOTHÉRAPIE À BASE DE BÉVACIZUMAB ET/OU DE PLATINE CHEZ DES PATIENTS ATTEINTS D'UN CANCER DE L'OVAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.10.2018   US 201862753274 P**

(43) Date of publication of application:
**08.09.2021   Bulletin 2021/36**

(73) Proprietor: **Regents of the University of Minnesota Minneapolis, MN 55455-2020 (US)**

(72) Inventors:
• **ALIFERIS, Konstantinos**
  **Minneapolis**
  **Minnesota 55455 (US)**
• **WINTERHOFF, Boris Jan nils**
  **Minneapolis**
  **Minnesota 55455 (US)**
• **MA, Sisi**
  **Minneapolis**
  **Minnesota 55455 (US)**
• **WANG, Jinhua**
  **Minneapolis**
  **Minnesota 55455 (US)**

(74) Representative: **Potter Clarkson**
**Chapel Quarter**
**Mount Street**
**Nottingham NG1 6HQ (GB)**

(56) References cited:
**WO-A1-2015/118353      US-A1- 2010 292 303**
**US-A1- 2017 253 933**

• **SAMUEL C. MOK ET AL: "A Gene Signature Predictive for Outcome in Advanced Ovarian Cancer Identifies a Survival Factor: Microfibril-Associated Glycoprotein 2", CANCER CELL, vol. 16, no. 6, 1 December 2009 (2009-12-01), pages 521-532, XP055663336, US ISSN: 1535-6108, DOI: 10.1016/j.ccr.2009.10.018**
• **KRISTIN SPIVEY ET AL: "A prognostic gene signature in advanced ovarian cancer reveals a microfibril-associated protein (MAGP2) as a promoter of tumor cell survival and angiogenesis", CELL ADHESION AND MIGRATION, vol. 4, no. 2, 1 April 2010 (2010-04-01), pages 169-171, XP055663327, US ISSN: 1933-6918, DOI: 10.4161/cam.4.2.11716**

**Description**

GOVERNMENT FUNDING

This invention was made with government support under TR002494 and CA077598 awarded by National Institutes of Health. The government has certain rights in the invention.

BACKGROUND

Epithelial ovarian cancer has the highest mortality rate of all gynecologic cancers with most patients diagnosed with stage III or IV disease. Additionally, up to one-third of patients will not respond to standard initial treatment including cytoreductive surgery and platinum-based chemotherapy. Although significant improvements in median progression-free survival (PFS) have been observed when bevacizumab was added to standard therapy, a subgroup of patients do not benefit from the treatment.

WO 2015/118353 describes a molecular diagnostic test for predicting response to anti-angiogenic drugs and prognosis of cancer.

US 2017/0253933 describes compositions and methods for treating and diagnosing chemotherapy-resistant cancers.

US 2010/0292303 describes a gene expression profile for predicting ovarian cancer patient survival.

Mok et al., 2009 (Cancer Cell, 16(6):521-532) describes how a gene signature predictive for outcome in advanced ovarian cancer identifies a survival factor.

Spivey et al., 2010 (Cell Adhesion and Migration, 4(2):169-171) describes a prognostic gene signature in advanced ovarian cancer reveals a microfibril-associated protein (MAGP2) as a promoter of tumor cell survival and angiogenesis.

SUMMARY OF THE INVENTION

The invention is defined in the claims herein. To the extent that other subject matter is referred to herein, it is included merely for reference purposes. Any references to methods of treatment by therapy or surgery or *in vivo* diagnosis methods herein are to be interpreted as references to compounds, pharmaceutical compositions and medicaments for use in those methods.

This disclosure describes methods of predicting the response of a patient with ovarian cancer to platinum-based chemotherapy and/or treatment with a monoclonal antibody against VEGF-A, bevacizumab (also referred to by the brand name AVASTIN), using clinical and molecular tumor characteristics in patients. This disclosure further provides methods of treating patients with ovarian cancer based on those predictions.

The invention provides bevacizumab for use in treating a patient suffering from ovarian cancer following removal of a tumor, wherein the patient has been predicted to benefit from the administration of bevacizumab by a method comprising:

determining whether the patient is predicted to benefit from the administration of bevacizumab, wherein such determination comprises:

determining the patient's gene expression level of microfibril associated protein 2 (MFAP); and
determining the patient's gene expression level of vascular endothelial growth factor A (VEGFA); and

determining the patient's predicted progression-free survival time with the administration of a platinum-based chemotherapy without bevacizumab, wherein such determination comprises:

determining the patient's gene expression level of microfibril associated protein 2 (MFAP);
determining the patient's gene expression level of vascular endothelial growth factor A (VEGFA);
determining the patient's International Federation of Gynecology and Obstetrics (FIGO) stage;
determining the patient's Eastern Cooperative Oncology Group (ECOG) performance status; and
determining the size of the tumor tissue remaining post-removal of the tumor, wherein:
a gene expression level of MFAP greater than a threshold gene expression level indicates a decreased likelihood of benefit from platinum-based chemotherapy, wherein the threshold gene expression level is selected based on a clinical outcome;
a gene expression level of VEGFA greater than a threshold gene expression level indicates an increased likelihood of benefit from the administration of platinum-based chemotherapy, wherein the threshold gene expression level is selected based on a clinical outcome;

a FIGO stage greater than 1 indicates a decreased likelihood of benefit from platinum-based chemotherapy,

an ECOG performance status greater than 0 indicates a decreased likelihood of benefit from platinum-based chemotherapy, and

a tumor size smaller than 1 cm indicates an increased likelihood of benefit from platinum-based chemotherapy.

In an embodiment of the invention, determining whether the patient is predicted to benefit from the administration of bevacizumab comprises determining whether the patient is predicted to benefit from the administration of bevacizumab in addition to the administration of platinum-based chemotherapy.

In an embodiment of the invention, the clinical outcome comprises increased time of progression-free survival. The predicted increase in progression-free survival may be at least 3 months, at least 4 months, at least 5 months, at least 6 months, at least 7 months, at least 8 months, at least 9 months, or at least 10 months.

In an embodiment of the invention, determining whether the patient is predicted to benefit from the administration of bevacizumab further comprises determining if the patient's predicted progression-free survival time with the administration of a platinum-based chemotherapy and bevacizumab is greater than the patient's predicted progression-free survival time with the administration of a platinum-based chemotherapy without bevacizumab.

The patient may be predicted to benefit from the administration of bevacizumab if the patient's predicted increase in progression-free survival is clinically meaningful. For example, the patient may be predicted to benefit from the administration of bevacizumab if the patient's predicted increase in progression-free survival is at least 3 months, at least 4 months, at least 5 months, at least 6 months, at least 7 months, at least 8 months, at least 9 months, or at least 10 months.

In an embodiment of the invention, determining whether the patient is predicted to benefit from the administration of bevacizumab comprises defining a benefit threshold.

In an embodiment of the invention, determining whether the patient is predicted to benefit from the administration of bevacizumab comprises applying a Cox model.

In an embodiment of the invention, the method comprises administering platinum-based chemotherapy.

In an embodiment of the invention, the tumor comprises a primary tumor or a secondary tumor.

In an embodiment of the invention, the method further comprises:

receiving an identified set of biomarkers determined based on a set of predetermined data comprising clinical data, gene expression data, or both, wherein the identified set of biomarkers comprises at least MFAP2 and VEGFA;

identifying other sets of biomarkers based on the identified set of biomarkers and remaining data comprising the set of predetermined data excluding the identified set of biomarkers; and

generating a signature for each set of biomarkers to predict an outcome for a patient having ovarian cancer,

wherein determining whether the patient is predicted to benefit from the administration of bevacizumab is based on an ensemble prediction using a plurality of signatures and patient test data comprising clinical data, gene expression data, or both.

Also disclosed for reference purposes is a method for treating a patient suffering from ovarian cancer following removal of a tumor. In some embodiments of the disclosure, the method includes determining whether the patient is predicted to benefit from the administration of bevacizumab and, if the patient is predicted to benefit from the administration of bevacizumab, administering bevacizumab. Determining whether the patient is predicted to benefit from the administration of bevacizumab may include determining whether the patient is predicted to benefit from the administration of bevacizumab in addition to the administration of platinum-based chemotherapy.

Determining whether the patient is predicted to benefit from the administration of bevacizumab may include determining the patient's gene expression level of microfibril associated protein 2 (MFAP2) and determining the patient's gene expression level of vascular endothelial growth factor A (VEGFA). Determining whether the patient is predicted to benefit from the administration of bevacizumab may further include at least one of determining the patient's International Federation of Gynecology and Obstetrics (FIGO) stage; determining the patient's Eastern Cooperative Oncology Group (ECOG) performance status; and determining the size of the tumor tissue remaining post-removal of the tumor.

Also disclosed herein for reference purposes is a method for treating a patient suffering from ovarian cancer following removal of a tumor, the method comprising determining whether the patient is predicted to benefit from the administration of a platinum-based chemotherapy and, if the patient is predicted to benefit from the administration of platinum-based chemotherapy, administering platinum-based chemotherapy.

Also disclosed herein for reference purposes is determining whether the patient is predicted to respond to the administration a platinum-based chemotherapy includes determining the patient's gene expression level of microfibril associated protein 2 (MFAP2); determining the patient's International Federation of Gynecology and Obstetrics (FIGO) stage; determining the patient's Eastern Cooperative Oncology Group (ECOG) performance status; and determining the size of the tumor tissue remaining post-removal of the tumor. Determining whether the patient is predicted to benefit from the administration of a platinum-based chemotherapy may further include determining the patient's gene expression level of vascular endothelial growth factor A (VEGFA).

Also disclosed herein for reference purposes is a method that includes identifying a patient with ovarian cancer, and determining the patient's gene expression levels of microfibril associated protein 2 (MFAP2) and vascular endothelial growth factor A (VEGFA) in a biological sample containing cancer cells obtained from the patient, determining the patient's International Federation of Gynecology and Obstetrics (FIGO) stage, determining the patient's Eastern Cooperative Oncology Group (ECOG) performance status, determining the size of the tumor tissue remaining post-removal of a tumor, and calculating a patient risk score for the patient.

Also disclosed herein for reference purposes is a method for predicting the response of a patient with ovarian cancer to treatment with bevacizumab. The method disclosed herein includes: determining gene expression levels of VEGFA and MFAP2; calculating a FIGO numeric score, wherein the FIGO stage is coded as an integer; calculating a surgical outcome score, wherein the score is -1 if the surgical outcome was suboptimal; 0 if the surgical outcome was optimal but tumor tissue smaller than 1 cm remained; or +1 if the surgical outcome was optimal and no visible macroscopic tumor tissue remained; calculating an ECOG score of 0 to 2, based on ECOG performance status; and applying the expression levels, FIGO numeric score, surgical outcome score, and ECOG score to a predictive model that relates the variables with progression-free survival of ovarian cancer; and evaluating an output of the predictive model to predict progression-free survival of the patient.

Also disclosed herein for reference purposes is a method for predicting the response of a patient with ovarian cancer to treatment with bevacizumab wherein the method includes determining gene expression levels of a collection of genes taken from a biological sample of the patient, applying the expression levels to a predictive model that relates the expression levels of the collection of genes the likelihood of progression-free survival of the patient; and evaluating an output of the predictive model to predict the likelihood of progression-free survival of the patient. The collection of genes disclosed herein may comprise at least 80%, at least 90%, at least 95%, at least 98%, or 100% of the genes of any one of Tables 9-12. The collection of genes disclosed herein may comprise the genes of any one of Tables 9-12. The method disclosed herein may further include applying at least one of FIGO stage, surgical outcome, ECOG score, and tumor histology to the predictive model.

Also disclosed herein for reference purposes is a method for predicting progression-free survival of a patient with ovarian cancer. The method may include determining gene expression levels of a collection of genes taken from a biological sample of the patient, applying the expression levels to a predictive model that relates the expression levels of the collection of genes with progression-free survival of ovarian cancer; and evaluating an output of the predictive model to predict progression-free survival of the patient.

The collection of genes may include at least 80%, at least 90%, at least 95%, at least 98%, or 100% of the genes of any one of Tables 6, 7, or 13-68. The collection of genes may include the genes of any one of Tables 6, 7, or 13-68. The method may further include applying at least one of FIGO stage, surgical outcome, ECOG score, and tumor histology to the predictive model.

Also disclosed herein for reference purposes is a method for predicting an outcome for a patient, the method including: receiving an identified set of biomarkers determined based on a set of predetermined data comprising clinical data, gene expression data, or both; identifying other sets of biomarkers based on the identified set of biomarkers and remaining data comprising the set of predetermined data excluding the identified set of biomarkers; generating a signature for each set of biomarkers to predict an outcome for a patient having ovarian cancer; and determining a prediction of an outcome for a patient having ovarian cancer based on one or more signatures and patient test data comprising clinical data, gene expression data, or both.

As used herein, the term "ovarian cancer" is used in the broadest sense and refers to all stages and all forms of cancer arising from the ovary.

As used herein, the term "signature" refers to a computational or mathematical model including a set of variables and corresponding coefficients. The variables may include clinical variables or molecular variables (for example, gene expression) or both. A signature may be used to evaluate patient test data.

As used herein, the term "ensemble" refers to a collection of or catalogue of signatures.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful and is not intended to exclude other embodiments from the scope of the invention. The terms "comprises" and variations thereof do not have a limiting meaning where these terms appear in the description and claims.

Unless otherwise specified, "a," "an," "the," and "at least one" are used interchangeably and mean one or more than one.

Also herein, the recitations of numerical ranges by endpoints include all numbers subsumed within that range (for example, 1 to 5 includes 1, 1.5, 2, 2.75, 3, 3.80, 4, 5, etc.).

For any method disclosed herein that includes discrete steps, the steps may be conducted in any feasible order.

And, as appropriate, any combination of two or more steps may be conducted simultaneously.

The above summary of the present invention is not intended to describe each disclosed embodiment or every implementation of the present invention. The description that follows more particularly exemplifies illustrative embodiments. In several places throughout the application, guidance is provided through lists of examples, which examples can be used in various combinations. In each instance, the recited list serves only as a representative group and should not be interpreted as an exclusive list.

Reference throughout this specification to "one embodiment," "an embodiment," "certain embodiments," or "some embodiments," etc., means that a particular feature, configuration, composition, or characteristic described in connection with the embodiment is included in at least one embodiment of the disclosure. Thus, the appearances of such phrases in various places throughout this specification are not necessarily referring to the same embodiment of the disclosure. Furthermore, the particular features, configurations, compositions, or characteristics may be combined in any suitable manner in one or more embodiments.

All headings are for the convenience of the reader and should not be used to limit the meaning of the text that follows the heading, unless so specified.

BRIEF DESCRIPTION OF THE FIGURES

FIG. 1 shows the methodological benefits (for example, computational modeling advantages) of tying development of precision medicine tests to randomized clinical trials (RCTs).

FIG. 2 shows sequential Nested N-Fold Cross-Validation model selection and error estimation design (NNFCV) used for overfitting-resistant multi-stage analysis as new methods and new data become available.

FIG. 3 shows Kaplan-Meier curves (top) and heatmaps (bottom) corresponding to subgroups and predictor variables in the reduced model identifying patients and subgroups that will benefit the most or the least from bevacizumab, as further described in Example 1.

FIG. 4A - FIG. 4B shows exemplary clinical strategies using precision treatment models/tests as described herein. FIG. 4A identifies a "clear benefit" group that should receive bevacizumab; a "no benefit" group; and an intermediate group with "minor/questionable benefit" from bevacizumab. FIG. 4B shows a strategy that combines the "no benefit" and "minor/questionable benefit" subgroups of FIG. 4A.

DETAILED DESCRIPTION OF ILLUSTRATIVE EMBODIMENTS

The invention is defined in the claims herein. To the extent that other subject matter is referred to herein, it is included merely for reference purposes. Any references to methods of treatment by therapy or surgery or *in vivo* diagnosis methods herein are to be interpreted as references to compounds, pharmaceutical compositions and medicaments for use in those methods. Disclosed herein are methods of determining whether a patient with ovarian cancer is predicted to benefit from platinum-based chemotherapy and/or administration of bevacizumab. The prediction may be based on the patient's clinical characteristics or molecular tumor characteristics or both. Disclosed herein are methods of treating patients with ovarian cancer. The patients disclosed herein may be treated based on the predictions. Also disclosed herein is a method of determining a risk score for a patient with ovarian cancer. Also disclosed herein is predicting progression-free survival of a patient with ovarian cancer. Also disclosed herein is a system, and a kit for performing all or part of the methods described herein.

**Need for and Benefit of a Predictive Test**

Patients are considered platinum-refractory if they progress while on treatment or platinumresistant if their disease recurs less than six months from completion of the initial platinum-based chemotherapy. Even in patients who have a complete initial response to chemotherapy, 80% will recur and eventually develop resistance to multiple drugs and die from drug-resistant disease. Efforts are ongoing to study novel, targeted agents, including bevacizumab, an anti-angiogenic monoclonal antibody against vascular endothelial growth factor (VEGF). Two phase III frontline trials in ovarian cancer (ICON7 and GOG 218) showed statistically significant improvements in median progression-free survival (PFS) of 2.3 and 3.8 months, respectively, when bevacizumab was added to standard first-line chemotherapy (Kommoss et al. Clin Cancer Res Off JAm Assoc Cancer Res. 2017; 23(14):3794-801; Perren et al. N Engl J Med. 2011; 365(26):2484-96.) A subgroup of patients benefits significantly whereas the majority benefit moderately or do not benefit. The problem is further compounded by the high cost of bevacizumab which is currently $400,000 per progression-free life saved in the USA, thus making treatment of all patients economically infeasible. Moreover, the patients who can afford the drug are not necessarily the ones who will benefit from it. These problems underscore the pressing clinical need for more individualized treatment strategies.

At the time of the invention, gene expression analysis of ovarian cancers performed in The Cancer Genome Atlas (TCGA) had led to a molecular classification of ovarian cancer into four subtypes (Tothill et al. Clin Cancer Res Off JAm Assoc Cancer Res. 2008; 14(16): 5198-208; Konecny et al. J Natl Cancer Inst. 2014; 106(10):dju249; Winterhoff et al. Gynecol Oncol. 2016; 141(1):95-100.) These four subgroups have some prognostic significance. (Winterhoff et al. Gynecol Oncol. 2016; 141(1):95-100; Konecny et al. J Natl Cancer Inst. 2014; 106(10):dju249.) Although differential response to bevacizumab and platinum-based chemotherapy within those four molecular subtypes had been demonstrated using formalin-fixed paraffin-embedded (FFPE) tumor samples (Kommoss et al. Clin Cancer Res Off JAm Assoc Cancer Res. 2017; 23(14):3794-801), development and statistical validation of a clinico-molecular stratification model with sufficient accuracy was needed to allow these observations to be clinically actionable. Development of such a model is described in the present disclosure (Example 1).

The potential for health economic impact of a precision test based on the predictivity of the models and corresponding clinical strategies described herein is enormous. For example, if only patients who were predicted to strongly benefit from treatment with bevacizumab were treated instead of all patients, up to $90 billion in savings globally could be realized over 10 years. Moreover, the methods described herein may identify patients who will not benefit from either conventional or bevacizumab treatment, allowing them to be routed to alternative experimental treatments, providing additional survival and economic benefits.

## Determining Gene Expression Levels

In the context of the invention, the methods described herein involve determining a gene expression level of a patient as defined in the claims. To the extent that other determinations of gene expression levels are included herein, they are included merely for reference purposes.

In some embodiments, a gene expression level may be measured using a standard biochemical technique and/or assay and may be converted to a quantitative gene expression level using an appropriate value transformation for that technology. In some embodiments, the gene expression level may be used as an input in a model, as described herein.

In the context of the invention, the genes include microfibril associated protein 2 (MFAP2) and vascular endothelial growth factor A (VEGFA).

Thus, determining a gene expression level of a patient includes determining the gene expression level of a collection of genes taken from a biological sample of the patient.

A collection of genes may include the genes of any one of Tables 6, 7, or 9-68. In some embodiments, some of the genes of a table may be excluded from the collection of genes at the cost of some reduction in predictive performance. In some embodiments, the collection of genes includes at least two genes, at least 14 genes, at least 18 genes, at least 20 genes, or at least 30 genes selected from the genes of any one of Tables 6, 7, or 9-68. In some embodiments, the collection of genes includes at least 50%, at least 60%, at least 70%, at least 80%, at least 90%, at least 95%, at least 98%, or 100% of the genes of any one of Tables 6, 7, or 9-68.

In some embodiments, the collection of genes of any one of Tables 6, 7, or 9-68 may be selected by excluding only those genes of that table that do not significantly affect predictivity.

In some embodiments, the collection of genes may be selected by optimizing predictivity with a constraint or a set of constraints. A constraint may include, for example, cost or user convenience.

In some embodiments, determining a gene expression level includes assessing the amount (for example, absolute amount, relative amount, or concentration) of a gene product in a sample. A gene product may include, for example, a protein or RNA transcript encoded by the gene, or a fragment of the protein or RNA transcript. In some embodiments, determining a gene expression level includes receiving the results of such an assessment. In some embodiments, determining a gene expression level includes converting the results of such an assessment to a quantitative gene expression level.

A sample may include a biological sample of the patient. In some embodiments, the sample may be a biological sample containing cancer cells. For example, the sample may include a tissue sample obtained by biopsy of a patient, a bodily fluid (for example, blood, plasma, serum, urine, etc.), a cell that is the progeny of a patient's tumor cell, or a sample enriched for tumor cells.

The sample may be subjected to a variety of well-known post-collection preparative and storage techniques (for example, fixation, storage, freezing, lysis, homogenization, DNA or RNA extraction, ultrafiltration, concentration, evaporation, centrifugation, etc.) prior to assessing the gene expression level in the sample.

The amount of the gene product may be assessed by any suitable method known to a person having skill in the art. For example, gene expression may be identified using sequencing, quantitative RT-PCR, microarray analysis, and/or immunohistochemistry as described in, for example, U.S. Patent No. 8,725,426 and WO 2015/109234. Standard assay normalization methods and batch effect correction methods suitable to each type of assay may also be employed.

In some embodiments, the method includes normalizing the gene expression levels including, for example, normalizing the level of the RNA transcripts to obtain normalized gene expression levels.

### International Federation of Gynecology and Obstetrics (FIGO) Stage

In the context of the invention, the methods of this disclosure involve determining a patient's International Federation of Gynecology and Obstetrics (FIGO) stage, as described at, for example, www.cancer.org/cancer/ovarian-cancer/detection-diagnosis-staging/staging.html. In some embodiments, the FIGO stage may be coded as an integer for the purposes of calculating a risk score for a patient. For example, FIGO stage IA=1, FIGO stage IB=2, FIGO stage IC=3, FIGO stage IIA=4, FIGO stage IIB=5, FIGO stage IIC=5, FIGO stage IIIA=7, FIGO stage IIIB=8, FIGO stage IIIC=9, and FIGO stage IV=10.

### Eastern Cooperative Oncology Group (ECOG) Performance Status

In the context of the invention, the methods of this disclosure involve determining a patient's Eastern Cooperative Oncology Group (ECOG) performance status. Oken et al. Am J Clin Oncol. 1982; 5:649-655. A patient has an ECOG performance status of 0 if the patient is fully active and able to carry on all pre-disease performance without restriction. A patient has an ECOG performance status of 1 if the patient is restricted in physically strenuous activity but ambulatory and able to carry out work of a light or sedentary nature including, for example, light house work, office work, etc. A patient has an ECOG performance status of 2 if the patient is ambulatory and capable of all selfcare but unable to carry out any work activities and is up and about more than 50% of waking hours. A patient has an ECOG performance status of 3 if the patient is capable of only limited selfcare; confined to bed or chair more than 50% of waking hours. A patient has an ECOG performance status of 4 if the patient is completely disabled.

### Removal of the Tumor and Size of the Tumor Tissue

In the context of the invention, the methods of this disclosure involve treating a patient with bevacizumab, after removal of a tumor for example by surgery. In the context of the invention, the methods of this disclosure include determining the size of a patient's tumor after removal of the tumor.

Removal of the ovarian cancer (including, for example, the tumor) by surgery may include any surgical method undertaken for the removal of cancerous surgery including, for example, hysterectomy, oophorectomy, salpingo-oophorectomy, omentectomy, and/or removal of any visible cancer within the abdomen including, for example, resection of bowel, parts of the liver spleen, a lymph node, diaphragm, parts of the stomach and or pancreas, gallbladder, and any other involved tissue or organ.

In some embodiments, the tumor may be a primary tumor (for example, from the ovary, fallopian tube or primary peritoneum). In some embodiments, the tumor may be a secondary tumor (for example, a metastatic tumor from a different organ to the ovary and or fallopian tube).

In some embodiments, a patient may be characterized based on whether the surgical outcome was suboptimal (that is, tumor tissue greater than 1 centimeter (cm) remained); the surgical outcome was optimal (that is, no tumor tissue greater than 1 cm remained) but tumor tissue smaller than 1 cm remained; or the surgical outcome was optimal and no visible macroscopic tumor tissue remained. In some embodiments, the patient's surgical outcome may be converted to a score (surg_outcome), where surg_outcome is -1 if the surgical outcome was suboptimal; surg_outcome is 0 if the surgical outcome was optimal but tumor tissue smaller than 1 cm remained; and surg_outcome is +1 if the surgical outcome was optimal and no visible macroscopic tumor tissue remained.

### Tumor Histology

In some embodiments, a patient may be characterized based on the histology of the tumor as determined by a pathologist. For example, microscopic examination of tumor tissue by a pathologist may be used to determine whether a patient has a serous borderline ovarian tumor (hist_rev_SBOT) or a metastatic tumor (hist_rev_metastais). If the patient is found to have a tumor (for example, either a serous borderline ovarian tumor or a metastatic tumor), the patient may be assigned a value: 1; if a tumor is present, 0 if a tumor is not present.

### Platinum-Based Chemotherapy and Administration of Platinum-Based Chemotherapy

In some embodiments, the methods described herein include determining whether a patient is predicted to benefit from the administration of platinum-based chemotherapy. In some embodiments, platinum-based chemotherapy is administered to the patient. In some embodiments, platinum-based chemotherapy is administered to a patient if the patient is predicted to benefit from the administration of platinum-based chemotherapy. In some embodiments, platinum-based chemotherapy is administered to a patient in combination with bevacizumab.

Platinum-based chemotherapy may include any suitable platinum-based chemotherapy. Platinum-based chemotherapy may include, for example, one or more of cisplatin, carboplatin, oxaliplatin, nedaplatin, lobaplatin, heptaplatin, triplatin tetranitrate, phenanthriplatin, picoplatin, and satraplatin.

Platinum-based chemotherapy may be administered by any suitable method. The selected dosage level will depend upon a variety of factors including the activity of the particular compound of the present disclosure employed, the route of administration, the time of administration, the rate of excretion of the particular compound being employed, the duration of the treatment, other drugs, compounds and/or materials used in combination with the chemotherapy, the age, sex, weight, condition, general health and prior medical history of the subject being treated, and like factors well known in the medical arts.

**Bevacizumab and Administration of Bevacizumab**

In the context of the invention, bevacizumab is provided for use in treating a patient suffering from ovarian cancer following removal of a tumor, wherein the patient has been predicted to benefit from the administration of bevacizumab as described in the claims. In some embodiments, bevacizumab is administered in combination with platinum-based chemotherapy. In some embodiments, bevacizumab is administered in combination with platinum-based chemotherapy if a patient is predicted to benefit from the administration of bevacizumab in combination with platinum-based chemotherapy.

In the context of the invention determining whether the patient is predicted to benefit from the administration of bevacizumab, comprises:

determining the patient's gene expression level of microfibril associated protein 2 (MFAP2); and
determining the patient's gene expression level of vascular endothelial growth factor A (VEGFA); and
determining the patient's predicted progression-free survival time with the administration of a platinum-based chemotherapy without bevacizumab, as recited in the claims. To the extent that other determinations are described herein, they are included merely for reference purposes. Determining whether a patient is predicted to benefit from the administration of bevacizumab may include using one or more of the sets of variables enumerated in Tables 9-12. A set of variables (that is the set of genes and other biomarkers) as enumerated in one of Tables 9-12 may be used in combination with the corresponding coefficients described in those tables. A set of variables (as enumerated in one of Tables 9-12 may be used in combination with alternative coefficients including, for example, coefficients obtained using a fitting protocol and classifier as described herein.

Determining whether a patient is predicted to benefit from the administration of bevacizumab may include using one or more of the sets of genes enumerated in Tables 9-12. A set of gene as enumerated in one of Tables 9-12 may be used in combination with the corresponding coefficients described in those tables. A set of genes of one of Tables 9-12 may be used in combination with alternative coefficients including, for example, coefficients obtained using a fitting protocol and classifier as described herein.

Determining whether a patient is predicted to benefit from the administration of bevacizumab may include using one or more of the sets of genes enumerated in Tables 9-12.

Bevacizumab may be administered by any suitable method. The selected dosage level will depend upon a variety of factors including the activity of the particular compound employed, the route of administration, the time of administration, the rate of excretion of bevacizumab, the rate of metabolism of bevacizumab, the duration of the treatment, other drugs, compounds and/or materials used in combination with bevacizumab, the age, sex, weight, condition, general health and prior medical history of the subject being treated, and like factors well known in the medical arts.

**Predictive Model**

In the context of the use of bevacizumab of the invention, , it is determined if a patient is predicted to benefit from the administration of bevacizumab, including the administration of bevacizumab in combination with platinum-based chemotherapy. In some embodiments, it is determinined if a patient is predicted to benefit from the administration of a platinum-based chemotherapy (for example, a platinum-based chemotherapy with bevacizumab or a platinum-based chemotherapy without bevacizumab). In some embodiments, it is determined if a patient is predicted to benefit from the administration of bevacizumab in combination with the administration of platinum-based chemotherapy. In the context of the invention, the patient's predicted progression-free survival time with the administration of a platinum-based chemotherapy without bevacizumab is determined. In some embodiments, the difference in progression-free survival of the patient is predicted depending on which therapy is administered.

In the context of the invention the patient's gene expression level of microfibril associated protein 2 (MFAP2) is determined; and the patient's gene expression level of vascular endothelial growth factor A (VEGFA) is determined; and the patient's International Federation of Gynecology and Obstetrics (FIGO) stage is determined; and the patient's Eastern Cooperative Oncology Group (ECOG) performance status is determined; and the size of the tumor tissue remaining post-removal of the tumor is determined.

In some embodiments, a threshold gene expression level of MFAP may be selected based on a clinical outcome (for example, a certain increase in progression free survival), and an expression level greater than that threshold expression may indicate an increased likelihood of benefit from the administration of bevacizumab, In some embodiments, a threshold gene expression level of VEGFA may be selected based on a clinical outcome (for example, a certain increase in progression free survival) and a gene expression level greater than that threshold expression may indicate a decreased likelihood of benefit from the administration of bevacizumab. In some embodiments, a FIGO stage greater than 1 may indicate a decreased likelihood of benefit from the administration of bevacizumab. In some embodiments, an ECOG performance status greater than 0 may indicate an increased likelihood of benefit from the administration of bevacizumab. In some embodiments, a tumor size smaller than 1 cm may indicate an increased likelihood of benefit from the administration of bevacizumab. In some embodiments, a threshold value of the combinations of the MFAP, VEGFA, FIGO stage and ECOG values may be selected based on a clinical outcome (for example, a certain increase in progression free survival) and a value of the combination greater than that threshold expression may indicate a decreased likelihood of benefit from the administration of bevacizumab.

In the context of the invention, a threshold gene expression level of MFAP is selected based on a clinical outcome (for example, a certain increase in progression free survival), and a gene expression level greater than that threshold gene expression level indicates a decreased likelihood of benefit from the administration of platinum-based chemotherapy. In the context of the invention, a threshold gene expression level of VEGFA is selected based on a clinical outcome (for example, a certain increase in progression free survival) and an expression level greater than that threshold gene expression level indicates an increased likelihood of benefit from the administration of platinum-based chemotherapy. In the context of the invention, a FIGO stage greater than 1 indicates a decreased likelihood of benefit from the administration of platinum-based chemotherapy. In the context of the invention, an ECOG performance status greater than 0 indicates a decreased likelihood of benefit from the administration of platinum-based chemotherapy. In the context of the invention, a tumor size smaller than 1 cm indicates an increased likelihood of benefit from the administration of platinum-based chemotherapy. In some embodiments, a threshold value of the combinations of the MFAP, VEGFA, FIGO stage and ECOG values may be selected based on a clinical outcome (for example, a certain increase in progression free survival) and a value of the combination greater than that threshold expression may indicate an increased likelihood of benefit from the administration of platinum-based chemotherapy.

A patient's predicted progression-free survival may be determined. For example, a patient's predicted progression-free survival time with the administration of a platinum-based chemotherapy and bevacizumab may be determined. In the context of the invention the patient's predicted progression-free survival time with the administration of a platinum-based chemotherapy without bevacizumab is determined wherein such determination comprises:

> determining the patient's gene expression level of microfibril associated protein 2 (MFAP2);
> determining the patient's gene expression level of vascular endothelial growth factor A (VEGFA);
> determining the patient's International Federation of Gynecology and Obstetrics (FIGO) stage;
> determining the patient's Eastern Cooperative Oncology Group (ECOG) performance status; and
> determining the size of the tumor tissue remaining post-removal of the tumor. To the extent that other determinations of the patient's predicted progression-free survival time with the administration of a platinum-based chemotherapy without bevacizumab, is described herein, it is included merely for reference purposes.

In some embodiments, the patient's predicted progression-free survival time with the administration of a platinum-based chemotherapy and bevacizumab may be compared with the patient's predicted progression-free survival time with the administration of a platinum-based chemotherapy without bevacizumab.

Determining a patient's predicted progression-free survival may include using one or more of the sets of variables enumerated in Table 6, Table 7, or one or more of the sets of variables described in Example 6 (Tables 13-68). A set of variables (that is the set of genes and other biomarkers) as enumerated in one of Tables 6, 7, or 13-68 may be used in combination with the corresponding coefficients described in those tables. A set of variables (as enumerated in one of Tables 6, 7, or 13-68 may be used in combination with alternative coefficients including, for example, coefficients obtained using a fitting protocol and classifier as described herein.

Determining a patient's predicted progression-free survival may include using one or more of the sets of variables enumerated in Table 6, Table 7, or one or more of the sets of variables described in Example 6 (Tables 13-68). A set of variables (that is the set of genes and other biomarkers) as enumerated in one of Tables 6, 7, or 13-68 may be used in combination with the corresponding coefficients described in those tables. A set of variables (as enumerated in one of Tables 6, 7, or 13-68 may be used in combination with alternative coefficients including, for example, coefficients obtained using a fitting protocol and classifier as described herein.

Determining a patient's predicted progression-free survival may include using one or more of the sets of genes enumerated in Table 6, Table 7, or one or more of the sets of genes described in Example 6 (Tables 13-68). A set of gene as enumerated in one of Tables 6, 7, or 13-68 may be used in combination with the corresponding coefficients described in those tables. A set of genes of one of Tables 6, 7, or 13-68 may be used in combination with alternative coefficients including, for example, coefficients obtained using a fitting protocol and classifier as described herein.

In some embodiments, it is determined whether a patient's predicted increase in progression-free survival time with the administration of a platinum-based chemotherapy and bevacizumab compared to the patient's predicted progression-free survival time with the administration of a platinum-based chemotherapy without bevacizumab is clinically meaningful. In some embodiments, a "clinically meaningful" increase in progression-free survival time may be determined by the treating physician. In some embodiments, the method may include defining a benefit threshold.

In some embodiments, a patient may be predicted to benefit from the administration of bevacizumab if the patient's predicted increase in progression-free survival is at least 3 months, at least 4 months, at least 5 months, at least 6 months, at least 7 months, at least 8 months, at least 9 months, or at least 10 months.

In some embodiments, a model may be applied for modeling time-to-event outcomes (for example, progression-free survival). Any model suitable for modeling time-to-event outcomes may be used including, for example, a Cox model or an accelerated failure time model. In some embodiments, a model may be applied for modeling binary outcomes (for example, progression-free survival up to a certain time point). Any modeling procedure suitable for modeling binary outcomes may be used including, for example, a support vector machine model or another classification method appropriate for biomedical data classification. In some embodiments, Nested N-Fold Cross-Validation (NNFCV) may be applied.

In some embodiments, a patient risk score may be calculated. For example, in some embodiments, a patient's risk score may be calculated as described in Example 3.

In some embodiments, a patient's risk of recurrence at time t may be calculated. For example, in some embodiments, a patient's risk of recurrence at time t may be calculated as described in Example 3.

In some embodiments, a patient's gene expression level (or levels) is applied to a predictive model that relates the expression level (or levels) with progression-free survival of ovarian cancer. In some embodiments, the expression levels of a collection of genes are applied to a predictive model that relates the expression levels of that collection of genes with progression-free survival of ovarian cancer. Examples of such collections of genes are provided herein. In some embodiments, the method may further include applying, or determining and applying one or more of: the patient's International Federation of Gynecology and Obstetrics (FIGO) stage; the patient's Eastern Cooperative Oncology Group (ECOG) performance status; the size of the tumor tissue remaining post-removal of the tumor; tumor histology indicating a serous borderline ovarian tumor (hist_rev_SBOT); and tumor histology indicating a metastatic tumor (hist_rev_metastasis).

In some embodiments, the expression level of a gene or a collection of genes are determined multiple times.

In some embodiments, an additional biomarker of progression-free survival of the patient is detected. Such biomarkers may include, for example, a germline mutation, a somatic mutation, a DNA methylation marker, and/or a protein marker.

**Predictive Ensemble Model**

Methods for predicting an outcome for a patient include receiving an identified set of biomarkers determined based on a set of predetermined data including clinical data, gene expression data, or both; identifying other sets of biomarkers based on the identified set of biomarkers and remaining data includes the set of predetermined data excluding the identified set of biomarkers; generating a signature for each set of biomarkers to predict an outcome for a patient having ovarian cancer; and determining a prediction of an outcome for a patient having ovarian cancer based on one or more of the signatures and patient test data including clinical data, gene expression data, or both.

The identified set of biomarkers may be determined to have optimal predictivity. The identified set of biomarkers may also be determined to have non-redundancy and may be described as a "Markov Boundary" biomarker set.

The outcome relates to progression-free survival for a patient with ovarian cancer. The outcome relates to benefitting from the administration of bevacizumab, platinum-based chemotherapy, or both for a patient with ovarian cancer.

Any suitable identified set of biomarkers may be used. In some embodiments, the identified set of biomarkers is a member of an ensemble, which is described herein in more detail. In some embodiments, the signatures of the ensemble include some or all genes of any one of Table 6, Table 7, and Tables 9-68.

A TIE* algorithm (or other multiplicity discovery technique) may be used to identify the remaining Markov Boundary sets of biomarkers in the data other than the previously identified set of biomarkers. In some embodiments, identifying other sets of biomarkers includes feeding the previously identified set of biomarkers and remaining data into a TIE* algorithm to provide the other equivalent sets of biomarkers. In particular, the TIE* algorithm may provide equivalent sets of biomarkers to the previously identified set of biomarkers. Any other appropriate biomarker and signature multiplicity discovery technique may be used in place of the TIE* algorithm known to one skilled in the art having the benefit of this disclosure.

Any suitable instantiation of the TIE* algorithm (or algorithms with similar functionality) may be used. (Statnikov and Aliferis. PLoS Computational Biology 2010; 6(5), p.e1000790; U.S. Patent 8,805,761; Aliferis et al. Journal of Machine Learning Research 2010; 11(Jan), pp. 171-234; Statnikov et al. Journal of Machine Learning Research 2013; 14(Feb), pp.499-566; U.S. Patent 8,655,821.)

The TIE* algorithm may systematically examine information equivalences in the "seed" biomarker set (and by extension to all corresponding optimal signatures) with variables in the remainder of the data (for example, full set of variables minus the seed). Replacement of a subset of the "seed" and execution of a subroutine may be performed to identify the Markov Boundary set of biomarkers in the remainder of the data (for example, running the subroutine once for each time a subset of the "seed" is excluded). The replacement of the subset of the "seed" and execution of the subroutine may be repeated recursively until all existing sets of biomarkers have been identified and output by the TIE* algorithm. As the TIE* algorithm, traverses the space of replacement subsets the remainder of the data shrinks. The TIE* algorithm may terminate when no biomarker replacement can generate new equivalent biomarker sets.

Generating a signature for each set of biomarkers sets identified by TIE* (or other multiplicity algorithm) may include feeding each set of biomarkers into a machine learning classifier fitting and model "pipeline". The pipeline may incorporate model selection and error estimation. The pipeline may apply one or more of the following: a repeated nested n-fold cross validation with grid parameter choice, a support vector machine classifier, a random forest classifier, a lasso classifier, or any other suitable technique in the field of "omics" based classification by molecular signature construction. The output of the TIE* algorithm may provide a catalogue, or database, of biomarker sets.

Each set of biomarkers may be fed into a machine learning classifier fitting and model pipeline that typically incorporates model selection and error estimation. (Statnikov. A gentle introduction to support vector machines in biomedicine: Theory and methods; Vol. 1. World Scientific Pub. Co.; 2011; Statnikov et al. A Gentle Introduction to Support Vector Machines in Biomedicine: Volume 2: Case Studies and Benchmarks. World Scientific Pub. Co.; 2013.) One or more methods for deriving signatures, or models, from datasets may be used. Different models may be generated by the pipeline. Different models can be generated by a machine learning classifier fitting and model pipeline. Different models can have the same underlying sets of biomarkers but with different coefficients for each biomarker in the set. For example, a plurality of classifier models can be produced for each set of biomarkers, each having different coefficients. Although the models may have different coefficients, the models can be constructed so that they will have functional (input-output) equivalency. Further, coefficients in each model may be refit as new data is acquired.

Still further, coefficients may be tuned to a particular measuring platform used to generate the biomarker data, such as clinical or gene expression data. Different measuring platforms may require slightly different coefficients.

The output of the pipeline for each set of biomarkers, or each member of the equivalency catalogue, may be used as a signature for predicting patient outcomes, for example, in response to treatment. Typically, a signature does not include data used for its construction or validation. These signatures may be implemented as a companion test, or companion diagnostic, according to usual methods that combine: assaying of the biomarkers from tumor tissue specimens and processing of the generated measurements via fitting and application of classifiers to create clinical decision guidance and support that is delivered in clinical practice.

The signatures may be statistically indistinguishable from one another for a particular predictivity level. Each signature may be a minimal (for example, non-reducible without degradation of predictivity) set of biomarkers for a particular predictivity level.

The catalogue of signatures may be described as an ensemble. Determining a prediction of an outcome for a patient having ovarian cancer may be based on an ensemble prediction using a plurality of the signatures. The catalogue of signatures may be used to provide an ensemble prediction. Use of the ensemble prediction may reduce, or even minimize, the variance of prediction accuracy when compared to using single signatures.

In one example, the ensemble prediction may average outputs of each of the signatures. A prediction may be obtained from every signature in the catalogue, and the predictions may be averaged to obtain a consolidated ensemble prediction.

In another example, the ensemble prediction may use a plurality of the signatures based on available patient test data. A prediction may be obtained from only a select number of signatures in the catalogue, or ensemble, and the predictions may be averaged to obtain a consolidated ensemble prediction. The signatures may be selected based on availability. One or a few signatures (for example, up to the full ensemble) may be used for prediction. Factors contributing to availability, or choice of signature to use, may include one or more of: convenience, cost, and ease of collection. The companion test may be personalized or customized for different patients by means of choice of members of the ensemble of signatures.

### Testing Whether A Signature Belongs in the Ensemble

A signature may be tested by a party who does not have a full ensemble to determine whether the signature belongs in an existing ensemble used to predict a particular outcome. In one example, when the full ensemble of signatures is known the inventor simply needs perform a table lookup for the signature against the ensemble. When the ensemble is not disclosed a method may determine whether the signature belongs to the existing ensemble even if all the signatures in the ensemble are unknown to the party. In general, determining the full ensemble (for example, determining all the equivalent sets of biomarkers.

The predictivity level of a signature under consideration may be determined as being statistically indistinguishable from the known predictivity of the existing set of signatures in the ensemble. Any suitable statistical technique for testing differences of predictivity measures of classifiers may be used to compare the predictivity levels to determine whether the predictivity levels are statistically indistinguishable as known to one skilled having the benefit of this disclosure. (Statnikov et al. A Gentle Introduction to Support Vector Machines in Biomedicine: Volume 2: Case Studies and Benchmarks. World Scientific Pub. Co.; 2013.)

It may be determined whether new signature is minimal for the related predictivity level. Minimality of the new signature may be established by testing and verifying that removal of at least one subset of markers does not leave the predictivity level intact.

If the signature has a predictivity level that is statistically indistinguishable from the predictivity of signatures in the existing ensemble and the signature is minimal, then the signature may be determined to belong in the existing ensemble.

If the new signature has a predictivity level that is statistically distinguishable, then the signature is not part of the ensemble.

If the new signature has a predictivity level that is statistically indistinguishable from a known signature in the existing ensemble but is not minimal, then the method may determine that the signature includes a signature that is part of the existing ensemble (whether known or unknown) plus some noise, or redundant markers. Noise or redundant markers may be described as adding no predictive value to the signature of the ensemble.

In general, the addition of biomarkers beyond the minimal level required for optimal predictivity should not confer any predictive advantage and thus would not constitute an enhanced or otherwise improved signature. Therefore, any predictively optimal biomarker set and signature that is minimal also corresponds to a large number of biomarker sets and signatures that may be constructed by "padding" essential biomarkers with predictively unnecessary (and potentially costly and cumbersome) biomarkers.

### Apparatus and Systems

Merely for reference purposes, the present disclosure further provides exemplary apparatuses and systems for executing all or part of the methods described herein. Also disclosed herein is an apparatus which may include, for example, a computer, a processor, or a group of processors. Also disclosed herein is an apparatus which may include a microarray, a sequencer, and/or a device capable of performing PCR. Also disclosed herein is a system which may include, for example, a computer program, a computer-readable medium, or an algorithm.

### Kits

Merely for reference purposes, this disclosure describes a kit that may be used to perform all or part of a method described herein. A kit disclosed herein may include reagent suitable for determining gene expression levels. A kit disclosed herein may include a system for executing a computer program described herein.

### Exemplary Method Embodiments Including Administration of a Platinum-Based Chemotherapy, included herein for reference purposes only.

1. A method for treating a patient suffering from ovarian cancer following removal of a tumor, the method comprising: determining whether the patient is predicted to benefit from the administration of a platinum-based chemotherapy, wherein such determination comprises:

determining the patient's gene expression level of microfibril associated protein 2 (MFAP2);
determining the patient's International Federation of Gynecology and Obstetrics (FIGO) stage;
determining the patient's Eastern Cooperative Oncology Group (ECOG) performance status; and

determining the size of the tumor tissue remaining post-removal of the tumor; and
if the patient is predicted to benefit from the administration of platinum-based chemotherapy, administering platinum-based chemotherapy.

2. The method of Embodiment 1, wherein determining whether the patient is predicted to benefit from the administration of a platinum-based chemotherapy further comprises:
determining the patient's gene expression level of vascular endothelial growth factor A (VEGFA).

3. The method of Embodiment 2, wherein

a gene expression level of MFAP greater than a threshold gene expression level indicates a decreased likelihood of benefit from the administration of platinum-based chemotherapy, wherein the threshold gene expression level is selected based on a clinical outcome;
a gene expression level of VEGFA greater than a threshold gene expression level indicates an increased likelihood of benefit from the administration of platinum-based chemotherapy, wherein the threshold gene expression level is selected based on a clinical outcome;
a FIGO stage greater than 1 indicates a decreased likelihood of benefit from platinum-based chemotherapy,
an ECOG performance status greater than 0 indicates aa decreased likelihood of benefit from platinum-based chemotherapy, and
a tumor size smaller than 1 cm indicates an increased likelihood of benefit from platinum-based chemotherapy.

4. The method of Embodiment 3, wherein the clinical outcome comprises increased time of progression-free survival.

5. The method of Embodiment 4, wherein the patient's predicted increase in progression-free survival is at least 3 months, at least 4 months, at least 5 months, at least 6 months, at least 7 months, at least 8 months, at least 9 months, or at least 10 months.

6. The method of any one of the preceding Embodiments, wherein determining whether the patient is predicted to benefit from the administration of a platinum-based chemotherapy further comprises determining the patient's predicted progression-free survival time.

7. The method of any one of the preceding Embodiments, wherein determining whether the patient is predicted to benefit from the administration of a platinum-based chemotherapy comprises applying a Cox model.

8. The method of any one of the preceding Embodiments, wherein the method comprises administering bevacizumab.

9. The method of any one of the preceding Embodiments, wherein the tumor is a primary tumor.

**Exemplary Method Embodiments Including Calculating a Quantitative Score, included herein for reference purposes only.**

1. A method comprising:

identifying a patient with ovarian cancer;
determining a patient's gene expression levels of microfibril associated protein 2 (MFAP2) and vascular endothelial growth factor A (VEGFA) in a biological sample containing cancer cells obtained from the patient,
determining the patient's International Federation of Gynecology and Obstetrics (FIGO) stage,
determining the patient's Eastern Cooperative Oncology Group (ECOG) performance status,
determining the size of the tumor tissue remaining post-removal of a tumor, and
calculating a patient risk score for the patient.

2. The method of Embodiment 1, wherein the patient risk score (recurrence_score) is calculated as follows:

recurrence_score = 0.31*figo_numeric-

0.35*surg_outcome+0.23*MFAP2+0.48*ECOG+0.19* VEGFA*Bevacizumab-

0.15*MFAP2*Bevacizumab-0.44*ECOG*Bevacizumab

wherein figo_numeric = FIGO stage coded as integers,
wherein surg_outcome is -1 if the surgical outcome was suboptimal; 0 if the surgical outcome was optimal but tumor tissue smaller than 1 cm remained; or +1 if the surgical outcome was optimal and no visible macroscopic tumor tissue remained;
wherein MFAP2 = gene expression level of MFAP2;
wherein ECOG = ECOG performance status; and
wherein VEGFA = gene expression level of VEGFA.

3. The method of Embodiment 1 or 2, the method further comprising calculating the patient's risk of recurrence at time t ($\lambda(t)$) wherein

$$\lambda(t) = \lambda_0(t)e^{recurrence\_score}$$

wherein $\lambda_0(t)$ is the baseline hazard function estimated with a non-parametric strategy.

4. The method of any one of the preceding Embodiments, wherein determining the expression levels of MFAP2 and VEGFA comprises measuring levels of RNA transcripts

5. The method of Embodiment 4, wherein the method further comprises normalizing the level of the RNA transcripts to obtain normalized gene expression levels.

6. The method of any one of the preceding Embodiments, wherein the biological sample containing cancer cells is fixed, paraffin-embedded, fresh, or frozen.

7. The method of any one of the preceding Embodiments, wherein the method further comprises computing the patient's risk of recurrence at time t if the patient receives platinum-based therapy.

8. The method of any one of the preceding Embodiments, wherein the method further comprises computing the patient's risk of recurrence at time t if the patient receives bevacizumab.

9. The method of Embodiment 8, wherein the method comprises calculating the benefit of the patient receiving bevacizumab and platinum-based therapy versus platinum-based therapy without bevacizumab.

10. The method of any one of the preceding Embodiments, wherein the method further comprises administering bevacizumab or platinum-based therapy or both.

11. The method of Embodiment 10, wherein the method comprises administering bevacizumab only if the patient's risk of recurrence at time t of the patient receiving bevacizumab is greater than the patient's risk of recurrence at time t of the patient receiving platinum-based therapy without bevacizumab.

12. The method of Embodiment 11, wherein the difference in the patient's risk of recurrence at time t is at least 1 month, at least 2 months, at least 3 months, at least 4 months, at least 5 months, or at least 6 months.

The present invention is illustrated by the following examples. To the extent that any of the following examples contain subject matter that falls outside the scope of the accompanying claims, the subject matter is included merely for reference purposes.

EXAMPLES

**EXAMPLE 1**

To address which ovarian cancer patients will benefit from bevacizumab and which ovarian cancer patients will benefit from conventional platinum-based chemotherapy, predictive and causal models attributing treatment benefit and predicting benefit from alternate treatment paths were developed. The development included determining the relative information value of clinical and of molecular information and how to optimally combine them with the goal of creating viable clinical strategies that incorporate health economics constraints so that all patients who benefit from bevacizumab will receive it and those who will not benefit, will not burden the health care system and will not suffer adverse reactions and toxicities.

*A. Tying modeling to Randomized Clinical Trials (RCTs) facilitates estimating clinical benefits of alternative treatments.*

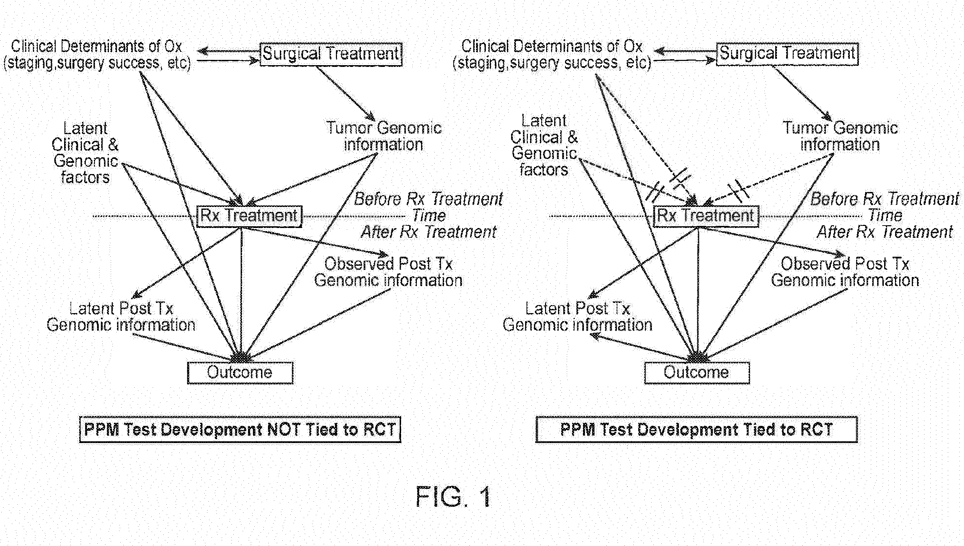

In designs where treatments are not randomized (left panel of FIG. 1) the effects of the treatment post-surgery are confounded by observed and latent (unmeasured) clinical and genomic factors. Whereas a variety of design and analytic solutions exist (including matching to known confounders, analytical control of known and suspected confounders, propensity scoring, and causal graph-based do-calculus), they leave open the possibility of residual confounding (matching, analytical controls), are subject to bias (propensity scoring), are subject to undetectable latent confounding (all methods), or are not practical to apply in genome-wide scale (do-calculus).

In contrast, development of a precision test based on a randomized clinical trial (RCT) design eliminates confounding both from measured and latent variables. The causal effects of posttreatment factors regardless of observed or latent status are incorporated into the total estimated causal effect of the treatment variables. When factors co-determining the outcome are observed, they can be used a covariates in models that individualize the predicted effect on outcome on the basis of these measured factors.

*B. Nested N-Fold Cross-Validation (NNFCV) model selection and error estimation design allows for sequential (phased) modeling without overfitting of model error estimates.*

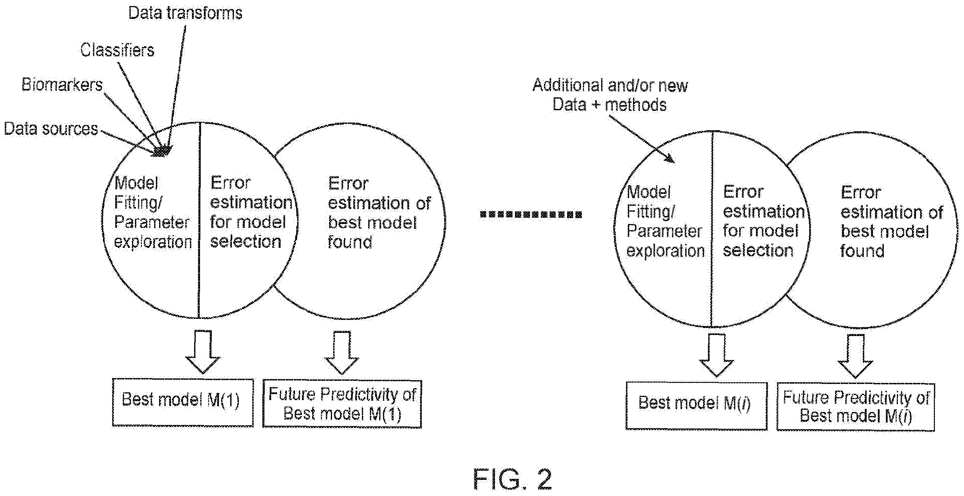

Nested N-Fold Cross-Validation (NNFCV) is an established state-of-the-art design for powerful model selection and unbiased error estimation. But an aspect of this design that is not widely recognized is its ability to perform an analysis in stages as new data and methods become available without overfitting the error estimates of the best models. (*See* FIG. 2.) This ability is achieved because each time the new models or data compete with the older ones against multiple internal validation tests, without ever accessing the final test set. Only after a winning model has been found, the error estimates are produced up to that round of analysis. This estimate never affects the choice of best model(s) thus avoiding overfitting. In a multi-center, multi-investigator, multi-modality, setting with data obtained in discrete stages, with evolving analytical methods, and with expanding molecular assays, the ability for ongoing, sequential analyses is very important.

*C. Data & Specimens*

Specimens and clinical data for the present study come from the OVAR-11 (German part of the ICON-7 phase III RCT). (Kommoss et al. Clin Cancer Res Off JAm Assoc Cancer Res. 2017; 23(14):3794-801; Perren et al. N Engl J Med. 2011; 365(26):2484-96.) Clinical data used for analysis were: age, race, International Federation of Gynecology and Obstetrics (FIGO) stage, histology, treatment, progression-free survival (PFS), overall survival (OS), surgical outcome (for example, debulking status), Eastern Cooperative Oncology Group (ECOG) performance status, independent path review diagnosis and visits.

Specimens were randomly allocated to RNA extraction and assay run order. In brief, 200 ng of RNA was analyzed using the Illumina Whole-Genome DASL array with the HumanRef-8 Bead Chip with 29K gene transcripts or 21K unique genes according to the manufacturer's protocol. (Kommoss et al. Clin Cancer Res Off JAm Assoc Cancer Res. 2017; 23(14):3794-801.) Gene expression data quality was assessed via residual minus vs average plots, box plots and jitter plots, to detect experimental artifacts such as batch effects. In addition, numerical measures such as stress and dfbeta, and measures of the magnitude of change due to normalization, were utilized. (Kommoss et al. Clin Cancer Res Off JAm Assoc Cancer Res. 2017; 23(14):3794-801; Konecny et al. J Natl Cancer Inst. 2014; 106(10):dju249.)

*D. Classifiers and Causal effect modeling - Supervised dichotomous prediction models for PFS.*

Models were built that predict whether patients would relapse within 12, 24, 36, 48, and 60 months from entering the trial and receiving treatment. This analysis excluded patients that dropped out before each prediction point and they were relapse negative. Support Vector Machines (SVMs) (Vapnik V. The Nature of Statistical Learning Theory. 2nd ed. New York: Springer-Verlag; 2000; Boser et al. A Training Algorithm for Optimal Margin Classifiers. In: Proceedings of the Fifth Annual Workshop on Computational Learning Theory. New York, NY, USA: ACM; 1992. p. 144-152. (COLT '92)) with polynomial kernel of degree from 1 to 3, c parameter from 0.1, 1 and 10 optimized with a nested 10-fold cross-validation (NNFCV, that is, inner fold performing grid model selection and outer fold providing unbiased estimates of generalization error measure via ROC AUC) were used.

Features entering the analysis included: clinical variables (n=20), and gene expression microarray variables (n=29,000).

Feature selectors for binary prediction models explored: all features, Markov Boundary induction (via HITON-PC (Aliferis et al. J Mach Learn Res. 2010; 11: 171-234; Aliferis et al. J Mach Learn Res. 2010; 11:235-284) with fixed k parameter to 1), and the 106 ovarian cancer genes from the CLOVAR signature obtained by TCGA analysis and previously reported (Konecny et al. J Natl Cancer Inst. 2014; 106(10):dju249; Verhaak et al. J Clin Invest. 2013; 123(1):517-25).

Multi-modal data combination strategies_for clinical+gene expression data included: clinical only, gene expression only and clinical+gene expression in a single input vector. Feature selection and multi-modal combinations evaluation were fully nested in the NNFCV to avoid over-fitting the genes selected to the data.

### E. Classifiers and Causal effect modeling - Time-to-event models that predict risk of relapse under different treatments and identify the patients that will benefit from bevacizumab.

Cox modeling combined with Markov Boundary induction (Aliferis et al. J Mach Learn Res. 2010; 11:171-234; Aliferis et al. J Mach Learn Res. 2010; 11:235-284) was used for feature selection to model the risk for relapse as a function of treatment and of other measured possible determinants of relapse. Cox modeling uses all available information whereas dichotomous prediction at a fixed time point methods discard information due to censoring. (Efron J Am Stat Assoc. 1977; 72(359):557-65.) Because the data came from a randomized trial, all possible confounders effects relating treatment and outcome were eliminating by randomization, thus the estimation of the treatment effect does not require an adjustment for confounders. The multivariate analysis separates the effect of treatment from the effect of other measured co-determinants of relapse, however. The interaction terms were constructed between potential co-determinants of relapse and the treatment. A significant interaction effect indicates a differential treatment effect for different values/levels of a co-determinant, thus results in differential treatment response from patients.

Once a model was fit, the model setting bevacizumab=yes was used as a prognostic model for the group receiving bevacizumab to estimate the outcome in that group. Similar for bevacizumab=no. The difference between the model risk predictions for individual patients setting bevacizumab=yes and then bevacizumab=no was calculated to estimate the benefit of receiving bevacizumab (for example, patients for which the estimated risk difference is negative will benefit from bevacizumab). 100-repeated 20-fold nested cross-validation was used. Treatment effects were then estimated for every subject in the testing set. Different threshold values were applied on the estimated treatment effect to group people into three groups: (1) predicted to strongly benefit; (2) predicted to achieve minor benefit; or (3) predict to not benefit. For patients in each of the three groups, the actual observed benefit in terms of relapse between the treated and untreated patients was compared. The relapse outcome was evaluated with Hazard Ratio (HR) and median survival difference between treatment and control. (Clark et al. Br J Cancer. 2003; 89(2):232-8.)

Markov Boundary induction (GLL-PC instantiated with a Cox regression model as the conditional independent test used by the algorithm (Aliferis et al. J Mach Learn Res. 2010; 11:171-234; Aliferis et al. J Mach Learn Res. 2010; 11:235-284), referred to as *GLL-PC-Cox*) combined with a knowledge-driven gene selection strategy was used for knowledge-driven and de novo feature selection for Cox modeling as follows: genes related to VEGF were selected from the literature and pathway databases strictly based on literature support without reference to the data in hand.

The following genes were selected: VEGFA, VEGFR2, VEGFB, VEGFC, VEGFR1, VEGFR3, CLDN6, TUBB2B, FGF12, MFAP2, and KIF1A. In the dataset, there are 16 probes measuring 9 of the above genes. A candidate set comprising the 16 gene probes + clinical data variables was formed, and Markov Boundary induction was applied on that set using Cox as a conditional independence test when performing feature selection, and then the selected features were fitted with a Cox model. All these steps were fully embedded inside the inner loop of the NNFCV design.

### F. Results

#### 1. Prognostic Models (binarized time points)

Models predicting Progression-Free Survival (PFS) with predictivities and selected feature types/numbers are shown in Table 1. In bold are models with sufficient predictivity to be potentially clinically actionable. The best models have sufficient predictivity to support for clinically actionable prognosis since they match the predictivity of other FDA-approved precision tests. The de novo feature selection resulted in the models having the AUCs indicated in Table 1 and outperformed the predictivity of the 106 genes (CLOVAR signature) previously reported in literature (AUC=0.63). Also notable for this type of model, just 3 clinical variables achieved an AUC of 0.75 (as shown in row 1 of Table 1, column 6). A slightly less predictive model (AUC of 0.74) can be obtained with gene expression only (as shown in row 2 of Table 1, column 6). Because clinical variables are highly subjective, however, these factors may not translate to other providers and could be biased to favor decisions towards specific treatment options. For example, residual disease after surgical cytoreduction is determined by the surgeon and may not translate to other surgeons. This bias could be overcome by using an objective gene expression models. Predictivity was observed to drop after 48 months because many patients had exited the trial at that time.

### *2. Time to Event Model.*

The final Cox Model (complete model) is shown in Table 2. Out of 16 genes + clinical variables and their interaction with the treatment, 7 variables remained in the final model after feature selection with GLL-PC-Cox.

VEGFA, MFAP2, and ECOG have a significant interaction effect with the treatment, indicating that the effects of these variables on progression-free survival depends on if the treatment was administered. For example, MFAP2 show a significant main effect with coefficient of 0.23, a significant interaction with treatment with coefficient of -0.15. In the treatment group, MFAP2 have an overall coefficient of 0.23+(-0.15)*1=0.08 (HR=1.08). In the control group, MRAP2 have an overall coefficient of 0.23+(-0.15)*0=0.23 (HR=1.25).

**Table 1.** Dichotomous prognostic models.

| Time point: | | 12 mo | 24 mo | 36 mo | 48 mo | 60 mo |
|---|---|---|---|---|---|---|
| Models with clinical features only | AUC | 0.71 ± 0.03 | 0.75 ± 0.03 | 0.73 ± 0.02 | 0.75 ± 0.02 | 0.71 ± 0.04 |
| | # of features | 5 | 4 | 4 | 3 | 3 |
| Models with gene expression only | AUC | 0.56 ± 0.03 | 0.58 ± 0.03 | 0.68 ± 0.03 | 0.74 ± 0.03 | 0.42 ± 0.05 |
| | # of features | 149 | 153 | 222 | 215 | 94 |
| Models with clinical + gene expression | AUC | 0.62 ± 0.02 | 0.65 ± 0.03 | 0.72 ± 0.03 | 0.77 ± 0.02 | 0.57 ± 0.03 |
| | # of features | 4 + 149 | 3 + 142 | 3 + 202 | 3 + 176 | 3 + 79 |
| Models with 106 genes from prior work (CLOVAR signature) | AUC | 0.62 ± 0.04 | 0.59 ± 0.03 | 0.62 ± 0.03 | 0.62 ± 0.02 | 0.47 ± 0.06 |
| | # of features | 8 | 4 | 6 | 7 | 2 |

**Table 3:** Examples of using the Cox models to identify patient subgroups that will benefit the most and the least from bevacizumab

| | | Predict to Not Benefit | | | | Gray Zone | | | | Predict to Benefit | | | |
|---|---|---|---|---|---|---|---|---|---|---|---|---|---|
| | | Median Surv Diff | | HR | | Median Surv Diff | | HR | | Median Surv Diff | | HR | |
| Perc. | Thre. | mean | sd | mean | sd | mean | sd | mean | sd | mean | sd | mean | sd |
| 40% | 60% | 1.28 | 1.45 | 0.95 | 0.07 | 7.99 | 4.60 | 0.82 | 0.13 | 7.74 | 0.86 | 0.62 | 0.05 |
| **40%** | **80%** | **1.28** | **1.45** | **0.95** | **0.07** | **5.79** | **2.12** | **0.77** | **0.06** | **9.95** | **1.53** | **0.49** | **0.07** |
| 60% | 80% | 3.34 | 0.77 | 0.90 | 0.04 | 5.63 | 2.49 | 0.73 | 0.12 | 9.95 | 1.53 | 0.49 | 0.07 |

**Table 2.** Time-to-event causal effect and prognostic models.

| Variables | Coef | exp (Coef) | se exp (Coef) | z | pval |
|---|---|---|---|---|---|
| **figo_numeric:** figo stage coded as integers, 10 levels, 1 = IA, 2 = IB, ..., 9 = IIIC, and 10 = IV | 0.31 | 1.37 | 0.06 | 5.58 | 2.39E-08 |
| **surg_outcome:** 3 levels, -1 = suboptimal; 0 = optimal but remaining tissue smaller than 1cm; +1 = optimal or no macroscopic tissue remaining | -0.35 | 0.71 | 0.08 | -4.61 | 3.98E-06 |

(continued)

| Variables | Coef | exp (Coef) | se exp (Coef) | z | pval |
|---|---|---|---|---|---|
| **MFAP2**: gene expression level of MFAP2, Microfibril Associated Protein 2, ranges from 6.7 to 15.9 with mean of 13.1 | 0.23 | 1.26 | 0.06 | 3.70 | 0.000215 |
| **ECOG:** ECOG performance status, 3 levels, 0 = Fully active, able to carry on all pre-disease performance without restriction; 1 = Restricted in physically strenuous activity but ambulatory and able to carry out work of a light or sedentary nature, 2 = Ambulatory and capable of all selfcare but unable to carry out any work activities; up and about more than 50% of waking hours. | 048 | 1.61 | 0.14 | 3.34 | 0.000851 |
| VEGFAxrndid **VEGFA:** gene expression level of MFAP2, Vascular Endothelial Growth Factor A, ranges from 4.9 to 13.3 with mean of 10.5 **Rndid:** 1= bevacizumab+Carboplatin, 0=Carboplatin. VEGFAxrndid, MFAP2xrndid,ECOGxrndid indicate interaction effects. | 0.19 | 1.20 | 0.07 | 2.76 | 0.005818 |
| **MFAP2xrndid** | -0.15 | 0.86 | 0.05 | -2.83 | 0.004651 |
| **ECOGxrndid** | -0.44 | 0.64 | 0.19 | -2.26 | 0.023707 |
| Concordance= 0_693 (se = 0.019 ), Rsquare= 0.281 (max possible= 0.999 ), Likelihood ratio test= 125_2 on 7 df, p=0, Wald test= 97.88 on 7 df, p=0, and Score (logrank) test = 108.7 on 7 df, p=0. | | | | | |

### 3. Identifying subpopulations who benefit from bevacizumab.

By exploring different thresholds on the PFS risk produced by the Cox models, individual patients and subpopulations that will benefit the most, the least, and in between can be identified. Table 3 shows examples of subpopulation identification.

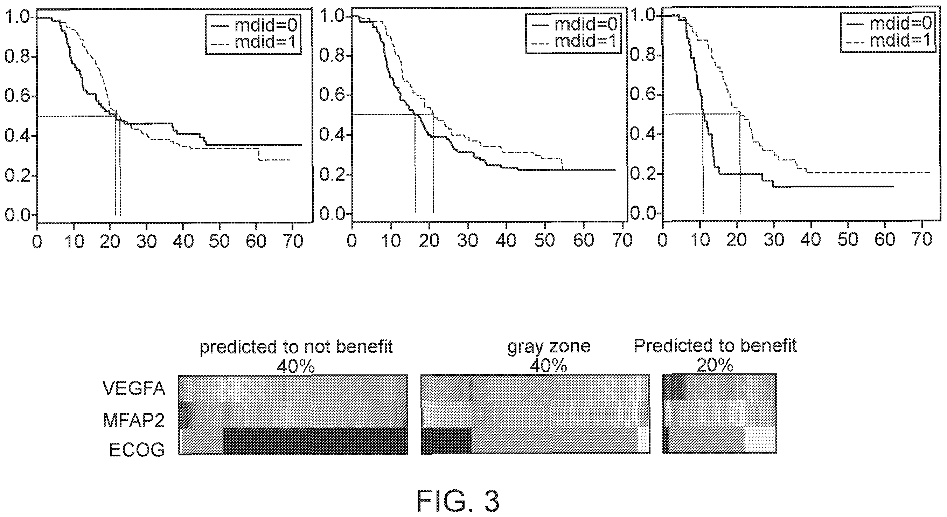

For example, the second row of Table 3 (bolded) depicts separation of a subgroup equal to 20% of the total patient population that will benefit (approximately 10 months for survival), or on the other end a subgroup equal to 40% of the total population without benefit (nominal benefit of 1.3 months which is not statistically significant). FIG. 3 depicts Kaplan-Meier curves (top) and heatmaps (bottom) corresponding to these subgroups and predictor variables in the reduced model. Kaplan-Meier curves (top) and heatmaps (bottom) corresponding to subgroups and predictor variables in the reduced model identifying patients and subgroups that will benefit the most or the least from Bevacizumab. Patients that benefit more from the addition of bevacizumab have lower expression level of VEGF-A, higher expression level of MFAP2 and worse EGOC performance status. Each column in the lower panel indicates a patient. Yellow indicates higher value, green indicates intermediate value and blue indicates lower value. All variables were scaled between 0 to 1 to assist visualization.

### 4. Construction of Treatment Strategies

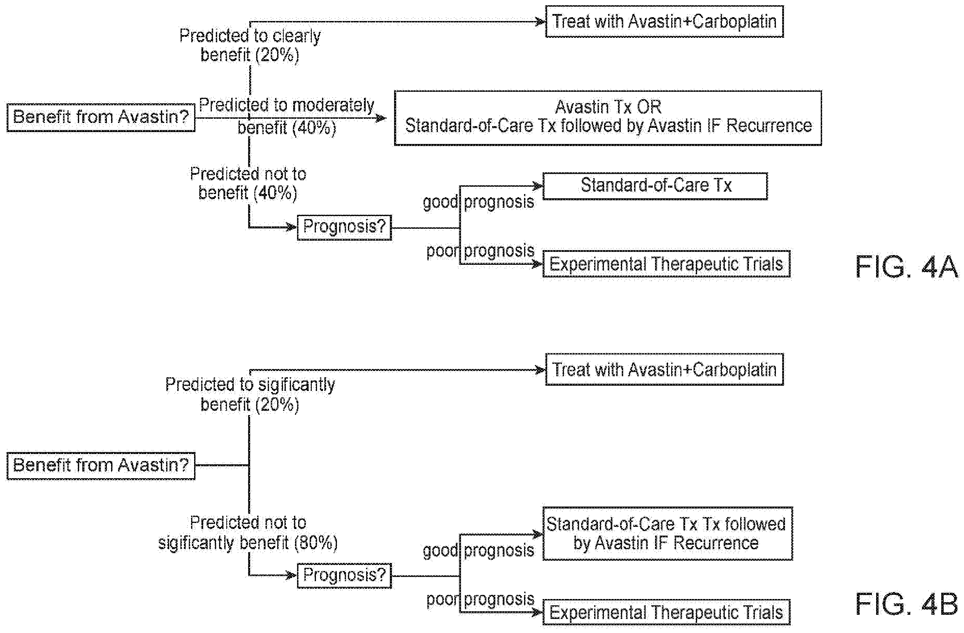

By using the analytical models described in this Example, clinical treatment strategies can be constructed and evaluated. Two possible strategies are depicted in FIG. 4A - FIG. 4B. FIG. 4A identifies a "clear benefit" group that should receive bevacizumab, a "no benefit" group that should receive standard treatment if the dichotomous prognosis models predict good response to Carboplatin or should be routed to experimental therapeutics if predicted response is not good. An intermediate group with "minor/questionable benefit" from bevacizumab may receive standard care plus bevacizumab in case of recurrence. An alternative binary strategy is depicted in FIG. 4B where the "no benefit" and "minor/questionable benefit" groups are merged.

### EXAMPLE 2

As shown in Example 1 and Table 1, models predicting Progression-Free Survival (PFS) were developed. The models exhibiting an AUC of 0.75 or greater are further described in this Example.

Determination of figo _numeric and surg_outcome are described in Table 2. hist_rev_SBOT was determined by microscopic examination of tumor tissue by a pathologist: a patient determined to have a serous borderline ovarian tumor was assigned a value of 1; a patient without a serous borderline ovarian tumor was assigned a value of 0.

hist_rev_metastais was determined by microscopic examination of tumor tissue by a pathologist: a patient determined to have a metastatic tumor was assigned a value of 1; a patient without a metastatic tumor was assigned a value of 0.

The model with 4 clinical features providing an AUC of $0.75\pm0.03$ (row 1, 24 months column of Table 1) included the clinical factors and coefficients shown in Table 4.

Table 4.

| Clinical Factor | Coefficient |
| --- | --- |
| figo_numeric | 0.499594 |
| surg_outcome | 0.000775 |
| hist_rev_SBOT | 2.497971 |
| hist_rev_metastasis | 2.998709 |

The model with 3 clinical features providing an AUC of $0.75\pm0.02$ (row 1, 48 months column of Table 1) included the clinical factors and coefficients shown in Table 5.

Table 5.

| Clinical Factor | Coefficient |
| --- | --- |
| figo_numeric | 0.400073 |
| surg_outcome | 0.00005 |
| hist_rev_SBOT | 2.000265 |

The model with 215 genes (and no clinical features) providing an AUC of $0.74\pm0.02$ (row 2, 48 months column of Table 1) included the genes and coefficients shown in Table 6.

The model with 3 clinical features and 176 genes providing an AUC of $0.77\pm0.02$ (row 3, 48 months column of Table 1) included the genes and coefficients shown in Table 7 and the clinical factors and coefficients shown in Table 8.

Table 8.

| Clinical Factor | Coefficient |
| --- | --- |
| figo_numeric | 0.231416 |
| hist_rev_SBOT | 0.173699 |
| surg_outcome | 0.068338 |

Table 6

| Gene Name | Coefficient | Gene Name | Coefficient | Gene Name | Coefficient | Gene Name | Coefficient |
| --- | --- | --- | --- | --- | --- | --- | --- |
| SERPINB2 | 0.03622 | EEF1E1 | 0.173467 | RNF7 | 0.01282 | IQCA1 | 0.116866 |
| C1orf168 | 0.138901 | PITX2 | 0.115383 | PCSK6 | 0.101694 | TPM2 | 0.069739 |
| MIDN | 0.041086 | ZNF75D | 0.025308 | ABHD3 | 0.054748 | EDN3 | 0.086092 |
| HBA2 | 0.175207 | RARG | 0.190947 | AXL | 0.038725 | ADAMTS1 | 0.000471 |
| MCAM | 0.051688 | UPK3B | 0.106369 | KCNIP3 | 0.171931 | NFATC4 | 0.096882 |
| PLAC9 | 0.076069 | RAD54B | 0.026128 | DSC3 | 0.113964 | EPYC | 0.122943 |
| SELENBP1 | 0.025843 | GAD1 | 0.086734 | C17orf106 | 0.062762 | CD34 | 0.092926 |
| HCFC1R1 | 0.102289 | PPAPDC1A | 0.020161 | KIF3C | 0.018418 | DUT | 0.201835 |
| FAM70A | 0.053427 | MYOHD1 | 0.14274 | PKN1 | 0.147588 | ORC1L | 0.340407 |
| IGSF9 | 0.04932 | FLJ33360 | 0.130302 | TMEM52 | 0.114855 | YARS2 | 0.071752 |
| METRNL | 0.149908 | CALD1 | 0.059619 | KCNQ2 | 0.003826 | OTUD7A | 0.224324 |
| NYX | 0.073665 | C10orf116 | 0.090491 | HPRT1 | 0.155877 | CASP8AP2 | 0.001789 |
| MMP12 | 0.049893 | LBH | 0.055515 | GRIN3A | 0.065821 | PNMAS | 0.009767 |

(continued)

| Gene Name | Coefficient | Gene Name | Coefficient | Gene Name | Coefficient | Gene Name | Coefficient |
|---|---|---|---|---|---|---|---|
| SFN | 0.120181 | KRT80 | 0.005235 | ADORA1 | 0.202699 | NR6A1 | 0.038371 |
| FBXO48 | 0.155071 | ODF2 | 0.035257 | SFRS4 | 0.040789 | NLRP9 | 0.161918 |
| ENPEP | 0.204423 | HIC1 | 0.056785 | PSMC6 | 0.08759 | TAF15 | 0.039363 |
| GJAS | 0.115978 | HDAC7 | 0.062167 | TCEAL8 | 0.087723 | CLDN6 | 0.073599 |
| C17orf58 | 0.161763 | UBR7 | 0.013314 | FAM187B | 0.058209 | CXCL13 | 0.07641 |
| GSR | 0.001917 | BTF3 | 0.148726 | ICAM4 | 0.119818 | WARS | 0.011903 |
| SATB2 | 0.157891 | C11orf24 | 0.033189 | MIR212 | 0.048242 | TESC | 0.064945 |
| TRIM58 | 0.140981 | NTRK2 | 0.02828 | ALS2CL | 0.015398 | CYP1A2 | 0.052665 |
| DNAH11 | 0.0699 | DBNDD2 | 0.228329 | ICAM2 | 0.080758 | TM2D3 | 0.246656 |
| HLXB9 | 0.058337 | VANGL2 | 0.003238 | RARA | 0.027594 | SNORD93 | 0.081411 |
| JUNB | 0.025915 | SERPINB5 | 0.060212 | NFATC3 | 0.103829 | TNFRSF18 | 0.165332 |
| CCL13 | 0.049223 | PRKAA2 | 0.210635 | IL1RAP | 0.10806 | RASGEFLC | 0.124793 |
| FKBP10 | 0.057389 | C8orf79 | 0.081366 | NET1 | 0.032067 | CCR2 | 0.019484 |
| ADAM17 | 0.074427 | XBP1 | 0.119153 | LGI3 | 0.038461 | GMNN | 0.115653 |
| FOSB | 0.011615 | EZH2 | 0.107034 | ARL6IP1 | 0.101664 | ROD1 | 0.073321 |
| EMP1 | 0.014821 | THBS3 | 0.027919 | C17orf58 | 0.092084 | BDNF | 0.033912 |
| C18orf56 | 0.00339 | PLSCR4 | 0.100974 | SHC1 | 0.086425 | NP | 0.150271 |
| MFSD11 | 0.03905 | CDC42BPA | 0.004402 | C11orf49 | 0.195174 | SBSN | 0.15035 |
| TMEM62 | 0.044461 | ERI2 | 0.070412 | GBP7 | 0.052231 | ARMCX3 | 0.072789 |
| TNNT2 | 0.122743 | FMNL3 | 0.207885 | RAP1A | 0.001336 | SPANXD | 0.080842 |
| LRRTM4 | 0.11724 | DNMT3L | 0.194431 | PLEKHGS | 0.142552 | CRYBA1 | 0.095109 |
| NUP155 | 0.027639 | ZSWIM4 | 0.107025 | ALX3 | 0.017065 | TOMM20L | 0.042679 |
| PRSS27 | 0.063727 | HPS4 | 0.079177 | SLC9A10 | 0.038537 | | |
| BMPRLA | 0.124556 | MFRP | 0.094868 | HCG9 | 0.106585 | | |
| HDLBP | 0.050078 | EPHB1 | 0.062946 | LRRC14B | 0.108694 | | |
| SLC25A34 | 0.086934 | SLC23A1 | 0.025963 | DOCK7 | 0.096171 | | |
| PRAMEFS | 0.19769 | C1orf64 | 0.172403 | RNASEK | 0.061792 | | |
| SYTL3 | 0.006225 | PMEPA1 | 0.079342 | ATXN10 | 0.191254 | | |
| ASBS | 0.06092 | CECR4 | 0.145267 | FOXN1 | 0.068077 | | |
| STC2 | 0.028435 | FBXO48 | 0.014442 | MYCN | 0.007338 | | |
| BCAS1 | 0.063785 | NRXN3 | 0.117417 | UBR7 | 0.081387 | | |
| HR | 0.218781 | MACC1 | 0.104212 | SEC22C | 0.233998 | | |
| ADAMTS9 | 0.051007 | PDLIM2 | 0.105603 | FU43752 | 0.084094 | | |
| GBE1 | 0.125008 | HOOK1 | 0.104046 | LOC441150 | 0.075526 | | |
| ESPNL | 0.026457 | CYB5R3 | 0.044329 | MIR654 | 0.132396 | | |
| ZNF114 | 0.11843 | SLC4A5 | 0.080003 | LENEP | 0.035236 | | |
| STC1 | 0.066473 | SOX2 | 0.088092 | MIR571 | 0.142624 | | |
| MANSC1 | 0.114537 | STYX | 0.030971 | HSD11B1 | 0.016267 | | |

(continued)

| Gene Name | Coefficient | Gene Name | Coefficient | Gene Name | Coefficient | Gene Name | Coefficient |
|---|---|---|---|---|---|---|---|
| NT5DC1 | 0.194833 | MIR942 | 0.062775 | C14orf102 | 0.085657 | | |
| MCART6 | 0.064187 | MIA2 | 0.099157 | MIR1914 | 0.133341 | | |
| PANK4 | 0.046483 | KRTAP10.10 | 0.203315 | KIAA0773 | 0.016884 | | |
| GLDN | 0.06358 | XRN2 | 0.110497 | CREB5 | 0.14742 | | |
| BAI1 | 0.067673 | SERPINB6 | 0.163358 | OTOP1 | 0.012675 | | |
| RBP4 | 0.042606 | MIR576 | 0.066863 | EIF2C2 | 0.041661 | | |
| ENO1 | 0.028603 | LOC492303 | 0.107718 | ANO7 | 0.153893 | | |
| FAM13AOS | 0.299714 | GFRA3 | 0.039813 | ANKRD30A | 0.133547 | | |
| SCXB | 0.054135 | LRRC37A4 | 0.16319 | ZNF599 | 0.121019 | | |

Table 7

| Gene Name | Coefficient | Gene Name | Coefficient | Gene Name | Coefficient |
|---|---|---|---|---|---|
| C1orf168 | 0.142046 | GAD1 | 0.050425 | TMEM52 | 0.003004 |
| MIDN | 0.0359 | PPAPDC1A | 0.002159 | KCNQ2 | 0.020539 |
| HBA2 | 0.108688 | MYOHD1 | 0.180576 | HPRT1 | 0.086891 |
| MCAM | 0.04625 | FLJ33360 | 0.205058 | SFRS4 | 0.15813 |
| PLAC9 | 0.124332 | CALD1 | 0.022523 | PSMC6 | 0.083801 |
| SELENBP1 | 0.010922 | C10orf116 | 0.126446 | TCEAL8 | 0.083907 |
| HCFC1R1 | 0.044686 | LBH | 0.026799 | FAM187B | 0.066754 |
| FAM70A | 0.050927 | KRT80 | 0.101739 | ICAM4 | 0.101648 |
| SERPINB2 | 0.025977 | ODF2 | 0.061025 | MIR212 | 0.050117 |
| NYX | 0.033832 | HIC1 | 0.044034 | FOSL2 | 0.041694 |
| MMP12 | 0.009991 | HDAC7 | 0.157829 | ALS2CL | 0.082645 |
| SFN | 0.135709 | UBR7 | 0.046341 | ICAM2 | 0.033457 |
| FBXO48 | 0.188484 | BTF3 | 0.132272 | RARA | 0.019454 |
| ENPEP | 0.290998 | C11orf24 | 0.068234 | NFATC3 | 0.122866 |
| GJAS | 0.200544 | NTRK2 | 0.007944 | IL1RAP | 0.126467 |
| C17orf58 | 0.108486 | DBNDD2 | 0.139397 | LGI3 | 0.062777 |
| GSR | 0.00945 | SERPINBS | 0.072663 | ARL6IP1 | 0.107493 |
| SATB2 | 0.117074 | PRKAA2 | 0.214928 | C17orf58 | 0.032018 |
| TRIM58 | 0.153599 | C8orf79 | 0.087576 | SHC1 | 0.0814 |
| DNAH11 | 0.074143 | XBP1 | 0.148784 | IQCA1 | 0.179486 |
| CCL13 | 0.027153 | EZH2 | 0.08015 | TPM2 | 0.125612 |
| FKBP10 | 0.043095 | THBS3 | 0.008082 | ADAMTS1 | 0.030315 |
| ADAM17 | 0.06098 | PLSCR4 | 0.130711 | NFATC4 | 0.096009 |
| FOSB | 0.023202 | RNF7 | 0.063844 | EPYC | 0.070795 |
| EMP1 | 0.037216 | ABHD3 | 0.106972 | CD34 | 0.113475 |

(continued)

| Gene Name | Coefficient | Gene Name | Coefficient | Gene Name | Coefficient |
|---|---|---|---|---|---|
| C18orf56 | 0.028461 | AXL | 0.107418 | DUT | 0.186273 |
| EEF1E1 | 0.135893 | KCNIP3 | 0.109267 | ORC1L | 0.238539 |
| PITX2 | 0.028185 | DSC3 | 0.120844 | YARS2 | 0.016456 |
| ZNF75D | 0.057275 | C17orf106 | 0.037081 | OTUD7A | 0.201115 |
| RARG | 0.216165 | KIF3C | 0.034227 | CASP8AP2 | 0.016062 |
| RAD54B | 0.045267 | PKN1 | 0.170888 | PNMAS | 0.135075 |
| NR6A1 | 0.006141 | STC1 | 0.006462 | XRN2 | 0.161955 |
| NLRP9 | 0.152894 | MANSC1 | 0.218641 | MIR576 | 0.136067 |
| TAF15 | 0.057532 | NT5DC1 | 0.174405 | LOC492303 | 0.166097 |
| CLDN6 | 0.075814 | MCART6 | 0.067483 | LRRC37A4 | 0.138503 |
| CXCL13 | 0.110036 | PANK4 | 0.003817 | C11orf49 | 0.236135 |
| WARS | 0.000433 | BAI1 | 0.112174 | GBP7 | 0.039005 |
| CYP1A2 | 0.025302 | CDC42SE2 | 0.021331 | RAP1A | 0.062414 |
| L3MBTL2 | 0.113922 | ENO1 | 0.033418 | PLEKHGS | 0.124847 |
| NOVA2 | 0.097248 | FAM13AOS | 0.265658 | SLC9A10 | 0.001898 |
| TM2D3 | 0.263952 | SCXB | 0.005665 | LRRC14B | 0.120427 |
| SNORD93 | 0.130103 | PIGA | 0.259665 | DOCK7 | 0.086846 |
| TNFRSF18 | 0.176799 | CDC42BPA | 0.018359 | RNASEK | 0.058433 |
| CCR2 | 0.019608 | ERI2 | 0.048111 | ATXN10 | 0.328539 |
| GMNN | 0.056982 | FMNL3 | 0.268819 | FOXN1 | 0.130011 |
| ROD1 | 0.00363 | DNMT3L | 0.11955 | MYCN | 0.05342 |
| BDNF | 0.033034 | ZSWIM4 | 0.00694 | UBR7 | 0.130303 |
| NP | 0.185919 | HPS4 | 0.054637 | SEC22C | 0.198633 |
| TMEM62 | 0.042722 | MFRP | 0.105931 | FU43752 | 0.025543 |
| TNNT2 | 0.11036 | EPHB1 | 0.038068 | MIR654 | 0.141295 |
| LRRTM4 | 0.017028 | SLC23A1 | 0.082779 | LENEP | 0.016182 |
| NUP155 | 0.030303 | C1orf64 | 0.132788 | MIR571 | 0.1286 |
| BMPR1A | 0.179979 | PMEPA1 | 0.010494 | HSD11B1 | 0.054315 |
| HDLBP | 0.063327 | NRXN3 | 0.047603 | C14orf102 | 0.045687 |
| SLC25A34 | 0.160687 | MACC1 | 0.132316 | MIR1914 | 0.11015 |
| PRAMEFS | 0.179546 | PDLIM2 | 0.092791 | CREB5 | 0.18562 |
| SYTL3 | 0.101981 | CYB5R3 | 0.042923 | ANO7 | 0.204686 |
| STC2 | 0.004501 | SLC4A5 | 0.079908 | SBSN | 0.192868 |
| C14orf109 | 0.025836 | SOX2 | 0.048221 | ARMCX3 | 0.028017 |
| BCAS1 | 0.101035 | STYX | 0.038973 | CRYBA1 | 0.063877 |
| HR | 0.275219 | MIR942 | 0.093471 | TOMM20L | 0.060286 |
| GBE1 | 0.097187 | PHYH | 0.02152 | | |
| ESPNL | 0.011079 | KRTAP10.10 | 0.226854 | | |

**EXAMPLE 3**

Example 3 provides further information about the Time to Event Model (Cox model) of Example 2, Table 2.

*A. Definitions:*

Patient risk score function is defined as:

$$\text{recurrence\_score} = 0.31 * \text{figo\_numeric} - 0.35 * \text{surg\_outcome} + 0.23 * \text{MFAP2} + 0.48 * \text{ECOG} + 0.19 * \text{VEGFA} * \text{Bevacizumab} - 0.15 * \text{MFAP2} * \text{Bevacizumab} - 0.44 * \text{ECOG} * \text{Bevacizumab} \ldots\ldots\ldots\ldots\ldots \text{Equation (1)}$$

wherein figo _numeric = FIGO stage coded as integers,
wherein surg_outcome is -1 if the surgical outcome was suboptimal; 0 if the surgical outcome was optimal but tumor tissue smaller than 1 cm remained; or +1 if the surgical outcome was optimal and no visible macroscopic tumor tissue remained;
wherein MFAP2 = gene expression level of MFAP2;
wherein ECOG = ECOG performance status; and
wherein VEGFA = gene expression level of VEGFA.

The Cox proportional hazard function is defined as:

$$\lambda(t) = \lambda_0(t) e^{recurrence\_score} \quad \ldots\ldots\ldots\ldots\ldots \text{Equation (2)}$$

Where $\lambda(t)$ is the risk of recurrence at time t and Ao(t) is the baseline hazard function estimated with a non-parametric strategy, describing how the risk of event per time unit changes over time at baseline levels of covariates. recurrence_score is computed from Equation (1).

*B. Compute patient risk of death at time t if platin based therapy is given:*

1. Compute risk score using equation (1): use equation in (1), plug in Bevacizumab=0 and patient value for figo_numeric, surg_outcome, MFAP2, ECOG, VEGFA, MFAP2
2. Compute risk at time t: plug score obtained in step B.1 into recurrence_score in Equation (2), plug in t (time when risk need to be estimated).
3. Compute time to reach a given risk: use step B.2 to compute risk at a series of time points, look up time that correspond to the risk in questions.

*C. Compute patient risk of death at time t if platin based therapy+ Bevacizumab is given:*

1. Compute risk score using Equation (1): use Equation in (1), plug in Bevacizumab=1 and patient value for figo_numeric, surg_outcome, MFAP2, ECOG, VEGFA, MFAP2

2. Compute risk at time t: plug score obtained in step C. 1 into recurrence_score in Equation (2), plug in t (time when risk need to be estimated).

3. Compute time to reach a given risk: use step C.2 to compute risk at a series of time points, look up time that correspond to the risk in questions.

**D. Compute benefit from platin based therapy+ Bevacizumab:**

1. Subtract probability obtained in C.2 from probability obtained in B.2, resulting estimated difference in risk of death if Bevacizumab were given in addition to platin based therapy.

2. Pick a risk value, compare time to reach the risk computed from C.2 and B.2, the difference between the two estimated time represents the estimated improvement in/reducing of recurrence.

## EXAMPLE 4

Example 4 provides a procedure for creating an ensemble of signatures for ovarian cancer. In particular, an ensemble of signatures were created for both dichotomous outcomes and survival analysis (Cox) signatures.

**Step 1.** The procedure included identifying a single best set of biomarkers, or "seed," produced by Example 1 from predetermined data including clinical data only, gene expression data only, or clinical and gene expression data.

**Step 2.** The set of biomarkers were fed into a TIE* algorithm with the remainder of the predetermined data. The TIE* algorithm was used with GLL-PC as a subroutine (parameter X=GLL-PC) with the seed provided by GLL-PC and conditional independence criterion (Y=IGS) and Z=INDEPENDENCE. (Statnikov and Aliferis. PLoS Computational Biology 2010; 6(5), p.e1000790; U.S. Patent 8,805,761; Aliferis et al. Journal of Machine Learning Research 2010; 11(Jan), pp.171-234; Statnikov et al. Journal of Machine Learning Research 2013; 14(Feb), pp.499-566; U.S. Patent 8,655,821.)

The TIE* algorithm systematically examined information equivalences in the "seed" with variables in the remainder of the data (for example, full set of variables minus the seed). Replacement of a subset of the "seed" and execution of a subroutine was performed to identify the Markov Boundary set of biomarkers in the remainder of the data (for example, running the subroutine once for each time a subset of the "seed" is excluded).

**Step 3.** The replacement of the subset of the "seed" and execution of the subroutine was repeated recursively until all existing sets of biomarkers were identified and output by the TIE* algorithm. The TIE* algorithm was then terminated.

**Step 4.** The output of the TIE* algorithm provided a catalogue, or database, of biomarker sets. Each set of biomarkers was fed into a machine learning classifier fitting and model pipeline that incorporated model selection and error estimation. (Statnikov. A gentle introduction to support vector machines in biomedicine: Theory and methods; Vol. 1. World Scientific Pub. Co.; 2011; Statnikov et al. A Gentle Introduction to Support Vector Machines in Biomedicine: Volume 2: Case Studies and Benchmarks. World Scientific Pub. Co.; 2013.) A plurality of methods for deriving signatures from datasets were used. In particular, one or more of the following methods were used be used: a repeated nested n-fold cross validation with grid parameter choice, a support vector machine (SVM) classifier, a random forest (RF) classifier, and a lasso classifier.

**Step 5.** The output of the pipeline for each set of biomarkers, or each member of the equivalency catalogue, was a signature for predicting patient outcomes, for example, in response to treatment. The catalogue of signatures may be described as an ensemble.

**Step 6.** The catalogue of signatures may be used to provide an ensemble prediction. In a first example, a prediction would be obtained from every signature in the catalogue, and the predictions would be averaged to obtain a consolidated ensemble prediction. The ensemble prediction may minimize variance of prediction accuracy. In a second example, a prediction would be obtained from only a select number of signatures in the catalogue, and the predictions would be averaged to obtain a consolidated ensemble prediction. The signatures would be selected based on availability. Factors contributing to availability would include one or more of: convenience, cost, and ease of collection. In the second example, the companion test may be personalized or customized for different patients.

## EXAMPLE 5

This Example describes the identification of sets of variables and signatures (that is, the set of variables and their coefficients) that predict a response to bevacizumab, developed as described in Example 4.

**Methods**

Predictor Set: Clinical features (21) and Gene expression features (29377)

Target: time to relapse
N: 380; N events: 269
Performance estimation: 20 fold 5 repeat cross validation
Performance Metric: c-index
Method: TIE with max-k=1, max-card=1, p=0.05, seeded with original MB.

**Results:**

Final Model: 4 TIE signatures
CV performance estimation:
With lasso cox:

Original MB (Seed): 0.68+/-0.08
TIE signatures: 0.64+/-0.08

With regular cox:

Original MB (Seed): 0.68+/-0.10
TIE signature: 0.56+/-0.10

Exemplary results are shown in Tables 9-12, wherein figo _numeric and surg_outcome are described in Table 2; hist_rev_SBOT and hist_rev_metastais are determined as described in Example 2; ECOG = ECOG performance status, "xrndid" after a variable name indicates interaction with treatment. For example, if the variables include MFAP2_3 and MFAP2_3xrndid, MFAP2 3 indicates expression of MFAP2_3 and MFAP2_3xrndid indicates expression of MFAP2_3, wherein the coefficient is only applied when the patient is treated

**Table 9**

| Variable Name | Coefficient |
|---|---|
| surg_outcome | -0.44082714 |
| figo_numeric | 0.31301932 |
| ECOG | 0.45061864 |
| MFAP2_3 | 0.16628139 |
| surg_outcomexrndid | 0.18204931 |
| MFAP2_1xrndid | -0.09522372 |
| VEGFA_3xrndid | 0.1375739 |
| ECOGxrndid | -0.42221603 |
| MFAP2_3xrndid | -0.07687417 |

**Table 10**

| Variable Name | Coefficient | Variable Name | Coefficient |
|---|---|---|---|
| figo_numeric | 0.222512662 | ALKBH7 | -0.024312142 |
| MCAM | 0.080289559 | LOC388503_1 | 0.04485732 |
| REG4 | 0.124861797 | PRDM2_3 | 0.000751499 |
| C18orf56 | -0.276812329 | C20orf77 | 0.00869733 |
| PREP | 0.000281229 | C8orf79_1 | -0.070446044 |
| PRRG4_2 | -0.007129649 | LRRIQ4 | 0.070624165 |

(continued)

| Variable Name | Coefficient | Variable Name | Coefficient |
|---|---|---|---|
| EXOC3L2 | 0.055025506 | RAD54B_2 | -0.041598424 |
| AXL_1 | 0.025469171 | CARD17_1 | 0.131333116 |
| RNF7_1 | 0.034214255 | EIF4E2 | 0.091643106 |
| C1orf168 | -0.072824665 | YARS2 | 0.005687757 |
| RPS27L_2 | -0.024708305 | FBXO48_2 | -0.136651878 |
| TM2D3_2 | -0.209582854 | GZMB | -0.130786174 |
| C11orf24 | 0.1545701 | ZNF550 | -0.06531994 |
| SLC35C2_2 | 0.140504621 | REXO1L1 | -0.051039716 |
| CCDC114 | -0.010359055 | ZSWIM4_1 | 0.243783625 |
| MYOHD1 | -0.146095296 | LOC387720 | -0.104943347 |
| B3GAT1_3 | -0.025250575 | TCTEX1D4 | -0.022025733 |
| PNPLA3 | -0.044912936 | SATB2 | -0.058100575 |
| C12orf39 | 0.063856301 | CCL18 | -0.000428123 |
| EIF4G3 | 0.0376753 | ECOG | 0.00843997 |
| C10orf32_1 | -0.073368282 | surg_outcomexrndid | -0.195468114 |
| ANKRD30A_2 | 0.122310931 | GRIK5xrndid | -0.039724252 |
| PCNP | -0.08554762 | | |
| DNAH9_3 | -0.01715795 | | |

**Table 11**

| Variable Name | Coefficient | Variable Name | Coefficient |
|---|---|---|---|
| figo_numeric | 2.97E-01 | NF2_3xrndid | -5.53E-02 |
| surg_outcome | -4.80E-01 | DNAH11xrndid | -1.65E-01 |
| ECOG | 4.93E-01 | TTRxrndid | 4.93E-02 |
| MFAP2_3 | 2.02E-01 | MRPS11_2xrndid | -1.01E-02 |
| surg_outcomexrndid | 3.18E-01 | ZNF530xrndid | 1.86E-01 |
| SERPINB2_2xrndid | 1.76E-05 | CLEC2D_3xrndid | -1.33E-01 |
| BCAS1_1xrndid | 7.79E-02 | RAD9Bxrndid | -1.81E-01 |
| ZBTB25_1xrndid | -2.46E-02 | TMEM90Axrndid | -1.30E-01 |
| NNAT_1xrndid | 2.31E-01 | ECOGxrndid | -4.16E-01 |
| CD2xrndid | -9.78E-02 | MFAP2_3xrndid | -1.65E-01 |
| CECR1_2xrndid | -3.20E-02 | | |
| PDE3Axrndid | 2.20E-02 | | |
| ENTPD8_2xrndid | 1.19E-01 | | |
| GUSBL2xrndid | -8.56E-02 | | |
| ANKRD30A_1xrndid | 1.13E-01 | | |
| ENPEP_2xrndid | 1.50E-02 | | |

(continued)

| Variable Name | Coefficient | Variable Name | Coefficient |
|---|---|---|---|
| MIR1914xrndid | 7.58E-02 | | |
| ZNF276xrndid | -3.50E-02 | | |
| REEP1xrndid | 4.13E-02 | | |
| P4HA1_2xrndid | -1.46E-01 | | |
| HARBI1_1xrndid | 2.05E-01 | | |
| TNFRSF17xrndid | -2.74E-02 | | |
| ANKRD30A_2xrndid | 5.56E-03 | | |
| GATA6xrndid | 1.54E-01 | | |
| GAD1_2xrndid | -2.38E-02 | | |
| ADAM5Pxrndid | 4.89E-02 | | |
| XPNPEP2xrndid | 3.24E-03 | | |
| TAS2R7xrndid | 3.87E-01 | | |
| NFATC4xrndid | 3.73E-02 | | |
| PDE4DIP_1xrndid | 1.16E-01 | | |
| SH2D6xrndid | 4.96E-02 | | |
| PCDHA7_3xrndid | 1.71E-01 | | |
| DUT_3xrndid | -1.44E-02 | | |
| PHLDB2_1xrndid | 1.36E-01 | | |
| PAICS_1xrndid | -2.25E-02 | | |
| CCDC50_2xrndid | 6.59E-02 | | |
| BHLHA15xrndid | -1.29E-01 | | |
| SORBS3_1xrndid | -1.64E-01 | | |
| NAPSAxrndid | -1.26E-01 | | |
| CDC14B_3xrndid | -7.89E-02 | | |
| GPR34_2xrndid | 7.45E-03 | | |
| PCSK6_1xrndid | -3.92E-02 | | |
| C7orf55_2xrndid | -4.43E-02 | | |

**Table 12**

| Variable Name | Coefficient | Variable Name | Coefficient |
|---|---|---|---|
| figo_numeric | 0.230997068 | ECOG | 0.02312799 |
| ANKRD30A_2 | 0.125678399 | surg_outcomexrndid | -0.198492853 |
| MCAM | 0.072331724 | GUSBL2xrndid | -0.008814662 |
| REG4 | 0.130660811 | BHLHA15xrndid | -0.045906491 |
| C18orf56 | -0.261439816 | | |
| PREP | 0.007826764 | | |
| PRRG4_2 | -0.011529826 | | |

(continued)

| Variable Name | Coefficient | Variable Name | Coefficient |
|---|---|---|---|
| EXOC3L2 | 0.05973232 | | |
| AXL_1 | 0.019925605 | | |
| RNF7_1 | 0.025954975 | | |
| C1orf168 | -0.075746531 | | |
| RPS27L_2 | -0.023185125 | | |
| TM2D3_2 | -0.198849821 | | |
| C11orf24 | 0.159456193 | | |
| SLC35C2_2 | 0.135432627 | | |
| CCDC114 | -0.015655845 | | |
| MYOHD1 | -0.158958318 | | |
| B3GAT1_3 | -0.024714285 | | |
| PNPLA3 | -0.058457746 | | |
| C12orf39 | 0.065472862 | | |
| EIF4G3 | 0.050656249 | | |
| C10orf32_1 | -0.088038114 | | |
| PCNP | -0.13688082 | | |
| DNAH9_3 | -0.025475558 | | |
| ALKBH7 | -0.029561969 | | |
| LOC388503_1 | 0.063993063 | | |
| PRDM2_3 | 0.01079284 | | |
| C20orf77 | 0.022530994 | | |
| FLJ37587 | 0.005155198 | | |
| C8orf79_1 | -0.062888761 | | |
| LRRIQ4 | 0.076642753 | | |
| RAD54B_2 | -0.048442085 | | |
| CARD17_1 | 0.164118694 | | |
| EIF4E2 | 0.102255429 | | |
| YARS2 | 0.021797945 | | |
| FBXO48_2 | -0.142665906 | | |
| GZMB | -0.132781409 | | |
| ZNF550 | -0.071905525 | | |
| REXO1L1 | -0.050514064 | | |
| ZSWIM4_1 | 0.33711993 | | |
| LOC387720 | -0.117252043 | | |
| TCTEX1D4 | -0.032385501 | | |
| SATB2 | -0.056044084 | | |

**EXAMPLE 6**

This Example describes the identification of sets of variables and signatures (that is, the set of variables and their coefficients) that predict ovarian cancer 48 month progression free survival, developed as described in Example 4.

Predictor Set: Clinical features (21) and Gene expression features (29377)
Target: 48 month survival binary outcome
N: 351 (265 dead and 86 alive)
Performance estimation: 10 fold 5 repeat cross validation

**Method: TIE Independence test**

# MB: 56
Median(# MB members): 193
min(# MB members): 190
max(# MB members): 198
# vars in at least one MB: 215
CV AUC (mean+/-sd)*: 0.76 +/- 0.02

*mean is taken first over multiple signatures within each cross validation run resulting in 50 values, then averaged across folds resulting in 5 values where computation of CV AUC mean and standard deviation are based on.

Exemplary results are shown in Tables 13-65, wherein figo_numeric and surg_outcome are described in Table 2; hist_rev_SBOT and hist_rev_metastais are determined as described in Example 2; ECOG = ECOG performance status, "xrndid" after a variable name indicates interaction with treatment.

Table 13

| | | | | | | | |
|---|---|---|---|---|---|---|---|
| ABHD3 | 0.0683 | DBNDD2 | 0.11 | HR_1 | 0.044 | NR6A1_2 | 0.1529 | SFRS4 | 0.026 |
| ADAM17_2 | 0.2314 | DNAH11 | 0.0429 | HSD11B1_1 | 0.0869 | NRXN3_3 | 0.0972 | SHC1_3 | 0.0727 |
| ADAMTS1 | 0.1737 | DNMT3L_2 | 0.0253 | ICAM2 | 0.0546 | NTSDC1_2 | 0.1859 | SLC23A1_2 | 0.1357 |
| ALS2CL_3 | 0.107 | DOCK7_1 | 0.1394 | ICAM4_1 | 0.2752 | NTRK2_3 | 0.0061 | SLC25A34 | 0.1581 |
| ANO7_3 | 0.061 | DSC3_1 | 0.0741 | ILIRAP_2 | 0.0543 | NUP155_1 | 0.0476 | SLC4A5_3 | 0.0814 |
| ARL6IP1_1 | 0.0303 | DUT_3 | 0.1195 | IQCA1_2 | 0.0335 | NYX | 0.1744 | SLC9A10 | 0.0828 |
| ARMCX3_2 | 0.0826 | EEF1E1_1 | 0.0868 | KCNIP3_1 | 0.1016 | ODF2_3 | 0.0079 | SNORD93 | 0.1607 |
| ATXN10_1 | 0.2047 | EMP1 | 0.1208 | KCNQ2_1 | 0.1265 | ORC1L | 0.0303 | SOX2_1 | 0.0799 |
| AXL_1 | 0.1075 | ENO1 | 0.1863 | KIF3C | 0.1795 | OTUD7A_3 | 0.0338 | STC1 | 0.0019 |
| BAI1_3 | 0.028 | ENPEP_2 | 0.1359 | KRT80_2 | 0.1093 | PANK4 | 0.061 | STC2 | 0.1301 |
| BCAS1_1 | 0.3285 | EPHB1 | 0.0372 | KRTAP10.10_2 | 0.0205 | PDLIM2_2 | 0.2385 | STYX_2 | 0.0482 |
| BDNF_2 | 0.1074 | EPYC | 0.0334 | L3MBTL2_3 | 0.0342 | PHYH_1 | 0.2011 | SYTL3 | 0.0065 |
| BMPR1A | 0.1122 | ERI2_2 | 0.291 | LBH_2 | 0.1017 | PIGA_1 | 0.0038 | TAF15_1 | 0.0045 |
| BTF3_3 | 0.101 | ESPNL | 0.0381 | LENEP | 0.2269 | PITX2_1 | 0.0928 | TCEAL8_1 | 0.039 |
| C10orf116 | 0.033 | EZH2_1 | 0.0708 | LGI3 | 0.1139 | PKN1_3 | 0.0215 | THBS3 | 0.102 |
| C11orf24 | 0.18 | FAM13AOS | 0.0481 | LOC492303 | 0.0268 | PLAC9 | 0.2597 | TM2D3_2 | 0.0575 |
| C11orf49_3 | 0.1323 | FAM187B_2 | 0.0111 | LRRC14B | 0.0162 | PLEKHG5_5 | 0.0282 | TMEM52 | 0.0839 |
| C14orf102_2 | 0.1264 | FAM70A_1 | 0.0802 | LRRC37A4_2 | 0.0628 | PLSCR4 | 0.1709 | TMEM62 | 0.0081 |
| C14orf109_2 | 0.0682 | FBXO48_2 | 0.2657 | LRRTM4 | 0.1661 | PMEPA1_4 | 0.1243 | TNFRSF18_1 | 0.264 |
| C17orf106 | 0.2361 | FKBP10 | 0.0668 | MACC1 | 0.1204 | PNMAS | 0.1248 | TNNT2_1 | 0.003 |
| C17orf58_2 | 0.0457 | FLJ33360 | 0.0509 | MANSC1_1 | 0.1385 | PPAPDC1A | 0.1307 | TOMM20L | 0.0427 |
| C17orf58_3 | 0.0258 | FLJ43752 | 0.1885 | MCAM | 0.017 | PRAMEF_5 | 0.0105 | TPM2_2 | 0.1768 |
| C18orf56 | 0.0371 | FMNL3_2 | 0.0431 | MCART6_1 | 0.1323 | PRKAA2 | 0.1351 | TRIM58 | 0.1104 |
| C1orf168 | 0.032 | FOSB | 0.2051 | MFRP | 0.2186 | PSMC6_1 | 0.0022 | UBR7_1 | 0.0603 |
| C1orf64 | 0.1085 | FOSL2 | 0.0255 | MIDN | 0.0462 | RAD54B 2 | 0.1795 | UBR7_2 | 0.1256 |
| C8orf79_1 | 0.0285 | FOXN1 | 0.2688 | MIR1914 | 0.0675 | RAP1A_1 | 0.2149 | WARS_2 | 0.1536 |

| | | | | | | | | |
|---|---|---|---|---|---|---|---|---|
| CALD1_2 | 0.142 | GAD1_2 | 0.0232 | MIR212 | 0.1059 | RARA_3 | 0.0838 | XBP1_2 | 0.1303 |
| CASP8AP2 | 0.1328 | GBE1 | 0.0417 | MIR571 | 0.0359 | RARG | 0.0453 | XRN2_1 | 0.0463 |
| CCL13 | 0.0876 | GBP7 | 0.13 | MIR576 | 0.1102 | RNASEK | 0.0624 | YARS2 | 0.0004 |
| CCR2_3 | 0.0225 | GJA5_1 | 0.0504 | MIR654 | 0.0501 | RNF7_1 | 0.0195 | ZNF75D_2 | 0.1488 |
| CD34_1 | 0.0161 | GMNN | 0.0972 | MIR942 | 0.1286 | ROD1_1 | 0.2162 | ZSWIM4_2 | 0.162 |
| CDC42BPA_2 | 0.0272 | GSR_2 | 0.039 | MMP12_1 | 0.1361 | SATB2 | 0.0584 | figo _numeric | 0.0165 |
| CDC42SE2_2 | 0.0196 | HBA2 | 0.2005 | MYCN_2 | 0.1413 | SBSN | 0.0638 | hist_rev_SBOT | 0.0573 |
| CLDN6 | 0.1135 | HCFC1R1_1 | 0.057 | MYOHD1 | 0.0935 | SCXB | 0.0036 | surg_outcome | 0.0069 |
| CREB5_2 | 0.0184 | HDAC7_2 | 0.0094 | NFATC3_5 | 0.01 | SEC22C_3 | 0.1171 | | |
| CRYBA1 | 0.0213 | HDLBP_3 | 0.1087 | NFATC4 | 0.0534 | SELENBP1 | 0.1929 | | |
| CXCL13 | 0.0758 | HIC1 | 0.0447 | NLRP9 | 0.1806 | SERPINB2_2 | 0.0057 | | |
| CYBSR3_2 | 0.1856 | HPRT1_1 | 0.1578 | NOVA2 | 0.1229 | SERPINB5 | 0.1986 | | |
| CYP1A2 | 0.0639 | HPS4_1 | 0.0633 | NP | 0.096 | SFN | 0.0109 | | |

Table 14

| | | | | | | | | | |
|---|---|---|---|---|---|---|---|---|---|
| ABHD3 | 0.0691 | DBNDD2 | 0.1093 | HIC1 | 0.0395 | NOV A2 | 0.1234 | SERPINB5 | 0.2043 |
| ADAM17_2 | 0.2301 | DNAH11 | 0.047 | HPRT1_1 | 0.1532 | NP | 0.0859 | SFN | 0.0172 |
| ADAMTS1 | 0.1681 | DNMT3L_2 | 0.0249 | HPS4 1 | 0.0696 | NR6A1_2 | 0.1562 | SFRS4 | 0.0302 |
| ALS2CL 3 | 0.1144 | DOCK7_1 | 0.1356 | HR 1 | 0.0444 | NRXN3_3 | 0.0972 | SHC1_3 | 0.0715 |
| ANO7_3 | 0.0721 | DSC3_1 | 0.0723 | HSD11B1_1 | 0.0979 | NT5DC1_2 | 0.1975 | SLC23A1_2 | 0.1325 |
| ARL6IP1_1 | 0.0276 | DUT 3 | 0.1209 | ICAM2 | 0.0583 | NTRK2_3 | 0.0024 | SLC25A34 | 0.1748 |
| ARMCX3 2 | 0.0869 | EEF1E1_1 | 0.1031 | ICAM4 1 | 0.2757 | NUP155_1 | 0.0631 | SLC4A5 3 | 0.0833 |
| ATXN10_1 | 0.2027 | EIF4ENIF 1 | 0.1243 | IL1RAP_2 | 0.0628 | NYX | 0.1779 | SLC9A10 | 0.0831 |
| AXL_1 | 0.1173 | EMP1 | 0.1714 | IQCA1_2 | 0.0279 | ODF2 3 | 0.0096 | SNORD93 | 0.165 |
| BAII 3 | 0.04 | ENOI | 0.1384 | KCNIP3 1 | 0.1018 | ORC1L | 0.0229 | SOX2 1 | 0.0776 |
| BCAS1_1 | 0.3333 | ENPEP_2 | 0.0147 | KCNQ2_1 | 0.1292 | OTUD7A_3 | 0.0364 | STC1 | 0.0081 |
| BDNF 2 | 0.1205 | EPHB1 | 0.0247 | KIF3C | 0.1922 | PANK4 | 0.0633 | STC2 | 0.1336 |
| BMPR1A | 0.1078 | EPYC | 0.0331 | KRT80 2 | 0.1117 | PDLIM2_2 | 0.233 | STYX 2 | 0.0487 |
| BTF3 3 | 0.1014 | ERI2 2 | 0.3022 | KRTAP10.10_2 | 0.0225 | PHYH_1 | 0.2002 | SYTL3 | 0.0061 |
| C10orf116 | 0.0327 | ESPNL | 0.0445 | L3MBTL2_3 | 0.032 | PIGA_1 | 0.0086 | TAF15_1 | 0.0023 |
| C11orf24 | 0.1899 | EZH2_1 | 0.069 | LBH 2 | 0.0989 | PITX2_1 | 0.0912 | TCEAL8 1 | 0.0419 |
| C11orf49_3 | 0.1274 | FAM13AOS | 0.0401 | LENEP | 0.2252 | PKN1_3 | 0.0198 | THBS3 | 0.103 |
| C14orf102_2 | 0.1343 | FAM187B_2 | 0.0085 | LGI3 | 0.1244 | PLAC9 | 0.2491 | TM2D3_2 | 0.062 |
| C14orf109_2 | 0.0732 | FAM70A_1 | 0.0737 | LOC492303 | 0.0327 | PLEKHGS_5 | 0.0182 | TMEM52 | 0.083 |
| C17orf106 | 0.244 | FBX048_2 | 0.2627 | LRRC14B | 0.0225 | PLSCR4 | 0.1645 | TMEM62 | 0.0104 |
| C17orf58_2 | 0.0461 | FGF5_1 | 0.0708 | LRRC37A4 2 | 0.0656 | PMEPA1_4 | 0.1301 | TNFRSF18_1 | 0.2692 |
| C17orf58_3 | 0.027 | FKBP10 | 0.0415 | LRRTM4 | 0.1751 | PNMAS | 0.1142 | TNNT2_1 | 0.0018 |
| C18orf56 | 0.0469 | FLJ33360 | 0.2007 | MACC1 | 0.1365 | PPAPDC1A | 0.1266 | TOMM20L | 0.0437 |
| C1orf168 | 0.0365 | FLJ43752 | 0.0712 | MANSC1_1 | 0.1403 | PRAMEFS | 0.0035 | TPM2 2 | 0.1748 |
| C1orf64 | 0.1125 | FMNL3_2 | 0.04 | MCAM | 0.0266 | PRKAA2 | 0.1445 | TRIM58 | 0.1078 |
| C8orf79_1 | 0.0188 | FMOD | 0.2067 | MCART6 1 | 0.1474 | PSMC6_1 | 0.0097 | UBR7 1 | 0.0702 |

| | | | | | | | |
|---|---|---|---|---|---|---|---|
| CALD1_2 | 0.1376 | FOSB | 0.0195 | MFRP | 0.2211 | RAD54B 2 | 0.1778 | UBR7 2 | 0.1186 |
| CASP8AP2 | 0.1369 | FOSL2 | 0.275 | MIDN | 0.0471 | RAP1A_1 | 0.2138 | WARS 2 | 0.1435 |
| CCL13 | 0.0982 | FOXN1 | 0.0258 | MIR1914 | 0.0636 | RARA 3 | 0.0826 | XBP1_2 | 0.1283 |
| CCR2 3 | 0.0247 | GAD1_2 | 0.019 | MIR212 | 0.1054 | RARG | 0.0438 | XRN2_1 | 0.0466 |
| CD34 1 | 0.0027 | GBE1 | 0.0448 | MIR571 | 0.0396 | RNASEK | 0.0706 | YARS2 | 0.0054 |
| CDC42BPA 2 | 0.0175 | GBP7 | 0.1269 | MIR576 | 0.1071 | RNF7_1 | 0.0197 | ZNF75D 2 | 0.1609 |
| CDC42SE2 2 | 0.0274 | GJA5_1 | 0.0503 | MIR654 | 0.0564 | ROD1_1 | 0.2173 | ZSWIM4 2 | 0.1605 |
| CLDN6 | 0.1012 | GMNN | 0.0934 | MIR942 | 0.139 | SATB2 | 0.0606 | figo _numeric | 0.0106 |
| CREB5 2 | 0.022 | GSR 2 | 0.0444 | MMP12_1 | 0.1332 | SBSN | 0.0556 | hist rev SBOT | 0.0666 |
| CRYBA1 | 0.0213 | HBA2 | 0.2067 | MYCN_2 | 0.1428 | SCXB | 0.0085 | surg_outcome | 0.0011 |
| CXCL13 | 0.0802 | HCFC1R1_1 | 0.0574 | NFATC3 3 | 0.1025 | SEC22C 3 | 0.1087 | | |
| CYBSR3 2 | 0.1887 | HDAC7_2 | 0.0057 | NFATC4 | 0.0074 | SELENBP1 | 0.1865 | | |
| CYP1A2 | 0.0623 | HDLBP 3 | 0.097 | NLRP9 | 0.0542 | SERPINB2_2 | 0.0086 | | |

Table 15

| Gene | Value | Gene | Value | Gene | Value | Gene | Value | Gene | Value |
|---|---|---|---|---|---|---|---|---|---|
| ABHD3 | 0.017 | CYP1A2 | 0.0806 | HR_1 | 0.0278 | NR6A1_2 | 0.1279 | SFRS4 | 0.041 |
| ADAM17_2 | 0.2178 | DBNDD2 | 0.1056 | HSD11B1_1 | 0.1166 | NRXN3_3 | 0.0986 | SHC1_3 | 0.0616 |
| ADAMTS1 | 0.1513 | DNAH11 | 0.0465 | ICAM2 | 0.0296 | NT5DC1_2 | 0.1927 | SLC23A1_2 | 0.0775 |
| ALS2CL_3 | 0.0869 | DNMT3L_2 | 0.0109 | ICAM4_1 | 0.2485 | NTRK2_3 | 0.007 | SLC25A34 | 0.1748 |
| ANO7_3 | 0.0093 | DOCK7_1 | 0.0962 | IL1RAP_2 | 0.0406 | NUP155_1 | 0.0258 | SLC4A5_3 | 0.0545 |
| ARL6IP1_1 | 0.039 | DSC3_1 | 0.0865 | IQCA1_2 | 0.0634 | NYX | 0.1517 | SLC9A10 | 0.0644 |
| ARMCX3_2 | 0.114 | DUT_3 | 0.1196 | KCNIP3_1 | 0.1136 | ODF2_3 | 0.031 | SNORD93 | 0.1602 |
| ATXN10_1 | 0.2204 | EEF1E1_1 | 0.1118 | KCNQ2_1 | 0.1423 | ORC1L | 0.0202 | SOX2_1 | 0.0722 |
| AURKA_1 | 0.107 | EMP1 | 0.1077 | KIF3C | 0.1857 | OTUD7A_3 | 0.0067 | STC1 | 0.017 |
| AXL_1 | 0.0976 | ENOI | 0.2069 | KRT80_2 | 0.1431 | PANK4 | 0.0503 | STC2 | 0.1174 |
| BAI1_3 | 0.2864 | ENPEP_2 | 0.1358 | KRTAP10.10_2 | 0.0013 | PDLIM2_2 | 0.2085 | STYX_2 | 0.0447 |
| BCAS1_1 | 0.1898 | EPHB1 | 0.04 | L3MBTL2_3 | 0.0236 | PHYH_1 | 0.1832 | SYTL3 | 0.0231 |
| BDNF_2 | 0.1284 | EPYC | 0.0359 | LBH_2 | 0.1133 | PIGA_1 | 0.0184 | TAF15_1 | 0.0384 |
| BMPR1A | 0.0733 | ERI2_2 | 0.2463 | LENEP | 0.1974 | PITX2_1 | 0.1464 | TCEAL8_1 | 0.0641 |
| BTF3_3 | 0.0703 | ESPNL | 0.0146 | LGI3 | 0.1402 | PKN1_3 | 0.0467 | THBS3 | 0.0535 |
| C10orf116 | 0.046 | FAM13AOS | 0.0501 | LOC492303 | 0.049 | PLAC9 | 0.201 | TM2D3_2 | 0.0597 |
| C11orf24 | 0.1475 | FAM187B_2 | 0.0008 | LRRC14B | 0.0347 | PLEKHG5_5 | 0.0054 | TMEM52 | 0.0905 |
| C11orf49_3 | 0.1114 | FAM70A_1 | 0.0226 | LRRC37A4_2 | 0.0681 | PLSCR4 | 0.1996 | TMEM62 | 0.0353 |
| C14orf102_2 | 0.0717 | FBXO48_2 | 0.2865 | LRRTM4 | 0.1938 | PMEPA1_4 | 0.1614 | TNFRSF18_1 | 0.2073 |
| C14orf109_2 | 0.0896 | FKBP10 | 0.0455 | MACC1 | 0.0885 | PNMA5 | 0.1364 | TNNT2_1 | 0.0036 |
| C17orf106 | 0.2203 | FLJ33360 | 0.0508 | MANSC1_1 | 0.1009 | PPAPDC1A | 0.1327 | TOMM20L | 0.0199 |
| C17orf58_2 | 0.0689 | FLJ43752 | 0.1805 | MCAM | 0.0045 | PRAMEF5 | 0.0077 | TPM2_2 | 0.1779 |
| C17orf58_3 | 0.0309 | FMNL3_2 | 0.0098 | MCART6_1 | 0.142 | PRKAA2 | 0.0733 | TRIM58 | 0.0972 |
| C18orf56 | 0.0005 | FOSB | 0.2 | MFRP | 0.2163 | PSMC6_1 | 0.0126 | UBR7_1 | 0.0564 |
| C1orf168 | 0.0392 | FOSL2 | 0.0571 | MIDN | 0.0208 | RAD54B_2 | 0.1822 | UBR7_2 | 0.1055 |
| C1orf64 | 0.1062 | FOXN1 | 0.2266 | MIR1914 | 0.0797 | RAP1A_1 | 0.1883 | WARS_2 | 0.1344 |

(continued)

| | | | | | | | | |
|---|---|---|---|---|---|---|---|---|
| C8orf79_1 | 0.0099 | GAD1_2 | 0.0281 | MIR212 | 0.0822 | RARA_3 | 0.0844 | WDR76 | 0.1029 |
| CALD1_2 | 0.14 | GBE1 | 0.039 | MIR571 | 0.0335 | RARG | 0.0525 | XBP1_2 | 0.0411 |
| CASP8AP2 | 0.1131 | GBP7 | 0.095 | MIR576 | 0.1208 | RNASEK | 0.0791 | XRN2_1 | 0.0238 |
| CCL13 | 0.0461 | GJA5_1 | 0.0386 | MIR654 | 0.0169 | RNF7_1 | 0.074 | YARS2 | 0.2448 |
| CCR2_3 | 0.03 | GMNN | 0.077 | MIR942 | 0.1718 | ROD1_1 | 0.1579 | ZNF75D_2 | 0.1373 |
| CD34_1 | 0.0066 | GSR_2 | 0.0027 | MMP12_1 | 0.0955 | SATB2 | 0.0435 | ZSWIM4_2 | 0.1486 |
| CDC42BPA_2 | 0.0174 | HBA2 | 0.1406 | MYCN_2 | 0.066 | SBSN | 0.0119 | figo _numeric | 0.0116 |
| CDC42SE2_2 | 0.0321 | HCFC1R1_1 | 0.0402 | MYOHD1 | 0.082 | SCXB | 0.0168 | hist_rev_SBOT | 0.0544 |
| CLDN6 | 0.1156 | HDAC7_2 | 0.0238 | NFATC3_5 | 0.0152 | SEC22C_3 | 0.1048 | surg_outcome | 0.0173 |
| CREB5_2 | 0.0101 | HDLBP_3 | 0.1024 | NFATC4 | 0.0671 | SELENBP1 | 0.1497 | | |
| CRYBA1 | 0.0287 | HIC1 | 0.032 | NLRP9 | 0.1677 | SERPINB2_2 | 0.0248 | | |
| CXCL13 | 0.1119 | HPRT1_1 | 0.0882 | NOVA2 | 0.0844 | SERPINB5 | 0.1755 | | |
| CYB5R3_2 | 0.1371 | HPS4_1 | 0.0776 | NP | 0.1041 | SFN | 0.0234 | | |

Table 16

| | | | | | | | | | |
|---|---|---|---|---|---|---|---|---|---|
| ABHD3 | 0.0747 | DBNDD2 | 0.1028 | HR_1 | 0.0683 | NR6A1_2 | 0.1415 | SFRS4 | 0.0454 |
| ADAM17_2 | 0.2317 | DNAH11 | 0.046 | HSD11B1_1 | 0.0791 | NRXN3_3 | 0.1 | SHC1_3 | 0.0867 |
| ADAMTS1 | 0.1658 | DNMT3L_2 | 0.0307 | ICAM2 | 0.0553 | NT5DC1_2 | 0.1905 | SLC23A1_2 | 0.1344 |
| ALS2CL_3 | 0.0808 | DOCK7_1 | 0.1517 | ICAM4_1 | 0.2718 | NTRK2_3 | 0.0049 | SLC25A34 | 0.1652 |
| ANO7_3 | 0.0363 | DSC3_1 | 0.0958 | IL1RAP_2 | 0.0666 | NUP155_1 | 0.0442 | SLC4A5_3 | 0.077 |
| ARL6IP1_1 | 0.0278 | DUT_3 | 0.1344 | IQCA1_ 2 | 0.0458 | NYX | 0.169 | SLC9A10 | 0.0804 |
| ARMCX3_2 | 0.0847 | EEF1E1_1 | 0.1017 | KCNIP3_1 | 0.1062 | ODF2_3 | 0.0024 | SNORD93 | 0.1576 |
| ATXN10_1 | 0.1749 | EMP1 | 0.1196 | KCNQ2_1 | 0.1298 | ORC1L | 0.0312 | SOX2_1 | 0.0576 |
| AXL_1 | 0.1004 | ENOI | 0.1976 | KIF3C | 0.1888 | OTUD7A_3 | 0.024 | STC1 | 0.0072 |
| BAI1_3 | 0.0291 | ENPEP_2 | 0.1452 | KRT80_2 | 0.1043 | PANK4 | 0.0574 | STC2 | 0.1268 |
| BCAS1_2 | 0.3377 | EPHB1 | 0.0422 | KRTAP10.10_2 | 0.0252 | PDLIM2_2 | 0.2424 | STYX_2 | 0.0469 |
| BDNF_2 | 0.082 | EPYC | 0.0263 | L3MBTL2_3 | 0.0224 | PHYH_1 | 0.2254 | SYTL3 | 0.0415 |
| BMPR1A | 0.1275 | ERI2_2 | 0.3104 | LBH_2 | 0.1201 | PIGA_1 | 0.0076 | TAF15_1 | 0.0093 |
| BTF3_3 | 0.1258 | ESPNL | 0.0371 | LENEP | 0.2267 | PITX2_1 | 0.1073 | TCEAL8_1 | 0.0306 |
| C10orf116 | 0.009 | EZH2_1 | 0.0793 | LGI3 | 0.0942 | PKN1_3 | 0.0335 | THBS3 | 0.1029 |
| C11orf24 | 0.1986 | FAM13AOS | 0.0488 | LOC492303 | 0.0283 | PLAC9 | 0.255 | TM2D3_2 | 0.0536 |
| C11orf49_3 | 0.1205 | FAM187B_2 | 0.003 | LRRC14B | 0.002 | PLEKHG5_5 | 0.0223 | TMEM52 | 0.0764 |
| C14orf102_2 | 0.1068 | FAM70A_1 | 0.0692 | LRRC37A4_2 | 0.0748 | PLSCR4 | 0.1482 | TMEM62 | 0.0115 |
| C14orf109_2 | 0.0823 | FBXO48_2 | 0.2424 | LRRTM4 | 0.1456 | PMEPA1_4 | 0.1317 | TNFRSF18_1 | 0.2552 |
| C17orf106 | 0.2146 | FKBP10 | 0.0708 | MACC1 | 0.1269 | PNMA5 | 0.1286 | TNNT2_1 | 0.0025 |
| C17orf58_2 | 0.0416 | FLJ33360 | 0.0337 | MANSC1_1 | 0.1122 | PPAPDC1A | 0.1167 | TOMM20L | 0.0431 |
| C17orf58_3 | 0.0174 | FLJ43752 | 0.1703 | MCAM | 0.0051 | PRAMEF 5 | 0.0087 | TPM2_2 | 0.1772 |
| C18orf56 | 0.0652 | FMNL3_2 | 0.0497 | MCART6_1 | 0.1513 | PRKAA2 | 0.1363 | TRIM58 | 0.0949 |
| C1orf168 | 0.0495 | FOSB | 0.1989 | MFRP | 0.2472 | PSMC6_ 1 | 0.0136 | UBR7_1 | 0.0817 |
| C1orf64 | 0.11 | FOSL2 | 0.0207 | MIDN | 0.0353 | RAD54B_2 | 0.171 | UBR7_2 | 0.1309 |
| C8orf79_1 | 0.024 | FOXN1 | 0.2588 | MIR1914 | 0.0721 | RAP1A_1 | 0.2223 | WARS_2 | 0.1811 |

(continued)

| | | | | | | | | | |
|---|---|---|---|---|---|---|---|---|---|
| CALD1_2 | 0.1349 | GAD1_2 | 0.011 | MIR212 | 0.1101 | RARA_3 | 0.0814 | XBP1_2 | 0.1364 |
| CASP8AP2 | 0.1386 | GBE1 | 0.052 | MIR571 | 0.0105 | RARG | 0.0542 | XRN2_1 | 0.0408 |
| CCL13 | 0.0976 | GBP7 | 0.1297 | MIR576 | 0.1185 | RNASEK | 0.0725 | YARS2 | 0.0021 |
| CCR2_3 | 0.042 | GJA5_1 | 0.0608 | MIR654 | 0.0532 | RNF7_1 | 0.0007 | ZNF75D_2 | 0.1606 |
| CD34_1 | 0.0276 | GMNN | 0.0927 | MIR942 | 0.1205 | ROD1_1 | 0.2151 | ZSWIM4_2 | 0.1737 |
| CDC42BPA_2 | 0.0327 | GSR_2 | 0.0347 | MMP12_1 | 0.1358 | SATB2 | 0.0497 | figo_numeric | 0.0311 |
| CDC42SE2_2 | 0.0358 | HBA2 | 0.1888 | MYCN_2 | 0.1492 | SBSN | 0.0558 | hist_rev_SBOT | 0.0587 |
| CLDN6 | 0.1204 | HCFC1R1_1 | 0.0557 | MYOHD1 | 0.0898 | SCXB | 0.0084 | surg_outcome | 0.0173 |
| CREB5_2 | 0.0007 | HDAC7_2 | 0.0085 | NFATC3_5 | 0.0112 | SEC22C_3 | 0.115 | | |
| CRYBA1 | 0.0133 | HDLBP_3 | 0.08 | NFATC4 | 0.0474 | SELENBP1 | 0.1832 | | |
| CXCL_13 | 0.0859 | HIC1 | 0.0079 | NLRP9 | 0.1736 | SERPINB2_2 | 0.0166 | | |
| CYB5R3_2 | 0.1771 | HPRT1_1 | 0.1413 | NOVA2 | 0.1253 | SERPINB5 | 0.2045 | | |
| CYP1A2 | 0.0533 | HPS4_1 | 0.0578 | NP | 0.1082 | SFN | 0.0067 | | |

Table 17

| | | | | | | | | |
|---|---|---|---|---|---|---|---|---|
| ABHD3 | 0.0849 | CYP1A2 | 0.0445 | HPRT1_1 | 0.1415 | NLRP9 | 0.1584 | SERPINB2_2 | 0 |
| ADAM17_2 | 0.2224 | DBNDD2 | 0.0769 | HPS4_1 | 0.0392 | NOV_A2 | 0.0925 | SERPINB5 | 0.2241 |
| ADAMTS1 | 0.1657 | DFFB_2 | 0.0489 | HR_1 | 0.0907 | NP | 0.0944 | SFN | 0.0073 |
| ALS2CL_3 | 0.1006 | DNAH11 | 0.0361 | HSD11B1_1 | 0.078 | NR6A1_2 | 0.1293 | SFRS4 | 0.061 |
| ANO7_3 | 0.0182 | DNMT3L_2 | 0.1396 | ICAM2 | 0.0379 | NRXN3_3 | 0.0854 | SHC1_3 | 0.0821 |
| ARL6IP1_1 | 0.0285 | DOCK7_1 | 0.0392 | ICAM4_1 | 0.2654 | NT5DC1_2 | 0.2065 | SLC23A1_2 | 0.0993 |
| ARMCX3_2 | 0.0788 | DSC3_1 | 0.0815 | IL1RAP_2 | 0.0582 | NTRK2_3 | 0.0069 | SLC25A34 | 0.1422 |
| ATXN10_1 | 0.145 | DUT_3 | 0.1487 | IQCA1_2 | 0.0154 | NUP155_1 | 0.0424 | SLC4A5_3 | 0.0807 |
| AXL_1 | 0.0852 | EEF1E1_1 | 0.0939 | KCNIP3_1 | 0.0947 | NYX | 0.1168 | SLC9A10 | 0.0695 |
| BAI1_3 | 0.0498 | EMP1 | 0.1023 | KCNQ2_1 | 0.1368 | ODF2_3 | 0.0324 | SNORD93 | 0.1626 |
| BCAS1_2 | 0.3253 | ENO1 | 0.1574 | KIF3C | 0.2001 | ORC1L | 0.0686 | SOX2_1 | 0.0384 |
| BDNF_2 | 0.0542 | ENPEP_2 | 0.123 | KRT80_2 | 0.0777 | OTUD7A_3 | 0.0408 | STC1 | 0.0055 |
| BMPR1A | 0.1279 | EPHB1 | 0.0441 | KRTAP10.10_2 | 0.017 | PANK4 | 0.0531 | STC2 | 0.0906 |
| BTF3_3 | 0.1219 | EPYC | 0.0215 | L3MBTL2_3 | 0.0297 | PDLIM2_2 | 0.2123 | STYX_2 | 0.06 |
| C10orf116 | 0.0347 | ERI2_2 | 0.3043 | LBH_2 | 0.115 | PHYH_1 | 0.2441 | SYTL3 | 0.0395 |
| C11orf24 | 0.135 | ESPNL | 0.0812 | LENEP | 0.227 | PIGA_1 | 0.0191 | TAF15_1 | 0.0068 |
| C11orf49_3 | 0.1129 | EZH2_1 | 0.0696 | LGI3 | 0.108 | PITX2_1 | 0.1065 | TCEAL8_1 | 0.0377 |
| C14orf102_2 | 0.0886 | FAM13AOS | 0.0348 | LOC492303 | 0.0652 | PKN1_3 | 0.0469 | THBS3 | 0.0909 |
| C14orf109_2 | 0.0653 | FAM187B_2 | 0.0133 | LRRC14B | 0.0074 | PLAC9 | 0.2449 | TM2D3_2 | 0.0473 |
| C17orf106 | 0.186 | FAM70A_1 | 0.1001 | LRRC37A4_2 | 0.0756 | PLEKHG5_5 | 0.012 | TMEM52 | 0.0514 |
| C17orf58_2 | 0.0173 | FBXO48_2 | 0.1998 | LRRTM4 | 0.1404 | PLSCR4 | 0.1373 | TMEM62 | 0.0034 |
| C17orf58_3 | 0.0224 | FKBP10 | 0.1051 | MACC1 | 0.1261 | PMEPA1_4 | 0.1187 | TNFRSF18_1 | 0.2597 |
| C18orf56 | 0.069 | FLJ33360 | 0.0309 | MANSC1_1 | 0.1005 | PNMA5 | 0.1309 | TNNT2_1 | 0.0028 |
| C1orf168 | 0.0417 | FLJ43752 | 0.1597 | MAPK3_1 | 0.0421 | PPAPDC1A | 0.1066 | TOMM20L | 0.0343 |
| C1orf64 | 0.0966 | FMNL3_2 | 0.0093 | MCAM | 0.1193 | PRAMEF5 | 0.0252 | TPM2_2 | 0.1535 |
| C8orf79_1 | 0.0556 | FOSB | 0.1793 | MCART6 1 | 0.245 | PRKAA2 | 0.1312 | TRIM58 | 0.0861 |

| | | | | | | | |
|---|---|---|---|---|---|---|---|
| CALD1_2 | 0.1387 | FOSL2 | 0.0245 | MFRP | 0.0322 | PSMC6_1 | 0.0277 | UBR7_1 | 0.0507 |
| CASP8AP2 | 0.1287 | FOXN1 | 0.2707 | MIDN | 0.0405 | RAD54B_2 | 0.194 | UBR7_2 | 0.1277 |
| CCL13 | 0.129 | GAD1_2 | 0.0169 | MIR1914 | 0.0603 | RAP1A_1 | 0.2216 | WARS_2 | 0.1917 |
| CCR2_3 | 0.0384 | GBE1 | 0.0579 | MIR212 | 0.105 | RARA_3 | 0.0738 | XBP1_2 | 0.1677 |
| CD34_1 | 0.0467 | GBP7 | 0.096 | MIR571 | 0.0175 | RARG | 0.0353 | XRN2_1 | 0.0257 |
| CDC42BPA_2 | 0.0402 | GJA5_1 | 0.0592 | MIR576 | 0.0932 | RNASEK | 0.0754 | YARS2 | 0.0047 |
| CDC42SE2_2 | 0.0171 | GMNN | 0.0831 | MIR654 | 0.0046 | RNF7_1 | 0.0307 | ZNF75D_2 | 0.1573 |
| CLDN6 | 0.1193 | GSR_2 | 0.0323 | MIR942 | 0.0898 | ROD1_1 | 0.215 | ZSWIM4_2 | 0.1616 |
| CREB5_2 | 0.0082 | GUSBL2 | 0.1796 | MMP12_1 | 0.1345 | SATB2 | 0.0451 | figo_numeric | 0.0422 |
| CREBBP_1 | 0.0336 | HBA2 | 0.0535 | MYCN_2 | 0.1567 | SBSN | 0.0509 | hist_rev_SBOT | 0.0621 |
| CRYBA1 | 0.0946 | HDAC7_2 | 0.0236 | MYOHD1 | 0.0838 | SCXB | 0.0046 | surg_outcome | 0.017 |
| CXCL13 | 0.1656 | HDLBP_3 | 0.2023 | NFATC3 5 | 0.0215 | SEC22C_3 | 0.107 | | |
| CYB5R3_2 | 0.1641 | HIC1 | 0.0583 | NFATC4 | 0.0458 | SELENBP1 | 0.187 | | |

Table 18

| | | | | | | | | | |
|---|---|---|---|---|---|---|---|---|---|
| ABHD3 | 0.0358 | CYP1A2 | 0.0741 | HR_1 | 0.0355 | NR6A1_2 | 0.1216 | SFRS4 | 0.0202 |
| ADAM17_2 | 0.2175 | DBNDD2 | 0.098 | HSD11B1_1 | 0.1016 | NRXN3_3 | 0.0929 | SHC1_3 | 0.0728 |
| ADAMTS1 | 0.1475 | DNAH11 | 0.0412 | ICAM2 | 0.0264 | NT5DC1_2 | 0.1956 | SLC23A1_2 | 0.0726 |
| ALS2CL_3 | 0.0718 | DNMT3L_2 | 0.0177 | ICAM4_1 | 0.2407 | NTRK2_3 | 0.0019 | SLC25A34 | 0.1777 |
| ANO7_3 | 0.0026 | DOCK7_1 | 0.1137 | IL1RAP_2 | 0.0502 | NUP155_1 | 0.0124 | SLC4A5_3 | 0.0493 |
| ARL6IP1_1 | 0.0301 | DSC3_1 | 0.1013 | IQCA1_2 | 0.0688 | NYX | 0.1302 | SLC9A10 | 0.0661 |
| ARMCX3_2 | 0.1154 | DUT_3 | 0.1326 | KCNIP3_1 | 0.121 | ODF2_3 | 0.0364 | SNORD93 | 0.1527 |
| ATXN10_1 | 0.2003 | EEF1E1_1 | 0.1225 | KCNQ2_1 | 0.1444 | ORC1L | 0.0235 | SOX2_1 | 0.064 |
| AURKA_1 | 0.097 | EMP1 | 0.1073 | KIF3C | 0.1813 | OTUD7A_3 | 0.0004 | STC1 | 0.0261 |
| AXL_1 | 0.098 | ENO1 | 0.2154 | KRT80_2 | 0.1373 | PANK4 | 0.0478 | STC2 | 0.11 |
| BAII_3 | 0.2848 | ENPEP_2 | 0.1391 | KRTAP10.10_2 | 0.0006 | PDLIM2_2 | 0.2134 | STYX_2 | 0.0508 |
| BCAS1_2 | 0.1934 | EPHB1 | 0.037 | L3MBTL2_3 | 0.0243 | PHYH_1 | 0.1987 | SYTL3 | 0.0402 |
| BDNF_2 | 0.1042 | EPYC | 0.0317 | LBH_2 | 0.1357 | PIGA_1 | 0.0208 | TAF15_1 | 0.039 |
| BMPR1A | 0.0773 | ERI2_2 | 0.2626 | LENEP | 0.1929 | PITX2_1 | 0.1588 | TCEAL8_1 | 0.0633 |
| BTF3_3 | 0.1061 | ESPNL | 0.0144 | LGI3 | 0.1337 | PKN1_3 | 0.0585 | THBS3 | 0.0541 |
| C10orf116 | 0.0394 | FAM13AOS | 0.0531 | LOC492303 | 0.0623 | PLAC9 | 0.1971 | TM2D3_2 | 0.0553 |
| C11orf24 | 0.1559 | FAM187B_2 | 0.0063 | LRRC14B | 0.0203 | PLEKHG5_5 | 0.0088 | TMEM52 | 0.0882 |
| C11orf49_3 | 0.1075 | FAM70A_1 | 0.0312 | LRRC37A4_2 | 0.0692 | PLSCR4 | 0.1785 | TMEM62 | 0.0349 |
| C14orf102_2 | 0.061 | FBXO48_2 | 0.2751 | LRRTM4 | 0.1867 | PMEPA1_4 | 0.1644 | TNFRSF18_1 | 0.1996 |
| C14orf109_2 | 0.0944 | FKBP10 | 0.0421 | MACC1 | 0.0958 | PNMA5 | 0.1479 | TNNT2_1 | 0.0012 |
| C17orf16 | 0.2116 | FLJ33360 | 0.0369 | MANSCI_1 | 0.0871 | PPAPDC1A | 0.1292 | TOMM20L | 0.0207 |
| C17orf58_2 | 0.0678 | FLJ43752 | 0.1619 | MCAM | 0.0151 | PRAMEF 5 | 0.0158 | TPM2_2 | 0.1747 |
| C17orf58_3 | 0.0153 | FMNL3_2 | 0.0038 | MCART6_1 | 0.1587 | PRKAA2 | 0.0749 | TRIM58 | 0.0846 |
| C18orf56 | 0.0143 | FOSB | 0.2003 | MFRP | 0.2311 | PSMC6_1 | 0.0165 | UBR7_1 | 0.0724 |
| Clorf168 | 0.0481 | FOSL2 | 0.0605 | MIDN | 0.0149 | RAD54B_2 | 0.1786 | UBR7_2 | 0.1081 |
| Clorf64 | 0.1025 | FOXN1 | 0.2122 | MIR1914 | 0.0871 | RAP1A_1 | 0.1964 | WARS_2 | 0.1504 |

(continued)

| C8orf79_1 | 0.0143 | GAD1_2 | 0.0339 | MIR212 | 0.0853 | RARA_3 | 0.0843 | WDR76 | 0.1055 |
|---|---|---|---|---|---|---|---|---|---|
| CALD1_2 | 0.1427 | GBE1 | 0.0371 | MIR571 | 0.0262 | RARG | 0.0599 | XBP1_2 | 0.0507 |
| CASP8AP2 | 0.1075 | GBP7 | 0.1079 | MIR576 | 0.1224 | RNASEK | 0.086 | XRN2_1 | 0.0154 |
| CCL13 | 0.0573 | GJA5_1 | 0.0488 | MIR654 | 0.0165 | RNF7_1 | 0.0603 | YARS2 | 0.2493 |
| CCR2_3 | 0.0416 | GMNN | 0.0748 | MIR942 | 0.1649 | ROD1_1 | 0.1465 | ZNF75D_2 | 0.1434 |
| CD34_1 | 0.0012 | GSR_2 | 0.0024 | MMP12_1 | 0.0964 | SATB2 | 0.0455 | ZSWIM4_2 | 0.1542 |
| CDC42BPA_2 | 0.0142 | HBA2 | 0.1338 | MYCN_2 | 0.0799 | SBSN | 0.0009 | figo_numeric | 0.017 |
| CDC42SE2_2 | 0.0393 | HCFC1R1_1 | 0.0335 | MYOHD1 | 0.0809 | SCXB | 0.0096 | hist rev SBOT | 0.0598 |
| CLDN6 | 0.1119 | HDAC7_2 | 0.0236 | NFATC3_5 | 0.0184 | SEC22C_3 | 0.1034 | surg_outcome | 0.0325 |
| CREB5_2 | 0.0003 | HDLBP_3 | 0.0856 | NFATC4 | 0.0587 | SELENBP1 | 0.1436 | | |
| CRYBA1 | 0.0128 | HIC1 | 0.0437 | NLRP9 | 0.1608 | SERPINB2_2 | 0.0398 | | |
| CXCL 13 | 0.1187 | HPRT1_1 | 0.0759 | NOVA2 | 0.0823 | SERPINB 5 | 0.182 | | |
| CYB5R3_2 | 0.1309 | HPS4_1 | 0.0729 | NP | 0.1078 | SFN | 0.0272 | | |

Table 19

| | | | | | | | | | |
|---|---|---|---|---|---|---|---|---|---|
| ABHD3 | 0.0867 | CYP1A2 | 0.0615 | HPRT1_1 | 0.153 | NOV_A2 | 0.0939 | SERPINB5 | 0.2102 |
| ADAM17_2 | 0.2243 | DBNDD2 | 0.0776 | HPS4_1 | 0.0374 | NP | 0.0856 | SFN | 0.0075 |
| ADAMTS1 | 0.1794 | DFFB_2 | 0.0471 | HR_1 | 0.0572 | NR6A1_2 | 0.1322 | SFRS4 | 0.0402 |
| ALS2CL_3 | 0.1263 | DNAH11 | 0.0366 | HSD11B1_1 | 0.0885 | NRXN3_3 | 0.0775 | SHC1_3 | 0.0816 |
| ANO7_3 | 0.0411 | DNMT3L_2 | 0.1082 | ICAM2 | 0.0476 | NT5DC1_2 | 0.2081 | SLC23A1_2 | 0.0991 |
| ARL6IP1_1 | 0.0351 | DOCK7_1 | 0.0236 | ICAM4_1 | 0.2756 | NTRK2_3 | 0.0021 | SLC25A34 | 0.1145 |
| ARMCX3_2 | 0.0851 | DSC3_1 | 0.0613 | IL1RAP_2 | 0.0478 | NUP155_1 | 0.0426 | SLC4A5_3 | 0.0837 |
| ATXN10_1 | 0.1618 | DUT_3 | 0.1296 | IQCA1_2 | 0.0159 | NYX | 0.1089 | SLC9A10 | 0.0845 |
| AXL_1 | 0.0848 | EEF1E1_1 | 0.0553 | KCNIP3_1 | 0.0903 | ODF2_3 | 0.031 | SNORD93 | 0.1611 |
| BAI1_3 | 0.0502 | EMP1 | 0.1035 | KCNQ2_1 | 0.1439 | ORC1L | 0.0606 | SOX2_1 | 0.0554 |
| BCAS1_1 | 0.3153 | ENO1 | 0.1501 | KIF3C | 0.1887 | OTUD7A_3 | 0.0437 | STC1 | 0.0034 |
| BDNF_2 | 0.0933 | ENPEP_2 | 0.1261 | KRT80_2 | 0.0722 | PANK4 | 0.0523 | STC2 | 0.087 |
| BMPR1A | 0.117 | EPHB1 | 0.039 | KRTAP10.10_2 | 0.007 | PDLIM2_2 | 0.2126 | STYX_2 | 0.0552 |
| BTF3_3 | 0.1172 | EPYC | 0.0286 | L3MBTL2_3 | 0.0389 | PHYH_1 | 0.2226 | SYTL3 | 0.0023 |
| C10orf116 | 0.0561 | ERI2_2 | 0.2795 | LBH_2 | 0.1057 | PIGA_1 | 0.0139 | TAF15_1 | 0.0001 |
| C11orf24 | 0.1261 | ESPNL | 0.0821 | LENEP | 0.2159 | PITX2_1 | 0.0894 | TCEAL8_1 | 0.0511 |
| C11orf49_3 | 0.1216 | EZH2_1 | 0.0578 | LGI3 | 0.1292 | PKN1_3 | 0.0564 | THBS3 | 0.0877 |
| C14orf102_2 | 0.1004 | FAM13AOS | 0.0376 | LOC492303 | 0.054 | PLAC9 | 0.2581 | TM2D3_2 | 0.0459 |
| C14orf109_2 | 0.0679 | FAM187B_2 | 0.0233 | LRRC14B | 0.0258 | PLEKHG5_5 | 0.0187 | TMEM52 | 0.0589 |
| C17orf106 | 0.2023 | FAM70A_1 | 0.1041 | LRRC37A4_2 | 0.0709 | PLSCR4 | 0.16 | TMEM62 | 0.0064 |
| C17orf58_2 | 0.0266 | FBXO48_2 | 0.2125 | LRRTM4 | 0.1619 | PMEPA1_4 | 0.112 | TNFRSF18_1 | 0.2544 |
| C17orf58_3 | 0.0287 | FKBP10 | 0.1071 | MACC1 | 0.1254 | PNMA5 | 0.1346 | TNNT2_1 | 0.0027 |
| C18orf56 | 0.0405 | FLJ33360 | 0.0473 | MANSC1_1 | 0.1334 | PPAPDC1A | 0.1058 | TOMM20L | 0.0407 |
| C1orf168 | 0.0309 | FLJ43752 | 0.1767 | MCAM | 0.0693 | PRAMEF5 | 0.0239 | TPM2_2 | 0.1518 |
| C1orf64 | 0.1031 | FMNL3_2 | 0.0002 | MCART6_1 | 0.1011 | PRKAA2 | 0.1246 | TRIM58 | 0.111 |
| C8orf79_1 | 0.0769 | FOSB | 0.183 | MFRP | 0.2146 | PSMC6_1 | 0.0096 | UBR7_1 | 0.0246 |

| | | | | | | | | | |
|---|---|---|---|---|---|---|---|---|---|
| CALD1_2 | 0.1442 | FOSL2 | 0.0192 | MIDN | 0.0485 | RAD54B 2 | 0.1877 | UBR7_2 | 0.1237 |
| CASP8AP2 | 0.1236 | FOXN1 | 0.2739 | MIR1914 | 0.063 | RAP1A_1 | 0.212 | WARS_2 | 0.1836 |
| CCL13 | 0.1216 | GAD1_2 | 0.0157 | MIR212 | 0.0949 | RARA_3 | 0.0857 | XBP1_2 | 0.1624 |
| CCR2_3 | 0.0345 | GBE1 | 0.0527 | MIR571 | 0.007 | RARG | 0.017 | XRN2_1 | 0.0277 |
| CD34_1 | 0.0393 | GBP7 | 0.0937 | MIR576 | 0.097 | RNASEK | 0.0678 | YARS2 | 0.0053 |
| CDC42BPA_2 | 0.0358 | GJA5_1 | 0.0517 | MIR654 | 0.0006 | RNF7_1 | 0.0169 | ZNF75D_2 | 0.1444 |
| CDC42SE2_2 | 0.0007 | GMNN | 0.0868 | MIR942 | 0.107 | ROD1_1 | 0.2162 | ZSWIM4_2 | 0.157 |
| CLDN6 | 0.1183 | GSR_2 | 0.0316 | MMP12_1 | 0.135 | SATB2 | 0.054 | figo_numeric | 0.0381 |
| CREB5_2 | 0.0028 | GUSBL2 | 0.1966 | MYCN_2 | 0.1539 | SBSN | 0.0626 | hist_rev_SBOT | 0.0579 |
| CREBBP_1 | 0.0384 | HBA2 | 0.0744 | MYOHD1 | 0.0868 | SCXB | 0.002 | surg_outcome | 0.0071 |
| CRYBA1 | 0.0852 | HDAC7_2 | 0.0462 | NFATC3_5 | 0.0261 | SEC22C_3 | 0.1031 | | |
| CXCL13 | 0.1743 | HDLBP_3 | 0.2167 | NFATC4 | 0.0564 | SELENBP1 | 0.1888 | | |
| CYB5R3_2 | 0.1549 | HIC1 | 0.0817 | NLRP9 | 0.159 | SERPINB2_2 | 0.006 | | |

Table 20

| | | | | | | | | | |
|---|---|---|---|---|---|---|---|---|---|
| ABHD3 | 0.0624 | DNAH11 | 0.0467 | HSD11B1_1 | 0.0931 | NRXN3_3 | 0.0999 | SHC1_3 | 0.072 |
| ADAM17_2 | 0.2343 | DNMT3L_2 | 0.0206 | ICAM2 | 0.0493 | NT5DC1_2 | 0.1855 | SLC23A1_2 | 0.1388 |
| ADAMTS1 | 0.1768 | DOCK7_1 | 0.1317 | ICAM4_1 | 0.279 | NTRK2_3 | 0.0077 | SLC25A34 | 0.1531 |
| ALS2CL_3 | 0.1061 | DSC3_1 | 0.0661 | IL1RAP 2 | 0.06 | NUP155_1 | 0.0488 | SLC4A5_3 | 0.0803 |
| ANO7_3 | 0.0694 | DUT_3 | 0.121 | IQCA1_2 | 0.0294 | NYX | 0.1733 | SLC9A10 | 0.0867 |
| ARL6IP1_1 | 0.037 | EEF1E1_1 | 0.0871 | KCNIP3_1 | 0.1039 | ODF2_3 | 0.0153 | SNORD93 | 0.1624 |
| ARMCX3_2 | 0.0811 | EMP1 | 0.1112 | KCNQ2_1 | 0.1248 | ORC1L | 0.0294 | SOX2_1 | 0.0764 |
| ATXN10_1 | 0.2064 | ENOI | 0.1821 | KIF3C | 0.1802 | OTUD7A_3 | 0.0342 | STC1 | 0.0073 |
| AXL_1 | 0.1046 | ENPEP_2 | 0.1326 | KRT80_2 | 0.1107 | PANK4 | 0.055 | STC2 | 0.1324 |
| BAII_3 | 0.0284 | EPHB1 | 0.0452 | KRTAP10.10_2 | 0.0206 | PDLIM2_2 | 0.2387 | STYX 2 | 0.0448 |
| BCAS1_1 | 0.3214 | EPYC | 0.0338 | L3MBTL2_3 | 0.034 | PHYH_1 | 0.1976 | SYTL3 | 0.0108 |
| BDNF_2 | 0.1134 | ERI2_2 | 0.2957 | LBH_2 | 0.0952 | PIGA_1 | 0.0024 | TAF15_1 | 0.0108 |
| BMPR1A | 0.11 | ESPNL | 0.0367 | LENEP | 0.2321 | PITX2_1 | 0.0924 | TCEAL8_1 | 0.0417 |
| BTF3_3 | 0.1052 | EZH2_1 | 0.0785 | LGI3 | 0.1201 | PKN1_3 | 0.0216 | THBS3 | 0.1047 |
| C10orf116 | 0.0302 | FAM13AOS | 0.0433 | LOC492303 | 0.0295 | PLAC9 | 0.2492 | THY1 | 0.0575 |
| C11orf24 | 0.1733 | FAM187B_2 | 0.0131 | LRRC14B | 0.0148 | PLEKHG5_5 | 0.0323 | TIMP2_2 | 0.0816 |
| C11orf49_3 | 0.1351 | FAM70A_1 | 0.0792 | LRRC37A4_2 | 0.0563 | PLSCR4 | 0.1766 | TM2D3_2 | 0.005 |
| C14orf102_2 | 0.1246 | FBXO48_2 | 0.2631 | LRRTM4 | 0.167 | PMEPA1_4 | 0.1204 | TMEM52 | 0.0275 |
| C14orf109_2 | 0.0694 | FKBP10 | 0.0694 | MACC1 | 0.1174 | PNMA5 | 0.1295 | TMEM62 | 0.0704 |
| C17orf106 | 0.2355 | FLJ33360 | 0.0483 | MANSC1_1 | 0.1393 | PPAPDC1A | 0.1315 | TNFRSF18_1 | 0.2567 |
| C17orf58_2 | 0.0328 | FLJ43752 | 0.1925 | MCAM | 0.0176 | PRAMEF5 | 0.0165 | TNNT2_1 | 0.0008 |
| C17orf58_3 | 0.0253 | FMNL3_2 | 0.0428 | MCART6_1 | 0.1302 | PRKAA2 | 0.1306 | TOMM20L | 0.0434 |
| C18orf56 | 0.0356 | FOSB | 0.1926 | MFRP | 0.2149 | PSMC6_1 | 0.0029 | TPM2_2 | 0.1799 |
| C1orf168 | 0.0309 | FOSL2 | 0.0287 | MIDN | 0.0442 | RAD54B_2 | 0.1842 | TRIM58 | 0.1137 |
| C1orf64 | 0.1075 | FOXN1 | 0.261 | MIR1914 | 0.0697 | RAP1A_1 | 0.2169 | UBR7_1 | 0.0577 |
| C8orf79_1 | 0.021 | GAD1_2 | 0.0214 | MIR212 | 0.1069 | RARA_3 | 0.0856 | UBR7_2 | 0.1274 |

| | | | | | | | | | |
|---|---|---|---|---|---|---|---|---|---|
| CASP8AP2 | 0.141 | GBE1 | 0.0453 | MIR571 | 0.0316 | RARG | 0.0481 | WARS_2 | 0.1613 |
| CCL13 | 0.146 | GBP7 | 0.133 | MIR576 | 0.1023 | RNASEK | 0.064 | XBP1_2 | 0.1397 |
| CCR2_3 | 0.0827 | GJA5_1 | 0.0525 | MIR654 | 0.0539 | RNF7_1 | 0.0209 | XRN2_1 | 0.0525 |
| CD34_1 | 0.0204 | GMNN | 0.0973 | MIR942 | 0.1338 | ROD1_1 | 0.2196 | YARS2 | 0.0062 |
| CDC42BPA_2 | 0.0281 | GSR_2 | 0.0421 | MMP12_1 | 0.1307 | SATB2 | 0.057 | ZNF75D_2 | 0.1498 |
| CDC42SE2_2 | 0.0175 | HBA2 | 0.2048 | MYCN_2 | 0.1396 | SBSN | 0.0581 | ZSWIM4_2 | 0.1618 |
| CLDN6 | 0.1155 | HCFC1R1_1 | 0.0572 | MYOHD1 | 0.0939 | SCXB | 0.0069 | figo_numeric | 0.021 |
| CREB5_2 | 0.0101 | HDAC7_2 | 0.0043 | NFATC3_5 | 0.008 | SEC22C_3 | 0.1229 | hist_rev_SBOT | 0.048 |
| CRYBA1 | 0.0182 | HDLBP_3 | 0.1153 | NFATC4 | 0.0521 | SELENBP1 | 0.1943 | surg_outcome | 0.0088 |
| CXCL13 | 0.0736 | HIC1 | 0.0396 | NLRP9 | 0.18 | SERPINB2_2 | 0.0123 | | |
| CYB5R3_2 | 0.1819 | HPRT1_1 | 0.1514 | NOVA2 | 0.1202 | SERPINB5 | 0.198 | | |
| CYP1A2 | 0.0568 | HPS4_1 | 0.0653 | NP | 0.0885 | SFN | 0.0091 | | |
| DBNDD2 | 0.1052 | HR_1 | 0.0434 | NR6A1_2 | 0.1446 | SFRS4 | 0.0329 | | |

Table 21

| Gene | Value | Gene | Value | Gene | Value | Gene | Value | Gene | Value |
|---|---|---|---|---|---|---|---|---|---|
| ABHD3 | 0.0663 | DNAH11 | 0.0475 | HSD11B1_1 | 0.0915 | NR6A1_2 | 0.1487 | SFRS4 | 0.0329 |
| ADAM17_2 | 0.2308 | DNMT3L_2 | 0.0228 | ICAM2 | 0.0608 | NRXN3_3 | 0.1005 | SHC1_3 | 0.0696 |
| ADAMTS1 | 0.175 | DOCK7_1 | 0.14 | ICAM4_1 | 0.2742 | NT5DC1_2 | 0.1878 | SLC23A1_2 | 0.1397 |
| ALS2CL_3 | 0.1066 | DSC3_1 | 0.0737 | IL1RAP_2 | 0.0589 | NTRK2_3 | 0.0059 | SLC25A34 | 0.155 |
| ANO7_3 | 0.0621 | DUT_3 | 0.1195 | IQCA1_2 | 0.0298 | NUP155_1 | 0.0484 | SLC4A5_3 | 0.0813 |
| ARL6IP1_1 | 0.0271 | EEF1E1_1 | 0.0883 | KCNIP3_1 | 0.1058 | NYX | 0.1782 | SLC9A10 | 0.0816 |
| ARMCX3_2 | 0.0823 | EMP1 | 0.1186 | KCNQ2_1 | 0.1317 | ODF2_3 | 0.0118 | SNORD93 | 0.1585 |
| ATXN10_1 | 0.2065 | ENOI | 0.1822 | KIF3C | 0.1789 | ORC1L | 0.0299 | SOX2_1 | 0.0771 |
| AXL_1 | 0.1063 | ENPEP_2 | 0.1303 | KRT80_2 | 0.1081 | OTUD7A_3 | 0.0332 | STC1 | 0.0091 |
| BAII_3 | 0.0239 | EPHB1 | 0.0369 | KRTAP10.10_2 | 0.0215 | PANK4 | 0.0559 | STC2 | 0.1293 |
| BCAS1_1 | 0.3215 | EPYC | 0.0297 | L3MBTL2_3 | 0.0311 | PDLIM2_2 | 0.2435 | STYX_2 | 0.0471 |
| BDNF_2 | 0.1088 | ERI2_2 | 0.2948 | LBH_2 | 0.0943 | PHYH_1 | 0.1998 | SYTL3 | 0.008 |
| BMPR1A | 0.1123 | ESPNL | 0.0342 | LENEP | 0.2325 | PIGA_1 | 0.0015 | TAF15_1 | 0.0012 |
| BTF3_3 | 0.1045 | EZH2_1 | 0.0734 | LGI3 | 0.1111 | PITX2_1 | 0.0912 | TCEAL8_1 | 0.0388 |
| C10orf116 | 0.0333 | FAM13AOS | 0.0438 | LOC492303 | 0.0252 | PKN1_3 | 0.018 | THBS3 | 0.1054 |
| C11orf24 | 0.1704 | FAM187B_2 | 0.011 | LRRC14B | 0.0127 | PLAC9 | 0.2485 | TIMP2_2 | 0.0614 |
| C11orf49_3 | 0.1322 | FAM70A_1 | 0.081 | LRRC37A4_2 | 0.061 | PLEKHG5_5 | 0.0248 | TM2D3_2 | 0.0737 |
| C14orf102_2 | 0.1184 | FBXO48_2 | 0.2591 | LRRTM4 | 0.1675 | PLSCR4 | 0.1735 | TMEM52 | 0.0072 |
| C14orf109_2 | 0.0685 | FKBP10 | 0.0693 | MACC1 | 0.1186 | PMEPA1_4 | 0.1229 | TMEM62 | 0.0699 |
| C17orf06 | 0.2339 | FLJ33360 | 0.0537 | MANSC1_1 | 0.1364 | PNMA5 | 0.1265 | TNFRSF18_1 | 0.2674 |
| C17orf58_2 | 0.0463 | FLJ43752 | 0.1899 | MCAM | 0.013 | PPAPDC1A | 0.1353 | TNNT2_1 | 0.0025 |
| C17orf58_3 | 0.0226 | FMNL3_2 | 0.0457 | MCART6_1 | 0.1314 | PRAMEF5 | 0.0079 | TOMM20L | 0.0407 |
| C18orf56 | 0.0371 | FOSB | 0.2007 | MFRP | 0.2201 | PRKAA2 | 0.1319 | TPM2_2 | 0.1772 |
| C1orfl68 | 0.0353 | FOSL2 | 0.0284 | MIDN | 0.0394 | PSMC6_1 | 0.0012 | TRIM58 | 0.1118 |
| C1orf64 | 0.1083 | FOXN1 | 0.2708 | MIR1914 | 0.0643 | RAD54B_2 | 0.1809 | UBR7_1 | 0.0622 |
| C8orf79_1 | 0.0248 | GAD1_2 | 0.0186 | MIR212 | 0.1082 | RAP1A_1 | 0.2108 | UBR7_2 | 0.1264 |

(continued)

| | | | | | | | | | |
|---|---|---|---|---|---|---|---|---|---|
| CASP8AP2 | 0.1364 | GBE1 | 0.0467 | MIR571 | 0.0339 | RARA_3 | 0.0834 | WARS_2 | 0.1566 |
| CCL13 | 0.1382 | GBP7 | 0.1322 | MIR576 | 0.104 | RARG | 0.0468 | XBP1_2 | 0.1366 |
| CCR2_3 | 0.083 | GJA5_1 | 0.0489 | MIR654 | 0.0504 | RNASEK | 0.0632 | XRN2_1 | 0.0525 |
| CD34_1 | 0.015 | GMNN | 0.1011 | MIR942 | 0.1245 | RNF7_1 | 0.0209 | YARS2 | 0.0045 |
| CDC42BPA_2 | 0.0272 | GSR_2 | 0.0408 | MMP12_1 | 0.131 | ROD1_1 | 0.2223 | ZNF75D_2 | 0.1493 |
| CDC42SE2_2 | 0.0209 | HBA2 | 0.1972 | MYCN_2 | 0.144 | SATB2 | 0.0592 | ZSWIM4_2 | 0.1622 |
| CLDN6 | 0.114 | HCFC1R1_1 | 0.0584 | MYL9_2 | 0.0911 | SBSN | 0.0579 | figo_numeric | 0.0199 |
| CREB5_2 | 0.014 | HDAC7_2 | 0.0084 | MYOHD1 | 0.0077 | SCXB | 0.0053 | hist_rev_SBOT | 0.0508 |
| CRYBA1 | 0.0281 | HDLBP_3 | 0.1136 | NFATC3_5 | 0.0536 | SEC22C_3 | 0.1148 | surg_outcome | 0.0057 |
| CXCL13 | 0.0738 | HIC1 | 0.0397 | NFATC4 | 0.0635 | SELENBP1 | 0.1917 | | |
| CYB5R3_2 | 0.18 | HPRT1_1 | 0.1549 | NLRP9 | 0.181 | SERPINB2 2 | 0.004 | | |
| CYP1A2 | 0.0588 | HPS4_1 | 0.0624 | NOVA2 | 0.1239 | SERPINB5 | 0.1982 | | |
| DBNDD2 | 0.1084 | HR_1 | 0.041 | NP | 0.0898 | SFN | 0.0117 | | |

Table 22

| Gene | Value | Gene | Value | Gene | Value | Gene | Value | Gene | Value |
|---|---|---|---|---|---|---|---|---|---|
| ABHD3 | 0.0702 | DNAH11 | 0.0458 | HSD11B1_1 | 0.0927 | NRXN3_3 | 0.0945 | SFRS4 | 0.0298 |
| ADAM17_2 | 0.24 | DNMT3L_2 | 0.019 | ICAM2 | 0.0457 | NT5DC1_2 | 0.1882 | SHC1_3 | 0.0655 |
| ADAMTS1 | 0.1767 | DOCK7_1 | 0.1366 | ICAM4_1 | 0.2788 | NTRK2_3 | 0.0009 | SLC23A1_2 | 0.141 |
| ALS2CL_3 | 0.1037 | DSC3_1 | 0.0765 | IL1RAP_2 | 0.0514 | NUP155_1 | 0.0572 | SLC25A34 | 0.1681 |
| ANO7_3 | 0.0614 | DUT_3 | 0.1146 | IQCA1_2 | 0.0262 | NYX | 0.1804 | SLC4A5_3 | 0.0826 |
| ARL6IP1_1 | 0.0381 | EEF1E1_1 | 0.0742 | KCNIP3_1 | 0.1058 | ODF2_3 | 0.0208 | SLC9A10 | 0.0799 |
| ARMCX3_2 | 0.082 | EMP1 | 0.1256 | KCNQ2_1 | 0.1243 | ORC1L | 0.0268 | SNORD93 | 0.1647 |
| ATXN10_1 | 0.1984 | ENOI | 0.1956 | KIF3C | 0.1741 | OTUD7A_3 | 0.0356 | SOX2_1 | 0.0848 |
| AXL_1 | 0.1098 | ENPEP_2 | 0.1362 | KRT80_2 | 0.1197 | PANK4 | 0.0582 | STC1 | 0.0087 |
| BAI1_3 | 0.0235 | EPHB1 | 0.0311 | KRTAP10.10_2 | 0.0223 | PDLIM2_2 | 0.2471 | STC2 | 0.1232 |
| BCAS1_1 | 0.3327 | EPYC | 0.0385 | L3MBTL2_3 | 0.032 | PHYH_1 | 0.1962 | STYX_2 | 0.0512 |
| BDNF_2 | 0.11 | ERI2_2 | 0.2922 | LBH_2 | 0.0926 | PIGA_1 | 0.0032 | SYTL3 | 0.0226 |
| BMPR1A | 0.1201 | ESPNL | 0.0338 | LENEP | 0.231 | PITX2_1 | 0.0989 | TAF15_1 | 0.0036 |
| BTF3_3 | 0.1057 | EZH2_1 | 0.0821 | LGI3 | 0.1303 | PKN1_3 | 0.0161 | TCEAL8_1 | 0.0349 |
| C10orf116 | 0.038 | FAM13AOS | 0.0551 | LOC492303 | 0.0326 | PLAC9 | 0.2729 | THBS3 | 0.0901 |
| C11orf24 | 0.1905 | FAM187B_2 | 0.0037 | LRRC14B | 0.0188 | PLEKHG5_5 | 0.0299 | TM2D3_2 | 0.058 |
| C11orf49_3 | 0.1248 | FAM70A_1 | 0.1031 | LRRC37A4_2 | 0.0536 | PLSCR4 | 0.1546 | TMEM52 | 0.0888 |
| C14orf102_2 | 0.1242 | FBXO48_2 | 0.2667 | LRRTM4 | 0.1687 | PMEPA1_4 | 0.1226 | TMEM62 | 0.0037 |
| C14orf109_2 | 0.0629 | FKBP10 | 0.0661 | MACC1 | 0.124 | PNMA5 | 0.1159 | TNFRSF18_1 | 0.2615 |
| C17orf106 | 0.2391 | FLJ33360 | 0.048 | MANSC1_1 | 0.1326 | PPAPDC1A | 0.1284 | TNNT2_1 | 0.0125 |
| C17orf58_2 | 0.0316 | FLJ43752 | 0.2006 | MCAM | 0.0075 | PRAMEF_5 | 0.0196 | TOMM20L | 0.0402 |
| C17orf58_3 | 0.0302 | FMNL3_2 | 0.0538 | MCART6_1 | 0.1271 | PRKAA2 | 0.1281 | TPM2_2 | 0.1775 |
| C18orf56 | 0.0364 | FOSB | 0.2041 | MFRP | 0.2258 | PSMC6_1 | 0.0134 | TRIM58 | 0.1153 |
| C1orf168 | 0.0316 | FOSL2 | 0.0243 | MIDN | 0.048 | RAD54B_2 | 0.1807 | UBR7_1 | 0.0551 |
| C8orf79_1 | 0.1135 | FOXN1 | 0.2702 | MIR1914 | 0.0695 | RAP1A_1 | 0.2136 | UBR7_2 | 0.1342 |
| CALD1_2 | 0.0409 | GAD1_2 | 0.0071 | MIR212 | 0.102 | RARA_3 | 0.0868 | WARS_2 | 0.1524 |

(continued)

| CASP8AP2 | 0.1434 | GBE1 | 0.045 | MIR571 | 0.0301 | RARG | 0.0463 | XBP1_2 | 0.1231 |
|---|---|---|---|---|---|---|---|---|---|
| CCL13 | 0.0815 | GBP7 | 0.1204 | MIR576 | 0.1013 | RNASEK | 0.062 | XRN2_1 | 0.0467 |
| CCR2_3 | 0.0319 | GJA5_1 | 0.0543 | MIR654 | 0.0511 | RNF7_1 | 0.0136 | YARS2 | 0.0093 |
| CD34_1 | 0.0148 | GMNN | 0.1034 | MIR942 | 0.1348 | ROD1_1 | 0.2251 | ZNF75D_2 | 0.1453 |
| CDC42BPA_2 | 0.0307 | GSR_2 | 0.0442 | MMP12_1 | 0.1385 | SATB2 | 0.053 | ZSWIM4_2 | 0.1658 |
| CDC42SE2_2 | 0.0235 | HBA2 | 0.2027 | MYCN_2 | 0.143 | SBSN | 0.055 | figo_numeric | 0.0134 |
| CLDN6 | 0.1084 | HCFC1R1_1 | 0.0499 | MYOHD1 | 0.089 | SCXB | 0.0075 | hist_rev_SBOT | 0.0617 |
| CREB5_2 | 0.0169 | HDAC7_2 | 0.0025 | NFATC3_5 | 0.0118 | SEC22C_3 | 0.1238 | surg_outcome | 0.0173 |
| CRYBA1 | 0.0302 | HDLBP_3 | 0.1094 | NFATC4 | 0.0472 | SELENBP1 | 0.1967 | | |
| CXCL13 | 0.0792 | HIC1 | 0.0438 | NLRP9 | 0.1849 | SERPINA12 | 0.0282 | | |
| CYB5R3_2 | 0.1878 | HPRT1_1 | 0.1519 | NOVA2 | 0.1147 | SERPINB2_2 | 0.1935 | | |
| CYP1A2 | 0.0598 | HPS4_1 | 0.0643 | NP | 0.0941 | SERPINB5 | 0.003 | | |
| DBNDD2 | 0.1083 | HR_1 | 0.0448 | NR6A1_2 | 0.1439 | SFN | 0.0536 | | |

Table 23

| | | | | | | | | | |
|---|---|---|---|---|---|---|---|---|---|
| ABHD3 | 0.0752 | CYB5R3_2 | 0.1585 | HIC1 | 0.0844 | NFATC4 | 0.0592 | SERPINB2_2 | 0.0056 |
| ADAM17_2 | 0.2422 | CYP1A2 | 0.0603 | HPRT1_1 | 0.146 | NLRP9 | 0.156 | SERPINB5 | 0.1869 |
| ADAMTS1 | 0.1531 | DBNDD2 | 0.0978 | HPS4_1 | 0.0335 | NOVA2 | 0.0483 | SFN | 0.0032 |
| ADAMTS2_1 | 0.1 | DFFB_2 | 0.0433 | HR_1 | 0.0376 | NP | 0.0783 | SFRS4 | 0.063 |
| ALS2CL_3 | 0.0622 | DNAH11 | 0.0292 | HSD11B1_1 | 0.1071 | NR6A1_2 | 0.1239 | SHC1_3 | 0.0786 |
| ANO7_3 | 0.0333 | DNMT3L_2 | 0.0881 | ICAM2 | 0.009 | NRXN3_3 | 0.1232 | SLC23A1_2 | 0.0821 |
| ARL6IP1 1 | 0.0222 | DOCK7_1 | 0.0205 | ICAM4_1 | 0.2848 | NT5DC1_2 | 0.1835 | SLC25A34 | 0.0944 |
| ARMCX3_2 | 0.0627 | DSC3_1 | 0.0348 | IL1RAP_2 | 0.0627 | NTRK2_3 | 0.0091 | SLC4A5_3 | 0.0989 |
| ATXN10_1 | 0.1719 | DUT_3 | 0.116 | IQCA1_2 | 0.0016 | NUP155_1 | 0.036 | SLC9A10 | 0.0687 |
| AXL_1 | 0.0779 | EEF1E1_1 | 0.1036 | KCNIP3_1 | 0.082 | NYX | 0.0826 | SNORD93 | 0.1311 |
| BAII_3 | 0.0545 | ELN_2 | 0.118 | KCNQ2_1 | 0.1277 | ODF2_3 | 0.0205 | SOX2_1 | 0.0498 |
| BCAS1_1 | 0.316 | EMP1 | 0.1789 | KIF3C | 0.1765 | ORC1L | 0.047 | STC1 | 0.0123 |
| BDNF_2 | 0.0885 | ENO1 | 0.1485 | KRT80_2 | 0.0673 | OTUD7A_3 | 0.0436 | STC2 | 0.09 |
| BMPR1A | 0.1239 | ENPEP_2 | 0.0537 | KRTAP10.10_2 | 0.0301 | PANK4 | 0.0471 | STYX_2 | 0.0308 |
| BTF3_3 | 0.1092 | EPHB1 | 0.03 | L3MBTL2_3 | 0.0485 | PDLIM2_2 | 0.1911 | SYTL3 | 0.0161 |
| C10orfll6 | 0.0845 | EPYC | 0.0396 | LBH_2 | 0.0769 | PDZRN4_2 | 0.2271 | TAF15_1 | 0.0182 |
| C11orf24 | 0.1233 | ERI2_2 | 0.2726 | LENEP | 0.2266 | PHYH_1 | 0.0097 | TCEAL8_1 | 0.0291 |
| C11orf49_3 | 0.111 | ESPNL | 0.0801 | LGI3 | 0.1039 | PIGA_1 | 0.0838 | THBS3 | 0.0783 |
| C14orf102_2 | 0.0988 | EZH2_1 | 0.0464 | LOC340508 | 0.0295 | PITX2_1 | 0.1998 | TM2D3_2 | 0.0275 |
| C14orf109_2 | 0.1089 | FAM13AOS | 0.055 | LOC492303 | 0.035 | PKN1_3 | 0.0372 | TMEM52 | 0.0679 |
| C17orf106 | 0.1557 | FAM187B_2 | 0.0069 | LRRC14B | 0.0695 | PLEKHG5_5 | 0.2717 | TMEM62 | 0.0014 |
| C17orf58_2 | 0.0009 | FAM70A_1 | 0.1027 | LRRC37A4_2 | 0.0036 | PLSCR4 | 0.0178 | TNFRSF18_1 | 0.23 |
| C17orf58_3 | 0.0262 | FBXO48_2 | 0.1908 | LRRTM4 | 0.1592 | PMEPA1_4 | 0.1444 | TNNT2_1 | 0.0008 |
| C18orf56 | 0.0128 | FKBP10 | 0.0969 | MACC1 | 0.1494 | PNMA5 | 0.1694 | TOMM20L | 0.0044 |
| Clorf168 | 0.0266 | FLJ33360 | 0.0233 | MANSCI_1 | 0.1284 | PPAPDC1A | 0.087 | TPM2_2 | 0.1504 |
| Clorf64 | 0.1011 | FLJ43752 | 0.2125 | MAPK3_1 | 0.0788 | PRAMEF 5 | 0.0101 | TRIM58 | 0.1121 |

| | | | | | | | |
|---|---|---|---|---|---|---|---|
| C8orf79_1 | 0.0411 | FMNL3_2 | 0.0269 | MCAM | 0.0948 | PRKAA2 | 0.1108 | UBR7_1 | 0.0587 |
| CALD1_2 | 0.1497 | FOSB | 0.1983 | MCART6_1 | 0.2292 | PSMC6_1 | 0.0137 | UBR7_2 | 0.1435 |
| CASP8AP2 | 0.1247 | FOSL2 | 0.0424 | MFRP | 0.0249 | RAD54B_2 | 0.1908 | WARS_2 | 0.2033 |
| CCL13 | 0.1557 | FOXN1 | 0.2379 | MIDN | 0.0441 | RAP1A_1 | 0.1953 | XBP1_2 | 0.176 |
| CCR2_3 | 0.0359 | GAD1_2 | 0.0249 | MIR1914 | 0.0566 | RARA_3 | 0.0953 | XRN2_1 | 0.0354 |
| CD34_1 | 0.0391 | GBE1 | 0.0517 | MIR212 | 0.0952 | RARG | 0.0276 | YARS2 | 0.0318 |
| CDC42BPA_2 | 0.0028 | GBP7 | 0.069 | MIR571 | 0.0392 | RNASEK | 0.1092 | ZNF75D_2 | 0.1281 |
| CDC42SE2_2 | 0.0014 | GJA5_1 | 0.0574 | MIR576 | 0.0931 | RNF7_1 | 0.0409 | ZSWIM4_2 | 0.1684 |
| CIDEC_1 | 0.1111 | GMNN | 0.1028 | MIR654 | 0.0133 | ROD1_1 | 0.1859 | figo_numeric | 0.0233 |
| CLDN6 | 0.0245 | GSR_2 | 0.011 | MIR942 | 0.0942 | SATB2 | 0.0304 | hist_rev_SBOT | 0.0775 |
| CREB5_2 | 0.0192 | GUSBL2 | 0.1976 | MMP12_1 | 0.1263 | SBSN | 0.0903 | surg_outcome | 0.008 |
| CREBBP_1 | 0.0576 | HBA2 | 0.0682 | MYCN_2 | 0.1423 | SCXB | 0.006 | | |
| CRYBA1 | 0.0714 | HDAC7_2 | 0.0378 | MYOHD1 | 0.0937 | SEC22C_3 | 0.0935 | | |
| CXCL13 | 0.1734 | HDLBP_3 | 0.2046 | NFATC3_5 | 0.0344 | SELENBP1 | 0.1544 | | |

Table 24

| | | | | | | | | | |
|---|---|---|---|---|---|---|---|---|---|
| ABCC9_3 | 0.0684 | CXCL13 | 0.1738 | HIC1 | 0.0948 | NFATC4 | 0.0518 | SERPINB2_2 | 0.0194 |
| ABHD3 | 0.2415 | CYB5R3_2 | 0.1632 | HPRT1_1 | 0.1329 | NLRP9 | 0.1645 | SERPINB5 | 0.1876 |
| ADAM17_ 2 | 0.1509 | CYP1A2 | 0.0538 | HPS4_1 | 0.035 | NOVA2 | 0.0652 | SFN | 0.0072 |
| ADAMTS1 | 0.077 | DBNDD2 | 0.0963 | HR_1 | 0.0463 | NP | 0.0917 | SFRS4 | 0.0706 |
| ADAMTS2_1 | 0.1042 | DFFB_2 | 0.0411 | HSD11B1_1 | 0.1014 | NR6A1_2 | 0.1183 | SHC1_3 | 0.0852 |
| ALS2CL_3 | 0.0566 | DNAH11 | 0.0364 | ICAM2 | 0.0074 | NRXN3_3 | 0.1265 | SLC23A1_2 | 0.0937 |
| ANO7_3 | 0.0462 | DNMT3L_2 | 0.0966 | ICAM4_1 | 0.2673 | NT5DC1_2 | 0.1841 | SLC25A34 | 0.1048 |
| ARL6IP1_1 | 0.0085 | DOCK7_1 | 0.0181 | IL1RAP_2 | 0.0556 | NTRK2_3 | 0.0117 | SLC4A5_3 | 0.0947 |
| ARMCX3_2 | 0.0652 | DSC3_1 | 0.0424 | IQCA1_2 | 0.0019 | NUP155_1 | 0.0354 | SLC9A10 | 0.0692 |
| ATXN10_1 | 0.1727 | DUT_3 | 0.1173 | KCNIP3_1 | 0.0898 | NYX | 0.0627 | SNORD93 | 0.1264 |
| AXL_1 | 0.072 | EEF1E1_1 | 0.0994 | KCNQ2_1 | 0.135 | ODF2_3 | 0.0347 | SOX2_1 | 0.0569 |
| BAI1_3 | 0.0458 | EMP1 | 0.1047 | KIF3C | 0.1711 | ORC1L | 0.0411 | STC1 | 0.0117 |
| BCAS1_1 | 0.3113 | ENO1 | 0.1697 | KRT80_2 | 0.0795 | OTUD7A_3 | 0.0579 | STC2 | 0.0978 |
| BDNF_2 | 0.1029 | ENPEP_2 | 0.1446 | KRTAP10.10_2 | 0.0249 | PANK4 | 0.0507 | STYX_2 | 0.0393 |
| BMPR1A | 0.1241 | EPHB1 | 0.0415 | L3MBTL2_3 | 0.0536 | PDLIM2_2 | 0.1883 | SYTL3 | 0.0208 |
| BTF3_3 | 0.1138 | EPYC | 0.0292 | LBH_2 | 0.0829 | PDZRN4_2 | 0.2332 | TAF15_1 | 0.0158 |
| C10orf116 | 0.0767 | ERI2_2 | 0.2792 | LENEP | 0.2326 | PHYH_1 | 0.0127 | TCEAL8_1 | 0.0333 |
| Cllorf24 | 0.1289 | ESPNL | 0.0781 | LGI3 | 0.1066 | PIGA_1 | 0.0899 | THBS3 | 0.0884 |
| C11orf49_3 | 0.1095 | EZH2_1 | 0.0508 | LOC340508 | 0.0496 | PITX2_1 | 0.1944 | TM2D3_2 | 0.0378 |
| C14orf102_2 | 0.0891 | FAM13AOS | 0.0616 | LOC492303 | 0.0275 | PKN1_3 | 0.0315 | TMEM52 | 0.0732 |
| C14orf109_2 | 0.114 | FAM187B_2 | 0.0118 | LRRC14B | 0.0657 | PLEKHG5_5 | 0.2484 | TMEM62 | 0.0112 |
| C17orf106 | 0.1586 | FAM70A_1 | 0.0982 | LRRC37A4_2 | 0.0104 | PLSCR4 | 0.019 | TNFRSF18_1 | 0.2304 |
| C17orf58_2 | 0.0052 | FBXO48_2 | 0.1891 | LRRTM4 | 0.1747 | PMEPA1_4 | 0.1389 | TNNT2_1 | 0.0086 |
| C17orf58_3 | 0.0216 | FKBP10 | 0.1123 | MACC1 | 0.1582 | PNMA5 | 0.172 | TOMM20L | 0.0048 |
| C18orf56 | 0.0081 | FLJ33360 | 0.0243 | MANSCl_1 | 0.128 | PPAPDC1A | 0.0878 | TPM2_2 | 0.155 |
| Clorf168 | 0.0357 | FLJ43752 | 0.2297 | MAPK3_1 | 0.059 | PRAMEF 5 | 0.0026 | TRIM58 | 0.0944 |

(continued)

| | | | | | | | | |
|---|---|---|---|---|---|---|---|---|
| C1orf64 | 0.1059 | FMNL3_2 | 0.0231 | MCAM | 0.1059 | PRKAA2 | 0.1149 | UBR7_1 | 0.0538 |
| C8orf79_1 | 0.0398 | FOSB | 0.1828 | MCART6_1 | 0.2265 | PSMC6_1 | 0.0193 | UBR7_2 | 0.139 |
| CALD1_2 | 0.1445 | FOSL2 | 0.0439 | MFRP | 0.023 | RAD54B_2 | 0.1881 | WARS_2 | 0.1959 |
| CASP8AP2 | 0.126 | FOXN1 | 0.2469 | MIDN | 0.0172 | RAP1A_1 | 0.2007 | XBP1_2 | 0.1609 |
| CCL13 | 0.1388 | GAD1_2 | 0.0292 | MIR1914 | 0.0434 | RARA_3 | 0.0887 | XRN2_1 | 0.043 |
| CCR2_3 | 0.038 | GBE1 | 0.0479 | MIR212 | 0.0923 | RARG | 0.0307 | YARS2 | 0.0284 |
| CD34_1 | 0.0492 | GBP7 | 0.0792 | MIR571 | 0.0389 | RNASEK | 0.1066 | ZNF75D_2 | 0.1318 |
| CDC42BPA_2 | 0.0116 | GJA5_1 | 0.065 | MIR576 | 0.0846 | RNF7_1 | 0.0492 | ZSWIM4_2 | 0.1659 |
| CDC42SE2_2 | 0.0038 | GMNN | 0.1116 | MIR654 | 0.0019 | ROD1_1 | 0.193 | figo_numeric | 0.0217 |
| CIDEC_1 | 0.1085 | GSR_2 | 0.0206 | MIR942 | 0.0906 | SATB2 | 0.0326 | hist rev SBOT | 0.0682 |
| CLDN6 | 0.0179 | GUSBL2 | 0.2016 | MMP12_1 | 0.1295 | SBSN | 0.0699 | surg outcome | 0.003 |
| CREB5_2 | 0.0244 | HBA2 | 0.0675 | MYCN_2 | 0.15 | SCXB | 0.0074 | | |
| CREBBP_1 | 0.0478 | HDAC7_2 | 0.0442 | MYOHD1 | 0.0934 | SEC22C_3 | 0.0918 | | |
| CRYBA1 | 0.0722 | HDLBP_3 | 0.1963 | NFATC3_5 | 0.0162 | SELENBP1 | 0.1492 | | |

Table 25

| | | | | | | | | | |
|---|---|---|---|---|---|---|---|---|---|
| ABCC9_3 | 0.0682 | CYB5R3_2 | 0.1603 | HIC1 | 0.0854 | NFATC4 | 0.0532 | SERPINB2_2 | 0.0165 |
| ABHD3 | 0.2443 | CYP1A2 | 0.0663 | HPRT1_1 | 0.1421 | NLRP9 | 0.1573 | SERPINB5 | 0.1847 |
| ADAM17 2 | 0.1454 | DBNDD2 | 0.1017 | HPS4_1 | 0.029 | NOVA2 | 0.0573 | SFN | 0.0132 |
| ADAMTS1 | 0.0824 | DFFB_2 | 0.0413 | HR 1 | 0.0414 | NP | 0.0799 | SFRS4 | 0.0678 |
| ALS2CL 3 | 0.1078 | DNAH11 | 0.0317 | HSD11B1_1 | 0.104 | NR6A1 2 | 0.1194 | SHC1_3 | 0.0831 |
| ANO7_3 | 0.0537 | DNMT3L_2 | 0.0967 | ICAM2 | 0.0109 | NRXN3 3 | 0.1309 | SLC23A1_2 | 0.0904 |
| ARL6IP1 1 | 0.0393 | DOCK7_1 | 0.0128 | ICAM4_1 | 0.2758 | NT5DC1 2 | 0.1804 | SLC25A34 | 0.0975 |
| ARMCX3_2 | 0.061 | DSC3_1 | 0.0401 | IL1RAP_2 | 0.0583 | NTRK2 3 | 0.0104 | SLC4A5 3 | 0.0945 |
| ATXN10 1 | 0.1742 | DUT_3 | 0.122 | IQCA1_2 | 0.0014 | NUP155 1 | 0.0276 | SLC9A10 | 0.0638 |
| AXL 1 | 0.0704 | EEF1E1_1 | 0.1049 | KCNIP3_1 | 0.0838 | NYX | 0.0582 | SNORD93 | 0.1306 |
| BAIl 3 | 0.0545 | ELN 2 | 0.1082 | KCNQ2_1 | 0.1263 | ODF2 3 | 0.0258 | SOX2 1 | 0.0626 |
| BCAS1 1 | 0.3079 | EMP1 | 0.1789 | KIF3C | 0.182 | ORC1L | 0.0454 | STC1 | 0.0084 |
| BDNF 2 | 0.0952 | ENOI | 0.1426 | KRT80_2 | 0.0691 | OTUD7A 3 | 0.0526 | STC2 | 0.0892 |
| BMPR1A | 0.1185 | ENPEP 2 | 0.0575 | KRTAP10.10_2 | 0.0228 | PANK4 | 0.0511 | STYX 2 | 0.0331 |
| BTF3_3 | 0.1115 | EPHB1 | 0.0434 | L3MBTL2_3 | 0.0495 | PDLIM2 2 | 0.1911 | SYTL3 | 0.0208 |
| C10orf116 | 0.0781 | EPYC | 0.031 | LBH_2 | 0.0787 | PDZRN4 2 | 0.2309 | TAF15 1 | 0.0086 |
| C11orf24 | 0.1297 | ERI2_2 | 0.2677 | LENEP | 0.2331 | PHYH 1 | 0.0191 | TCEAL8 1 | 0.0316 |
| C11orf49_3 | 0.1091 | ESPNL | 0.0833 | LGI3 | 0.1062 | PIGA 1 | 0.0892 | THBS3 | 0.0873 |
| C14orf102_2 | 0.0892 | EZH2_1 | 0.0402 | LOC340508 | 0.042 | PITX2 1 | 0.1958 | TM2D3 2 | 0.0322 |
| C14orf109_2 | 0.1107 | FAM13AOS | 0.0554 | LOC492303 | 0.0288 | PKN1_3 | 0.0308 | TMEM52 | 0.0723 |
| C17orf106 | 0.1527 | FAM187B_2 | 0.0103 | LRRC14B | 0.0692 | PLEKHG5_5 | 0.2591 | TMEM62 | 0.0051 |
| C17orf58_2 | 0.0055 | FAM70A_1 | 0.1018 | LRRC37A4_2 | 0.0079 | PLSCR4 | 0.0174 | TNFRSF18 1 | 0.2355 |
| C17orf58_3 | 0.0287 | FBXO48_2 | 0.1877 | LRRTM4 | 0.1633 | PMEPA1 4 | 0.1368 | TNNT2 1 | 0.0045 |
| C18orf56 | 0.0055 | FKBP10 | 0.1051 | MACC1 | 0.1624 | PNMA5 | 0.1731 | TOMM20L | 0.0044 |
| C1orfl68 | 0.0317 | FLJ33360 | 0.0249 | MANSC1_1 | 0.1213 | PPAPDC1A | 0.093 | TPM2 2 | 0.1559 |
| C1orf64 | 0.1038 | FLJ43752 | 0.2266 | MAPK3_1 | 0.0602 | PRAMEF5 | 0.0086 | TRIM58 | 0.1018 |

| | | | | | | | | | |
|---|---|---|---|---|---|---|---|---|---|
| C8orf79_1 | 0.0412 | FMNL3 2 | 0.0365 | MCAM | 0.103 | PRKAA2 | 0.1125 | UBR7 1 | 0.0572 |
| CALD1 2 | 0.1514 | FOSB | 0.1925 | MCART6_1 | 0.2245 | PSMC6_1 | 0.018 | UBR7 2 | 0.1508 |
| CASP8AP2 | 0.1197 | FOSL2 | 0.0394 | MFRP | 0.0236 | RAD54B 2 | 0.1885 | WARS 2 | 0.1977 |
| CCL13 | 0.1514 | FOXN1 | 0.2509 | MIDN | 0.0246 | RAP1A _ 1 | 0.1957 | XBP1 2 | 0.161 |
| CCR2 3 | 0.0338 | GAD1_2 | 0.0272 | MIR1914 | 0.0441 | RARA 3 | 0.0886 | XRN2 1 | 0.026 |
| CD34 1 | 0.0492 | GBE1 | 0.0517 | MIR212 | 0.0936 | RARG | 0.0401 | YARS2 | 0.0281 |
| CDC42BPA 2 | 0.0003 | GBP7 | 0.0794 | MIR571 | 0.0381 | RNASEK | 0.1013 | ZNF75D 2 | 0.1315 |
| CDC42SE2 2 | 0 | GJA5_1 | 0.0623 | MIR576 | 0.0926 | RNF7_1 | 0.0468 | ZSWIM4 2 | 0.1654 |
| CIDEC 1 | 0.1061 | GMNN | 0.1058 | MIR654 | 0.0013 | ROD1 1 | 0.1929 | figo_numeric | 0.0208 |
| CLDN6 | 0.0199 | GSR_2 | 0.0111 | MIR942 | 0.0829 | SATB2 | 0.0271 | hist rev SBOT | 0.0748 |
| CREB5_2 | 0.0184 | GUSBL2 | 0.193 | MMP12_1 | 0.132 | SBSN | 0.0761 | surg outcome | 0.0014 |
| CREBBP 1 | 0.0514 | HBA2 | 0.069 | MYCN 2 | 0.1408 | SCXB | 0.0089 | | |
| CRYBA1 | 0.0675 | HDAC7 2 | 0.0304 | MYOHD1 | 0.0938 | SEC22C 3 | 0.0921 | | |
| CXCL13 | 0.1712 | HDLBP_3 | 0.1922 | NFATC3 _ 5 | 0.0259 | SELENBP1 | 0.1486 | | |

Table 26

| ABCC9_3 | 0.0476 | CXCL 13 | 0.1697 | HDLBP_3 | 0.2006 | NFATC3_5 | 0.0407 | SERPINB2_2 | 0.017 |
|---|---|---|---|---|---|---|---|---|---|
| ABHD3 | 0.2469 | CYB5R3 2 | 0.1687 | HIC1 | 0.0848 | NFATC4 | 0.0513 | SERPINB5 | 0.203 |
| ADAM17 2 | 0.16 | CYP1A2 | 0.0699 | HPRT1 1 | 0.1553 | NLRP9 | 0.1502 | SFN | 0.0329 |
| ADAMTS1 | 0.0982 | DBNDD2 | 0.084 | HPS4 1 | 0.0392 | NOV A2 | 0.085 | SFRS4 | 0.0619 |
| ADAMTS2 1 | 0.1272 | DFFB 2 | 0.037 | HR 1 | 0.0504 | NP | 0.0834 | SHC1_3 | 0.0753 |
| ALS2CL 3 | 0.05 | DNAH11 | 0.0235 | HSD11B1 1 | 0.0967 | NR6A1_2 | 0.1261 | SLC23A1_2 | 0.1103 |
| ANO7 3 | 0.0392 | DNMT3L_2 | 0.1057 | ICAM2 | 0.0054 | NRXN3_3 | 0.0891 | SLC25A34 | 0.0851 |
| ARL6IP1_1 | 0.0192 | DOCK7_1 | 0.0147 | ICAM4 1 | 0.2676 | NT5DC1_2 | 0.1823 | SLC4A5 3 | 0.083 |
| ARMCX3 2 | 0.0755 | DSC3 1 | 0.0535 | IL1RAP 2 | 0.0356 | NTRK2_3 | 0.0252 | SLC9A10 | 0.0945 |
| ATXN10 1 | 0.1707 | DUT 3 | 0.1181 | IQCA1_2 | 0.0114 | NUP155_1 | 0.0146 | SNORD93 | 0.1705 |
| AXL 1 | 0.0883 | EEF1E1_1 | 0.0877 | KCNIP3_1 | 0.0805 | NYX | 0.0789 | SOX2 1 | 0.0489 |
| BAII 3 | 0.0608 | ELN_2 | 0.1041 | KCNQ2_1 | 0.1399 | ODF2 3 | 0.0283 | STC1 | 0.001 |
| BCAS1 1 | 0.3288 | EMP1 | 0.1731 | KIF3C | 0.2155 | ORC1L | 0.0571 | STC2 | 0.0976 |
| BDNF 2 | 0.104 | ENOI | 0.1271 | KRT80 2 | 0.0639 | OTUD7A 3 | 0.045 | STYX 2 | 0.0549 |
| BMPR1A | 0.1257 | ENPEP_2 | 0.0578 | KRTAP10.10_2 | 0.0151 | PANK4 | 0.0423 | SYTL3 | 0.003 |
| BTF3 3 | 0.1173 | EPHB1 | 0.0574 | L3MBTL2_3 | 0.0464 | PDLIM2_2 | 0.2005 | TAF15 1 | 0.0041 |
| C10orfII6 | 0.044 | EPYC | 0.0271 | LBH_2 | 0.0991 | PHYH 1 | 0.2122 | TCEAL8 1 | 0.0288 |
| C11orf24 | 0.1453 | ERI2 2 | 0.2777 | LENEP | 0.2429 | PIGA 1 | 0.012 | THBS3 | 0.0823 |
| C11orf49_3 | 0.1311 | ESPNL | 0.0816 | LGI3 | 0.1157 | PITX2 1 | 0.0764 | TM2D3_2 | 0.0461 |
| C14orf102_2 | 0.0888 | EZH2_1 | 0.0374 | LOC340508 | 0.0435 | PKN1_3 | 0.0519 | TMEM52 | 0.0834 |
| C14orf109_2 | 0.0692 | FAM13AOS | 0.0287 | LOC492303 | 0.0199 | PLEKHG5_5 | 0.2777 | TMEM62 | 0.0011 |
| C17orf106 | 0.1665 | FAM187B_2 | 0.0124 | LRRC14B | 0.0696 | PLSCR4 | 0.0333 | TNFRSF18_1 | 0.2512 |
| C17orf58_2 | 0.01 | FAM70A_1 | 0.0974 | LRRC37A4 2 | 0.0045 | PMEPA1 4 | 0.1482 | TNNT2 1 | 0.0037 |
| C17orf58 3 | 0.0344 | FBXO48_2 | 0.1976 | LRRTM4 | 0.1548 | PNMA5 | 0.1554 | TOMM20L | 0.0464 |
| C18orf56 | 0.0318 | FKBP10 | 0.0997 | MACC1 | 0.1409 | PPAPDC1A | 0.1215 | TPM2 2 | 0.1557 |
| Clorf168 | 0.0381 | FLJ33360 | 0.0363 | MANSCI 1 | 0.1432 | PRAMEF 5 | 0.0287 | TRIM58 | 0.106 |

56

| | | | | | | | |
|---|---|---|---|---|---|---|---|
| C1orf64 | 0.1247 | FLJ43752 | 0.2224 | MAPK3 1 | 0.0687 | PRKAA2 | 0.1182 | UBR7 1 | 0.0139 |
| C8orf79_1 | 0.0568 | FMNL3_2 | 0.0145 | MCAM | 0.1114 | PSMC6_1 | 0.0133 | UBR7 2 | 0.1407 |
| CALD1_2 | 0.1613 | FOSB | 0.1895 | MCART6 1 | 0.2171 | RAD54B 2 | 0.1973 | WARS 2 | 0.1709 |
| CASP8AP2 | 0.1143 | FOSL2 | 0.0201 | MFRP | 0.0204 | RAP1A_1 | 0.2038 | XBP1 2 | 0.1367 |
| CCL 13 | 0.1617 | FOXN1 | 0.2817 | MIDN | 0.0342 | RARA 3 | 0.0831 | XRN2 1 | 0.0079 |
| CCR2 3 | 0.0119 | GAD1 2 | 0.0171 | MIR1914 | 0.0392 | RARG | 0.0136 | YARS2 | 0.0026 |
| CD34 1 | 0.0599 | GBE1 | 0.0639 | MIR212 | 0.0991 | RNASEK | 0.0596 | ZNF75D 2 | 0.1368 |
| CDC42BPA 2 | 0.0156 | GBP7 | 0.1032 | MIR571 | 0.0311 | RNF7 1 | 0.066 | ZSWIM4 2 | 0.1669 |
| CDC42SE2 2 | 0.017 | GJA5 1 | 0.051 | MIR576 | 0.0854 | ROD1_1 | 0.2187 | figo_numeric | 0.0267 |
| CIDEC_1 | 0.1153 | GMNN | 0.0776 | MIR654 | 0.0168 | SATB2 | 0.0385 | hist rev SBOT | 0.0627 |
| CLDN6 | 0.0052 | GSR 2 | 0.0245 | MIR942 | 0.0906 | SBSN | 0.0849 | surg outcome | 0.0132 |
| CREB5 2 | 0.0516 | GUSBL2 | 0.188 | MMP12 1 | 0.1239 | SCXB | 0.0097 | | |
| CREBBP 1 | 0.0369 | HBA2 | 0.0817 | MYCN 2 | 0.1542 | SEC22C_3 | 0.0968 | | |
| CRYBA1 | 0.0801 | HDAC7_2 | 0.0295 | MYOHD1 | 0.0972 | SELENBP1 | 0.174 | | |

Table 27

| | | | | | | | | | |
|---|---|---|---|---|---|---|---|---|---|
| ABCC9_3 | 0.065 | CYB5R3_2 | 0.1798 | HIC1 | 0.1045 | NFATC4 | 0.058 | SERPINB2_2 | 0.0345 |
| ABHD3 | 0.2364 | CYP1A2 | 0.0773 | HPRT1_1 | 0.1451 | NLRP9 | 0.1587 | SERPINB5 | 0.1967 |
| ADAM17_2 | 0.1517 | DBNDD2 | 0.0986 | HPS4_1 | 0.004 | NOVA2 | 0.0556 | SFN | 0.0191 |
| ADAMTS1 | 0.1015 | DFFB_2 | 0.0369 | HR_1 | 0.045 | NP | 0.0842 | SFRS4 | 0.061 |
| ADAMTS2_1 | 0.111 | DNAH11 | 0.0356 | HSD11B1_1 | 0.1073 | NR6A1_2 | 0.1202 | SHC1_3 | 0.089 |
| ALS2CL_3 | 0.0631 | DNMT3L_2 | 0.113 | ICAM2 | 0.0219 | NRXN3_3 | 0.1317 | SLC23A1_2 | 0.0882 |
| ANO7_3 | 0.0177 | DOCK7_1 | 0.0058 | ICAM4_1 | 0.2635 | NT5DC1_2 | 0.1844 | SLC25A34 | 0.0937 |
| ARL6IP1_1 | 0.0002 | DSC3_1 | 0.0561 | IL1RAP_2 | 0.0726 | NTRK2_3 | 0.0283 | SLC4A5_3 | 0.0897 |
| ARMCX3_2 | 0.0492 | DUT_3 | 0.1277 | IQCA1_2 | 0.0176 | NUP155_1 | 0.0382 | SLC9A10 | 0.0675 |
| ATXN10_1 | 0.1864 | EEF1E1_1 | 0.1034 | KCNIP3_1 | 0.0945 | NYX | 0.0625 | SNORD93 | 0.1369 |
| AXL_1 | 0.0812 | ELN_2 | 0.109 | KCNQ2_1 | 0.1335 | ODF2_3 | 0.0315 | SOX2_1 | 0.0599 |
| BAI1_3 | 0.0399 | EMP1 | 0.1754 | KIF3C | 0.193 | ORC1L | 0.0513 | STC1 | 0.0115 |
| BCAS1_1 | 0.2986 | ENO1 | 0.1403 | KRT80_2 | 0.0765 | OTUD7A_3 | 0.073 | STC2 | 0.0823 |
| BDNF_2 | 0.0907 | ENPEP_2 | 0.0449 | KRTAP10.10_2 | 0.0138 | PANK4 | 0.0475 | STYX_2 | 0.0391 |
| BMPR1A | 0.1242 | EPHB1 | 0.0394 | L3MBTL2 3 | 0.0427 | PDLIM2_2 | 0.1872 | SYTL3 | 0.0069 |
| BTF3_3 | 0.11 | EPYC | 0.0314 | LBH_2 | 0.0826 | PDZRN4_2 | 0.2358 | TAF15_1 | 0.0071 |
| C10orf116 | 0.0759 | ERI2_2 | 0.2791 | LENEP | 0.2258 | PHYH_1 | 0.0063 | TCEAL8_1 | 0.0398 |
| C11orf24 | 0.1217 | ESPNL | 0.0955 | LGI3 | 0.1079 | PIGA_1 | 0.1012 | THBS3 | 0.0768 |
| C11orf49_3 | 0.1088 | EZH2_1 | 0.0336 | LOC340508 | 0.0632 | PITX2_1 | 0.1804 | TM2D3_2 | 0.0367 |
| C14orf102_2 | 0.0804 | FAM13AOS | 0.0556 | LOC492303 | 0.0294 | PKN1_3 | 0.0399 | TMEM52 | 0.0746 |
| C14orf109_2 | 0.1262 | FAM187B_2 | 0.0291 | LRRC14B | 0.0808 | PLEKHG5_5 | 0.2662 | TMEM62 | 0.0034 |
| C17orf106 | 0.1575 | FAM70A_1 | 0.094 | LRRC37A4_2 | 0.0079 | PLSCR4 | 0.027 | TNFRSF18_1 | 0.2372 |
| C17orf58_2 | 0.0313 | FBXO48_2 | 0.1923 | LRRTM4 | 0.181 | PMEPA1_4 | 0.1375 | TNNT2_1 | 0.0008 |
| C17orf58_3 | 0.0388 | FKBP10 | 0.1219 | MACC1 | 0.1689 | PNMA5 | 0.1794 | TOMM20L | 0.0068 |
| C18orf56 | 0.0067 | FLJ33360 | 0.0077 | MANSC1_1 | 0.1203 | PPAPDC1A | 0.0921 | TPM2_2 | 0.1513 |
| C1orf168 | 0.0427 | FLJ43752 | 0.2354 | MAPK3_1 | 0.0447 | PRAMEF_5 | 0.003 | TRIM58 | 0.102 |

| | | | | | | | |
|---|---|---|---|---|---|---|---|
| C1orf64 | 0.1084 | FMNL3_2 | 0.0352 | MCAM | 0.1012 | PRKAA2 | 0.0835 | UBR7_1 | 0.0338 |
| C8orf79_1 | 0.0602 | FOSB | 0.2097 | MCART6_1 | 0.2168 | PSMC6_1 | 0.001 | UBR7_2 | 0.1467 |
| CALD1_2 | 0.1315 | FOSL2 | 0.0224 | MFRP | 0.0342 | RAD54B_2 | 0.1935 | WARS_2 | 0.1962 |
| CASP8AP2 | 0.1172 | FOXN1 | 0.2375 | MIDN | 0.0277 | RAP1A_1 | 0.208 | XBP1_2 | 0.1619 |
| CCL13 | 0.1255 | GAD1_2 | 0.0205 | MIR1914 | 0.0621 | RARA_3 | 0.0748 | XRN2_1 | 0.0064 |
| CCR2_3 | 0.0423 | GBE1 | 0.07 | MIR212 | 0.0887 | RARG | 0.0289 | YARS2 | 0.0057 |
| CD34_1 | 0.0422 | GBP7 | 0.0943 | MIR571 | 0.0229 | RNASEK | 0.098 | ZNF75D_2 | 0.1134 |
| CDC42BPA_2 | 0.015 | GJA5_1 | 0.0504 | MIR576 | 0.0855 | RNF7_1 | 0.0311 | ZSWIM4_2 | 0.1535 |
| CDC42SE2_2 | 0.0232 | GMNN | 0.0833 | MIR654 | 0.0092 | ROD1_1 | 0.2203 | figo_numeric | 0.0079 |
| CLDN6 | 0.1183 | GSR_2 | 0.0126 | MIR942 | 0.0891 | SATB2 | 0.0192 | hist_rev_SBOT | 0.0662 |
| CREB5_2 | 0.0239 | GUSBL2 | 0.2013 | MMP12_1 | 0.1221 | SBSN | 0.0578 | surg_outcome | 0.0034 |
| CREBBP_1 | 0.0347 | HBA2 | 0.0841 | MYCN_2 | 0.1217 | SCXB | 0.012 | | |
| CRYBA1 | 0.0762 | HDAC7_2 | 0.023 | MYOHD1 | 0.0882 | SEC22C_3 | 0.0927 | | |
| CXCL13 | 0.1625 | HDLBP_3 | 0.1929 | NFATC3_5 | 0.0152 | SELENBP1 | 0.137 | | |

Table 28

| | | | | | | | | | |
|---|---|---|---|---|---|---|---|---|---|
| ABCC9_3 | 0.0685 | CXCL13 | 0.1724 | HDLBP_3 | 0.1918 | NFATC4 | 0.0531 | SERPINB2_2 | 0.0152 |
| ABHD3 | 0.244 | CYB5R3 2 | 0.16 | HIC1 | 0.0858 | NLRP9 | 0.1566 | SERPINB5 | 0.1862 |
| ADAM17 2 | 0.1456 | CYP1A2 | 0.0667 | HPRT1 1 | 0.1429 | NOV A2 | 0.0572 | SFN | 0.014 |
| ADAMTS1 | 0.0804 | DBNDD2 | 0.1008 | HPS4 1 | 0.0275 | NP | 0.0798 | SFRS4 | 0.0682 |
| ADAMTS2_1 | 0.1088 | DFFB 2 | 0.0414 | HR 1 | 0.0396 | NR6A1 2 | 0.1202 | SHC1_3 | 0.0832 |
| ALS2CL 3 | 0.0534 | DNAH11 | 0.0309 | HSD11B1 1 | 0.1048 | NRXN3_3 | 0.1303 | SLC23A1_2 | 0.0905 |
| ANO7_3 | 0.0387 | DNMT3L_2 | 0.0979 | ICAM2 | 0.0101 | NT5DC1_2 | 0.1811 | SLC25A34 | 0.097 |
| ARL6IP1_1 | 0.0062 | DOCK7_1 | 0.0132 | ICAM4 1 | 0.2764 | NTRK2_3 | 0.0106 | SLC4A5 3 | 0.0945 |
| ARMCX3 2 | 0.0603 | DSC3 1 | 0.0382 | IL1RAP 2 | 0.0589 | NUP155_1 | 0.0284 | SLC9A10 | 0.0638 |
| ATXN10 1 | 0.1744 | DUT 3 | 0.1216 | IQCA1_2 | 0.0019 | NYX | 0.0589 | SNORD93 | 0.1296 |
| AXL 1 | 0.0709 | EEF1E1_1 | 0.1052 | KCNIP3 1 | 0.0836 | ODF2 3 | 0.0259 | SOX2 1 | 0.0626 |
| BAII 3 | 0.0546 | ELN_2 | 0.1082 | KCNQ2 _ 1 | 0.1263 | ORC1L | 0.0456 | STC1 | 0.0083 |
| BCAS1 1 | 0.307 | EMP1 | 0.1791 | KIF3C | 0.1822 | OTUD7A 3 | 0.0528 | STC2 | 0.0902 |
| BDNF 2 | 0.0947 | ENOI | 0.1418 | KRT80 2 | 0.0699 | PANK4 | 0.0518 | STYX 2 | 0.0325 |
| BMPR1A | 0.1185 | ENPEP_2 | 0.0594 | KRTAP10.10_2 | 0.0235 | PDLIM2_2 | 0.1921 | SYTL3 | 0.0211 |
| BTF3 3 | 0.1107 | EPHB1 | 0.0427 | L3MBTL2_3 | 0.0499 | PDZRN4 2 | 0.2307 | TAF15 1 | 0.0091 |
| C10orfll6 | 0.0779 | EPYC | 0.0308 | LBH 2 | 0.0784 | PHYH 1 | 0.0186 | TCEAL8 1 | 0.0323 |
| C11orf24 | 0.1292 | ERI2 2 | 0.2675 | LENEP | 0.2324 | PIGA 1 | 0.0892 | THBS3 | 0.0868 |
| C11orf49_3 | 0.1097 | ESPNL | 0.0834 | LGI3 | 0.1069 | PITX2 1 | 0.1948 | TM2D3 2 | 0.0321 |
| C14orf102_2 | 0.0891 | EZH2_1 | 0.0414 | LOC492303 | 0.0413 | PKN1_3 | 0.0313 | TMEM52 | 0.0706 |
| C14orf109_2 | 0.11 | FAM13AOS | 0.055 | LRRC14B | 0.0286 | PLEKHG5_5 | 0.2595 | TMEM62 | 0.0054 |
| C17orf106 | 0.1543 | FAM187B_2 | 0.0098 | LRRC37A4 2 | 0.069 | PLSCR4 | 0.0171 | TNFRSF18 1 | 0.2353 |
| C17orf58_2 | 0.0053 | FAM70A_1 | 0.1018 | LRRTM4 | 0.1636 | PMEPA1_4 | 0.1383 | TNNT2 1 | 0.005 |
| C17orf58 3 | 0.028 | FBXO48_2 | 0.1878 | MACC1 | 0.1621 | PNMA5 | 0.1722 | TOMM20L | 0.0051 |
| C18orf56 | 0.0048 | FKBP10 | 0.1057 | MANSCI 1 | 0.1209 | PPAPDC1A | 0.093 | TPM2 2 | 0.1559 |
| Clorf168 | 0.0315 | FLJ33360 | 0.0249 | MAPK3 1 | 0.0616 | PRAMEF 5 | 0.0075 | TRIM58 | 0.1018 |

(continued)

| | | | | | | | | |
|---|---|---|---|---|---|---|---|---|
| Clorf64 | 0.1037 | FLJ43752 | 0.226 | MCAM | 0.1033 | PRKAA2 | 0.1133 | UBR7 1 | 0.0569 |
| C8orf79_1 | 0.042 | FMNL3_2 | 0.0365 | MCART6 1 | 0.2257 | PSMC6_1 | 0.0177 | UBR7 2 | 0.1509 |
| CALD1_2 | 0.1513 | FOSB | 0.1933 | MFRP | 0.0231 | RAD54B 2 | 0.1882 | WARS 2 | 0.197 |
| CASP8AP2 | 0.1192 | FOSL2 | 0.0384 | MIDN | 0.0249 | RAP1A_1 | 0.194 | XBP1 2 | 0.1612 |
| CCL 13 | 0.151 | FOXN1 | 0.2511 | MIR1914 | 0.0424 | RARA 3 | 0.0881 | XRN2 1 | 0.0263 |
| CCR2 3 | 0.034 | GAD1 2 | 0.0273 | MIR212 | 0.0931 | RARG | 0.0404 | YARS2 | 0.0284 |
| CD34 1 | 0.0494 | GBE1 | 0.0526 | MIR571 | 0.0375 | RNASEK | 0.1022 | ZNF75D 2 | 0.1315 |
| CDC42BPA 2 | 0.0004 | GBP7 | 0.0796 | MIR576 | 0.0931 | RNF7 1 | 0.0459 | ZSWIM4 2 | 0.1654 |
| CDC42SE2 2 | 0.0005 | GJA5 1 | 0.0627 | MIR654 | 0.0012 | ROD1_1 | 0.1934 | figo_numeric | 0.0217 |
| CIDEC_1 | 0.1068 | GMNN | 0.106 | MIR942 | 0.0823 | SATB2 | 0.0276 | hist rev SBOT | 0.0745 |
| CLDN6 | 0.0201 | GSR 2 | 0.0097 | MMP12 1 | 0.1315 | SBSN | 0.0758 | surg outcome | 0.0002 |
| CREB5 2 | 0.0193 | GUSBL2 | 0.1927 | MYCN 2 | 0.1405 | SCXB | 0.009 | | |
| CREBBP 1 | 0.0516 | HBA2 | 0.0699 | MYOHD1 | 0.0938 | SEC22C_3 | 0.0927 | | |
| CRYBA1 | 0.0675 | HDAC7_2 | 0.0315 | NFATC3 5 | 0.0265 | SELENBP1 | 0.1487 | | |

EP 3 874 274 B1

Table 29

| Gene | Value | Gene | Value | Gene | Value | Gene | Value | Gene | Value |
|---|---|---|---|---|---|---|---|---|---|
| ABHD3 | 0.0618 | DBNDD2 | 0.1261 | HR_1 | 0.0199 | NP | 0.0742 | SFRS4 | 0.0628 |
| ADAM17_2 | 0.2475 | DNAH11 | 0.0454 | HSD11B1_1 | 0.0988 | NR6A1_2 | 0.1413 | SHC1_3 | 0.0513 |
| ADAMTS_1 | 0.1461 | DNMT3L_2 | 0.0123 | ICAM2 | 0.0189 | NRXN3_3 | 0.1729 | SLC23A1_2 | 0.1159 |
| ADAMTS2_1 | 0.0871 | DOCK7_1 | 0.1005 | ICAM4_1 | 0.3077 | NT5DC1_2 | 0.1804 | SLC25A34 | 0.1291 |
| ALS2CL_3 | 0.077 | DSC3_1 | 0.0364 | IL1RAP_2 | 0.0827 | NTRK2_3 | 0.0071 | SLC4A5_3 | 0.0937 |
| ANO7_3 | 0.0212 | DUT_3 | 0.1169 | IQCA1_2 | 0.014 | NUP155_1 | 0.0366 | SLC9A10 | 0.0669 |
| ARL6IP1_1 | 0.0217 | EEF1E1_1 | 0.1311 | KCNIP3_1 | 0.0954 | NYX | 0.1525 | SNORD93 | 0.134 |
| ARMCX3_2 | 0.0673 | ELN_2 | 0.1234 | KCNQ2_1 | 0.1123 | ODF2_3 | 0.0055 | SOX2_1 | 0.0735 |
| ATXN10_1 | 0.2132 | EMP1 | 0.2053 | KIF3C | 0.1782 | ORC1L | 0.0279 | STC1 | 0.0015 |
| AXL_1 | 0.095 | ENOI | 0.1684 | KRT80_2 | 0.0941 | OTUD7A_3 | 0.0312 | STC2 | 0.1212 |
| BAI1_3 | 0.0392 | ENPEP_2 | 0.0695 | KRTAP10.10_2 | 0.0339 | PANK4 | 0.0578 | STYX_2 | 0.0093 |
| BCAS1_1 | 0.3166 | EPHB1 | 0.0221 | L3MBTL2_3 | 0.0422 | PDLIM2_2 | 0.2134 | SYTL3 | 0.0182 |
| BDNF_2 | 0.1039 | EPYC | 0.0518 | LBH_2 | 0.0695 | PDZRN4_2 | 0.1932 | TAF15_1 | 0.0303 |
| BMPR1A | 0.1113 | ERI2_2 | 0.281 | LENEP | 0.2316 | PHYH_1 | 0.0049 | TCEAL8 1 | 0.0055 |
| BTF3_3 | 0.099 | ESPNL | 0.0508 | LGI3 | 0.0948 | PIGA_1 | 0.0808 | THBS3 | 0.0788 |
| C10orf116 | 0.0686 | EZH2_1 | 0.0486 | LOC340508 | 0.0133 | PITX2_1 | 0.2057 | THY1 | 0.0272 |
| C11orf24 | 0.1691 | FAM13AOS | 0.0603 | LOC492303 | 0.037 | PKN1_3 | 0.0038 | TIMP2_2 | 0.0904 |
| C11orf49_3 | 0.1217 | FAM187B_2 | 0.0061 | LRRC14B | 0.072 | PLEKHG5_5 | 0.2623 | TM2D3_2 | 0.0107 |
| C14orf102_2 | 0.1211 | FAM70A_1 | 0.0744 | LRRC37A4_2 | 0.0148 | PLSCR4 | 0.0168 | TMEM52 | 0.0317 |
| C14orf109_2 | 0.1057 | FBXO48_2 | 0.2395 | LRRTM4 | 0.1616 | PMEPA1_4 | 0.1561 | TMEM62 | 0.0753 |
| C17orf106 | 0.1712 | FKBP10 | 0.0433 | MACC1 | 0.1462 | PNMA5 | 0.1577 | TNFRSF18_1 | 0.2291 |
| C17orf58_2 | 0.0212 | FLJ33360 | 0.0163 | MANSC1_1 | 0.1217 | PPAPDC1A | 0.1222 | TNNT2_1 | 0.0027 |
| C17orf58_3 | 0.0262 | FLJ43752 | 0.2253 | MCAM | 0.0331 | PRAMEF 5 | 0.0044 | TOMM20L | 0.0123 |
| C18orf56 | 0.0087 | FMNL3_2 | 0.0011 | MCART6_1 | 0.114 | PRKAA2 | 0.1197 | TPM2_2 | 0.1782 |
| C1orf168 | 0.0234 | FOSB | 0.2168 | MFRP | 0.2341 | PSMC6_1 | 0.0273 | TRIM58 | 0.1209 |
| C1orf64 | 0.1021 | FOSL2 | 0.0488 | MIDN | 0.0273 | RAD54B_2 | 0.1907 | UBR7_1 | 0.0869 |

(continued)

| | | | | | | | | | |
|---|---|---|---|---|---|---|---|---|---|
| C8orf79_1 | 0.005 | FOXN1 | 0.2391 | MIR1914 | 0.0737 | RAP1A_1 | 0.1828 | UBR7_2 | 0.1318 |
| CASP8AP2 | 0.1346 | GAD1_2 | 0.0218 | MIR212 | 0.105 | RARA_3 | 0.0998 | WARS_2 | 0.1787 |
| CCL13 | 0.1363 | GBE1 | 0.0402 | MIR571 | 0.0079 | RARG | 0.065 | XBP1_2 | 0.1588 |
| CCR2_3 | 0.1265 | GBP7 | 0.1302 | MIR576 | 0.1016 | RNASEK | 0.0781 | XRN2_1 | 0.0623 |
| CD34_1 | 0.012 | GJA5_1 | 0.0633 | MIR654 | 0.0606 | RNF7_1 | 0.0041 | YARS2 | 0.0364 |
| CDC42BPA_2 | 0.0006 | GMNN | 0.1023 | MIR942 | 0.1115 | ROD1_1 | 0.1907 | ZCCHC24 | 0.1336 |
| CDC42SE2_2 | 0.0196 | GSR_2 | 0.019 | MMP12_1 | 0.114 | SATB2 | 0.0351 | ZNF75D_2 | 0.178 |
| CIDEC_1 | 0.0995 | HBA2 | 0.2143 | MYCN_2 | 0.1289 | SBSN | 0.102 | ZSWIM4_2 | 0.005 |
| CLDN6 | 0.0116 | HCFC1R1_1 | 0.0428 | MYL9_2 | 0.1078 | SCXB | 0.0184 | figo_numeric | 0.042 |
| CREB5_2 | 0.0031 | HDAC7_2 | 0.003 | MYOHD1 | 0.0231 | SEC22C_3 | 0.1137 | hist_rev_SBOT | 0.0462 |
| CRYBA1 | 0.0607 | HDLBP_3 | 0.0974 | NFATC3_5 | 0.0414 | SELENBP1 | 0.1525 | surg_outcome | 0.0032 |
| CXCL13 | 0.0615 | HIC1 | 0.0161 | NFATC4 | 0.0648 | SERPINB2_2 | 0.0294 | | |
| CYB5R3_2 | 0.1912 | HPRT1_1 | 0.1425 | NLRP9 | 0.1888 | SERPINB5 | 0.1806 | | |
| CYP1A2 | 0.0598 | HPS4_1 | 0.0712 | NOV A2 | 0.0538 | SFN | 0.0045 | | |

Table 30

| Gene | Value | Gene | Value | Gene | Value | Gene | Value | Gene | Value |
|---|---|---|---|---|---|---|---|---|---|
| ABHD3 | 0.0616 | CYB5R3_2 | 0.1925 | HPRT1_1 | 0.1315 | NLRP9 | 0.1998 | SERPINB2_2 | 0.0096 |
| ADAM17_2 | 0.2471 | CYP1A2 | 0.0545 | HPS4 1 | 0.0742 | NOVA2 | 0.0714 | SERPINBS | 0.1899 |
| ADAMTS1 | 0.1489 | DBNDD2 | 0.1222 | HR_1 | 0.0307 | NP | 0.084 | SFN | 0.0083 |
| ADAMTS2 1 | 0.0826 | DNAH11 | 0.043 | HSD11B1_1 | 0.0998 | NR6A1 2 | 0.1442 | SFRS4 | 0.0566 |
| ALS2CL 3 | 0.0755 | DNMT3L_2 | 0.0228 | ICAM2 | 0.0132 | NRXN3 3 | 0.1708 | SHC1_3 | 0.0563 |
| ANO7_3 | 0.0368 | DOCK7 1 | 0.1114 | ICAM4 1 | 0.2908 | NT5DC1 2 | 0.1851 | SLC23A1_2 | 0.1265 |
| ARL6IP1 1 | 0.0047 | DSC3 1 | 0.05 | IL1RAP 2 | 0.0712 | NTRK2 3 | 0.0054 | SLC25A34 | 0.1342 |
| ARMCX3_2 | 0.075 | DUT 3 | 0.0994 | IQCA1_2 | 0.0221 | NUP155 1 | 0.0373 | SLC4A5 _3 | 0.0946 |
| ATXN10 1 | 0.2066 | EEF1E1 1 | 0.1284 | KCNIP3_1 | 0.102 | NYX | 0.1587 | SLC9A10 | 0.0674 |
| AXL 1 | 0.0994 | EMP1 | 0.1304 | KCNQ2_1 | 0.1221 | ODF2 3 | 0.0093 | SNORD93 | 0.1338 |
| BAII 3 | 0.0299 | ENOI | 0.207 | KIF3C | 0.158 | ORC1L | 0.0107 | SOX2 1 | 0.0749 |
| BCAS1 1 | 0.3377 | ENPEP 2 | 0.1684 | KRT80 2 | 0.1047 | OTUD7A 3 | 0.0394 | STC1 | 0.0153 |
| BDNF 2 | 0.1184 | EPHB1 | 0.0222 | KRTAP10.10_2 | 0.0351 | PANK4 | 0.0564 | STC2 | 0.1306 |
| BMPR1A | 0.1141 | EPYC | 0.0453 | L3MBTL2_3 | 0.0462 | PDLIM2 2 | 0.2098 | STYX 2 | 0.0142 |
| BTF3_3 | 0.1065 | ERI2 2 | 0.2904 | LBH_2 | 0.0773 | PDZRN4 2 | 0.205 | SYTL3 | 0.0214 |
| C10orf116 | 0.0741 | ESPNL | 0.0471 | LENEP | 0.2262 | PHYH 1 | 0.0038 | TAF15 1 | 0.0329 |
| C11orf24 | 0.1923 | EZH2 1 | 0.0561 | LGI3 | 0.0872 | PIGA 1 | 0.0836 | TCEAL8 1 | 0.012 |
| C11orf49_3 | 0.107 | FAM13AOS | 0.066 | LOC340508 | 0.0228 | PITX2 1 | 0.216 | THBS3 | 0.0896 |
| C14orf102_2 | 0.1262 | FAM187B_2 | 0.0127 | LOC492303 | 0.04 | PKN1 3 | 0.0099 | TM2D3 2 | 0.0347 |
| C14orf109_2 | 0.1112 | FAM70A_1 | 0.0735 | LRRC14B | 0.077 | PLEKHG5_5 | 0.2613 | TMEM52 | 0.0974 |
| C17orf106 | 0.1828 | FBXO48_2 | 0.2406 | LRRC37A4_2 | 0.0128 | PLSCR4 | 0.0138 | TMEM62 | 0.0064 |
| C17orf58_2 | 0.0177 | FKBP10 | 0.0634 | LRRTM4 | 0.1688 | PMEPA1 4 | 0.1447 | TNFRSF18_1 | 0.23 |
| C17orf58_3 | 0.0241 | FLJ33360 | 0.0159 | MACC1 | 0.1328 | PNMA5 | 0.1631 | TNNT2 1 | 0.0087 |
| C18orf56 | 0.0112 | FLJ43752 | 0.2325 | MANSC1_1 | 0.1301 | PPAPDC1A | 0.1032 | TOMM20L | 0.0148 |
| C1orf168 | 0.0283 | FMNL3 2 | 0.0124 | MCAM | 0.0322 | PRAMEF5 | 0.0098 | TPM2 2 | 0.1766 |
| C1orf64 | 0.1091 | FOSB | 0.2212 | MCART6_1 | 0.1191 | PRKAA2 | 0.1284 | TRIM58 | 0.1201 |

| | | | | | | | |
|---|---|---|---|---|---|---|---|
| C8orf79_1 | 0.0087 | FOSL2 | 0.0487 | MFRP | 0.2311 | PSMC6_1 | 0.0248 | UBR7 1 | 0.0894 |
| CALD1 2 | 0.1208 | FOXN1 | 0.2383 | MIDN | 0.0232 | RAD54B _ 2 | 0.1832 | UBR7 2 | 0.1281 |
| CASP8AP2 | 0.1425 | GAD1 2 | 0.0286 | MIR1914 | 0.0637 | RAP1A 1 | 0.1961 | WARS 2 | 0.1675 |
| CCL 13 | 0.127 | GBE1 | 0.0374 | MIR212 | 0.0967 | RARA_3 | 0.1 | XBP1 2 | 0.1486 |
| CCR2 3 | 0.0256 | GBP7 | 0.1255 | MIR571 | 0.0043 | RARG | 0.0489 | XRN2_1 | 0.067 |
| CD34_1 | 0.0151 | GJA5_1 | 0.0629 | MIR576 | 0.1015 | RNASEK | 0.0889 | YARS2 | 0.0371 |
| CDC42BPA 2 | 0.0088 | GMNN | 0.1049 | MIR654 | 0.0586 | RNF7 1 | 0.0022 | ZNF75D 2 | 0.1348 |
| CDC42SE2 2 | 0.0086 | GSR_2 | 0.0323 | MIR942 | 0.1229 | ROD1_1 | 0.1789 | ZSWIM4 2 | 0.1814 |
| CIDEC 1 | 0.0993 | HBA2 | 0.2133 | MMP12 1 | 0.1182 | SATB2 | 0.0348 | figo_numeric | 0.0002 |
| CLDN6 | 0.0009 | HCFC1R1_1 | 0.0402 | MYCN 2 | 0.1248 | SBSN | 0.0947 | hist rev SBOT | 0.0543 |
| CREB5 2 | 0.0038 | HDAC7 2 | 0.0084 | MYOHD1 | 0.1121 | SCXB | 0.0091 | surg outcome | 0.006 |
| CRYBA1 | 0.0576 | HDLBP _ 3 | 0.1079 | NFATC3 _ 5 | 0.0145 | SEC22C 3 | 0.1053 | | |
| CXCL13 | 0.0679 | HIC1 | 0.0192 | NFATC4 | 0.0439 | SELENBP1 | 0.1512 | | |

Table 31

| | | | | | | | | | |
|---|---|---|---|---|---|---|---|---|---|
| ABHD3 | 0.0611 | CYB5R3_2 | 0.19 | HIC1 | 0.0148 | NLRP9 | 0.1932 | SERPINB2_2 | 0.0195 |
| ADAM17 2 | 0.2467 | CYP1A2 | 0.0702 | HPRT1_1 | 0.1453 | NOVA2 | 0.0611 | SERPINB5 | 0.184 |
| ADAMTS1 | 0.1481 | DBNDD2 | 0.1289 | HPS4 1 | 0.0659 | NP | 0.0779 | SFN | 0.0013 |
| ADAMTS2 1 | 0.0878 | DNAH11 | 0.0438 | HR 1 | 0.0255 | NR6A1_2 | 0.1484 | SFRS4 | 0.0584 |
| ALS2CL 3 | 0.0732 | DNMT3L_2 | 0.0171 | HSD11B1_1 | 0.1035 | NRXN3 _ 3 | 0.1753 | SHC1_3 | 0.0549 |
| ANO7_3 | 0.026 | DOCK7_1 | 0.1044 | ICAM2 | 0.0171 | NT5DC1_2 | 0.1814 | SLC23A1_2 | 0.1183 |
| ARL6IP1 1 | 0.0234 | DSC3_1 | 0.0436 | ICAM4_1 | 0.3048 | NTRK2_3 | 0.0048 | SLC25A34 | 0.1292 |
| ARMCX3_2 | 0.0699 | DUT_3 | 0.103 | IL1RAP_2 | 0.0743 | NUP155 _ 1 | 0.0327 | SLC4A5 3 | 0.0944 |
| ATXN10 1 | 0.2139 | EEF1E1_1 | 0.1359 | IQCA1_2 | 0.014 | NYX | 0.1538 | SLC9A10 | 0.0613 |
| AXL 1 | 0.0988 | ELN 2 | 0.1332 | KCNIP3_1 | 0.0921 | ODF2_3 | 0.0051 | SNORD93 | 0.1383 |
| BAII 3 | 0.0483 | EMP1 | 0.2134 | KCNQ2 _ 1 | 0.1057 | ORC1L | 0.0197 | SOX2 1 | 0.0796 |
| BCAS1 1 | 0.3278 | ENOI | 0.1671 | KIF3C | 0.1706 | OTUD7A_3 | 0.0402 | STC1 | 0.0062 |
| BDNF 2 | 0.1097 | ENPEP 2 | 0.0697 | KRT80_2 | 0.0934 | PANK4 | 0.0561 | STC2 | 0.1198 |
| BMPR1A | 0.1125 | EPHB1 | 0.0203 | KRTAP10.10_2 | 0.0361 | PDLIM2_2 | 0.2129 | STYX 2 | 0.0119 |
| BTF3_3 | 0.0995 | EPYC | 0.0499 | L3MBTL2_3 | 0.0435 | PDZRN4_2 | 0.1996 | SYTL3 | 0.0268 |
| C10orfII6 | 0.0784 | ERI2 2 | 0.276 | LBH_2 | 0.0715 | PHYH_1 | 0.0036 | TAF15 1 | 0.0313 |
| C11orf24 | 0.1862 | ESPNL | 0.0502 | LENEP | 0.2254 | PIGA 1 | 0.0841 | TCEAL8 1 | 0.0042 |
| C11orf49_3 | 0.1119 | EZH2_1 | 0.0405 | LGI3 | 0.0879 | PITX2_1 | 0.2154 | THBS3 | 0.0851 |
| C14orf102_2 | 0.1243 | FAM13AOS | 0.0591 | LOC492303 | 0.0159 | PKN1 3 | 0.0059 | TM2D3_2 | 0.0269 |
| C14orf109_2 | 0.1031 | FAM187B_2 | 0.0072 | LRRC14B | 0.0399 | PLEKHG5_5 | 0.2748 | TMEM52 | 0.0942 |
| C17orf106 | 0.1714 | FAM70A_1 | 0.0775 | LRRC37A4_2 | 0.0755 | PLSCR4 | 0.0108 | TMEM62 | 0.0135 |
| C17orf58_2 | 0.0228 | FBXO48_2 | 0.2457 | LRRTM4 | 0.1586 | PMEPA1_4 | 0.1442 | TNFRSF18_1 | 0.2313 |
| C17orf58_3 | 0.03 | FKBP10 | 0.0488 | MACC1 | 0.1379 | PNMA5 | 0.1597 | TNNT2_1 | 0.012 |
| C18orf56 | 0.0098 | FLJ33360 | 0.0155 | MANSC1_1 | 0.1215 | PPAPDC1A | 0.1134 | TOMM20L | 0.0136 |
| Clorf168 | 0.0231 | FLJ43752 | 0.2301 | MCAM | 0.0381 | PRAMEF5 | 0.0017 | TPM2 2 | 0.1755 |
| Clorf64 | 0.106 | FMNL3_2 | 0.0004 | MCART6_1 | 0.1141 | PRKAA2 | 0.1194 | TRIM58 | 0.1229 |

(continued)

| | | | | | | | | | |
|---|---|---|---|---|---|---|---|---|---|
| C8orf79_1 | 0.002 | FOSB | 0.2262 | MFRP | 0.23 | PSMC6_1 | 0.0261 | UBR7 1 | 0.0884 |
| CALD1 2 | 0.1331 | FOSL2 | 0.0439 | MIDN | 0.0298 | RAD54B_2 | 0.194 | UBR7 2 | 0.1352 |
| CASP8AP2 | 0.1365 | FOXN1 | 0.2459 | MIR1914 | 0.0671 | RAP1A_1 | 0.1843 | WARS 2 | 0.1689 |
| CCL 13 | 0.14 | GAD1_2 | 0.0267 | MIR212 | 0.0994 | RARA_3 | 0.099 | XBP1 2 | 0.1514 |
| CCR2 3 | 0.0175 | GBE1 | 0.0406 | MIR571 | 0.0046 | RARG | 0.0566 | XRN2_1 | 0.0559 |
| CD34 1 | 0.0126 | GBP7 | 0.1254 | MIR576 | 0.1041 | RNASEK | 0.0867 | YARS2 | 0.037 |
| CDC42BPA 2 | 0.0028 | GJA5_1 | 0.0606 | MIR654 | 0.0554 | RNF7_1 | 0.0004 | ZNF75D 2 | 0.1337 |
| CDC42SE2 2 | 0.0138 | GMNN | 0.1019 | MIR942 | 0.1137 | ROD1_1 | 0.1804 | ZSWIM4 2 | 0.1807 |
| CIDEC 1 | 0.0988 | GSR_2 | 0.0222 | MMP12_1 | 0.1168 | SATB2 | 0.0317 | figo_numeric | 0.0053 |
| CLDN6 | 0.0011 | HBA2 | 0.2105 | MYCN_2 | 0.1202 | SBSN | 0.1026 | hist rev SBOT | 0.0588 |
| CREB5_2 | 0.003 | HCFC1R1 1 | 0.0388 | MYOHD1 | 0.1078 | SCXB | 0.016 | surg outcome | 0.0047 |
| CRYBA1 | 0.0657 | HDAC7_2 | 0.0036 | NFATC3_5 | 0.0247 | SEC22C_3 | 0.1117 | | |
| CXCL13 | 0.0628 | HDLBP_3 | 0.1 | NFATC4 | 0.0433 | SELENBP1 | 0.1501 | | |

Table 32

| | | | | | | | | | |
|---|---|---|---|---|---|---|---|---|---|
| ABCC9_3 | 0.0529 | CYP1A2 | 0.0554 | HR_1 | 0.0256 | NP | 0.0832 | SFRS4 | 0.0689 |
| ABHD3 | 0.2424 | DBNDD2 | 0.1234 | HSD11B1_1 | 0.0858 | NR6A1_2 | 0.1279 | SHC1_3 | 0.0778 |
| ADAM17_2 | 0.1512 | DNAH11 | 0.0447 | ICAM2 | 0.0136 | NRXN3_3 | 0.1643 | SLC23A1_2 | 0.1388 |
| ADAMTS1 | 0.1088 | DNMT3L_2 | 0.0282 | ICAM4_1 | 0.285 | NT5DC1_2 | 0.186 | SLC25A34 | 0.1157 |
| ADAMTS2_1 | 0.0942 | DOCK7_1 | 0.1119 | IL1RAP_2 | 0.0786 | NTRK2_3 | 0.0092 | SLC4A5_3 | 0.0883 |
| ALS2CL_3 | 0.065 | DSC3_1 | 0.0486 | IQCA1_2 | 0.0276 | NUP155_1 | 0.0304 | SLC9A10 | 0.0756 |
| ANO7_3 | 0.0491 | DUT_3 | 0.1142 | KCNIP3_1 | 0.1029 | NYX | 0.1206 | SNORD93 | 0.1274 |
| ARL6IP1_1 | 0.0162 | EEF1E1_1 | 0.1242 | KCNQ2_1 | 0.1189 | ODF2_3 | 0.0217 | SOX2_1 | 0.0692 |
| ARMCX3_2 | 0.0691 | EMP1 | 0.1118 | KIF3C | 0.1695 | ORC1L | 0.0297 | STC1 | 0.0055 |
| ATXN10_1 | 0.198 | ENOI | 0.1908 | KRT80 2 | 0.1099 | OTUD7A_3 | 0.0403 | STC2 | 0.1273 |
| AXL_1 | 0.0809 | ENPEP_2 | 0.166 | KRTAP10.10_2 | 0.0252 | PANK4 | 0.0439 | STYX 2 | 0.0154 |
| BAI1_3 | 0.0175 | EPHB1 | 0.0417 | L3MBTL2_3 | 0.0478 | PDLIM2_2 | 0.2151 | SYTL3 | 0.0196 |
| BCAS1_1 | 0.3169 | EPYC | 0.0312 | LBH_2 | 0.0792 | PDZRN4_2 | 0.2076 | TAF15_1 | 0.0258 |
| BDNF_2 | 0.1303 | ERI2_2 | 0.2846 | LENEP | 0.2379 | PHYH_1 | 0.0078 | TCEAL8_1 | 0.0227 |
| BMPR1A | 0.1153 | ESPNL | 0.0526 | LGI3 | 0.0883 | PIGA_1 | 0.0915 | THBS3 | 0.1018 |
| BTF3_3 | 0.1156 | EZH2_1 | 0.0598 | LOC340508 | 0.0366 | PITX2_1 | 0.2042 | THY1 | 0.0426 |
| C10orf1 16 | 0.0674 | FAM13AOS | 0.0796 | LOC492303 | 0.0211 | PKN1_3 | 0.0078 | TIMP2_2 | 0.0947 |
| C11orf24 | 0.1849 | FAM187B_2 | 0.0084 | LRRC14B | 0.0744 | PLEKHG5_5 | 0.2383 | TM2D3_2 | 0.0076 |
| C11orf49_3 | 0.1023 | FAM70A_1 | 0.0708 | LRRC37A4_2 | 0.0238 | PLSCR4 | 0.0206 | TMEM52 | 0.0201 |
| C14orf102_2 | 0.1041 | FBXO48_2 | 0.2201 | LRRTM4 | 0.179 | PMEPA1_4 | 0.1431 | TMEM62 | 0.0621 |
| C14orf109_2 | 0.1215 | FKBP10 | 0.0794 | MACC1 | 0.1569 | PNMA5 | 0.1693 | TNFRSF18_1 | 0.2192 |
| C17orf106 | 0.1711 | FLJ33360 | 0.0187 | MANSC1_1 | 0.1193 | PPAPDC1A | 0.114 | TNNT2_1 | 0.0004 |
| C17orf58_2 | 0.009 | FLJ43752 | 0.2468 | MCAM | 0.0131 | PRAMEF5 | 0.0136 | TOMM20L | 0.0057 |
| C17orf58_3 | 0.0117 | FMNL3_2 | 0.0007 | MCART6_1 | 0.1301 | PRKAA2 | 0.1277 | TPM2_2 | 0.1835 |
| C18orf56 | 0.001 | FOSB | 0.2028 | MFRP | 0.2287 | PSMC6_1 | 0.0415 | TRIM58 | 0.1045 |
| C1orf168 | 0.0387 | FOSL2 | 0.0376 | MIDN | 0.0079 | RAD54B_2 | 0.1692 | UBR7_1 | 0.0805 |

| | | | | | | | |
|---|---|---|---|---|---|---|---|
| Clorf64 | 0.1176 | FOXN1 | 0.2508 | MIR1914 | 0.0582 | RAP1A_1 | 0.2019 | UBR7_2 | 0.1223 |
| C8orf79_1 | 0.0116 | GAD1_2 | 0.0232 | MIR212 | 0.0976 | RARA_3 | 0.0999 | WARS_2 | 0.1854 |
| CASP8AP2 | 0.1278 | GBE1 | 0.0526 | MIR571 | 0.0029 | RARG | 0.0712 | XBP1_2 | 0.144 |
| CCL13 | 0.1316 | GBP7 | 0.1402 | MIR576 | 0.1028 | RNASEK | 0.0808 | XRN2_1 | 0.0651 |
| CCR2_3 | 0.1087 | GJA5_1 | 0.0714 | MIR654 | 0.0464 | RNF7_1 | 0.0279 | YARS2 | 0.0288 |
| CD34_1 | 0.0323 | GMNN | 0.1076 | MIR942 | 0.1057 | ROD1_1 | 0.2035 | ZNF75D_2 | 0.1394 |
| CDC42BPA_2 | 0.0092 | GSR_2 | 0.0338 | MMP12_1 | 0.1202 | SATB2 | 0.0406 | ZSWIM4_2 | 0.1758 |
| CDC42SE2_2 | 0.0096 | HBA2 | 0.2092 | MYCN_2 | 0.1352 | SBSN | 0.0642 | figo_numeric | 0.0182 |
| CIDEC_1 | 0.1047 | HCFC1R1_1 | 0.0619 | MYL9 2 | 0.104 | SCXB | 0.0067 | hist_rev_SBOT | 0.0331 |
| CLDN6 | 0.0159 | HDAC7_2 | 0.0084 | MYOHD1 | 0.0049 | SEC22C_3 | 0.1018 | surg_outcome | 0.0106 |
| CREB5_2 | 0.0147 | HDLBP_3 | 0.1015 | NFATC3_5 | 0.0374 | SELENBP1 | 0.1488 | | |
| CRYBA1 | 0.0504 | HIC1 | 0.0072 | NFATC4 | 0.0738 | SERPINB2_2 | 0.0031 | | |
| CXCL13 | 0.0645 | HPRT1_1 | 0.1231 | NLRP9 | 0.1861 | SERPINB5 | 0.1804 | | |
| CYB5R3_2 | 0.1864 | HPS4_1 | 0.076 | NOV A2 | 0.0865 | SFN | 0.0011 | | |

Table 33

| | | | | | | | |
|---|---|---|---|---|---|---|---|
| ABCC9_3 | 0.0769 | DBNDD2 | 0.1382 | HPS4_1 | 0.0585 | NOVA2 | 0.0742 | SFN | 0.016 |
| ABHD3 | 0.2263 | DNAH11 | 0.041 | HR 1 | 0.0251 | NP | 0.0703 | SFRS4 | 0.062 |
| ADAM17 2 | 0.135 | DNMT3L 2 | 0.0247 | HSD11B1_1 | 0.0913 | NR6A1 2 | 0.1314 | SHC1_3 | 0.085 |
| ADAMTS1 | 0.1107 | DOCK7_1 | 0.1187 | ICAM2 | 0.0182 | NRXN3 _ 3 | 0.1686 | SLC23A1_2 | 0.144 |
| ALS2CL 3 | 0.0981 | DSC3_1 | 0.0468 | ICAM4_1 | 0.2767 | NT5DC1_2 | 0.1646 | SLC25A34 | 0.1005 |
| ANO7 3 | 0.0694 | DUT_3 | 0.1219 | IL1RAP_2 | 0.1004 | NTRK2_3 | 0.0005 | SLC4A5 3 | 0.0911 |
| ARL6IP1 1 | 0.0407 | EEF1E1 _ 1 | 0.1415 | IQCA1_2 | 0.0196 | NUP155 _ 1 | 0.054 | SLC9A10 | 0.0636 |
| ARMCX3_2 | 0.0676 | ELN 2 | 0.1253 | KCNIP3_1 | 0.0938 | NYX | 0.1204 | SNORD93 | 0.123 |
| ATXN10 1 | 0.1977 | EMP1 | 0.2016 | KCNQ2_1 | 0.1103 | ODF2_3 | 0.0096 | SOX2 1 | 0.0597 |
| AXL 1 | 0.0805 | ENOI | 0.1534 | KIF3C | 0.1884 | ORC1L | 0.0388 | STC1 | 0.001 |
| BAII 3 | 0.0393 | ENPEP 2 | 0.0998 | KRT80_2 | 0.0985 | OTUD7A 3 | 0.0475 | STC2 | 0.1239 |
| BCAS1 1 | 0.3046 | EPHB1 | 0.0503 | KRTAP10.10_2 | 0.0313 | PANK4 | 0.0329 | STYX 2 | 0.0093 |
| BDNF 2 | 0.1224 | EPYC | 0.0358 | L3MBTL2_3 | 0.0356 | PDLIM2_2 | 0.214 | SYTL3 | 0.0194 |
| BMPR1A | 0.115 | ERI2_2 | 0.2572 | LBH_2 | 0.068 | PDZRN4_2 | 0.2201 | TAF15 1 | 0.022 |
| BTF3_3 | 0.1162 | ESPNL | 0.0616 | LENEP | 0.2277 | PHYH_1 | 0.0164 | TCEAL8 1 | 0.0003 |
| C10orfll6 | 0.074 | EZH2_1 | 0.0412 | LGI3 | 0.0652 | PIGA_1 | 0.0739 | THBS3 | 0.0974 |
| C11orf24 | 0.1755 | FAM13AOS | 0.0663 | LOC340508 | 0.0296 | PITX2_1 | 0.194 | THY1 | 0.0381 |
| C11orf49_3 | 0.109 | FAM187B _ 2 | 0.0012 | LOC492303 | 0.0031 | PKN1_3 | 0.0126 | TIMP2 2 | 0.0828 |
| C14orf102_2 | 0.1056 | FAM70A_1 | 0.078 | LRRC14B | 0.0766 | PLEKHG5_5 | 0.2702 | TM2D3 2 | 0.0051 |
| C14orf109_2 | 0.1252 | FBXO48_2 | 0.2295 | LRRC37A4_2 | 0.0115 | PLSCR4 | 0.0288 | TMEM52 | 0.0268 |
| C17orf106 | 0.1576 | FKBP10 | 0.0568 | LRRTM4 | 0.1479 | PMEPA1_4 | 0.1262 | TMEM62 | 0.0673 |
| C17orf58_2 | 0.0012 | FLJ33360 | 0.0175 | MACC1 | 0.1498 | PNMA5 | 0.1737 | TNFRSF18_1 | 0.2093 |
| C17orf58_3 | 0.0209 | FLJ43752 | 0.2249 | MANSC1_1 | 0.1195 | PPAPDC1A | 0.1265 | TNNT2_1 | 0.0013 |
| C18orf56 | 0.0072 | FMNL3 2 | 0.008 | MCAM | 0.0017 | PRAMEF5 | 0.0046 | TOMM20L | 0.0085 |
| Clorf168 | 0.0443 | FOSB | 0.2095 | MCART6_1 | 0.1391 | PRKAA2 | 0.11 | TPM2 2 | 0.1867 |
| Clorf64 | 0.1247 | FOSL2 | 0.0203 | MFRP | 0.2329 | PSMC6_1 | 0.0405 | TRIM58 | 0.1035 |

| | | | | | | | |
|---|---|---|---|---|---|---|---|
| C8orf79_1 | 0.0056 | FOXN1 | 0.2606 | MIDN | 0.0063 | RAD54B 2 | 0.1786 | UBR7 1 | 0.0714 |
| CASP8AP2 | 0.1365 | FRMD6_3 | 0.0299 | MIR1914 | 0.0619 | RAP1A _ 1 | 0.187 | UBR7 2 | 0.1268 |
| CCL13 | 0.1089 | GAD1_2 | 0.0692 | MIR212 | 0.0944 | RARA_3 | 0.0946 | WARS 2 | 0.1952 |
| CCR2 3 | 0.1056 | GBE1 | 0.1563 | MIR571 | 0.0076 | RARG | 0.0879 | XBP1 2 | 0.1465 |
| CD34 1 | 0.0216 | GBP7 | 0.0956 | MIR576 | 0.1135 | RNASEK | 0.0679 | XRN2_1 | 0.0487 |
| CDC42BPA 2 | 0.0082 | GJA5_1 | 0.0806 | MIR654 | 0.047 | RNF7_1 | 0.0185 | YARS2 | 0.0242 |
| CDC42SE2 2 | 0.0016 | GMNN | 0.0938 | MIR942 | 0.1085 | ROD1_1 | 0.2005 | ZNF75D 2 | 0.136 |
| CIDEC 1 | 0.1023 | GSR 2 | 0.0251 | MMP12_1 | 0.109 | SATB2 | 0.0383 | ZSWIM4 2 | 0.1701 |
| CLDN6 | 0.0187 | HBA2 | 0.2097 | MYCN_2 | 0.1288 | SBSN | 0.0809 | figo_numeric | 0.0381 |
| CREB5_2 | 0.0012 | HCFC1R1_1 | 0.0701 | MYL9_2 | 0.0939 | SCXB | 0.0124 | hist rev SBOT | 0.0496 |
| CRYBA1 | 0.0604 | HDAC7 2 | 0.0164 | MYOHD1 | 0.0301 | SEC22C 3 | 0.0852 | surg outcome | 0.0085 |
| CXCL13 | 0.0559 | HDLBP_3 | 0.0931 | NFATC3 _ 5 | 0.0334 | SELENBP1 | 0.1419 | | |
| CYB5R3 2 | 0.1876 | HIC1 | 0.0231 | NFATC4 | 0.0658 | SERPINB2_2 | 0.0033 | | |
| CYP1A2 | 0.0567 | HPRT1_1 | 0.1342 | NLRP9 | 0.1667 | SERPINB5 | 0.1761 | | |

EP 3 874 274 B1

Table 34

| Gene | Value | Gene | Value | Gene | Value | Gene | Value | Gene | Value |
|---|---|---|---|---|---|---|---|---|---|
| ABCC9_3 | 0.0388 | CYP1A2 | 0.0849 | HPS4 1 | 0.0639 | NOVA2 | 0.1038 | SFRS4 | 0.0657 |
| ABHD3 | 0.2506 | DBNDD2 | 0.1068 | HR_1 | 0.0406 | NP | 0.0773 | SHC1_3 | 0.0652 |
| ADAM17 2 | 0.1571 | DNAH11 | 0.0327 | HSD11B1_1 | 0.0851 | NR6A1 2 | 0.1333 | SLC23A1_2 | 0.1524 |
| ADAMTS1 | 0.1332 | DNMT3L_2 | 0.0097 | ICAM2 | 0.0185 | NRXN3 3 | 0.1292 | SLC25A34 | 0.1104 |
| ADAMTS21 | 0.1159 | DOCK7 1 | 0.1207 | ICAM4_1 | 0.2705 | NT5DC1 2 | 0.1712 | SLC4A5 3 | 0.0766 |
| ALS2CL 3 | 0.0613 | DSC3_1 | 0.0423 | IL1RAP 2 | 0.0475 | NTRK2 3 | 0.0184 | SLC9A10 | 0.0965 |
| ANO7_3 | 0.0327 | DUT_3 | 0.126 | IQCA1_2 | 0.0304 | NUP155 1 | 0.0066 | SNORD93 | 0.1544 |
| ARL6IP1_1 | 0.0201 | EEF1E1 1 | 0.1036 | KCNIP3_1 | 0.0917 | NYX | 0.1169 | SOX2 1 | 0.0813 |
| ARMCX3_2 | 0.0782 | ELN_2 | 0.1072 | KCNQ2_1 | 0.1162 | ODF2_3 | 0.0103 | STC1 | 0.0126 |
| ATXN10 1 | 0.2094 | EMP1 | 0.1975 | KIF3C | 0.2075 | ORC1L | 0.0351 | STC2 | 0.1178 |
| AXL 1 | 0.0928 | ENOI | 0.1405 | KRT80_2 | 0.0952 | OTUD7A 3 | 0.0408 | STYX 2 | 0.0347 |
| BAII 3 | 0.0418 | ENPEP 2 | 0.0842 | KRTAP10.10_2 | 0.0191 | PANK4 | 0.0451 | SYTL3 | 0.008 |
| BCAS1 1 | 0.3188 | EPHB1 | 0.0612 | L3MBTL2_3 | 0.0438 | PDLIM2 2 | 0.2294 | TAF15 1 | 0.0138 |
| BDNF 2 | 0.1466 | EPYC | 0.0344 | LBH_2 | 0.083 | PHYH1 | 0.1882 | TCEAL8_1 | 0.0059 |
| BMPR1A | 0.126 | ERI2 2 | 0.2807 | LENEP | 0.2523 | PIGA 1 | 0.0089 | THBS3 | 0.0953 |
| BTF3 3 | 0.1093 | ESPNL | 0.0421 | LGI3 | 0.101 | PITX2 1 | 0.0681 | THY1 | 0.0587 |
| C10orf116 | 0.0292 | EZH2_1 | 0.0512 | LOC340508 | 0.0218 | PKN1_3 | 0.0189 | TIMP2 2 | 0.1112 |
| C11orf24 | 0.1893 | FAM13AOS | 0.0246 | LOC492303 | 0.0057 | PLEKHG5_5 | 0.2635 | TM2D3_2 | 0.0069 |
| C11orf49_3 | 0.152 | FAM187B_2 | 0.0024 | LRRC14B | 0.0674 | PLSCR4 | 0.0429 | TMEM52 | 0.014 |
| C14orf102_2 | 0.1004 | FAM70A_1 | 0.0769 | LRRC37A4_2 | 0.0004 | PMEPA1_4 | 0.1604 | TMEM62 | 0.0758 |
| C14orf109_2 | 0.0715 | FBXO48_2 | 0.2347 | LRRTM4 | 0.165 | PNMA5 | 0.1476 | TNFRSF18 1 | 0.2563 |
| C17orf106 | 0.1828 | FKBP10 | 0.05 | MACC1 | 0.1471 | PPAPDC1A | 0.1517 | TNNT2_1 | 0.0088 |
| C17orf58_2 | 0.0149 | FLJ33360 | 0.029 | MANSC1_1 | 0.1409 | PRAMEF5 | 0.0077 | TOMM20L | 0.0428 |
| C17orf58_3 | 0.0274 | FLJ43752 | 0.2396 | MCAM | 0.0217 | PRKAA2 | 0.1146 | TPM2 2 | 0.1822 |
| C18orf56 | 0.0323 | FMNL3 2 | 0.0106 | MCART6_1 | 0.1374 | PSMC6 1 | 0.0375 | TRIM58 | 0.1079 |
| Clorf168 | 0.0413 | FOSB | 0.207 | MFRP | 0.2239 | RAD54B_2 | 0.2 | UBR7 1 | 0.0384 |

| | | | | | | | |
|---|---|---|---|---|---|---|---|
| Clorf64 | 0.1345 | FOSL2 | 0.0321 | MIDN | 0.0195 | RAP1A 1 | 0.2053 | UBR7 2 | 0.1276 |
| C8orf79_1 | 0.026 | FOXN1 | 0.2979 | MIR1914 | 0.0494 | RARA_3 | 0.0872 | WARS 2 | 0.1626 |
| CASP8AP2 | 0.1526 | GAD1_2 | 0.0116 | MIR212 | 0.1098 | RARG | 0.0514 | XBP1 2 | 0.115 |
| CCL13 | 0.1129 | GBE1 | 0.0538 | MIR571 | 0.0105 | RNASEK | 0.0322 | XRN2 1 | 0.0221 |
| CCR2_3 | 0.1358 | GBP7 | 0.1576 | MIR576 | 0.1182 | RNF7 1 | 0.0384 | YARS2 | 0.0034 |
| CD34 1 | 0.0399 | GJA5_1 | 0.0537 | MIR654 | 0.0259 | ROD1_1 | 0.2271 | ZNF75D 2 | 0.1379 |
| CDC42BPA 2 | 0.0086 | GMNN | 0.0806 | MIR942 | 0.0974 | SATB2 | 0.0413 | ZSWIM4_2 | 0.1762 |
| CDC42SE2_2 | 0.0018 | GSR_2 | 0.0328 | MMP12_1 | 0.1164 | SBSN | 0.0873 | figo_numeric | 0.0245 |
| CIDEC 1 | 0.123 | HBA2 | 0.1962 | MYCN 2 | 0.1565 | SCXB | 0.0201 | hist rev SBOT | 0.0407 |
| CLDN6 | 0.0096 | HCFC1R1_1 | 0.0691 | MYL9_2 | 0.1105 | SEC22C 3 | 0.1031 | surg_outcome | 0.0258 |
| CREB5 2 | 0.0464 | HDAC7 2 | 0.0115 | MYOHD1 | 0.0317 | SELENBP1 | 0.1728 | | |
| CRYBA1 | 0.0438 | HDLBP_3 | 0.1051 | NFATC3 _ 5 | 0.0367 | SERPINB2 _ 2 | 0.0012 | | |
| CXCL 13 | 0.0717 | HIC1 | 0.001 | NFATC4 | 0.0743 | SERPINBS | 0.1955 | | |
| CYB5R3 2 | 0.1762 | HPRT1_1 | 0.1534 | NLRP9 | 0.1684 | SFN | 0.0434 | | |

Table 35

| Gene | Value | Gene | Value | Gene | Value | Gene | Value | Gene | Value |
|---|---|---|---|---|---|---|---|---|---|
| ABCC9_3 | 0.0381 | DBNDD2 | 0.1175 | HR 1 | 0.0274 | NP | 0.0712 | SFRS4 | 0.0625 |
| ABHD3 | 0.2338 | DNAH11 | 0.0373 | HSD11B11 | 0.092 | NR6A1_2 | 0.1267 | SHC1_3 | 0.0771 |
| ADAM17 2 | 0.1493 | DNMT3L_2 | 0.0276 | ICAM2 | 0.0318 | NRXN3 3 | 0.1699 | SLC23A1 2 | 0.1334 |
| ADAMTS1 | 0.126 | DOCK7_1 | 0.1329 | ICAM4 1 | 0.2845 | NT5DC1_2 | 0.1809 | SLC25A34 | 0.1103 |
| ADAMTS2 1 | 0.1136 | DSC3 1 | 0.0472 | IL1RAP 2 | 0.0946 | NTRK2_3 | 0.0264 | SLC4A5 3 | 0.0823 |
| ALS2CL 3 | 0.0775 | DUT 3 | 0.1334 | IQCA1_2 | 0.044 | NUP155_1 | 0.0358 | SLC9A10 | 0.0738 |
| ANO7 3 | 0.0196 | EEF1E1_1 | 0.117 | KCNIP3 1 | 0.098 | NYX | 0.1102 | SNORD93 | 0.1401 |
| ARL6IP1_1 | 0.0044 | ELN_2 | 0.1039 | KCNQ2_1 | 0.1143 | ODF2 3 | 0.018 | SOX2 1 | 0.0698 |
| ARMCX3 2 | 0.0442 | EMP1 | 0.1967 | KIF3C | 0.1992 | ORC1L | 0.0475 | STC1 | 0.0054 |
| ATXN10 1 | 0.2144 | ENOI | 0.1639 | KRT80 2 | 0.1022 | OTUD7A 3 | 0.0533 | STC2 | 0.1166 |
| AXL 1 | 0.0856 | ENPEP_2 | 0.0613 | KRTAP10.10_2 | 0.0127 | PANK4 | 0.0492 | STYX 2 | 0.0168 |
| BAII 3 | 0.0267 | EPHB1 | 0.0444 | L3MBTL2_3 | 0.0412 | PDLIM2_2 | 0.2254 | SYTL3 | 0.0068 |
| BCAS1 1 | 0.2954 | EPYC | 0.0412 | LBH 2 | 0.0802 | PDZRN4_2 | 0.2058 | TAF15 1 | 0.0143 |
| BDNF 2 | 0.1234 | ERI2 2 | 0.28 | LENEP | 0.2283 | PHYH 1 | 0.0062 | TCEAL8 1 | 0.0282 |
| BMPR1A | 0.111 | ESPNL | 0.0741 | LGI3 | 0.1008 | PIGA 1 | 0.0959 | THBS3 | 0.0785 |
| BTF3 3 | 0.1047 | EZH2 1 | 0.0341 | LOC340508 | 0.0476 | PITX2 1 | 0.1918 | THY1 | 0.0361 |
| C10orf116 | 0.0513 | FAM13AOS | 0.071 | LOC492303 | 0.0142 | PKN1_3 | 0.0113 | TIMP2 2 | 0.091 |
| C11orf24 | 0.1669 | FAM187B_2 | 0.0159 | LRRC14B | 0.0846 | PLEKHG5_5 | 0.2537 | TM2D3 2 | 0.0068 |
| C11orf49_3 | 0.1181 | FAM70A_1 | 0.0643 | LRRC37A4 2 | 0.0184 | PLSCR4 | 0.0363 | TMEM52 | 0.0479 |
| C14orf102_2 | 0.0933 | FBXO48_2 | 0.2243 | LRRTM4 | 0.1877 | PMEPA1 4 | 0.1511 | TMEM62 | 0.062 |
| C14orf109_2 | 0.1256 | FKBP10 | 0.0743 | MACC1 | 0.1835 | PNMA5 | 0.1668 | TNFRSF18_1 | 0.2197 |
| C17orf106 | 0.1735 | FLJ33360 | 0.0105 | MANSCl 1 | 0.1151 | PPAPDC1A | 0.1206 | TNNT2_1 | 0.0015 |
| C17orf58_2 | 0.0433 | FLJ43752 | 0.2547 | MCAM | 0.001 | PRAMEF 5 | 0.0026 | TOMM20L | 0.0009 |
| C17orf58 3 | 0.0244 | FMNL3_2 | 0.0115 | MCART6 1 | 0.1265 | PRKAA2 | 0.0848 | TPM2 2 | 0.1812 |
| C18orf56 | 0.0027 | FOSB | 0.2183 | MFRP | 0.2273 | PSMC6_1 | 0.0149 | TRIM58 | 0.1108 |
| Clorf168 | 0.0418 | FOSL2 | 0.021 | MIDN | 0.0193 | RAD54B_2 | 0.1833 | UBR7 1 | 0.0573 |

| | | | | | | | | | |
|---|---|---|---|---|---|---|---|---|---|
| C1orf64 | 0.1164 | FOXN1 | 0.2436 | MIR1914 | 0.0793 | RAP1A_1 | 0.2022 | UBR7 2 | 0.127 |
| C8orf79_1 | 0.0363 | GAD1_2 | 0.0205 | MIR212 | 0.0977 | RARA_3 | 0.0878 | WARS 2 | 0.1946 |
| CASP8AP2 | 0.1313 | GBE1 | 0.068 | MIR571 | 0.0082 | RARG | 0.0786 | XBP1 2 | 0.1632 |
| CCL 13 | 0.1206 | GBP7 | 0.1563 | MIR576 | 0.1163 | RNASEK | 0.0689 | XRN2_1 | 0.025 |
| CCR2 3 | 0.1162 | GJA5_1 | 0.0484 | MIR654 | 0.0305 | RNF7 1 | 0.0148 | YARS2 | 0.0083 |
| CD34 1 | 0.0218 | GMNN | 0.093 | MIR942 | 0.1017 | ROD1_1 | 0.2262 | ZNF75D 2 | 0.1132 |
| CDC42BPA 2 | 0.0145 | GSR 2 | 0.0154 | MMP12 1 | 0.1097 | SATB2 | 0.0257 | ZSWIM4 2 | 0.1604 |
| CDC42SE2 2 | 0.0079 | HBA2 | 0.2014 | MYCN_2 | 0.1174 | SBSN | 0.0632 | figo_numeric | 0.0078 |
| CLDN6 | 0.122 | HCFC1R1_1 | 0.0703 | MYL9 2 | 0.0971 | SCXB | 0.0105 | hist rev SBOT | 0.0391 |
| CREB5_2 | 0.0284 | HDAC7_2 | 0.0006 | MYOHD1 | 0.0014 | SEC22C _ 3 | 0.1011 | surg_outcome | 0.01 |
| CRYBA1 | 0.02 | HDLBP 3 | 0.1085 | NFATC3 5 | 0.0364 | SELENBP1 | 0.1474 | | |
| CXCL13 | 0.0631 | HIC1 | 0.0162 | NFATC4 | 0.0707 | SERPINB2_2 | 0.0031 | | |
| CYB5R3 2 | 0.177 | HPRT 1_1 | 0.1394 | NLRP9 | 0.1794 | SERPINBS | 0.1959 | | |
| CYP1A2 | 0.0825 | HPS4_1 | 0.0437 | NOV A2 | 0.0714 | SFN | 0.0091 | | |

Table 36

| | | | | | | | | | |
|---|---|---|---|---|---|---|---|---|---|
| ABCC9_3 | 0.0545 | CYP1A2 | 0.0737 | HPS4_1 | 0.0653 | NP | 0.0763 | SFRS4 | 0.0685 |
| ABHD3 | 0.2415 | DBNDD2 | 0.1287 | HR 1 | 0.0203 | NR6A1_2 | 0.1269 | SHC1_3 | 0.0692 |
| ADAM17_2 | 0.1477 | DNAH11 | 0.0425 | HSD11B1_1 | 0.0894 | NRXN3 3 | 0.171 | SLC23A1_2 | 0.1305 |
| ADAMTS1 | 0.1122 | DNMT3L_2 | 0.0196 | ICAM2 | 0.0173 | NT5DC1_2 | 0.1813 | SLC25A34 | 0.1051 |
| ADAMTS2_1 | 0.1032 | DOCK7_1 | 0.1078 | ICAM4_1 | 0.2972 | NTRK2_3 | 0.0073 | SLC4A5_3 | 0.0889 |
| ALS2CL_3 | 0.0595 | DSC3_1 | 0.0417 | IL1RA_2 | 0.0791 | NUP155_1 | 0.0266 | SLC9A10 | 0.0683 |
| ANO7_3 | 0.0362 | DUT_3 | 0.124 | IQCA1_2 | 0.0194 | NYX | 0.1089 | SNORD93 | 0.1272 |
| ARL6IP1_1 | 0.0031 | EEF1E1_1 | 0.1334 | KCNIP3_1 | 0.0924 | ODF2_3 | 0.0152 | SOX2_1 | 0.0728 |
| ARMCX3_2 | 0.0618 | ELN_2 | 0.1181 | KCNQ2_1 | 0.1029 | ORC1L | 0.0419 | STC1 | 0.0058 |
| ATXN10_1 | 0.2047 | EMP1 | 0.2003 | KIF3C | 0.1825 | OTUD7A 3 | 0.0423 | STC2 | 0.1154 |
| AXL_1 | 0.0783 | ENOI | 0.1596 | KRT80_2 | 0.095 | PANK4 | 0.0448 | STYX_2 | 0.0132 |
| BAI1_3 | 0.0391 | ENPEP_2 | 0.0809 | KRTAP10.10_2 | 0.0274 | PDLIM2_2 | 0.2176 | SYTL3 | 0.0257 |
| BCAS1_1 | 0.3048 | EPHB1 | 0.0459 | L3MBTL2_3 | 0.044 | PDZRN4_2 | 0.2035 | TAF15_1 | 0.0251 |
| BDNF_2 | 0.1216 | EPYC | 0.036 | LBH_2 | 0.0721 | PHYH_1 | 0.0109 | TCEAL8_1 | 0.0139 |
| BMPR1A | 0.1123 | ERI2_2 | 0.2708 | LENEP | 0.2393 | PIGA_1 | 0.0904 | THBS3 | 0.0963 |
| BTF3_3 | 0.1074 | ESPNL | 0.0581 | LGI3 | 0.0934 | PITX2_1 | 0.1997 | THY1 | 0.0386 |
| C10orf1 16 | 0.0716 | EZH2_1 | 0.0371 | LOC492303 | 0.0266 | PKN1_3 | 0.0013 | TIMP2_2 | 0.0924 |
| C11orf24 | 0.1755 | FAM13AOS | 0.0679 | LRRC14B | 0.0216 | PLEKHG5_5 | 0.2547 | TM2D3_2 | 0.0004 |
| C11orf49_3 | 0.1114 | FAM187B_2 | 0.0032 | LRRC37A4_2 | 0.0734 | PLSCR4 | 0.021 | TMEM52 | 0.02 |
| C14orf102_2 | 0.0991 | FAM70A_1 | 0.0779 | LRRTM4 | 0.1707 | PMEPA1_4 | 0.1405 | TMEM62 | 0.0682 |
| C14orf109_2 | 0.1138 | FBXO48_2 | 0.2245 | MACC1 | 0.1633 | PNMA5 | 0.1713 | TNFRSF18_1 | 0.2167 |
| C17orf106 | 0.1603 | FKBP10 | 0.0641 | MANSC1_1 | 0.1122 | PPAPDC1A | 0.1249 | TNNT2_1 | 0.0065 |
| C17orf58_2 | 0.0148 | FLJ33360 | 0.0162 | MCAM | 0.0193 | PRAMEF 5 | 0.0061 | TOMM20L | 0.0036 |
| C17orf58_3 | 0.0157 | FLJ43752 | 0.2442 | MCART6_1 | 0.1262 | PRKAA2 | 0.1218 | TPM2_2 | 0.1791 |
| C18orf56 | 0.0002 | FMNL3_2 | 0.0149 | MFRP | 0.2249 | PSMC6_1 | 0.0398 | TRIM58 | 0.1121 |
| C1orf168 | 0.0365 | FOSB | 0.2147 | MIDN | 0.0023 | RAD54B_2 | 0.1753 | UBR7_1 | 0.0797 |

| | | | | | | | |
|---|---|---|---|---|---|---|---|
| C1orf64 | 0.1172 | FOSL2 | 0.0302 | MIR1914 | 0.0565 | RAP1A_1 | 0.1949 | UBR7_2 | 0.1337 |
| C8orf79_1 | 0.0041 | FOXN1 | 0.2586 | MIR212 | 0.0981 | RARA_3 | 0.0966 | WARS_2 | 0.1886 |
| CASP8AP2 | 0.142 | GAD1_2 | 0.0218 | MIR571 | 0.0046 | RARG | 0.0824 | XBP1_2 | 0.1499 |
| CCL 13 | 0.1245 | GBE1 | 0.0561 | MIR576 | 0.1079 | RNASEK | 0.0752 | XRN2_1 | 0.0436 |
| CCR2_3 | 0.1264 | GBP7 | 0.1392 | MIR654 | 0.0442 | RNF7_1 | 0.0274 | YARS2 | 0.0291 |
| CD34_1 | 0.0294 | GJA5_1 | 0.0684 | MIR942 | 0.0995 | ROD1_1 | 0.2054 | ZNF75D_2 | 0.1336 |
| CDC42BPA_2 | 0.0043 | GMNN | 0.1047 | MMP12_1 | 0.1168 | SATB2 | 0.0387 | ZSWIM4_2 | 0.1728 |
| CDC42SE2_2 | 0.0164 | GSR_2 | 0.0197 | MYCN_2 | 0.133 | SBSN | 0.0728 | figo_numeric | 0.0272 |
| CIDEC_1 | 0.1042 | HBA2 | 0.2087 | MYL9_2 | 0.1032 | SCXB | 0.014 | hist_rev_SBOT | 0.0364 |
| CLDN6 | 0.0173 | HCFC1R1_1 | 0.0644 | MYOHD1 | 0.0204 | SEC22C_3 | 0.1054 | surg_outcome | 0.0109 |
| CREB5_2 | 0.0142 | HDAC7_2 | 0.0055 | NFATC3_5 | 0.0384 | SELENBP1 | 0.1467 | | |
| CRYBA1 | 0.0574 | HDLBP_3 | 0.0954 | NFATC4 | 0.0676 | SERPINB2_2 | 0.0143 | | |
| CXCL13 | 0.0592 | HIC1 | 0.0018 | NLRP9 | 0.1737 | SERPINB5 | 0.1786 | | |
| CYB5R3_2 | 0.1837 | HPRT1_1 | 0.1332 | NOVA2 | 0.0681 | SFN | 0.0177 | | |

Table 37

| | | | | | | | | | |
|---|---|---|---|---|---|---|---|---|---|
| ABCC9_3 | 0.053 | CYB5R3_2 | 0.1934 | HPRT1_1 | 0.123 | NLRP9 | 0.189 | SERPINB2_2 | 0.0008 |
| ABHD3 | 0.2403 | CYP1A2 | 0.0619 | HPS4_1 | 0.0684 | NOVA2 | 0.0873 | SERPINB5 | 0.1879 |
| ADAM17_2 | 0.1493 | DBNDD2 | 0.1231 | HR_1 | 0.0267 | NP | 0.0869 | SFN | 0.0016 |
| ADAMTS1 | 0.1085 | DNAH11 | 0.0407 | HSD11B1_1 | 0.0858 | NR6A1_2 | 0.1324 | SFRS4 | 0.0695 |
| ALS2CL_3 | 0.0948 | DNMT3L_2 | 0.0273 | ICAM2 | 0.0091 | NRXN3 _3 | 0.1628 | SHC1_3 | 0.0757 |
| ANO7_3 | 0.0613 | DOCK7_1 | 0.1244 | ICAM4_1 | 0.285 | NT5DC1_2 | 0.1884 | SLC23A1_2 | 0.1359 |
| ARL6IP1_1 | 0.0511 | DSC3_1 | 0.0458 | IL1RAP_2 | 0.0733 | NTRK2_3 | 0.0071 | SLC25A34 | 0.117 |
| ARMCX3_2 | 0.0684 | DUT_3 | 0.1107 | IQCA1_2 | 0.0312 | NUP155 _1 | 0.0294 | SLC4A5_3 | 0.0875 |
| ATXN10_1 | 0.1976 | EEF1E1_1 | 0.1213 | KCNIP3_1 | 0.1025 | NYX | 0.1243 | SLC9A10 | 0.0723 |
| AXL_1 | 0.0838 | EMP1 | 0.1142 | KCNQ2_1 | 0.1155 | ODF2_3 | 0.0249 | SNORD93 | 0.1242 |
| BAII_3 | 0.0217 | ENOI | 0.1996 | KIF3C | 0.1607 | ORC1L | 0.024 | SOX2_1 | 0.0772 |
| BCAS1_1 | 0.3211 | ENPEP_2 | 0.1619 | KRT80_2 | 0.1105 | OTUD7A_3 | 0.0485 | STC1 | 0.005 |
| BDNF_2 | 0.1348 | EPHB1 | 0.0395 | KRTAP10.10_2 | 0.0262 | PANK4 | 0.0507 | STC2 | 0.1287 |
| BMPR1A | 0.1172 | EPYC | 0.0303 | L3MBTL2_3 | 0.0524 | PDLIM2_2 | 0.215 | STYX 2 | 0.0175 |
| BTF3_3 | 0.1122 | ERI2_2 | 0.2787 | LBH_2 | 0.0853 | PDZRN4_2 | 0.2106 | SYTL3 | 0.0242 |
| C10orfII6 | 0.0744 | ESPNL | 0.0527 | LENEP | 0.2303 | PHYH 1 | 0.0083 | TAF15 1 | 0.0297 |
| C11orf24 | 0.1946 | EZH2_1 | 0.0572 | LGI3 | 0.0888 | PIGA 1 | 0.0914 | TCEAL8_1 | 0.024 |
| C11orf49_3 | 0.1039 | FAM13AOS | 0.0779 | LOC340508 | 0.0384 | PITX2_1 | 0.2038 | THBS3 | 0.1003 |
| C14orf102_2 | 0.1077 | FAM187B_2 | 0.0084 | LOC492303 | 0.0229 | PKN1_3 | 0.0132 | TM2D3_2 | 0.0396 |
| C14orf109_2 | 0.1196 | FAM70A_1 | 0.0738 | LRRC14B | 0.0792 | PLEKHG5_5 | 0.247 | TMEM52 | 0.099 |
| C17orf106 | 0.1789 | FBXO48 2 | 0.2285 | LRRC37A4 2 | 0.0204 | PLSCR4 | 0.0201 | TMEM62 | 0.0101 |
| C17orf58_2 | 0.0085 | FKBP10 | 0.0816 | LRRTM4 | 0.1778 | PMEPA1 4 | 0.1369 | TNFRSF18_1 | 0.2172 |
| C17orf58_3 | 0.0167 | FLJ33360 | 0.0127 | MACC1 | 0.1575 | PNMA5 | 0.1684 | TNNT2_1 | 0.0065 |
| C18orf56 | 0.0009 | FLJ43752 | 0.2482 | MANSC1_1 | 0.1242 | PPAPDC1A | 0.1058 | TOMM20L | 0.0067 |
| C1orf168 | 0.038 | FMNL3_2 | 0.001 | MCAM | 0.0185 | PRAMEF5 | 0.016 | TPM2_2 | 0.1822 |
| C1orf64 | 0.1189 | FOSB | 0.2151 | MCART6_1 | 0.1265 | PRKAA2 | 0.1326 | TRIM58 | 0.1077 |

(continued)

| | | | | | | | | |
|---|---|---|---|---|---|---|---|---|
| C8orf79_1 | 0.0219 | FOSL2 | 0.0328 | MFRP | 0.2275 | PSMC6_1 | 0.038 | UBR7_1 | 0.0832 |
| CALD1_2 | 0.1263 | FOXN1 | 0.2578 | MIDN | 0.0068 | RAD54B_2 | 0.1625 | UBR7_2 | 0.1286 |
| CASP8AP2 | 0.1316 | GAD1_2 | 0.0252 | MIR1914 | 0.0485 | RAP1A _ 1 | 0.2013 | WARS_2 | 0.1735 |
| CCL13 | 0.1129 | GBE1 | 0.0495 | MIR212 | 0.0913 | RARA_3 | 0.0969 | XBP1_2 | 0.1339 |
| CCR2_3 | 0.0422 | GBP7 | 0.1388 | MIR571 | 0.003 | RARG | 0.0689 | XRN2_1 | 0.0576 |
| CD34_1 | 0.0328 | GJA5_1 | 0.0702 | MIR576 | 0.1087 | RNASEK | 0.0856 | YARS2 | 0.0344 |
| CDC42BPA_2 | 0.0062 | GMNN | 0.1019 | MIR654 | 0.0426 | RNF7 1 | 0.0228 | ZNF75D_2 | 0.1385 |
| CDC42SE2_2 | 0.0047 | GSR 2 | 0.0348 | MIR942 | 0.1113 | ROD1_1 | 0.1961 | ZSWIM4 _ 2 | 0.1769 |
| CIDEC_1 | 0.1007 | HBA2 | 0.2093 | MMP12_1 | 0.1231 | SATB2 | 0.0377 | figo_numeric | 0.012 |
| CLDN6 | 0.0092 | HCFC1R1_1 | 0.0638 | MYCN_2 | 0.1306 | SBSN | 0.0676 | hist rev SBOT | 0.0396 |
| CREB5_2 | 0.0117 | HDAC7_2 | 0.0111 | MYOHD1 | 0.1081 | SCXB | 0.0075 | surg outcome | 0.0149 |
| CRYBA1 | 0.0523 | HDLBP_ 3 | 0.1043 | NFATC3_5 | 0.0114 | SEC22C_3 | 0.1025 | | |
| CXCL13 | 0.0657 | HIC1 | 0.007 | NFATC4 | 0.0383 | SELENBP1 | 0.1466 | | |

Table 38

| Gene | Value | Gene | Value | Gene | Value | Gene | Value | Gene | Value |
|---|---|---|---|---|---|---|---|---|---|
| ABCC9 3 | 0.0424 | CXCL13 | 0.0744 | HIC1 | 0.0015 | NFATC4 | 0.0371 | SERPINB2_2 | 0.0047 |
| ABHD3 | 0.2496 | CYB5R3_2 | 0.188 | HPRT1_1 | 0.1391 | NLRP9 | 0.1828 | SERPINB5 | 0.1987 |
| ADAM17_2 | 0.1599 | CYP1A2 | 0.0735 | HPS4 1 | 0.0719 | NOVA2 | 0.1187 | SFN | 0.0351 |
| ADAMTS1 | 0.1341 | DBNDD2 | 0.1055 | HR_1 | 0.0492 | NP | 0.0913 | SFRS4 | 0.0644 |
| ADAMTS2_1 | 0.1074 | DNAH11 | 0.033 | HSD11B1_1 | 0.08 | NR6A1_2 | 0.1321 | SHC1_3 | 0.0707 |
| ALS2CL_3 | 0.0646 | DNMT3L_2 | 0.0192 | ICAM2 | 0.0001 | NRXN3_3 | 0.121 | SLC23A1_2 | 0.1554 |
| ANO7_3 | 0.0491 | DOCK7_1 | 0.1234 | ICAM4_1 | 0.2621 | NT5DC1_2 | 0.1775 | SLC25A34 | 0.1192 |
| ARL6IP1_1 | 0.0019 | DSC3_1 | 0.0459 | IL1RAP_2 | 0.0496 | NTRK2_3 | 0.0178 | SLC4A5_3 | 0.0757 |
| ARMCX3_2 | 0.0757 | DUT_3 | 0.1053 | IQCA1_2 | 0.0424 | NUP155_1 | 0.0047 | SLC9A10 | 0.1008 |
| ATXN10_1 | 0.2048 | EEF1E1_1 | 0.1021 | KCNIP3_1 | 0.0947 | NYX | 0.1288 | SNORD93 | 0.1567 |
| AXL_1 | 0.0987 | EMP1 | 0.1095 | KCNQ2_1 | 0.1222 | ODF2_3 | 0.0161 | SOX2_1 | 0.0799 |
| BAII_3 | 0.0324 | ENOI | 0.1947 | KIF3C | 0.1963 | ORC1L | 0.0232 | STC1 | 0.0106 |
| BCAS1_1 | 0.3401 | ENPEP_2 | 0.148 | KRT80_2 | 0.1123 | OTUD7A_3 | 0.0454 | STC2 | 0.1382 |
| BDNF_2 | 0.1591 | EPHB1 | 0.0575 | KRTAP10.10_2 | 0.0199 | PANK4 | 0.0492 | STYX_2 | 0.0405 |
| BMPR1A | 0.1264 | EPYC | 0.0338 | L3MBTL2_3 | 0.0511 | PDLIM2_2 | 0.2231 | SYTL3 | 0.0078 |
| BTF3_3 | 0.1119 | ERI2_2 | 0.298 | LBH_2 | 0.0973 | PHYH_1 | 0.1936 | TAF15_1 | 0.0154 |
| C10orf116 | 0.0343 | ESPNL | 0.048 | LENEP | 0.2515 | PIGA_1 | 0.0078 | TCEAL8_1 | 0.0147 |
| C11orf24 | 0.2059 | EZH2_1 | 0.0645 | LGI3 | 0.1002 | PITX2_1 | 0.0748 | THBS3 | 0.1018 |
| C11orf49_3 | 0.1412 | FAM13AOS | 0.0394 | LOC340508 | 0.0293 | PKN1_3 | 0.0305 | TM2D3_2 | 0.058 |
| C14orf102_2 | 0.1018 | FAM187B_2 | 0.0083 | LOC492303 | 0.0123 | PLEKHG5_5 | 0.26 | TMEM52 | 0.1205 |
| C14orf109_2 | 0.0736 | FAM70A_1 | 0.0736 | LRRC14B | 0.0733 | PLSCR4 | 0.0469 | TMEM62 | 0.0022 |
| CI7orf106 | 0.1945 | FBXO48_2 | 0.2346 | LRRC37A4_2 | 0.0007 | PMEPA1_4 | 0.1514 | TNFRSF18_1 | 0.246 |
| C17orf58_2 | 0.0062 | FKBP10 | 0.0639 | LRRTM4 | 0.1658 | PNMA5 | 0.1499 | TNNT2_1 | 0.0012 |
| C17orf58_3 | 0.0227 | FLJ33360 | 0.0259 | MACC1 | 0.1345 | PPAPDC1A | 0.136 | TOMM20L | 0.0383 |
| C18orf56 | 0.0333 | FLJ43752 | 0.2398 | MANSC1_1 | 0.146 | PRAMEF5 | 0.0069 | TPM2_2 | 0.1829 |
| C1orf168 | 0.0383 | FMNL3_2 | 0.0212 | MCAM | 0.0157 | PRKAA2 | 0.126 | TRIM58 | 0.1059 |

| | | | | | | | |
|---|---|---|---|---|---|---|---|
| C1orf64 | 0.1355 | FOSB | 0.202 | MCART6_1 | 0.1389 | PSMC6_1 | 0.0339 | UBR7_1 | 0.0435 |
| C8orf79_1 | 0.0285 | FOSL2 | 0.0377 | MFRP | 0.2154 | RAD54B_2 | 0.1854 | UBR7_2 | 0.1202 |
| CALD1_2 | 0.1427 | FOXN1 | 0.2908 | MIDN | 0.0075 | RAP1A_1 | 0.2213 | WARS_2 | 0.1523 |
| CASP8AP2 | 0.1302 | GAD1_2 | 0.0145 | MIR1914 | 0.0498 | RARA_3 | 0.0912 | XBP1_2 | 0.1057 |
| CCL13 | 0.1286 | GBE1 | 0.0505 | MIR212 | 0.1042 | RARG | 0.043 | XRN2_1 | 0.0367 |
| CCR2_3 | 0.0076 | GBP7 | 0.1583 | MIR571 | 0.0109 | RNASEK | 0.0424 | YARS2 | 0.0092 |
| CD34_1 | 0.0375 | GJA5_1 | 0.0568 | MIR576 | 0.1081 | RNF7_1 | 0.0342 | ZNF75D_2 | 0.1434 |
| CDC42BPA_2 | 0.0167 | GMNN | 0.0856 | MIR654 | 0.029 | ROD1_1 | 0.2221 | ZSWIM4_2 | 0.1799 |
| CDC42SE2_2 | 0.0106 | GSR_2 | 0.0439 | MIR942 | 0.111 | SATB2 | 0.0456 | figo_numeric | 0.0132 |
| CIDEC_1 | 0.1188 | HBA2 | 0.2032 | MMP12_1 | 0.1258 | SBSN | 0.0832 | hist_rev_SBOT | 0.0424 |
| CLDN6 | 0.0114 | HCFC1R1_1 | 0.0689 | MYCN_2 | 0.1659 | SCXB | 0.0132 | surg_outcome | 0.0264 |
| CREB5_2 | 0.0509 | HDAC7_2 | 0.007 | MYOHD1 | 0.115 | SEC22C_3 | 0.106 | | |
| CRYBA1 | 0.0391 | HDLBP_3 | 0.107 | NFATC3_5 | 0.0204 | SELENBP1 | 0.1769 | | |

Table 39

| Gene | Value | Gene | Value | Gene | Value | Gene | Value | Gene | Value |
|---|---|---|---|---|---|---|---|---|---|
| ABCC9_3 | 0.0437 | CYB5R3_2 | 0.1779 | HPRT1_1 | 0.1297 | NLRP9 | 0.1917 | SERPINB2_2 | 0.0145 |
| ABHD3 | 0.2335 | CYP1A2 | 0.0781 | HPS4_1 | 0.0428 | NOVA2 | 0.0861 | SERPINB5 | 0.2 |
| ADAM17_2 | 0.1471 | DBNDD2 | 0.1158 | HR_1 | 0.0359 | NP | 0.0807 | SFN | 0.0027 |
| ADAMTS1 | 0.125 | DNAH11 | 0.0338 | HSD11B1_1 | 0.0878 | NR6A1_2 | 0.1299 | SFRS4 | 0.0606 |
| ADAMTS2_1 | 0.1082 | DNMT3L_2 | 0.035 | ICAM2 | 0.0247 | NRXN3_3 | 0.1635 | SHC1_3 | 0.0783 |
| ALS2CL_3 | 0.0673 | DOCK7_1 | 0.1459 | ICAM4_1 | 0.2693 | NT5DC1_2 | 0.1893 | SLC23A1_2 | 0.1316 |
| ANO7_3 | 0.028 | DSC3_1 | 0.0563 | IL1RAP_2 | 0.084 | NTRK2_3 | 0.0237 | SLC25A34 | 0.1141 |
| ARL6IP1_1 | 0.0196 | DUT_3 | 0.1267 | IQCA1_2 | 0.053 | NUP155_1 | 0.0329 | SLC4A5_3 | 0.0799 |
| ARMCX3_2 | 0.0532 | EEF1E1_1 | 0.1117 | KCNIP3_1 | 0.1079 | NYX | 0.1176 | SLC9A10 | 0.0728 |
| ATXN10_1 | 0.2092 | EMP1 | 0.11 | KCNQ2_1 | 0.1233 | ODF2_3 | 0.0268 | SNORD93 | 0.1344 |
| AXL_1 | 0.0898 | ENOI | 0.2058 | KIF3C | 0.1757 | ORC1L | 0.0328 | SOX2_1 | 0.0773 |
| BAII_3 | 0.0149 | ENPEP_2 | 0.1652 | KRT80_2 | 0.114 | OTUD7A_3 | 0.0567 | STC1 | 0.0038 |
| BCAS1_1 | 0.3127 | EPHB1 | 0.032 | KRTAP10.10_2 | 0.0114 | PANK4 | 0.0489 | STC2 | 0.1182 |
| BDNF_2 | 0.1379 | EPYC | 0.0339 | L3MBTL2_3 | 0.0448 | PDLIM2_2 | 0.2186 | STYX_2 | 0.0238 |
| BMPR1A | 0.1149 | ERI2_2 | 0.2901 | LBH_2 | 0.092 | PDZRN4_2 | 0.2162 | SYTL3 | 0.0103 |
| BTF3_3 | 0.107 | ESPNL | 0.0731 | LENEP | 0.2239 | PHYH_1 | 0.0042 | TAF15_1 | 0.0148 |
| C10orf116 | 0.0559 | EZH2_1 | 0.0436 | LGI3 | 0.0908 | PIGA_1 | 0.1044 | TCEAL8_1 | 0.033 |
| C11orf24 | 0.1941 | FAM13AOS | 0.0793 | LOC340508 | 0.0562 | PITX2_1 | 0.1952 | THBS3 | 0.0835 |
| C11orf49_3 | 0.1089 | FAM187B_2 | 0.0196 | LOC492303 | 0.02 | PKN1_3 | 0.0181 | TM2D3_2 | 0.0401 |
| C14orf102_2 | 0.0951 | FAM70A_1 | 0.0644 | LRRC14B | 0.0937 | PLEKHG5_5 | 0.2534 | TMEM52 | 0.099 |
| C14orf109_2 | 0.1318 | FBXO48_2 | 0.2315 | LRRC37A4_2 | 0.0203 | PLSCR4 | 0.031 | TMEM62 | 0.0043 |
| C17orf10_ | 0.1848 | FKBP10 | 0.0873 | LRRTM4 | 0.198 | PMEPA1_4 | 0.1353 | TNFRSF18_1 | 0.2257 |
| C17orf58_2 | 0.0402 | FLJ33360 | 0.0106 | MACC1 | 0.1688 | PNMA5 | 0.1673 | TNNT2_1 | 0.0041 |
| C17orf58_3 | 0.0224 | FLJ43752 | 0.2561 | MANSC1_1 | 0.1222 | PPAPDC1A | 0.1097 | TOMM20L | 0.0004 |
| C18orf56 | 0.003 | FMNL3_2 | 0.0038 | MCAM | 0.0005 | PRAMEF5 | 0.0097 | TPM2_2 | 0.1766 |
| C1orf168 | 0.047 | FOSB | 0.2306 | MCART6_1 | 0.1271 | PRKAA2 | 0.0972 | TRIM58 | 0.1115 |

| | | | | | | | | | |
|---|---|---|---|---|---|---|---|---|---|
| C1orf64 | 0.1194 | FOSL2 | 0.025 | MFRP | 0.2211 | PSMC6_1 | 0.0129 | UBR7_1 | 0.0699 |
| C8orf79_1 | 0.0394 | FOXN1 | 0.2475 | MIDN | 0.008 | RAD54B_2 | 0.1676 | UBR7_2 | 0.1313 |
| CALD1_2 | 0.1148 | GAD1_2 | 0.0174 | MIR1914 | 0.0703 | RAP1A_1 | 0.2097 | WARS_2 | 0.1744 |
| CASP8AP2 | 0.122 | GBE1 | 0.0637 | MIR212 | 0.0928 | RARA_3 | 0.0864 | XBP1_2 | 0.1496 |
| CCL_13 | 0.1135 | GBP7 | 0.1588 | MIR571 | 0.0125 | RARG | 0.0705 | XRN2_1 | 0.0279 |
| CCR2_3 | 0.0454 | GJA5_1 | 0.0467 | MIR576 | 0.114 | RNASEK | 0.0784 | YARS2 | 0.012 |
| CD34_1 | 0.0186 | GMNN | 0.0908 | MIR654 | 0.0306 | RNF7_1 | 0.0122 | ZNF75D_2 | 0.1209 |
| CDC42BPA_2 | 0.0209 | GSR_2 | 0.028 | MIR942 | 0.1136 | ROD1_1 | 0.2194 | ZSWIM4 2 | 0.1681 |
| CDC42SE2_2 | 0.0152 | HBA2 | 0.2021 | MMP12_1 | 0.1152 | SATB2 | 0.0246 | figo_numeric | 0.0044 |
| CLDN6 | 0.1179 | HCFC1R1_1 | 0.0685 | MYCN_2 | 0.1162 | SBSN | 0.0546 | hist_rev_SBOT | 0.0511 |
| CREB5_2 | 0.0171 | HDAC7_2 | 0.0048 | MYOHD1 | 0.1035 | SCXB | 0.0042 | surg_outcome | 0.0121 |
| CRYBA1 | 0.0193 | HDLBP_3 | 0.1149 | NFATC3_5 | 0.0005 | SEC22C_3 | 0.0938 | | |
| CXCL13 | 0.068 | HIC1 | 0.0175 | NFATC4 | 0.0387 | SELENBP1 | 0.1442 | | |

Table 40

| | | | | | | | | |
|---|---|---|---|---|---|---|---|---|
| ABCC9_3 | 0.0533 | CXCL13 | 0.0652 | HIC1 | 0.0059 | NLRP9 | 0.1901 | SERPINB2 2 | 0.0024 |
| ABHD3 | 0.2416 | CYB5R3 2 | 0.1903 | HPRT1_1 | 0.123 | NOVA2 | 0.0877 | SERPINB5 | 0.1847 |
| ADAM17_2 | 0.148 | CYPIA2 | 0.0627 | HPS4_1 | 0.0724 | NP | 0.0868 | SFN | 0.0027 |
| ADAMTS1 | 0.112 | DBNDD2 | 0.1258 | HR_1 | 0.0282 | NR6A1_2 | 0.1293 | SFRS4 | 0.0691 |
| ADAMTS2_1 | 0.0961 | DNAH11 | 0.0411 | HSD11B1_1 | 0.0849 | NRXN3_3 | 0.163 | SHC1_3 | 0.0782 |
| ALS2CL 3 | 0.0628 | DNMT3L_2 | 0.0282 | ICAM2 | 0.0088 | NT5DC1 2 | 0.1897 | SLC23A1_2 | 0.1364 |
| ANO7_3 | 0.0498 | DOCK7_1 | 0.1161 | ICAM4_1 | 0.2845 | NTRK2 3 | 0.0079 | SLC25A34 | 0.1162 |
| ARL6IP1_1 | 0.0137 | DSC3_1 | 0.0478 | IL1RAP_2 | 0.0729 | NUP155 1 | 0.0268 | SLC4A5_3 | 0.0874 |
| ARMCX3_2 | 0.0685 | DUT_3 | 0.1115 | IQCA1_2 | 0.0317 | NYX | 0.1178 | SLC9A10 | 0.0726 |
| ATXN10_1 | 0.1957 | EEF1E1_1 | 0.1222 | KCNIP3_1 | 0.102 | ODF2_3 | 0.0219 | SNORD93 | 0.1248 |
| AXL_1 | 0.0829 | EMP1 | 0.116 | KCNQ2_1 | 0.1156 | ORC1L | 0.0235 | SOX2_1 | 0.0778 |
| BAII_3 | 0.0209 | ENOI | 0.1972 | KIF3C | 0.1639 | OTUD7A 3 | 0.0497 | STC1 | 0.0055 |
| BCAS1_1 | 0.3223 | ENPEP_2 | 0.1664 | KRT80_2 | 0.11 | PANK4 | 0.0507 | STC2 | 0.1283 |
| BDNF_2 | 0.1353 | EPHB1 | 0.0401 | KRTAP10.10_2 | 0.0243 | PDLIM2 2 | 0.2123 | STYX_2 | 0.0171 |
| BMPR1A | 0.1158 | EPYC | 0.0303 | L3MBTL2_3 | 0.0525 | PDZRN4 2 | 0.2088 | SYTL3 | 0.0246 |
| BTF3_3 | 0.1138 | ERI2 2 | 0.2829 | LBH_2 | 0.0857 | PHYH 1 | 0.0108 | TAF15_1 | 0.0303 |
| C10orfII6 | 0.0743 | ESPNL | 0.0543 | LENEP | 0.233 | PIGA 1 | 0.0936 | TCEAL8_1 | 0.0237 |
| C11orf24 | 0.1957 | EZH2_1 | 0.0546 | LGI3 | 0.0878 | PITX2_1 | 0.2057 | THBS3 | 0.102 |
| C11orf49_3 | 0.102 | FAM13AOS | 0.0791 | LOC492303 | 0.0373 | PKN1_3 | 0.0116 | TM2D3_2 | 0.0399 |
| C14orf102_2 | 0.1078 | FAM187B_2 | 0.0105 | LRRC14B | 0.025 | PLEKHG5_5 | 0.2467 | TMEM52 | 0.1032 |
| C14orf109_2 | 0.1201 | FAM70A_1 | 0.0714 | LRRC37A4_2 | 0.0794 | PLSCR4 | 0.0204 | TMEM62 | 0.0084 |
| C17orf106 | 0.1726 | FBXO48_2 | 0.2243 | LRRTM4 | 0.179 | PMEPA1_4 | 0.1344 | TNFRSF18_1 | 0.2162 |
| C17orf58_2 | 0.0099 | FKBP10 | 0.081 | MACC1 | 0.1568 | PNMA5 | 0.1709 | TNNT2_1 | 0.0037 |
| C17orf58 3 | 0.0145 | FLJ33360 | 0.0135 | MANSC1_1 | 0.1233 | PPAPDC1A | 0.1055 | TOMM20L | 0.0051 |
| C18orf56 | 0.0003 | FLJ43752 | 0.2485 | MCAM | 0.0164 | PRAMEF5 | 0.0152 | TPM2 2 | 0.1824 |
| Clorfl68 | 0.0389 | FMNL3_2 | 0.0005 | MCART6_1 | 0.1279 | PRKAA2 | 0.133 | TRIM58 | 0.1067 |

| C1orf64 | 0.1191 | FOSB | 0.2147 | MFRP | 0.2234 | PSMC6 1 | 0.04 | UBR7_1 | 0.084 |
|---|---|---|---|---|---|---|---|---|---|
| C8orf79_1 | 0.0166 | FOSL2 | 0.0333 | MIDN | 0.008 | RAD54B_2 | 0.1622 | UBR7_2 | 0.1307 |
| CALD1_2 | 0.1284 | FOXN1 | 0.2566 | MIR1914 | 0.0516 | RAP1A 1 | 0.2022 | WARS_2 | 0.176 |
| CASP8AP2 | 0.1304 | GAD1_2 | 0.0249 | MIR212 | 0.0933 | RARA_3 | 0.0968 | XBP1_2 | 0.1358 |
| CCL13 | 0.1154 | GBE1 | 0.0473 | MIR571 | 0.0013 | RARG | 0.0719 | XRN2_1 | 0.0599 |
| CCR2_3 | 0.0417 | GBP7 | 0.1373 | MIR576 | 0.1094 | RNASEK | 0.0821 | YARS2 | 0.034 |
| CD34_1 | 0.0328 | GJA5_1 | 0.0723 | MIR654 | 0.0443 | RNF7_1 | 0.0257 | ZNF75D_2 | 0.1361 |
| CDC42BPA 2 | 0.0034 | GMNN | 0.1036 | MIR942 | 0.1108 | ROD1_1 | 0.1967 | ZSWIM4_2 | 0.1774 |
| CDC42SE2_2 | 0.0074 | GSR_2 | 0.0336 | MMP12_1 | 0.1245 | SATB2 | 0.0371 | figo_numeric | 0.0096 |
| CIDEC_1 | 0.1011 | HBA2 | 0.2112 | MYCN_2 | 0.1301 | SBSN | 0.0678 | hist rev SBOT | 0.0385 |
| CLDN6 | 0.0107 | HCFC1R1_1 | 0.061 | MYOHD1 | 0.1094 | SCXB | 0.0068 | surg outcome | 0.0116 |
| CREB5_2 | 0.0106 | HDAC7_2 | 0.0082 | NFATC3 _ 5 | 0.0121 | SEC22C_3 | 0.1023 | | |
| CRYBA1 | 0.0538 | HDLBP_3 | 0.102 | NFATC4 | 0.0385 | SELENBP1 | 0.1462 | | |

Table 41

| | | | | | | | | | |
|---|---|---|---|---|---|---|---|---|---|
| ABCC9_3 | 0.0397 | CYB5R3_2 | 0.1766 | HIC1 | 0.0001 | NFATC4 | 0.0346 | SERPINB2 2 | 0.0034 |
| ABHD3 | 0.2499 | CYP1A2 | 0.0889 | HPRT1 1 | 0.1525 | NLRP9 | 0.1737 | SERPINB5 | 0.1921 |
| ADAM17 2 | 0.1539 | DBNDD2 | 0.108 | HPS4 1 | 0.0655 | NOVA2 | 0.104 | SFN | 0.0433 |
| ADAMTS1 | 0.142 | DNAH11 | 0.0306 | HR 1 | 0.0481 | NP | 0.077 | SFRS4 | 0.0632 |
| ALS2CL 3 | 0.1129 | DNMT3L_2 | 0.0143 | HSD11B1 1 | 0.083 | NR6A1 2 | 0.1329 | SHC1_3 | 0.0668 |
| ANO7_3 | 0.059 | DOCK7_1 | 0.1172 | ICAM2 | 0.012 | NRXN3 3 | 0.1299 | SLC23A1_2 | 0.1474 |
| ARL6IP1 1 | 0.0407 | DSC3 1 | 0.0472 | ICAM4 1 | 0.2696 | NT5DC1 2 | 0.1761 | SLC25A34 | 0.1086 |
| ARMCX3 2 | 0.0754 | DUT 3 | 0.1225 | IL1RAP 2 | 0.0469 | NTRK2 3 | 0.0155 | SLC4A5 3 | 0.0741 |
| ATXN10 1 | 0.2072 | EEF1E1_1 | 0.1071 | IQCA1_2 | 0.0363 | NUP155 1 | 0.0032 | SLC9A10 | 0.098 |
| AXL 1 | 0.0942 | ELN 2 | 0.1114 | KCNIP3 1 | 0.0911 | NYX | 0.1139 | SNORD93 | 0.1599 |
| BAII 3 | 0.0426 | EMP1 | 0.2017 | KCNQ2_1 | 0.1135 | ODF2 3 | 0.0109 | SOX2 1 | 0.0826 |
| BCAS1 1 | 0.3299 | ENOI | 0.1477 | KIF3C | 0.2112 | ORC1L | 0.0328 | STC1 | 0.0136 |
| BDNF 2 | 0.1511 | ENPEP 2 | 0.0718 | KRT80 2 | 0.1004 | OTUD7A 3 | 0.0381 | STC2 | 0.1175 |
| BMPR1A | 0.1229 | EPHB1 | 0.0599 | KRTAP10.10_2 | 0.0162 | PANK4 | 0.0477 | STYX 2 | 0.0395 |
| BTF3 3 | 0.108 | EPYC | 0.0354 | L3MBTL2 3 | 0.0447 | PDLIM2 2 | 0.2231 | SYTL3 | 0.0075 |
| C10orfll6 | 0.0296 | ERI2 2 | 0.2846 | LBH 2 | 0.0936 | PHYH 1 | 0.1928 | TAF15 1 | 0.0141 |
| C11orf24 | 0.2047 | ESPNL | 0.0508 | LENEP | 0.2514 | PIGA 1 | 0.0149 | TCEAL8 1 | 0.0075 |
| C11orf49_3 | 0.1498 | EZH2_1 | 0.0488 | LGI3 | 0.1011 | PITX2_1 | 0.0749 | THBS3 | 0.0959 |
| C14orf102_2 | 0.1044 | FAM13AOS | 0.0304 | LOC340508 | 0.0265 | PKN1 3 | 0.0208 | TM2D3 2 | 0.055 |
| C14orf109_2 | 0.0708 | FAM187B_2 | 0.0104 | LOC492303 | 0.0131 | PLEKHG5_5 | 0.2748 | TMEM52 | 0.1215 |
| C17orf106 | 0.1763 | FAM70A_1 | 0.0757 | LRRC14B | 0.0724 | PLSCR4 | 0.0429 | TMEM62 | 0.0099 |
| C17orf58_2 | 0.0123 | FBXO48_2 | 0.2353 | LRRC37A4 _ 2 | 0.0026 | PMEPA1_4 | 0.1469 | TNFRSF18_1 | 0.256 |
| C17orf58_3 | 0.0281 | FKBP10 | 0.0533 | LRRTM4 | 0.1641 | PNMA5 | 0.1504 | TNNT2_1 | 0.0068 |
| Cl8orf56 | 0.029 | FLJ33360 | 0.0322 | MACC1 | 0.1444 | PPAPDC1A | 0.1486 | TOMM20L | 0.0466 |
| Clorf168 | 0.0419 | FLJ43752 | 0.2425 | MANSC1 1 | 0.1437 | PRAMEF5 | 0.0147 | TPM2 2 | 0.1813 |
| Clorf64 | 0.1374 | FMNL3_2 | 0.0113 | MCAM | 0.0178 | PRKAA2 | 0.1132 | TRIM58 | 0.1118 |

| | | | | | | | |
|---|---|---|---|---|---|---|---|
| C8orf79_1 | 0.0234 | FOSB | 0.2125 | MCART6 1 | 0.1369 | PSMC6 1 | 0.0322 | UBR7 1 | 0.0387 |
| CALD1_2 | 0.1552 | FOSL2 | 0.0292 | MFRP | 0.2153 | RAD54B _ 2 | 0.192 | UBR7 2 | 0.1325 |
| CASP8AP2 | 0.1138 | FOXN1 | 0.2988 | MIDN | 0.0203 | RAP1A 1 | 0.2103 | WARS 2 | 0.1551 |
| CCL 13 | 0.1448 | GAD1_2 | 0.0126 | MIR1914 | 0.0513 | RARA 3 | 0.0895 | XBP1 2 | 0.1108 |
| CCR2 3 | 0.0026 | GBE1 | 0.0504 | MIR212 | 0.1066 | RARG | 0.0525 | XRN2_1 | 0.0171 |
| CD34 1 | 0.037 | GBP7 | 0.1549 | MIR571 | 0.0077 | RNASEK | 0.0326 | YARS2 | 0.0048 |
| CDC42BPA 2 | 0.0056 | GJA5 1 | 0.0538 | MIR576 | 0.1208 | RNF7 1 | 0.0412 | ZNF75D 2 | 0.1391 |
| CDC42SE2 2 | 0.0015 | GMNN | 0.082 | MIR654 | 0.024 | ROD1_1 | 0.2198 | ZSWIM4_2 | 0.1784 |
| CIDEC 1 | 0.1194 | GSR 2 | 0.0361 | MIR942 | 0.1037 | SATB2 | 0.0405 | figo_numeric | 0.0128 |
| CLDN6 | 0.013 | HBA2 | 0.1962 | MMP12 1 | 0.1228 | SBSN | 0.0882 | hist rev SBOT | 0.0481 |
| CREB5 2 | 0.0427 | HCFC1R1_1 | 0.0678 | MYCN 2 | 0.1558 | SCXB | 0.0176 | surg outcome | 0.0218 |
| CRYBA1 | 0.0429 | HDAC7 2 | 0.0126 | MYOHD1 | 0.1153 | SEC22C_3 | 0.105 | | |
| CXCL13 | 0.0699 | HDLBP 3 | 0.0981 | NFATC3 5 | 0.0349 | SELENBP1 | 0.173 | | |

EP 3 874 274 B1

87

Table 42

| | | | | | | | | | |
|---|---|---|---|---|---|---|---|---|---|
| ABCC9_3 | 0.0425 | CYP1A2 | 0.0925 | HPRT1_1 | 0.141 | NLRP9 | 0.1836 | SERPINB2_2 | 0.0093 |
| ABHD3 | 0.2305 | DBNDD2 | 0.1199 | HPS4 1 | 0.0305 | NOVA2 | 0.0788 | SERPINB5 | 0.1985 |
| ADAM17 2 | 0.1466 | DNAH11 | 0.0324 | HR 1 | 0.0314 | NP | 0.0729 | SFN | 0.0125 |
| ADAMTS1 | 0.1315 | DNMT3L_2 | 0.0295 | HSD11B1_1 | 0.09 | NR6A1 _ 2 | 0.132 | SFRS4 | 0.0619 |
| ALS2CL 3 | 0.1149 | DOCK7_1 | 0.1454 | ICAM2 | 0.0303 | NRXN3 _ 3 | 0.1687 | SHC1_3 | 0.0786 |
| ANO7 3 | 0.0659 | DSC3_1 | 0.0494 | ICAM4_1 | 0.2776 | NT5DC1_2 | 0.1873 | SLC23A1_2 | 0.1282 |
| ARL6IP1 1 | 0.0178 | DUT_3 | 0.1321 | IL1RAP 2 | 0.0888 | NTRK2_3 | 0.0257 | SLC25A34 | 0.1047 |
| ARMCX3 2 | 0.0467 | EEF1E1_1 | 0.1159 | IQCA1_2 | 0.0508 | NUP155 _ 1 | 0.03 | SLC4A5 _ 3 | 0.0788 |
| ATXN10 1 | 0.216 | ELN 2 | 0.1108 | KCNIP3 1 | 0.0998 | NYX | 0.1113 | SLC9A10 | 0.0695 |
| AXL 1 | 0.0883 | EMP1 | 0.2116 | KCNQ2_1 | 0.1103 | ODF2_3 | 0.023 | SNORD93 | 0.1365 |
| BAII 3 | 0.0263 | ENOI | 0.1609 | KIF3C | 0.1865 | ORC1L | 0.0393 | SOX2 1 | 0.0821 |
| BCAS1 1 | 0.3029 | ENPEP_2 | 0.0584 | KRT80_2 | 0.1084 | OTUD7A_3 | 0.0605 | STC1 | 0.0002 |
| BDNF 2 | 0.1326 | EPHB1 | 0.0334 | KRTAP10.10_2 | 0.0109 | PANK4 | 0.0488 | STC2 | 0.1076 |
| BMPR1A | 0.1149 | EPYC | 0.0371 | L3MBTL2_3 | 0.0423 | PDLIM2_2 | 0.224 | STYX 2 | 0.0213 |
| BTF3 3 | 0.1015 | ERI2_2 | 0.2778 | LBH_2 | 0.0868 | PDZRN4_2 | 0.2142 | SYTL3 | 0.0124 |
| C10orfl6 | 0.0584 | ESPNL | 0.0754 | LENEP | 0.2223 | PHYH_1 | 0.0013 | TAF15 1 | 0.0116 |
| C11orf24 | 0.1867 | EZH2_1 | 0.0275 | LGI3 | 0.0912 | PIGA 1 | 0.1039 | TCEAL8 1 | 0.0282 |
| C11orf49_3 | 0.1161 | FAM13AOS | 0.074 | LOC340508 | 0.0526 | PITX2_1 | 0.1916 | THBS3 | 0.0768 |
| C14orf102_2 | 0.0909 | FAM187B_2 | 0.0166 | LOC492303 | 0.0173 | PKN1 3 | 0.0171 | TM2D3 2 | 0.035 |
| C14orf109_2 | 0.1302 | FAM70A_1 | 0.0699 | LRRC14B | 0.0959 | PLEKHG5_5 | 0.2654 | TMEM52 | 0.0977 |
| C17orf106 | 0.1793 | FBXO48_2 | 0.2364 | LRRC37A4_2 | 0.0175 | PLSCR4 | 0.0321 | TMEM62 | 0.0098 |
| C17orf58_2 | 0.0493 | FKBP10 | 0.0782 | LRRTM4 | 0.191 | PMEPA1_4 | 0.1345 | TNFRSF18_1 | 0.2255 |
| C17orf58_3 | 0.0259 | FLJ33360 | 0.0094 | MACC1 | 0.1757 | PNMA5 | 0.1658 | TNNT2_1 | 0.0087 |
| C18orf56 | 0.0048 | FLJ43752 | 0.253 | MANSC1_1 | 0.1188 | PPAPDC1A | 0.1172 | TOMM20L | 0.0036 |
| C1orf168 | 0.046 | FMNL3_2 | 0.0067 | MCAM | 0.004 | PRAMEF5 | 0.0033 | TPM2 2 | 0.1748 |
| C1orf64 | 0.1192 | FOSB | 0.2377 | MCART6_1 | 0.1223 | PRKAA2 | 0.0835 | TRIM58 | 0.1149 |

(continued)

| | | | | | | | | |
|---|---|---|---|---|---|---|---|---|
| C8orf79_1 | 0.0404 | FOSL2 | 0.0173 | MFRP | 0.2198 | PSMC6_1 | 0.0085 | UBR7 1 | 0.0621 |
| CALD1_2 | 0.1241 | FOXN1 | 0.2532 | MIDN | 0.0121 | RAD54B _ 2 | 0.1735 | UBR7 2 | 0.1383 |
| CASP8AP2 | 0.1146 | GAD1_2 | 0.0134 | MIR1914 | 0.0731 | RAP1A _ 1 | 0.202 | WARS 2 | 0.1778 |
| CCL 13 | 0.1245 | GBE1 | 0.0693 | MIR212 | 0.0946 | RARA_3 | 0.0836 | XBP1 2 | 0.1525 |
| CCR2 3 | 0.0408 | GBP7 | 0.1589 | MIR571 | 0.0141 | RARG | 0.0752 | XRN2_1 | 0.0126 |
| CD34 1 | 0.0143 | GJA5_1 | 0.0434 | MIR576 | 0.12 | RNASEK | 0.0797 | YARS2 | 0.0089 |
| CDC42BPA 2 | 0.0129 | GMNN | 0.0865 | MIR654 | 0.026 | RNF7_1 | 0.0084 | ZNF75D 2 | 0.1155 |
| CDC42SE2 2 | 0.0115 | GSR_2 | 0.0197 | MIR942 | 0.1063 | ROD1_1 | 0.2238 | ZSWIM4 2 | 0.165 |
| CLDN6 | 0.1193 | HBA2 | 0.1984 | MMP12 1 | 0.113 | SATB2 | 0.0195 | figo _numeric | 0.0013 |
| CREB5 2 | 0.0185 | HCFC1R1_1 | 0.0748 | MYCN 2 | 0.112 | SBSN | 0.0599 | hist rev SBOT | 0.0539 |
| CRYB AI | 0.0202 | HDAC7 2 | 0.0025 | MYOHD1 | 0.1003 | SCXB | 0.0079 | surg outcome | 0.0123 |
| CXCL13 | 0.0644 | HDLBP_3 | 0.1123 | NFATC3 _ 5 | 0.0061 | SEC22C_3 | 0.0985 | | |
| CYB5R3 2 | 0.1752 | HIC1 | 0.0216 | NFATC4 | 0.0379 | SELENBP1 | 0.141 | | |

Table 43

| ABCC9_3 | 0.0518 | CYB5R3_2 | 0.1875 | HIC1 | 0.0032 | NLRP9 | 0.1795 | SERPINB2_2 | 0.0054 |
|---|---|---|---|---|---|---|---|---|---|
| ABHD3 | 0.2416 | CYP1A2 | 0.0788 | HPRT1_1 | 0.1353 | NOVA2 | 0.0707 | SERPINB5 | 0.1773 |
| ADAM17 2 | 0.1421 | DBNDD2 | 0.1281 | HPS4 1 | 0.0631 | NP | 0.0758 | SFN | 0.0126 |
| ADAMTS1 | 0.1163 | DNAH11 | 0.0391 | HR 1 | 0.0243 | NR6A1_2 | 0.1303 | SFRS4 | 0.0664 |
| ALS2CL 3 | 0.1032 | DNMT3L_2 | 0.0233 | HSD11B1_1 | 0.0892 | NRXN3 _3 | 0.1671 | SHC1_3 | 0.0738 |
| ANO7 3 | 0.0577 | DOCK7_1 | 0.1142 | ICAM2 | 0.0138 | NT5DC1_2 | 0.1835 | SLC23A1_2 | 0.1263 |
| ARL6IP1 1 | 0.0383 | DSC3_1 | 0.0421 | ICAM4_1 | 0.2951 | NTRK2_3 | 0.0065 | SLC25A34 | 0.109 |
| ARMCX3 2 | 0.0621 | DUT_3 | 0.1213 | IL1RAP 2 | 0.0741 | NUP155 _1 | 0.0235 | SLC4A5 3 | 0.0866 |
| ATXN10 1 | 0.2002 | EEF1E1_1 | 0.1304 | IQCA1_2 | 0.0244 | NYX | 0.1072 | SLC9A10 | 0.0661 |
| AXL 1 | 0.0787 | ELN 2 | 0.1203 | KCNIP3_1 | 0.0938 | ODF2_3 | 0.0161 | SNORD93 | 0.1261 |
| BAII 3 | 0.039 | EMP1 | 0.2038 | KCNQ2 _1 | 0.1005 | ORC1L | 0.0346 | SOX2 1 | 0.0782 |
| BCAS1 1 | 0.3125 | ENOI | 0.1612 | KIF3C | 0.1804 | OTUD7A 3 | 0.0453 | STC1 | 0.0047 |
| BDNF 2 | 0.1249 | ENPEP_2 | 0.0755 | KRT80_2 | 0.0974 | PANK4 | 0.0512 | STC2 | 0.1147 |
| BMPR1A | 0.1127 | EPHB1 | 0.0435 | KRTAP10.10_2 | 0.0251 | PDLIM2_2 | 0.2133 | STYX 2 | 0.0145 |
| BTF3 3 | 0.1074 | EPYC | 0.0353 | L3MBTL2_3 | 0.047 | PDZRN4_2 | 0.2065 | SYTL3 | 0.0265 |
| C10orf116 | 0.0764 | ERI2 2 | 0.2661 | LBH_2 | 0.0788 | PHYH_1 | 0.0138 | TAF15 1 | 0.0283 |
| C11orf24 | 0.1919 | ESPNL | 0.0618 | LENEP | 0.2352 | PIGA 1 | 0.0954 | TCEAL8 1 | 0.0151 |
| C11orf49_3 | 0.1101 | EZH2_1 | 0.0349 | LGI3 | 0.0898 | PITX2_1 | 0.2052 | THBS3 | 0.0969 |
| C14orf102_2 | 0.1056 | FAM13AOS | 0.0713 | LOC492303 | 0.0287 | PKN1 3 | 0.0055 | TM2D3_2 | 0.0344 |
| C14orf109_2 | 0.1151 | FAM187B_2 | 0.0061 | LRRC14B | 0.0258 | PLEKHG5_5 | 0.2631 | TMEM52 | 0.1012 |
| C17orf106 | 0.1628 | FAM70A_1 | 0.0763 | LRRC37A4_2 | 0.0805 | PLSCR4 | 0.0174 | TMEM62 | 0.0003 |
| C17orf58_2 | 0.0165 | FBXO48_2 | 0.2271 | LRRTM4 | 0.171 | PMEPA1_4 | 0.1317 | TNFRSF18_1 | 0.22 |
| C17orf58_3 | 0.0188 | FKBP10 | 0.0694 | MACC1 | 0.1667 | PNMA5 | 0.1709 | TNNT2_1 | 0.0095 |
| C18orf56 | 0.0014 | FLJ33360 | 0.015 | MANSC1_1 | 0.1147 | PPAPDC1A | 0.1182 | TOMM20L | 0.0031 |
| C1orf168 | 0.0362 | FLJ43752 | 0.2482 | MCAM | 0.0177 | PRAMEF5 | 0.0079 | TPM2 2 | 0.1789 |
| C1orf64 | 0.117 | FMNL3_2 | 0.0148 | MCART6_1 | 0.1242 | PRKAA2 | 0.1228 | TRIM58 | 0.1141 |

| | | | | | | | |
|---|---|---|---|---|---|---|---|
| C8orf79_1 | 0.0116 | FOSB | 0.2242 | MFRP | 0.2218 | PSMC6_1 | 0.0374 | UBR7 1 | 0.0813 |
| CALD1 2 | 0.1444 | FOSL2 | 0.027 | MIDN | 0.0017 | RAD54B _ 2 | 0.17 | UBR7 2 | 0.1399 |
| CASP8AP2 | 0.1208 | FOXN1 | 0.2632 | MIR1914 | 0.0521 | RAP1A _ 1 | 0.1931 | WARS 2 | 0.1788 |
| CCL 13 | 0.1339 | GAD1_2 | 0.022 | MIR212 | 0.0938 | RARA_3 | 0.0943 | XBP1 2 | 0.1423 |
| CCR2 3 | 0.0306 | GBE1 | 0.0515 | MIR571 | 0.0018 | RARG | 0.0835 | XRN2_1 | 0.0391 |
| CD34 1 | 0.0302 | GBP7 | 0.1337 | MIR576 | 0.1152 | RNASEK | 0.0781 | YARS2 | 0.032 |
| CDC42BPA 2 | 0.008 | GJA5_1 | 0.0692 | MIR654 | 0.0402 | RNF7_1 | 0.0263 | ZNF75D 2 | 0.1331 |
| CDC42SE2 2 | 0.0158 | GMNN | 0.1028 | MIR942 | 0.1028 | ROD1_1 | 0.1957 | ZSWIM4 2 | 0.1751 |
| CIDEC 1 | 0.1023 | GSR_2 | 0.0217 | MMP12 1 | 0.1231 | SATB2 | 0.0337 | figo_numeric | 0.0156 |
| CLDN6 | 0.0101 | HBA2 | 0.2072 | MYCN_2 | 0.1288 | SBSN | 0.0787 | hist rev SBOT | 0.0427 |
| CREB5_2 | 0.0087 | HCFC1R1_1 | 0.0608 | MYOHD1 | 0.1095 | SCXB | 0.0128 | surg_outcome | 0.0116 |
| CRYBA1 | 0.0583 | HDAC7_2 | 0.006 | NFATC3 _ 5 | 0.0257 | SEC22C_3 | 0.1033 | | |
| CXCL13 | 0.0606 | HDLBP_3 | 0.0941 | NFATC4 | 0.0391 | SELENBP1 | 0.1464 | | |

Table 44

| | | | | | | | | | |
|---|---|---|---|---|---|---|---|---|---|
| ABCC9_3 | 0.036 | CYB5R3_2 | 0.1712 | HIC1 | 0.0256 | NFATC4 | 0.0453 | SERPINB2 2 | 0.0109 |
| ABHD3 | 0.2418 | CYP1A2 | 0.0968 | HPRT1 1 | 0.1566 | NLRP9 | 0.1706 | SERPINB5 | 0.2042 |
| ADAM17_2 | 0.1594 | DBNDD2 | 0.1126 | HPS4 1 | 0.0459 | NOVA2 | 0.1107 | SFN | 0.0343 |
| ADAMTS1 | 0.1413 | DNAH11 | 0.0285 | HR 1 | 0.0402 | NP | 0.0876 | SFRS4 | 0.0627 |
| ADAMTS2 1 | 0.121 | DNMT3L_2 | 0.0232 | HSD11B1 1 | 0.087 | NR6A1 2 | 0.1312 | SHC1_3 | 0.0789 |
| ALS2CL 3 | 0.0649 | DOCK7 1 | 0.1391 | ICAM2 | 0.0205 | NRXN3 3 | 0.1303 | SLC23A1_2 | 0.1388 |
| ANO7_3 | 0.0213 | DSC3 1 | 0.0513 | ICAM4 1 | 0.2616 | NT5DC1_2 | 0.184 | SLC25A34 | 0.1082 |
| ARL6IP1 1 | 0.0213 | DUT 3 | 0.1196 | IL1RAP 2 | 0.0513 | NTRK2 3 | 0.044 | SLC4A5 3 | 0.0717 |
| ARMCX3_2 | 0.0681 | EEF1E1_1 | 0.0951 | IQCA1_2 | 0.0447 | NUP155_1 | 0.0115 | SLC9A10 | 0.1028 |
| ATXN10 1 | 0.2199 | ELN 2 | 0.1071 | KCNIP3 1 | 0.1012 | NYX | 0.1203 | SNORD93 | 0.1652 |
| AXL 1 | 0.0968 | EMP1 | 0.2002 | KCNQ2_1 | 0.1135 | ODF2 3 | 0.0224 | SOX2 1 | 0.0838 |
| BAII 3 | 0.0412 | ENOI | 0.1533 | KIF3C | 0.2104 | ORC1L | 0.034 | STC1 | 0.0093 |
| BCAS1 1 | 0.3202 | ENPEP 2 | 0.0677 | KRT80 2 | 0.1038 | OTUD7A_3 | 0.0543 | STC2 | 0.1172 |
| BDNF 2 | 0.1502 | EPHB1 | 0.0571 | KRTAP10.10 2 | 0.0058 | PANK4 | 0.037 | STYX 2 | 0.0436 |
| BMPR1A | 0.1275 | EPYC | 0.0355 | L3MBTL2 3 | 0.0483 | PDLIM2_2 | 0.2288 | SYTL3 | 0.0048 |
| BTF3 3 | 0.1045 | ERI2_2 | 0.285 | LBH 2 | 0.092 | PHYH 1 | 0.194 | TAF15_1 | 0.002 |
| C10orf116 | 0.028 | ESPNL | 0.0581 | LENEP | 0.2431 | PIGA 1 | 0.0048 | TCEAL8_1 | 0.0188 |
| C11orf24 | 0.2 | EZH2_1 | 0.0411 | LGI3 | 0.0848 | PITX2 1 | 0.0845 | THBS3 | 0.0896 |
| C11orf49_3 | 0.1503 | FAM13AOS | 0.0424 | LOC340508 | 0.0351 | PKN1_3 | 0.0306 | TM2D3_2 | 0.0517 |
| C14orf102_2 | 0.083 | FAM187B_2 | 0.0158 | LOC492303 | 0.001 | PLEKHG5_5 | 0.2787 | TMEM52 | 0.1115 |
| C14orf109_2 | 0.0921 | FAM70A_1 | 0.0593 | LRRC14B | 0.0865 | PLSCR4 | 0.0479 | TMEM62 | 0.0171 |
| C17orf106 | 0.1908 | FBXO48_2 | 0.2378 | LRRC37A4 2 | 0.0078 | PMEPA1_4 | 0.1626 | TNFRSF18_1 | 0.2479 |
| C17orf58_2 | 0.039 | FKBP10 | 0.0718 | LRRTM4 | 0.1788 | PNMA5 | 0.1467 | TNNT2 1 | 0.0053 |
| C17orf58 3 | 0.0287 | FLJ33360 | 0.019 | MACC1 | 0.1593 | PPAPDC1A | 0.1399 | TOMM20L | 0.049 |
| C18orf56 | 0.0321 | FLJ43752 | 0.2474 | MANSC1 1 | 0.1468 | PRAMEF 5 | 0.0122 | TPM2 2 | 0.18 |
| C1orf168 | 0.0489 | FMNL3 2 | 0.0143 | MCAM | 0.0017 | PRKAA2 | 0.0937 | TRIM58 | 0.1134 |

| | | | | | | | | |
|---|---|---|---|---|---|---|---|---|
| C1orf64 | 0.135 | FOSB | 0.2264 | MCART6 1 | 0.1422 | PSMC6_1 | 0.0073 | UBR7 1 | 0.0324 |
| C8orf79_1 | 0.036 | FOSL2 | 0.02 | MFRP | 0.2188 | RAD54B_2 | 0.1946 | UBR7 2 | 0.1357 |
| CALD1 2 | 0.1435 | FOXN1 | 0.2808 | MIDN | 0.0097 | RAP1A_1 | 0.2211 | WARS 2 | 0.1513 |
| CASP8AP2 | 0.1065 | GAD1_2 | 0.0056 | MIR1914 | 0.0589 | RARA 3 | 0.0827 | XBP1 2 | 0.1115 |
| CCL13 | 0.1338 | GBE1 | 0.0656 | MIR212 | 0.112 | RARG | 0.0498 | XRN2_1 | 0.0002 |
| CCR2 3 | 0.017 | GBP7 | 0.1639 | MIR571 | 0.0143 | RNASEK | 0.0463 | YARS2 | 0.016 |
| CD34 1 | 0.0292 | GJA5 1 | 0.0425 | MIR576 | 0.1222 | RNF7 1 | 0.027 | ZNF75D 2 | 0.1219 |
| CDC42BPA 2 | 0.0121 | GMNN | 0.0697 | MIR654 | 0.0199 | ROD1 1 | 0.2439 | ZSWIM4_2 | 0.1727 |
| CDC42SE2_2 | 0.0321 | GSR 2 | 0.0249 | MIR942 | 0.1114 | SATB2 | 0.0247 | figo_numeric | 0.0137 |
| CLDN6 | 0.1355 | HBA2 | 0.1999 | MMP12 1 | 0.1088 | SBSN | 0.0737 | hist rev SBOT | 0.0484 |
| CREB5 2 | 0.0068 | HCFC1R1_1 | 0.0751 | MYCN_2 | 0.1385 | SCXB | 0.0121 | surg outcome | 0.0353 |
| CRYBA1 | 0.065 | HDAC7_2 | 0.0136 | MYOHD1 | 0.1035 | SEC22C 3 | 0.0979 | | |
| CXCL13 | 0.0787 | HDLBP 3 | 0.1099 | NFATC3 5 | 0.0304 | SELENBP1 | 0.1641 | | |

Table 45

| | | | | | | | |
|---|---|---|---|---|---|---|---|
| ABCC9_3 | 0.0405 | CXCL13 | 0.0712 | HDLBP 3 | 0.1009 | NFATC4 | 0.0353 | SERPINB2 2 | 0.0002 |
| ABHD3 | 0.248 | CYB5R3 2 | 0.1805 | HIC1 | 0.0023 | NLRP9 | 0.1721 | SERPINB5 | 0.1947 |
| ADAM17 2 | 0.1551 | CYP1A2 | 0.0895 | HPRT1_1 | 0.1547 | NOVA2 | 0.1049 | SFN | 0.0443 |
| ADAMTS1 | 0.1361 | DBNDD2 | 0.1079 | HPS4 1 | 0.0617 | NP | 0.0776 | SFRS4 | 0.064 |
| ADAMTS2 1 | 0.114 | DNAH11 | 0.0306 | HR 1 | 0.042 | NR6A1_2 | 0.1366 | SHC1_3 | 0.0668 |
| ALS2CL 3 | 0.0574 | DNMT3L_2 | 0.0125 | HSD11B1 _ 1 | 0.0838 | NRXN3 3 | 0.1304 | SLC23A1_2 | 0.1452 |
| ANO7 3 | 0.0398 | DOCK7 1 | 0.1253 | ICAM2 | 0.0132 | NT5DC1_2 | 0.1769 | SLC25A34 | 0.1091 |
| ARL6IP1 1 | 0.0203 | DSC3_1 | 0.0446 | ICAM4 1 | 0.2725 | NTRK2_3 | 0.0182 | SLC4A5 3 | 0.0736 |
| ARMCX3 2 | 0.0756 | DUT 3 | 0.1217 | IL1RAP 2 | 0.047 | NUP155_1 | 0.0071 | SLC9A10 | 0.0977 |
| ATXN10 1 | 0.2101 | EEF1E1 1 | 0.1053 | IQCA1_2 | 0.0335 | NYX | 0.1181 | SNORD93 | 0.1568 |
| AXL 1 | 0.0947 | ELN_2 | 0.1093 | KCNIP3 1 | 0.0906 | ODF2_3 | 0.0143 | SOX2 1 | 0.0829 |
| BAII 3 | 0.0448 | EMP1 | 0.2013 | KCNQ2_1 | 0.1123 | ORC1L | 0.0339 | STC1 | 0.0155 |
| BCAS1 1 | 0.3265 | ENOI | 0.1433 | KIF3C | 0.2087 | OTUD7A_3 | 0.0389 | STC2 | 0.1165 |
| BDNF 2 | 0.1484 | ENPEP_2 | 0.0787 | KRT80 2 | 0.1016 | PANK4 | 0.0493 | STYX 2 | 0.0385 |
| BMPR1A | 0.1254 | EPHB1 | 0.0585 | KRTAP10.10_2 | 0.0184 | PDLIM2 2 | 0.2253 | SYTL3 | 0.008 |
| BTF3 3 | 0.1066 | EPYC | 0.0343 | L3MBTL2 3 | 0.0458 | PHYH 1 | 0.1925 | TAF15 1 | 0.0146 |
| C10orfll6 | 0.032 | ERI2 2 | 0.2792 | LBH 2 | 0.0914 | PIGA 1 | 0.0109 | TCEAL8 1 | 0.0078 |
| C11orf24 | 0.2036 | ESPNL | 0.0496 | LENEP | 0.2476 | PITX2_1 | 0.0726 | THBS3 | 0.0944 |
| C11orf49_3 | 0.1528 | EZH2_1 | 0.0506 | LGI3 | 0.1018 | PKN1 3 | 0.0238 | TM2D3_2 | 0.0534 |
| C14orf102_2 | 0.1049 | FAM13AOS | 0.0286 | LOC492303 | 0.0262 | PLEKHG5_5 | 0.275 | TMEM52 | 0.1154 |
| C14orf109_2 | 0.0692 | FAM187B_2 | 0.0083 | LRRC14B | 0.0105 | PLSCR4 | 0.0404 | TMEM62 | 0.0076 |
| C17orf106 | 0.183 | FAM70A_1 | 0.077 | LRRC37A4 _ 2 | 0.0724 | PMEPA1_4 | 0.1528 | TNFRSF18_1 | 0.2551 |
| C17orf58_2 | 0.0131 | FBXO48_2 | 0.2388 | LRRTM4 | 0.162 | PNMA5 | 0.1469 | TNNT2_1 | 0.0097 |
| C17orf58_3 | 0.0296 | FKBP10 | 0.0553 | MACC1 | 0.147 | PPAPDC1A | 0.1491 | TOMM20L | 0.0442 |
| C18orf56 | 0.0308 | FLJ33360 | 0.0312 | MANSC1_1 | 0.1434 | PRAMEF5 | 0.0133 | TPM2 2 | 0.1828 |
| C1orf168 | 0.0421 | FLJ43752 | 0.2411 | MCAM | 0.0203 | PRKAA2 | 0.1131 | TRIM58 | 0.1129 |

(continued)

| | | | | | | | | | | |
|---|---|---|---|---|---|---|---|---|---|---|
| C1orf64 | 0.1371 | FMNL3 2 | 0.0121 | MCART6 1 | 0.1354 | PSMC6 1 | 0.0295 | UBR7 1 | 0.0366 |
| C8orf79_1 | 0.0283 | FOSB | 0.2136 | MFRP | 0.2209 | RAD54B_2 | 0.1927 | UBR7 2 | 0.1311 |
| CALD1 2 | 0.1557 | FOSL2 | 0.0265 | MIDN | 0.0197 | RAP1A_1 | 0.2064 | WARS 2 | 0.152 |
| CASP8AP2 | 0.1118 | FOXN1 | 0.3005 | MIR1914 | 0.0461 | RARA_3 | 0.0877 | XBP1 2 | 0.1099 |
| CCL13 | 0.1418 | GAD1_2 | 0.0126 | MIR212 | 0.1054 | RARG | 0.0507 | XRN2_1 | 0.0157 |
| CCR2 3 | 0.0027 | GBE1 | 0.0533 | MIR571 | 0.0122 | RNASEK | 0.0392 | YARS2 | 0.0047 |
| CD34 1 | 0.0382 | GBP7 | 0.1547 | MIR576 | 0.1217 | RNF7_1 | 0.0375 | ZNF75D 2 | 0.1409 |
| CDC42BPA 2 | 0.0056 | GJA5 1 | 0.0544 | MIR654 | 0.0234 | ROD1_1 | 0.2204 | ZSWIM4_2 | 0.1781 |
| CDC42SE2 2 | 0.002 | GMNN | 0.0815 | MIR942 | 0.104 | SATB2 | 0.0409 | figo_numeric | 0.0154 |
| CIDEC 1 | 0.1189 | GSR_2 | 0.0369 | MMP12_1 | 0.1214 | SBSN | 0.0881 | hist rev SBOT | 0.0474 |
| CLDN6 | 0.0145 | HBA2 | 0.1941 | MYCN 2 | 0.1555 | SCXB | 0.0192 | surg outcome | 0.026 |
| CREB5 2 | 0.0443 | HCFC1R1_1 | 0.0692 | MYOHD1 | 0.1145 | SEC22C 3 | 0.1055 | | |
| CRYBA1 | 0.0424 | HDAC7 2 | 0.0102 | NFATC3 _ 5 | 0.0357 | SELENBP1 | 0.1717 | | |

Table 46

| | | | | | | | | | |
|---|---|---|---|---|---|---|---|---|---|
| ABCC9_3 | 0.0433 | CYB5R3_2 | 0.1744 | HIC1 | 0.0219 | NLRP9 | 0.1847 | SERPINB2_2 | 0.0099 |
| ABHD3 | 0.2313 | CYP1A2 | 0.0928 | HPRT1 1 | 0.1409 | NOVA2 | 0.0786 | SERPINBS | 0.1984 |
| ADAM17 2 | 0.1463 | DBNDD2 | 0.1202 | HPS4 1 | 0.0314 | NP | 0.0737 | SFN | 0.0117 |
| ADAMTS1 | 0.1324 | DNAH11 | 0.0329 | HR 1 | 0.0314 | NR6A1 2 | 0.1323 | SFRS4 | 0.062 |
| ADAMTS2_1 | 0.1156 | DNMT3L_2 | 0.03 | HSD11B1 1 | 0.0913 | NRXN3 _ 3 | 0.1695 | SHC1_3 | 0.0792 |
| ALS2CL 3 | 0.0663 | DOCK7_1 | 0.1428 | ICAM2 | 0.0295 | NT5DC1_2 | 0.1883 | SLC23A1_2 | 0.1285 |
| ANO7 3 | 0.0165 | DSC3 1 | 0.0499 | ICAM4 1 | 0.2782 | NTRK2_3 | 0.0264 | SLC25A34 | 0.1046 |
| ARL6IP1 1 | 0.005 | DUT 3 | 0.1323 | IL1RAP 2 | 0.089 | NUP155 1 | 0.0286 | SLC4A5_3 | 0.0784 |
| ARMCX3 2 | 0.0473 | EEF1E1_1 | 0.1166 | IQCA1_2 | 0.051 | NYX | 0.1093 | SLC9A10 | 0.0692 |
| ATXN10 1 | 0.2153 | ELN_2 | 0.1113 | KCNIP3 1 | 0.0995 | ODF2 3 | 0.0222 | SNORD93 | 0.1354 |
| AXL 1 | 0.0883 | EMP1 | 0.2114 | KCNQ2_1 | 0.1115 | ORC1L | 0.0396 | SOX2 1 | 0.0821 |
| BAII 3 | 0.0263 | ENOI | 0.1618 | KIF3C | 0.1872 | OTUD7A 3 | 0.0602 | STC1 | 0.0006 |
| BCAS11 | 0.303 | ENPEP_2 | 0.0575 | KRT80 2 | 0.1091 | PANK4 | 0.0492 | STC2 | 0.1069 |
| BDNF 2 | 0.1325 | EPHB1 | 0.0325 | KRTAP10.10_2 | 0.0099 | PDLIM2_2 | 0.2233 | STYX 2 | 0.0206 |
| BMPR1A | 0.1136 | EPYC | 0.0368 | L3MBTL2 3 | 0.0423 | PDZRN4 2 | 0.213 | SYTL3 | 0.0128 |
| BTF3 3 | 0.1025 | ERI2_2 | 0.2801 | LBH_2 | 0.087 | PHYH1 | 0.002 | TAF15 1 | 0.0124 |
| C10orf116 | 0.0579 | ESPNL | 0.0762 | LENEP | 0.2226 | PIGA 1 | 0.1049 | TCEAL8_1 | 0.029 |
| C11orf24 | 0.1872 | EZH2 1 | 0.026 | LGI3 | 0.0909 | PITX2_1 | 0.1925 | THBS3 | 0.0772 |
| C11orf49_3 | 0.1148 | FAM13AOS | 0.0738 | LOC492303 | 0.0523 | PKN1_3 | 0.0164 | TM2D3_2 | 0.0352 |
| C14orf102_2 | 0.0905 | FAM187B_2 | 0.0162 | LRRC14B | 0.0181 | PLEKHG5_5 | 0.2641 | TMEM52 | 0.0973 |
| C14orf109_2 | 0.1288 | FAM70A_1 | 0.0679 | LRRC37A4 2 | 0.095 | PLSCR4 | 0.0318 | TMEM62 | 0.0099 |
| CI7orf106 | 0.1772 | FBXO48_2 | 0.2354 | LRRTM4 | 0.192 | PMEPA1 4 | 0.1339 | TNFRSF18_1 | 0.2259 |
| C17orf58_2 | 0.0491 | FKBP10 | 0.0781 | MACC1 | 0.1756 | PNMA5 | 0.1661 | TNNT2_1 | 0.007 |
| C17orf58 3 | 0.0249 | FLJ33360 | 0.0098 | MANSC1 1 | 0.1191 | PPAPDC1A | 0.1173 | TOMM20L | 0.0038 |
| C18orf56 | 0.0047 | FLJ43752 | 0.2543 | MCAM | 0.0035 | PRAMEF 5 | 0.0037 | TPM2 2 | 0.1746 |
| Clorf168 | 0.0462 | FMNL3_2 | 0.0068 | MCART6 1 | 0.1239 | PRKAA2 | 0.0845 | TRIM58 | 0.1143 |

| | | | | | | | |
|---|---|---|---|---|---|---|---|
| Clorf64 | 0.1187 | FOSB | 0.2374 | MFRP | 0.2181 | PSMC6_1 | 0.0092 | UBR7 1 | 0.0632 |
| C8orf79_1 | 0.039 | FOSL2 | 0.0174 | MIDN | 0.0112 | RAD54B_2 | 0.1734 | UBR7 2 | 0.1372 |
| CALD1 2 | 0.1237 | FOXN1 | 0.2537 | MIR1914 | 0.0735 | RAP1A _ 1 | 0.2019 | WARS 2 | 0.1773 |
| CASP8AP2 | 0.1133 | GAD1_2 | 0.0135 | MIR212 | 0.0953 | RARA_3 | 0.0839 | XBP1 2 | 0.1536 |
| CCL 13 | 0.1257 | GBE1 | 0.069 | MIR571 | 0.0131 | RARG | 0.076 | XRN2_1 | 0.0131 |
| CCR2 3 | 0.0405 | GBP7 | 0.1605 | MIR576 | 0.1188 | RNASEK | 0.0781 | YARS2 | 0.0087 |
| CD34 1 | 0.015 | GJA5_1 | 0.0438 | MIR654 | 0.0263 | RNF7 1 | 0.0092 | ZNF75D_2 | 0.1157 |
| CDC42BPA 2 | 0.0125 | GMNN | 0.0875 | MIR942 | 0.1061 | ROD1_1 | 0.2241 | ZSWIM4 _ 2 | 0.1653 |
| CDC42SE2 2 | 0.012 | GSR 2 | 0.019 | MMP12 1 | 0.1136 | SATB2 | 0.0194 | figo_numeric | 0.0012 |
| CLDN6 | 0.1194 | HBA2 | 0.198 | MYCN_2 | 0.112 | SBSN | 0.0605 | hist rev SBOT | 0.0544 |
| CREB5 2 | 0.0186 | HCFC1R1 1 | 0.0729 | MYOHD 1 | 0.1009 | SCXB | 0.0084 | surg outcome | 0.0116 |
| CRYBA1 | 0.0195 | HDAC7_2 | 0.003 | NFATC3 5 | 0.0066 | SEC22C_3 | 0.0984 | | |
| CXCL13 | 0.0646 | HDLBP 3 | 0.1117 | NFATC4 | 0.0369 | SELENBP1 | 0.1409 | | |

Table 47

| | | | | | | | | |
|---|---|---|---|---|---|---|---|---|
| ABCC9_3 | 0.0696 | CXCL13 | 0.0664 | HDLBP_3 | 0.1796 | NFATC3_5 | 0.0307 | SELENBP1 | 0.1444 |
| ABHD3 | 0.2533 | CYB5R3 _ 2 | 0.1655 | HIC1 | 0.0794 | NFATC4 | 0.046 | SERPINB2_2 | 0.025 |
| ADAM17 2 | 0.1436 | CYP1A2 | 0.0623 | HPRT1_1 | 0.135 | NLRP9 | 0.153 | SERPINBS | 0.1819 |
| ADAMTS1 | 0.0774 | DBNDD2 | 0.1079 | HPS4 1 | 0.0317 | NOVA2 | 0.058 | SFN | 0.0093 |
| ADAMTS2 1 | 0.0967 | DFFB_2 | 0.0435 | HR 1 | 0.0355 | NP | 0.081 | SFRS4 | 0.0715 |
| ALS2CL 3 | 0.0472 | DNAH11 | 0.0244 | HSD11B1_1 | 0.0991 | NR6A1_2 | 0.1229 | SHC1_3 | 0.1054 |
| ANO7_3 | 0.0388 | DNMT3L_2 | 0.0951 | ICAM2 | 0.0086 | NRXN3_3 | 0.1365 | SLC23A1_2 | 0.0915 |
| ARL6IP1 1 | 0.0119 | DOCK7_1 | 0.0083 | ICAM4_1 | 0.2797 | NT5DC1_2 | 0.1855 | SLC25A34 | 0.0864 |
| ARMCX3 2 | 0.0639 | DSC3_1 | 0.0316 | IL1RAP 2 | 0.0665 | NTRK2 3 | 0.0012 | SLC4A5 3 | 0.0891 |
| ATP2B1 3 | 0.1777 | DUT_3 | 0.1331 | IQCA1_2 | 0.005 | NUP155 1 | 0.0212 | SLC9A10 | 0.0702 |
| ATXN10_1 | 0.0694 | EEF1E1_1 | 0.1018 | KCNIP3_1 | 0.0803 | NYX | 0.0636 | SNORD93 | 0.121 |
| AXL 1 | 0.0588 | ELN 2 | 0.1057 | KCNQ2 _ 1 | 0.1234 | ODF2_3 | 0.0254 | SOX2 1 | 0.0692 |
| BAII 3 | 0.036 | EMP1 | 0.1805 | KIF3C | 0.1851 | ORC1L | 0.0528 | STC1 | 0.0048 |
| BCAS1 1 | 0.3111 | ENOI | 0.1502 | KRT80_2 | 0.0789 | OTUD7A 3 | 0.0414 | STC2 | 0.0886 |
| BDNF 2 | 0.1004 | ENPEP_2 | 0.0681 | KRTAP10.10_2 | 0.0252 | PANK4 | 0.0513 | STYX 2 | 0.0307 |
| BMPR1A | 0.1203 | EPHB1 | 0.0478 | L3MBTL2 3 | 0.045 | PDLIM2 2 | 0.2016 | SYTL3 | 0.0229 |
| BTF3 3 | 0.1159 | EPYC | 0.0254 | LBH_2 | 0.0781 | PDZRN4_2 | 0.2334 | TAF15 1 | 0.0307 |
| C10orf116 | 0.0819 | ERI2_2 | 0.2725 | LENEP | 0.2225 | PHYH_1 | 0.0129 | TCEAL8 1 | 0.0282 |
| Cllorf24 | 0.1375 | ESPNL | 0.0803 | LGI3 | 0.1071 | PIGA_1 | 0.0786 | THBS3 | 0.0887 |
| C11orf49_3 | 0.1207 | EZH2_1 | 0.0506 | LOC340508 | 0.0427 | PITX2_1 | 0.2039 | TM2D3_2 | 0.0286 |
| C14orf102_2 | 0.0873 | FAM13AOS | 0.046 | LOC492303 | 0.0279 | PKN1_3 | 0.0349 | TMEM52 | 0.0716 |
| C14orf109 _2 | 0.1053 | FAM187B_2 | 0.0052 | LRRC14B | 0.0689 | PLEKHG5_5 | 0.2594 | TMEM62 | 0.005 |
| C17orf106 | 0.1659 | FAM70A_1 | 0.1008 | LRRC37A4_2 | 0.0168 | PLSCR4 | 0.0257 | TNFRSF18_1 | 0.2254 |
| C17orf58_2 | 0.0033 | FBXO48_2 | 0.1965 | LRRTM4 | 0.1666 | PMEPA1 4 | 0.1513 | TNNT2_1 | 0.0102 |
| C17orf58_3 | 0.0289 | FKBP10 | 0.0944 | MACC1 | 0.1672 | PNMA5 | 0.1849 | TOMM20L | 0.0059 |
| C18orf56 | 0.0106 | FLJ33360 | 0.0228 | MANSC1_1 | 0.122 | PPAPDC1A | 0.1082 | TPM2 2 | 0.1709 |

EP 3 874 274 B1

(continued)

| Gene | Value | Gene | Value | Gene | Value | Gene | Value | Gene | Value |
|---|---|---|---|---|---|---|---|---|---|
| C1orf168 | 0.0384 | FLJ43752 | 0.2324 | MAPK3_1 | 0.0462 | PRAMEF5 | 0.0173 | TRIM58 | 0.0914 |
| C1orf64 | 0.1093 | FMNL3_2 | 0.0244 | MCAM | 0.093 | PRKAA2 | 0.1096 | UBR7 1 | 0.063 |
| C8orf79_1 | 0.0444 | FOSB | 0.1977 | MCART6_1 | 0.2299 | PSMC6_1 | 0.022 | UBR7 2 | 0.157 |
| CALD1 2 | 0.1526 | FOSL2 | 0.0472 | MFRP | 0.0347 | RAD54B_2 | 0.1948 | WARS 2 | 0.1918 |
| CASP8AP2 | 0.1126 | FOXN1 | 0.257 | MIDN | 0.0306 | RAP1A 1 | 0.2024 | XBP1 2 | 0.1665 |
| CCL13 | 0.1468 | GAD1 2 | 0.024 | MIR1914 | 0.0473 | RARA 3 | 0.0887 | XRN2 1 | 0.0272 |
| CCR2 3 | 0.0417 | GBE1 | 0.0549 | MIR212 | 0.0992 | RARG | 0.0268 | YARS2 | 0.0296 |
| CD34 1 | 0.0562 | GBP7 | 0.0954 | MIR571 | 0.0288 | RNASEK | 0.0969 | ZNF75D 2 | 0.1301 |
| CDC42BPA 2 | 0.0137 | GJA5_1 | 0.0628 | MIR576 | 0.0982 | RNF7_1 | 0.0546 | ZSWIM4 2 | 0.1703 |
| CDC42SE2 2 | 0.001 | GMNN | 0.1071 | MIR654 | 0.0045 | ROD1_1 | 0.1945 | figo_numeric | 0.025 |
| CIDEC 1 | 0.1086 | GSR 2 | 0.0117 | MIR942 | 0.0829 | SATB2 | 0.0246 | hist rev SBOT | 0.054 |
| CLDN6 | 0.0248 | GUSBL2 | 0.1966 | MMP12_1 | 0.1251 | SBSN | 0.0683 | surg_outcome | 0.0057 |
| CREB5_2 | 0.0103 | HBA2 | 0.0512 | MYCN 2 | 0.1504 | SCXB | 0.0162 | | |
| CRYBA1 | 0.0612 | HDAC7_2 | 0.0281 | MYOHD1 | 0.0906 | SEC22C_3 | 0.1006 | | |

Table 48

| | | | | | | | | | |
|---|---|---|---|---|---|---|---|---|---|
| ABCC9_3 | 0.0682 | CXCL13 | 0.1719 | HDLBP 3 | 0.1913 | NFATC4 | 0.0529 | SERPINB2_2 | 0.0149 |
| ABHD3 | 0.2441 | CYB5R3 2 | 0.1607 | HIC1 | 0.0851 | NLRP9 | 0.1568 | SERPINBS | 0.1863 |
| ADAM17 2 | 0.1457 | CYP1A2 | 0.0661 | HPRT1_1 | 0.1429 | NOV A2 | 0.0576 | SFN | 0.0136 |
| ADAMTS1 | 0.0811 | DBNDD2 | 0.1009 | HPS4 1 | 0.0271 | NP | 0.0796 | SFRS4 | 0.0676 |
| ADAMTS2 1 | 0.1086 | DFFB 2 | 0.0413 | HR 1 | 0.0393 | NR6A1_2 | 0.1199 | SHC1_3 | 0.0828 |
| ALS2CL 3 | 0.0528 | DNAH11 | 0.0309 | HSD11B1 1 | 0.105 | NRXN3_3 | 0.1311 | SLC23A1_2 | 0.0898 |
| ANO7 3 | 0.04 | DNMT3L_2 | 0.0976 | ICAM2 | 0.01 | NT5DC1_2 | 0.1811 | SLC25A34 | 0.0974 |
| ARL6IP1 1 | 0.0068 | DOCK7_1 | 0.0128 | ICAM4 1 | 0.2753 | NTRK2_3 | 0.0095 | SLC4A5 3 | 0.0942 |
| ARMCX3 2 | 0.0617 | DSC3 1 | 0.0381 | IL1RAP 2 | 0.0589 | NUP155_1 | 0.0292 | SLC9A10 | 0.0642 |
| ATXN10 1 | 0.1738 | DUT 3 | 0.1224 | IQCA1_2 | 0.0019 | NYX | 0.0596 | SNORD93 | 0.1309 |
| AXL 1 | 0.0704 | EEF1E1_1 | 0.1055 | KCNIP3_1 | 0.0834 | ODF2 3 | 0.0253 | SOX2 1 | 0.0629 |
| BAII 3 | 0.0552 | ELN_2 | 0.109 | KCNQ2_1 | 0.126 | ORC1L | 0.0455 | STC1 | 0.0078 |
| BCAS1 1 | 0.3069 | EMP1 | 0.1793 | KIF3C | 0.1827 | OTUD7A 3 | 0.053 | STC2 | 0.0898 |
| BDNF 2 | 0.0938 | ENOI | 0.1425 | KRT80 2 | 0.0686 | PANK4 | 0.0516 | STYX 2 | 0.0328 |
| BMPR1A | 0.118 | ENPEP_2 | 0.0593 | KRTAP10.10_2 | 0.0236 | PDLIM2_2 | 0.1925 | SYTL3 | 0.0217 |
| BTF3 3 | 0.1104 | EPHB1 | 0.0429 | L3MBTL2_3 | 0.049 | PDZRN4_2 | 0.2315 | TAF15 1 | 0.0082 |
| Cl0orf116 | 0.0783 | EPYC | 0.0307 | LBH_2 | 0.0793 | PHYH 1 | 0.0186 | TCEAL8 1 | 0.0327 |
| C11orf24 | 0.1293 | ERI2 2 | 0.2674 | LENEP | 0.2316 | PIGA 1 | 0.0884 | THBS3 | 0.0865 |
| C11orf49_3 | 0.1112 | ESPNL | 0.0826 | LGI3 | 0.1073 | PITX2 1 | 0.1951 | TM2D3_2 | 0.0325 |
| C14orf102_2 | 0.0893 | EZH2_1 | 0.0417 | LOC340508 | 0.0423 | PKN1_3 | 0.0311 | TMEM52 | 0.0704 |
| C14orf109_2 | 0.111 | FAM13AOS | 0.0552 | LOC492303 | 0.0284 | PLEKHG5_5 | 0.2597 | TMEM62 | 0.0053 |
| C17orf106 | 0.1548 | FAM187B_2 | 0.0099 | LRRC14B | 0.069 | PLSCR4 | 0.0168 | TNFRSF18_1 | 0.2353 |
| C17orf58_2 | 0.0048 | FAM70A_1 | 0.1014 | LRRC37A4 2 | 0.0079 | PMEPA1 4 | 0.1388 | TNNT2 1 | 0.0044 |
| C17orf58 3 | 0.0282 | FBXO48_2 | 0.1886 | LRRTM4 | 0.1632 | PNMA5 | 0.1728 | TOMM20L | 0.0053 |
| C18orf56 | 0.005 | FKBP10 | 0.1053 | MACC1 | 0.1621 | PPAPDC1A | 0.0931 | TPM2 2 | 0.1562 |
| Clorf168 | 0.0319 | FLJ33360 | 0.0252 | MANSC1_1 | 0.1219 | PRAMEF 5 | 0.0074 | TRIM58 | 0.1017 |

| | | | | | | | |
|---|---|---|---|---|---|---|---|
| C1orf64 | 0.1039 | FLJ43752 | 0.2252 | MCAM | 0.061 | PRKAA2 | 0.1125 | UBR7 1 | 0.0568 |
| C8orf79_1 | 0.0416 | FMNL3_2 | 0.0363 | MCART6 1 | 0.1036 | PSMC6_1 | 0.0175 | UBR7 2 | 0.1495 |
| CALD1 2 | 0.1521 | FOSB | 0.1936 | MFRP | 0.2262 | RAD54B 2 | 0.1883 | WARS 2 | 0.197 |
| CASP8AP2 | 0.1191 | FOSL2 | 0.0383 | MIDN | 0.0248 | RAP1A_1 | 0.1955 | XBP1 2 | 0.1608 |
| CCL 13 | 0.1516 | FOXN1 | 0.2519 | MIR1914 | 0.0427 | RARA 3 | 0.0884 | XRN2_1 | 0.0265 |
| CCR2 3 | 0.0349 | GAD1 2 | 0.0272 | MIR212 | 0.0933 | RARG | 0.0401 | YARS2 | 0.0284 |
| CD34 1 | 0.0491 | GBE1 | 0.0517 | MIR571 | 0.0368 | RNASEK | 0.1025 | ZNF75D 2 | 0.1311 |
| CDC42BPA 2 | 0.0004 | GBP7 | 0.0793 | MIR576 | 0.0928 | RNF7 1 | 0.0454 | ZSWIM4 2 | 0.1653 |
| CDC42SE2 2 | 0.0011 | GJA5 1 | 0.063 | MIR654 | 0.0014 | ROD1_1 | 0.1921 | figo_numeric | 0.0216 |
| CIDEC 1 | 0.1065 | GMNN | 0.1054 | MIR942 | 0.0824 | SATB2 | 0.0273 | hist rev SBOT | 0.0739 |
| CLDN6 | 0.0203 | GSR 2 | 0.0101 | MMP12 1 | 0.1313 | SBSN | 0.0751 | surg outcome | 0.0005 |
| CREB5_2 | 0.019 | GUSBL2 | 0.1925 | MYCN 2 | 0.1406 | SCXB | 0.0089 | | |
| CREBBP 1 | 0.052 | HBA2 | 0.0693 | MYOHD1 | 0.0937 | SEC22C_3 | 0.0932 | | |
| CRYBA1 | 0.0676 | HDAC7_2 | 0.031 | NFATC3 5 | 0.0264 | SELENBP1 | 0.1484 | | |

Table 49

| Gene | Value | Gene | Value | Gene | Value | Gene | Value |
|---|---|---|---|---|---|---|---|
| ABCC9_3 | 0.068 | CXCL_13 | 0.1716 | HIC1 | 0.086 | NFATC4 | 0.0529 | SERPINB2_2 | 0.0145 |
| ABHD3 | 0.2454 | CYB5R3 2 | 0.1593 | HPRT1_1 | 0.1412 | NLRP9 | 0.1562 | SERPINBS | 0.1864 |
| ADAM17_2 | 0.1462 | CYP1A2 | 0.0675 | HPS4_1 | 0.0263 | NOVA2 | 0.0577 | SFN | 0.0147 |
| ADAMTS1 | 0.0822 | DBNDD2 | 0.1017 | HR_1 | 0.0418 | NP | 0.0808 | SFRS4 | 0.066 |
| ADAMTS2_1 | 0.1063 | DNAH11 | 0.0416 | HSD11B1_1 | 0.1054 | NR6A1_2 | 0.1203 | SHC1_3 | 0.0846 |
| ALS2CL_3 | 0.0537 | DNMT3L_2 | 0.0309 | ICAM2 | 0.0105 | NRXN3_3 | 0.1293 | SLC23A1_2 | 0.0887 |
| ANO7_3 | 0.04 | DOCK7_1 | 0.0989 | ICAM4_1 | 0.2757 | NT5DC1_2 | 0.1823 | SLC25A34 | 0.0976 |
| ARL6IP1_1 | 0.0054 | DSC3 1 | 0.0388 | IL1RAP_2 | 0.0591 | NTRK2_3 | 0.0102 | SLC4A5_3 | 0.0939 |
| ARMCX3_2 | 0.0611 | DUT 3 | 0.1208 | IQCA1_2 | 0.002 | NUP155_1 | 0.0288 | SLC9A10 | 0.0629 |
| ATXN10_1 | 0.1742 | EEF1E1_1 | 0.1035 | KCNIP3_1 | 0.0836 | NYX | 0.0597 | SNORD93 | 0.1298 |
| AXL_1 | 0.0715 | ELN_2 | 0.1085 | KCNQ2_1 | 0.1249 | ODF2_3 | 0.0269 | SOX2_1 | 0.0601 |
| BAII_3 | 0.0543 | EMP1 | 0.179 | KIF3C | 0.1835 | ORC1L | 0.0462 | STC1 | 0.0078 |
| BCAS1_1 | 0.3087 | ENOI | 0.141 | KRT80_2 | 0.0706 | OTUD7A_3 | 0.0519 | STC2 | 0.0891 |
| BDNF_2 | 0.0934 | ENPEP_2 | 0.0603 | KRTAP10.10_2 | 0.024 | PANK4 | 0.0511 | STYX_2 | 0.0319 |
| BMPR1A | 0.1199 | EPHB1 | 0.0428 | L3MBTL2_3 | 0.0495 | PDLIM2_2 | 0.1909 | SYTL3 | 0.0197 |
| BTF3_3 | 0.1106 | EPYC | 0.0301 | LBH_2 | 0.0807 | PDZRN4_2 | 0.2316 | TAF15_1 | 0.0084 |
| C10orfll6 | 0.0796 | ERI2_2 | 0.2651 | LENEP | 0.2318 | PHYH_1 | 0.0171 | TCEAL8_1 | 0.0332 |
| C11orf24 | 0.1305 | ESPNL | 0.0841 | LGI3 | 0.1079 | PIGA_1 | 0.0902 | THBS3 | 0.0887 |
| C11orf49_3 | 0.1096 | EZH2 1 | 0.0416 | LOC340508 | 0.0398 | PITX2_1 | 0.1949 | TM2D3_2 | 0.0318 |
| C14orf102_2 | 0.0906 | FAM13AOS | 0.055 | LOC492303 | 0.0303 | PKN1_3 | 0.0318 | TMEM52 | 0.0702 |
| C14orf109_2 | 0.1105 | FAM187B_2 | 0.0096 | LRRC14B | 0.0689 | PLEKHG5_5 | 0.2619 | TMEM62 | 0.0059 |
| C17orf106 | 0.1558 | FAM70A_1 | 0.1017 | LRRC37A4 2 | 0.0073 | PLSCR4 | 0.0156 | TNFRSF18_1 | 0.236 |
| C17orf58_2 | 0.0049 | FBXO48_2 | 0.1866 | LRRTM4 | 0.1634 | PMEPA1_4 | 0.1371 | TNNT2_1 | 0.004 |
| C17orf58_3 | 0.0281 | FKBP10 | 0.1092 | MACC1 | 0.1622 | PNMA5 | 0.1746 | TOMM20L | 0.0018 |
| C18orf56 | 0.0053 | FLJ33360 | 0.0249 | MANSC1_1 | 0.1204 | PPAPDC1A | 0.0922 | TPM2 2 | 0.1568 |
| C1orf168 | 0.032 | FLJ43752 | 0.2269 | MAPK3_1 | 0.0606 | PRAMEF_5 | 0.008 | TRIM58 | 0.1038 |

| | | | | | | | |
|---|---|---|---|---|---|---|---|
| C1orf64 | 0.1042 | FMNL3_2 | 0.0362 | MCAM | 0.1022 | PRKAA2 | 0.1141 | UBR7_1 | 0.056 |
| C8orf79_1 | 0.0425 | FOSB | 0.1926 | MCART6_1 | 0.2249 | PSMC6_1 | 0.0188 | UBR7_2 | 0.1506 |
| CALD1_2 | 0.152 | FOSL2 | 0.0387 | MFRP | 0.0225 | RAD54B_2 | 0.1879 | WARS_2 | 0.1966 |
| CASP8AP2 | 0.1205 | FOXN1 | 0.2483 | MIDN | 0.0242 | RAP1A _ 1 | 0.194 | XBP1_2 | 0.1608 |
| CCL_13 | 0.1506 | GAD1_2 | 0.028 | MIR1914 | 0.0421 | RARA_3 | 0.0878 | XRN2_1 | 0.0261 |
| CCR2_3 | 0.035 | GBE1 | 0.0532 | MIR212 | 0.0922 | RARG | 0.04 | YARS2 | 0.0286 |
| CD34_1 | 0.0505 | GBP7 | 0.0782 | MIR571 | 0.0368 | RNASEK | 0.1015 | ZNF75D_2 | 0.1319 |
| CDC42BPA_2 | 0.0004 | GJA5_1 | 0.0632 | MIR576 | 0.0937 | RNF7_1 | 0.0434 | ZSWIM4 _ 2 | 0.1657 |
| CDC42SE2_2 | 0.0019 | GMNN | 0.1057 | MIR654 | 0.0009 | ROD1_1 | 0.1918 | figo_numeric | 0.0198 |
| CIDEC_1 | 0.1069 | GSR_2 | 0.0095 | MIR942 | 0.0813 | SATB2 | 0.0277 | hist rev SBOT | 0.0732 |
| CLDN6 | 0.0196 | GUSBL2 | 0.1919 | MMP12_1 | 0.1333 | SBSN | 0.0754 | surg outcome | 0 |
| CREB5_2 | 0.0181 | HBA2 | 0.0697 | MYCN_2 | 0.1392 | SCXB | 0.0086 | | |
| CREBBP_1 | 0.0508 | HDAC7 2 | 0.0309 | MYOHD1 | 0.0938 | SEC22C_3 | 0.0928 | | |
| CRYBA1 | 0.069 | HDLBP _ 3 | 0.1909 | NFATC3_5 | 0.0257 | SELENBP1 | 0.1495 | | |

Table 50

| Gene | Value | Gene | Value | Gene | Value | Gene | Value | Gene | Value |
|---|---|---|---|---|---|---|---|---|---|
| ABCC9_3 | 0.0489 | CXCL13 | 0.19 | HIC1 | 0.1042 | NFATC4 | 0.0421 | SERPINB2_2 | 0.0175 |
| ABHD3 | 0.2344 | CYB5R3_2 | 0.1335 | HPRT1_1 | 0.134 | NLRP9 | 0.1819 | SERPINB5 | 0.176 |
| ADAM17_2 | 0.1438 | CYP1A2 | 0.0835 | HPS4_1 | 0.055 | NOVA2 | 0.071 | SFN | 0.0187 |
| ADAMTS1 | 0.1209 | DBNDD2 | 0.1243 | HR_1 | 0.0333 | NP | 0.077 | SFRS4 | 0.0621 |
| ADAMTS2_1 | 0.1094 | DFFB_2 | 0.0369 | HSD11B1_1 | 0.0919 | NR6A1_2 | 0.1303 | SHC1_3 | 0.0571 |
| ALS2CL_3 | 0.0592 | DNAH11 | 0.0281 | ICAM2 | 0.0185 | NRXN3_3 | 0.1619 | SLC23A1_2 | 0.122 |
| ANO7_3 | 0.0383 | DNMT3L_2 | 0.1236 | ICAM4_1 | 0.2905 | NT5DC1_2 | 0.1764 | SLC25A34 | 0.1242 |
| ARL6IP1_1 | 0.0006 | DOCK7_1 | 0.0156 | IL1RAP_2 | 0.0676 | NTRK2_3 | 0.0156 | SLC4A5_3 | 0.0903 |
| ARMCX3_2 | 0.0553 | DSC3_1 | 0.0449 | IQCA1_2 | 0.0174 | NUP155_1 | 0.0311 | SLC9A10 | 0.0593 |
| ATXN10_1 | 0.2055 | DUT_3 | 0.1145 | KCNIP3_1 | 0.0952 | NYX | 0.1073 | SNORD93 | 0.1329 |
| AXL_1 | 0.0807 | EEF1E1_1 | 0.1242 | KCNQ2_1 | 0.1018 | ODF2_3 | 0.0177 | SOX2_1 | 0.0728 |
| BAII 3 | 0.0368 | ELN_2 | 0.1211 | KIF3C | 0.1764 | ORC1L | 0.0254 | STC1 | 0.0041 |
| BCAS1_1 | 0.3119 | EMP1 | 0.2024 | KRT80_2 | 0.095 | OTUD7A_3 | 0.059 | STC2 | 0.1165 |
| BDNF_2 | 0.1194 | ENOI | 0.1517 | KRTAP10.10_2 | 0.0248 | PANK4 | 0.052 | STYX_2 | 0.0169 |
| BMPR1A | 0.1171 | ENPEP_2 | 0.0722 | L3MBTL2_3 | 0.0482 | PDLIM2_2 | 0.2051 | SYTL3 | 0.0257 |
| BTF3_3 | 0.0979 | EPHB1 | 0.0435 | LBH_2 | 0.0836 | PDZRN4_2 | 0.2059 | TAF15_1 | 0.0093 |
| CI0orf116 | 0.0732 | EPYC | 0.039 | LENEP | 0.2374 | PHYH_1 | 0.0161 | TCEAL8_1 | 0.0123 |
| C11orf24 | 0.1901 | ERI2_2 | 0.2597 | LGI3 | 0.0934 | PIGA_1 | 0.1019 | THBS3 | 0.0978 |
| C11orf49_3 | 0.1068 | ESPNL | 0.064 | LOC340508 | 0.0261 | PITX2_1 | 0.199 | TM2D3_2 | 0.035 |
| C14orf102_2 | 0.1094 | EZH2_1 | 0.0284 | LOC492303 | 0.0233 | PKN1_3 | 0.0066 | TMEM52 | 0.0986 |
| C14orf109_2 | 0.1188 | FAM13AOS | 0.0739 | LRRC14B | 0.0775 | PLEKHG5_5 | 0.2619 | TMEM62 | 0.0011 |
| C17orf106 | 0.161 | FAM187B_2 | 0.0046 | LRRC37A4 _ 2 | 0.0065 | PLSCR4 | 0.0134 | TNFRSF18_1 | 0.2241 |
| C17orf58_2 | 0.0206 | FAM70A_1 | 0.0789 | LRRTM4 | 0.1714 | PMEPA1_4 | 0.1204 | TNNT2_1 | 0.0148 |
| C17orf58_3 | 0.0155 | FBXO48 2 | 0.221 | MACC1 | 0.165 | PNMA5 | 0.1591 | TOMM20L | 0.0028 |
| C 1 Sorf56 | 0.0044 | FKBP10 | 0.0756 | MANSC1_1 | 0.1128 | PPAPDC1A | 0.1056 | TPM2_2 | 0.1687 |
| Clorf168 | 0.0307 | FLJ33360 | 0.0213 | MAPK3_1 | 0.025 | PRAMEF5 | 0.0127 | TRIM58 | 0.1228 |

| | | | | | | | |
|---|---|---|---|---|---|---|---|
| C1orf64 | 0.1113 | FLJ43752 | 0.2432 | MCAM | 0.1315 | PRKAA2 | 0.1294 | UBR7_1 | 0.072 |
| C8orf79_1 | 0.009 | FMNL3_2 | 0.0217 | MCART6_1 | 0.216 | PSMC6_1 | 0.0359 | UBR7_2 | 0.1404 |
| CALD1 2 | 0.1443 | FOSB | 0.2156 | MFRP | 0.0168 | RAD54B_2 | 0.1662 | WARS_2 | 0.1834 |
| CASP8AP2 | 0.1307 | FOSL2 | 0.0239 | MIDN | 0.0071 | RAP1A _ 1 | 0.1802 | XBP1_2 | 0.1409 |
| CCL_13 | 0.1388 | FOXN1 | 0.2585 | MIR1914 | 0.047 | RARA_3 | 0.0875 | XRN2_1 | 0.0367 |
| CCR2_3 | 0.0199 | GAD1_2 | 0.0256 | MIR212 | 0.0885 | RARG | 0.0924 | YARS2 | 0.0318 |
| CD34_1 | 0.0238 | GBE1 | 0.0465 | MIR571 | 0.0024 | RNASEK | 0.0892 | ZNF75D_2 | 0.1337 |
| CDC42BPA_2 | 0.0086 | GBP7 | 0.123 | MIR576 | 0.1147 | RNF7_1 | 0.0137 | ZSWIM4_2 | 0.1715 |
| CDC42SE2_2 | 0.02 | GJA5_1 | 0.0669 | MIR654 | 0.0368 | ROD1_1 | 0.1936 | figo_numeric | 0.0098 |
| CIDEC 1 | 0.1064 | GMNN | 0.1039 | MIR942 | 0.0979 | SATB2 | 0.0363 | hist rev SBOT | 0.0556 |
| CLDN6 | 0.0006 | GSR_2 | 0.0198 | MMP12_1 | 0.1322 | SBSN | 0.0821 | surg outcome | 0.0089 |
| CREB5 2 | 0.0093 | HBA2 | 0.2079 | MYCN 2 | 0.1227 | SCXB | 0.0083 | | |
| CREBBP_1 | 0.0493 | HDAC7_2 | 0.0732 | MYOHD1 | 0.1099 | SEC22C_3 | 0.0939 | | |
| CRYBA1 | 0.0645 | HDLBP_3 | 0.0052 | NFATC3 _ 5 | 0.017 | SELENBP1 | 0.1504 | | |

Table 51

| | | | | | | | | | |
|---|---|---|---|---|---|---|---|---|---|
| ABHD3 | 0.0895 | CYP1A2 | 0.0607 | HPS4_1 | 0.0398 | NOVA2 | 0.0958 | SERPINB5 | 0.2131 |
| ADAM17_2 | 0.2342 | DBNDD2 | 0.0838 | HR_1 | 0.0544 | NP | 0.0913 | SFN | 0.0104 |
| ADAMTS1 | 0.1789 | DNAH11 | 0.0496 | HSD11B1_1 | 0.0892 | NR6A1_2 | 0.1335 | SFRS4 | 0.0424 |
| ALS2CL_3 | 0.1118 | DNMT3L_2 | 0.0335 | ICAM2 | 0.0475 | NRXN3 _ 3 | 0.077 | SHC1_3 | 0.1055 |
| ANO7_3 | 0.0427 | DOCK7_1 | 0.1066 | ICAM4_1 | 0.2773 | NT5DC1_2 | 0.2107 | SLC23A1_2 | 0.0986 |
| ARL6IP1_1 | 0.0328 | DSC3 _ 1 | 0.0589 | IL1RAP_2 | 0.0595 | NTRK2_3 | 0.0122 | SLC25A34 | 0.107 |
| ARMCX3_2 | 0.0876 | DUT_3 | 0.1352 | IQCA1_2 | 0.0233 | NUP155_1 | 0.0355 | SLC4A5_3 | 0.0793 |
| ATP2B1_3 | 0.1651 | EEF1E1_1 | 0.0554 | KCNIP3_1 | 0.0915 | NYX | 0.1133 | SLC9A10 | 0.0892 |
| ATXN10_1 | 0.0892 | EMP1 | 0.1048 | KCNQ2_1 | 0.147 | ODF2_3 | 0.0269 | SNORD93 | 0.1501 |
| AXL_1 | 0.0516 | ENO1 | 0.1538 | KIF3C | 0.191 | ORC1L | 0.0704 | SOX2_1 | 0.0608 |
| BAII-3 | 0.0156 | ENPEP 2 | 0.1276 | KRT80_2 | 0.0782 | OTUD7A_3 | 0.0327 | STC1 | 0.0086 |
| BCAS1_1 | 0.3163 | EPHB1 | 0.0403 | KRTAP10.10_2 | 0.009 | PANK4 | 0.0527 | STC2 | 0.0905 |
| BDNF_2 | 0.0983 | EPYC | 0.0208 | L3MBTL2_3 | 0.0308 | PDLIM2_2 | 0.2236 | STYX_2 | 0.0534 |
| BMPR1A | 0.1193 | ERI2_2 | 0.2871 | LBH_2 | 0.112 | PHYH_1 | 0.2248 | SYTL3 | 0.0026 |
| BTF3_3 | 0.1194 | ESPNL | 0.0816 | LENEP | 0.2121 | PIGA_1 | 0.0104 | TAF15_1 | 0.0179 |
| C10orfll6 | 0.0504 | EZH2_1 | 0.0653 | LGI3 | 0.1325 | PITX2_1 | 0.0894 | TCEAL8_1 | 0.0572 |
| C11orf24 | 0.1279 | FAM13AOS | 0.032 | LOC492303 | 0.0493 | PKN1_3 | 0.0599 | THBS3 | 0.0912 |
| C11orf49_3 | 0.1283 | FAM187B_2 | 0.0262 | LRRC14B | 0.0287 | PLAC9 | 0.2574 | TM2D3_2 | 0.047 |
| C14orf102_2 | 0.1 | FAM70A_1 | 0.104 | LRRC37A4 _ 2 | 0.0699 | PLEKHG5_5 | 0.0193 | TMEM52 | 0.0592 |
| C14orf109_2 | 0.0644 | FBXO48_2 | 0.2147 | LRRTM4 | 0.168 | PLSCR4 | 0.1686 | TMEM62 | 0.0063 |
| C17orf106 | 0.2144 | FKBP10 | 0.1034 | MACC1 | 0.1291 | PMEPA1 4 | 0.1266 | TNFRSF18_1 | 0.2489 |
| C17orf58_2 | 0.0323 | FLJ33360 | 0.0367 | MANSC1_1 | 0.128 | PNMA5 | 0.1496 | TNNT2_1 | 0.006 |
| C17orf58_3 | 0.0304 | FLJ43752 | 0.1831 | MAPK3_1 | 0.0622 | PPAPDC1A | 0.1127 | TOMM20L | 0.0459 |
| C18orf56 | 0.0422 | FMNL3_2 | 0.0158 | MCAM | 0.0933 | PRAMEF5 | 0.0323 | TPM2_2 | 0.1667 |
| C1orf168 | 0.0382 | FOSB | 0.1895 | MCART6_1 | 0.2201 | PRKAA2 | 0.1201 | TRIM58 | 0.1021 |
| C1orf64 | 0.1103 | FOSL2 | 0.0208 | MFRP | 0.0321 | PSMC6_1 | 0.0056 | UBR7_1 | 0.034 |

| | | | | | | | |
|---|---|---|---|---|---|---|---|
| C8orf79_1 | 0.0779 | FOXN1 | 0.2711 | MIDN | 0.0479 | RAD54B_2 | 0.1917 | UBR7_2 | 0.1325 |
| CALD1 2 | 0.1453 | GAD1_2 | 0.0091 | MIR1914 | 0.0663 | RAP1A_1 | 0.2144 | WARS_2 | 0.181 |
| CASP8AP2 | 0.1233 | GBE1 | 0.0599 | MIR212 | 0.0968 | RARA_3 | 0.0852 | XBP1_2 | 0.164 |
| CCL_13 | 0.1111 | GBP7 | 0.1071 | MIR571 | 0.0034 | RARG | 0.0034 | XRN2_1 | 0.0274 |
| CCR2_3 | 0.0465 | GJA5_1 | 0.0485 | MIR576 | 0.1 | RNASEK | 0.0584 | YARS2 | 0.0085 |
| CD34_1 | 0.0448 | GMNN | 0.0903 | MIR654 | 0.0046 | RNF7_1 | 0.017 | ZNF75D_2 | 0.1447 |
| CDC42BPA_2 | 0.0278 | GSR_2 | 0.0286 | MIR942 | 0.1109 | ROD1_1 | 0.2164 | ZSWIM4_2 | 0.1611 |
| CDC42SE2_2 | 0.0062 | GUSBL2 | 0.2001 | MMP12 1 | 0.1316 | SATB2 | 0.0525 | figo_numeric | 0.043 |
| CLDN6 | 0.1165 | HBA2 | 0.0605 | MYCN 2 | 0.1557 | SBSN | 0.059 | hist rev SBOT | 0.045 |
| CREB5_2 | 0.0067 | HDAC7_2 | 0.0429 | MYOHD1 | 0.0821 | SCXB | 0.0053 | surg_outcome | 0.0152 |
| CRYB_AI | 0.0333 | HDLBP_3 | 0.2083 | NFATC3_5 | 0.0231 | SEC22C_3 | 0.1068 | | |
| CXCL_13 | 0.0849 | HIC1 | 0.0782 | NFATC4 | 0.0519 | SELENBP1 | 0.1885 | | |
| CYB5R3_2 | 0.1675 | HPRT1_1 | 0.1481 | NLRP9 | 0.1553 | SERPINB2_2 | 0.0096 | | |

Table 52

| ABHD3 | 0.0643 | CYP1A2 | 0.0628 | HPS4_1 | 0.0647 | NOVA2 | 0.1207 | SERPINB5 | 0.1974 |
|---|---|---|---|---|---|---|---|---|---|
| ADAM17_2 | 0.2328 | DBNDD2 | 0.1096 | HR_1 | 0.0482 | NP | 0.0919 | SFN | 0.0056 |
| ADAMTS1 | 0.1768 | DFFB 2 | 0.0427 | HSD11B1_1 | 0.0797 | NR6A1 2 | 0.1526 | SFRS4 | 0.0285 |
| ALS2CL_3 | 0.1045 | DNAH11 | 0.0251 | ICAM2 | 0.0592 | NRXN3_3 | 0.1026 | SHCI_3 | 0.0709 |
| ANO7_3 | 0.0609 | DNMT3L_2 | 0.1376 | ICAM4_1 | 0.2765 | NT5DC1_2 | 0.1848 | SLC23A1_2 | 0.134 |
| ARL6IP1_1 | 0.0303 | DOCK7_1 | 0.0058 | IL1RAP_2 | 0.0478 | NTRK2_3 | 0.0046 | SLC25A34 | 0.155 |
| ARMCX3_2 | 0.0817 | DSC3_1 | 0.072 | IQCA1_2 | 0.0351 | NUP155_1 | 0.0486 | SLC4A5_3 | 0.0783 |
| ATP2B1_3 | 0.2063 | DUT_3 | 0.1169 | KCNIP3 1 | 0.1017 | NYX | 0.1717 | SLC9A10 | 0.0821 |
| ATXN10_1 | 0.1041 | EEF1E1_1 | 0.0798 | KCNQ2_1 | 0.1302 | ODF2_3 | 0.0126 | SNORD93 | 0.1554 |
| AXL_1 | 0.0323 | EMP1 | 0.1197 | KIF3C | 0.1759 | ORC1L | 0.0295 | SOX2_1 | 0.0805 |
| BAII_3 | 0.0262 | ENOI | 0.1874 | KRT80_2 | 0.1134 | OTUD7A 3 | 0.0328 | STC1 | 0.0033 |
| BCAS1_1 | 0.3374 | ENPEP_2 | 0.141 | KRTAP10.10_2 | 0.0208 | PANK4 | 0.0581 | STC2 | 0.1286 |
| BDNF_2 | 0.1102 | EPHB1 | 0.0359 | L3MBTL2_3 | 0.0365 | PDLIM2_2 | 0.2394 | STYX_2 | 0.0479 |
| BMPR1A | 0.1163 | EPYC | 0.0339 | LBH_2 | 0.1019 | PHYH 1 | 0.199 | SYTL3 | 0.0047 |
| BTF3_3 | 0.098 | ERI2_2 | 0.2917 | LENEP | 0.2237 | PIGA 1 | 0.0002 | TAF15_1 | 0.0001 |
| C10orf116 | 0.0264 | ESPNL | 0.0419 | LGI3 | 0.1147 | PITX2_1 | 0.0908 | TCEAL8_1 | 0.0337 |
| C11orf24 | 0.1822 | EZH2_1 | 0.0679 | LOC492303 | 0.0255 | PKN1_3 | 0.0275 | THBS3 | 0.0996 |
| C11orf49_3 | 0.1291 | FAM13AOS | 0.0482 | LRRC14B | 0.0144 | PLAC9 | 0.2579 | TM2D3_2 | 0.0554 |
| C14orf102_2 | 0.1272 | FAM187B_2 | 0.0133 | LRRC37A4_2 | 0.0611 | PLEKHG5_5 | 0.0328 | TMEM52 | 0.0839 |
| C14orf109_2 | 0.0647 | FAM70A_1 | 0.0779 | LRRTM4 | 0.1658 | PLSCR4 | 0.1771 | TMEM62 | 0.0056 |
| C17orf106 | 0.2377 | FBXO48_2 | 0.2662 | MACC1 | 0.1162 | PMEPA1_4 | 0.1204 | TNFRSF18_1 | 0.2606 |
| C17orf58_2 | 0.0515 | FKBP10 | 0.0632 | MANSC1_1 | 0.1357 | PNMA5 | 0.117 | TNNT2_1 | 0.0031 |
| C17orf58_3 | 0.0197 | FLJ33360 | 0.0563 | MAPK3_1 | 0.0175 | PPAPDC1A | 0.1296 | TOMM20L | 0.0531 |
| C18orf56 | 0.0336 | FLJ43752 | 0.1886 | MCAM | 0.1341 | PRAMEF5 | 0.0085 | TPM2_2 | 0.1772 |
| C1orfl68 | 0.0331 | FMNL3_2 | 0.0365 | MCART6_1 | 0.2205 | PRKAA2 | 0.1345 | TRIM58 | 0.1121 |
| C1orf64 | 0.1099 | FOSB | 0.2004 | MFRP | 0.0348 | PSMC6_1 | 0.0021 | UBR7_1 | 0.0582 |

(continued)

| C8orf79_1 | 0.0329 | FOSL2 | 0.0289 | MIDN | 0.0477 | RAD54B_2 | 0.1782 | UBR7_2 | 0.1274 |
|---|---|---|---|---|---|---|---|---|---|
| CALD1_2 | 0.1366 | FOXN1 | 0.2707 | MIR1914 | 0.0678 | RAP1A_1 | 0.2125 | WARS_2 | 0.1558 |
| CASP8AP2 | 0.1312 | GAD1_2 | 0.0238 | MIR212 | 0.1054 | RARA_3 | 0.0817 | XBP1_2 | 0.1344 |
| CCL_13 | 0.0888 | GBE1 | 0.0385 | MIR571 | 0.0357 | RARG | 0.0414 | XRN2_1 | 0.0507 |
| CCR2_3 | 0.0211 | GBP7 | 0.1356 | MIR576 | 0.1142 | RNASEK | 0.0641 | YARS2 | 0.001 |
| CD34_1 | 0.0174 | GJA5_1 | 0.0515 | MIR654 | 0.0496 | RNF7_1 | 0.0177 | ZNF75D_2 | 0.146 |
| CDC42BPA_2 | 0.0304 | GMNN | 0.1019 | MIR942 | 0.1318 | ROD1_1 | 0.2177 | ZSWIM4_2 | 0.1652 |
| CDC42SE2_2 | 0.0185 | GSR_2 | 0.0411 | MMP12_1 | 0.1354 | SATB2 | 0.0616 | figo_numeric | 0.0188 |
| CLDN6 | 0.1143 | HBA2 | 0.2058 | MYCN 2 | 0.148 | SBSN | 0.065 | hist rev SBOT | 0.0573 |
| CREB5 2 | 0.0211 | HDAC7_2 | 0.0611 | MYOHD1 | 0.0953 | SCXB | 0.0009 | surg_outcome | 0.0045 |
| CRYB_AI | 0.0238 | HDLBP_3 | 0.0135 | NFATC3_5 | 0.009 | SEC22C_3 | 0.1165 | | |
| CXCL_13 | 0.079 | HIC1 | 0.1066 | NFATC4 | 0.053 | SELENBP1 | 0.192 | | |
| CYB5R3_2 | 0.1854 | HPRT1_1 | 0.154 | NLRP9 | 0.1774 | SERPINB22 | 0.0118 | | |

Table 53

| ABHD3 | 0.0657 | CYP1A2 | 0.0645 | HR_1 | 0.0532 | NP | 0.091 | SFN | 0.0096 |
|---|---|---|---|---|---|---|---|---|---|
| ADAM17_2 | 0.2284 | DBNDD2 | 0.1086 | HSD11B1_1 | 0.0811 | NR6A1_2 | 0.1577 | SFRS4 | 0.0215 |
| ADAMTS1 | 0.1768 | DNAH11 | 0.0409 | ICAM2 | 0.0557 | NRXN3_3 | 0.1063 | SHC1_3 | 0.0541 |
| ALS2CL_3 | 0.1078 | DNMT3L_2 | 0.0275 | ICAM4_1 | 0.2758 | NT5DC1_2 | 0.176 | SLC23A1_2 | 0.1288 |
| ANO7_3 | 0.0644 | DOCK7_1 | 0.1407 | IL1RAP_2 | 0.0428 | NTRK2_3 | 0.003 | SLC25A34 | 0.1621 |
| ARL6IP1_1 | 0.0333 | DSC3_1 | 0.0755 | IQCA1_2 | 0.0281 | NUP155_1 | 0.0557 | SLC4A5_3 | 0.0816 |
| ARMCX3_2 | 0.0793 | DUT_3 | 0.1117 | KCNIP3 1 | 0.1006 | NYX | 0.1725 | SLC9A10 | 0.0744 |
| ATXN10_1 | 0.2139 | EEF1E1_1 | 0.0834 | KCNQ2_1 | 0.1265 | ODF2_3 | 0.0155 | SNORD93 | 0.1584 |
| AXL_1 | 0.107 | EMP1 | 0.1229 | KIF3C | 0.1707 | ORC1L | 0.0244 | SOX2_1 | 0.0751 |
| BAII_3 | 0.0256 | ENOI | 0.1858 | KRT80_2 | 0.111 | OTUD7A_3 | 0.0379 | STC1 | 0.0025 |
| BCAS1_1 | 0.3393 | ENPEP 2 | 0.1369 | KRTAP10.10_2 | 0.0202 | PANK4 | 0.0597 | STC2 | 0.1276 |
| BDNF_2 | 0.1033 | EPHB1 | 0.0251 | L3MBTL2 3 | 0.0415 | PDLIM2_2 | 0.2252 | STYX-2 | 0.0473 |
| BMPR1A | 0.1185 | EPYC | 0.0376 | LBH_2 | 0.1027 | PHYH_1 | 0.1951 | SYTL3 | 0.001 |
| BTF3_3 | 0.091 | ERI2_2 | 0.2825 | LENEP | 0.2253 | PIGA_1 | 0.003 | TAF15_1 | 0.0126 |
| C10orfl6 | 0.0269 | ESPNL | 0.044 | LGI3 | 0.1144 | PITX2_1 | 0.0961 | TCEAL8-1 | 0.0251 |
| C11orf24 | 0.1846 | EZH2_1 | 0.064 | LOC492303 | 0.0253 | PKN1 3 | 0.0207 | THBS3 | 0.0935 |
| C11orf49_3 | 0.1241 | FAM13AOS | 0.0489 | LRRC14B | 0.0162 | PLAC9 | 0.257 | TM2D3_2 | 0.0546 |
| C14orf102_2 | 0.1332 | FAM187B_2 | 0.013 | LRRC37A4_2 | 0.0579 | PLEKHG5_5 | 0.0261 | TMEM52 | 0.0831 |
| C14orf109_2 | 0.0686 | FAM70A_1 | 0.076 | LRRTM4 | 0.164 | PLSCR4 | 0.1668 | TMEM62 | 0.0049 |
| C17orf106 | 0.2275 | FBXO48_2 | 0.26 | MACC1 | 0.1121 | PMEPA1_4 | 0.1096 | TNFRSF18_1 | 0.2694 |
| C17orf58_2 | 0.052 | FKBP10 | 0.0638 | MANSC1_1 | 0.135 | PNMA5 | 0.1042 | TNNT2_1 | 0.0099 |
| C17orf58_3 | 0.0232 | FLJ33360 | 0.0603 | MAPK3_1 | 0.0256 | PPAPDC1A | 0.1256 | TOMM20L | 0.053 |
| C18orf56 | 0.0332 | FLJ43752 | 0.1886 | MCAM | 0.1396 | PRAMEF5 | 0.0042 | TPM2_2 | 0.167 |
| C1orfl68 | 0.0261 | FMNL3_2 | 0.032 | MCART6_1 | 0.2182 | PRKAA2 | 0.1387 | TRIM58 | 0.1201 |
| C1orf64 | 0.1053 | FOSB | 0.1974 | MFRP | 0.0284 | PSMC6_1 | 0.0044 | UBR7_1 | 0.0543 |
| C8orf79_1 | 0.0308 | FOSL2 | 0.0265 | MIDN | 0.0503 | RAD54B_2 | 0.1772 | UBR7_2 | 0.1156 |

(continued)

| CALD1_2 | 0.1359 | FOXN1 | 0.2699 | MIR1914 | 0.0648 | RAP1A_1 | 0.2049 | WARS_2 | 0.1563 |
|---|---|---|---|---|---|---|---|---|---|
| CASP8AP2 | 0.1334 | GAD1_2 | 0.0285 | MIR212 | 0.1032 | RARA_3 | 0.078 | XBP1_2 | 0.1348 |
| CCL_13 | 0.0936 | GBE1 | 0.0357 | MIR571 | 0.0362 | RARG | 0.047 | XRN2.1 | 0.0512 |
| CCR2_3 | 0.0134 | GBP7 | 0.1272 | MIR576 | 0.11 | RNASEK | 0.07 | YARS2 | 0.0014 |
| CD34_1 | 0.0137 | GJA5_1 | 0.0544 | MIR654 | 0.0493 | RNF7_1 | 0.0239 | ZNF75D_2 | 0.1477 |
| CDC42BPA 2 | 0.0398 | GMNN | 0.1028 | MIR942 | 0.1301 | ROD1_1 | 0.2187 | ZSWIM4_2 | 0.1654 |
| CDC42SE2 2 | 0.0157 | GSR_2 | 0.0467 | MMP12_1 | 0.1397 | SATB2 | 0.0632 | figo_numeric | 0.0092 |
| CLDN6 | 0.115 | HBA2 | 0.2041 | MYCN 2 | 0.1467 | SBSN | 0.0725 | hist rev SBOT | 0.071 |
| CREB5_2 | 0.0255 | HDAC7_2 | 0.0649 | MYOHD1 | 0.0968 | SCXB | 0.0007 | surg_outcome | 0.0015 |
| CREBBP_1 | 0.0262 | HDLBP_3 | 0.0122 | NFATC3_5 | 0.0088 | SEC22C_3 | 0.1111 | | |
| CRYB_AI | 0.0813 | HIC1 | 0.1098 | NFATC4 | 0.0519 | SELENBP1 | 0.194 | | |
| CXCL_13 | 0.1902 | HPRT1_1 | 0.1609 | NLRP9 | 0.1852 | SERPINB2 _ 2 | 0.0258 | | |
| CYB5R3_2 | 0.1199 | HPS4_1 | 0.0654 | NOVA2 | 0.1234 | SERPINB5 | 0.1961 | | |

111

Table 54

| | | | | | | | | | |
|---|---|---|---|---|---|---|---|---|---|
| ABCC9_3 | 0.0543 | CYP1A2 | 0.0776 | HPS4_1 | 0.0652 | NP | 0.0758 | SFRS4 | 0.0654 |
| ABHD3 | 0.2423 | DBNDD2 | 0.1256 | HR_1 | 0.0241 | NR6A1_2 | 0.1264 | SHC1_3 | 0.0707 |
| ADAM17_2 | 0.1473 | DNAH11 | 0.0414 | HSD11B11 | 0.0913 | NRXN3_3 | 0.1707 | SLC23A1_2 | 0.1276 |
| ADAMTS1 | 0.1127 | DNMT3L_2 | 0.0199 | ICAM2 | 0.0133 | NT5DC1_2 | 0.1807 | SLC25A34 | 0.1046 |
| ADAMTS2_1 | 0.1041 | DOCK7_1 | 0.1092 | ICAM4_1 | 0.2949 | NTRK2_3 | 0.0046 | SLC4A5_3 | 0.0855 |
| ALS2CL_3 | 0.0601 | DSC3_1 | 0.0425 | IL1RAP_2 | 0.0823 | NUP155_1 | 0.0259 | SLC9A10 | 0.0704 |
| ANO7_3 | 0.0425 | DUT 3 | 0.1247 | IQCA1_2 | 0.0227 | NYX | 0.1098 | SNORD93 | 0.1306 |
| ARL6IP1_1 | 0.0019 | EEF1E1_1 | 0.1296 | KCNIP3_1 | 0.0912 | ODF2_3 | 0.0179 | SOX2_1 | 0.0723 |
| ARMCX3_2 | 0.0636 | ELN 2 | 0.1167 | KCNQ2_1 | 0.0999 | ORC1L | 0.0388 | STC1 | 0.0051 |
| ATXN10_1 | 0.2046 | EMP1 | 0.2027 | KIF3C | 0.1819 | OTUD7A_3 | 0.0439 | STC2 | 0.1139 |
| AXL_1 | 0.0795 | ENOI | 0.1576 | KRT80_2 | 0.0972 | PANK4 | 0.0424 | STYX_2 | 0.0107 |
| BAII_3 | 0.0404 | ENPEP_2 | 0.0827 | KRTAP10.10_2 | 0.0269 | PDLIM2_2 | 0.2119 | SYTL3 | 0.0249 |
| BCAS1_1 | 0.3089 | EPHB1 | 0.0476 | L3MBTL2_3 | 0.0433 | PDZRN4_2 | 0.205 | TAF15_1 | 0.0259 |
| BDNF_2 | 0.1255 | EPYC | 0.0349 | LBH_2 | 0.0755 | PHYH_1 | 0.0138 | TCEAL8_1 | 0.0144 |
| BMPR1A | 0.1121 | ERI2_2 | 0.267 | LENEP | 0.2366 | PIGA_1 | 0.0917 | THBS3 | 0.0976 |
| BTF3_3 | 0.1063 | ESPNL | 0.0611 | LGI3 | 0.0985 | PITX2_1 | 0.201 | THY1 | 0.0373 |
| C10orfll6 | 0.0748 | EZH2 1 | 0.0368 | LOC340508 | 0.0304 | PKN1_3 | 0.0078 | TIMP2_2 | 0.0975 |
| Cllorf24 | 0.1832 | FAM13AOS | 0.0656 | LOC492303 | 0.022 | PLEKHG5_5 | 0.2566 | TM2D3_2 | 0.0021 |
| C11orf49_3 | 0.1119 | FAM187B_2 | 0.0044 | LRRC14B | 0.0718 | PLSCR4 | 0.0187 | TMEM52 | 0.0217 |
| C14orf102_2 | 0.1038 | FAM70A_1 | 0.082 | LRRC37A4 _ 2 | 0.0176 | PMEPA1_4 | 0.1384 | TMEM62 | 0.0646 |
| C14orf109_2 | 0.1136 | FBXO48_2 | 0.2301 | LRRTM4 | 0.1685 | PNMA5 | 0.1752 | TNFRSF18_1 | 0.2151 |
| C17orf106 | 0.1626 | FKBP10 | 0.064 | MACC1 | 0.1635 | PPAPDC1A | 0.1216 | TNNT2_1 | 0.0075 |
| C17orf58_2 | 0.0122 | FLJ33360 | 0.0153 | MANSC1_1 | 0.1141 | PRAMEF5 | 0.0036 | TOMM20L | 0.001 |
| C17orf58_3 | 0.0168 | FLJ43752 | 0.2483 | MCAM | 0.0229 | PRKAA2 | 0.1182 | TPM2 2 | 0.181 |
| C18orf56 | 0.0024 | FMNL3 2 | 0.0121 | MCART6_1 | 0.1238 | PSMC6_1 | 0.0364 | TRIM58 | 0.115 |
| Clorf168 | 0.0362 | FOSB | 0.2134 | MFRP | 0.2252 | RAD54B _ 2 | 0.1722 | UBR7_1 | 0.0759 |

(continued)

| | | | | | | | | | |
|---|---|---|---|---|---|---|---|---|---|
| C1orf64 | 0.1183 | FOSL2 | 0.0284 | MIDN | 0.0077 | RAP1A 1 | 0.1922 | UBR7_2 | 0.1396 |
| C8orf79_1 | 0.0052 | FOXN1 | 0.2589 | MIR1914 | 0.0573 | RARA_3 | 0.0942 | WARS_2 | 0.1866 |
| CASP8AP2 | 0.1416 | GAD1 2 | 0.019 | MIR212 | 0.0962 | RARG | 0.0807 | XBP1_2 | 0.1516 |
| CCL_13 | 0.1337 | GBE1 | 0.0572 | MIR571 | 0.0025 | RNASEK | 0.0762 | XRN2_1 | 0.0393 |
| CCR2_3 | 0.1294 | GBP7 | 0.1378 | MIR576 | 0.108 | RNF7_1 | 0.0257 | YARS2 | 0.0272 |
| CD34_1 | 0.034 | GJA5_1 | 0.0707 | MIR654 | 0.0409 | ROD1_1 | 0.1981 | ZNF75D_2 | 0.1344 |
| CDC42BPA_2 | 0.0047 | GMNN | 0.1035 | MIR942 | 0.1074 | SATB2 | 0.0347 | ZSWIM4 2 | 0.1752 |
| CDC42SE2_2 | 0.014 | GSR 2 | 0.0243 | MMP12_1 | 0.1182 | SBSN | 0.0724 | figo_numeric | 0.0248 |
| CIDEC_1 | 0.1045 | HBA2 | 0.2092 | MYCN_2 | 0.1305 | SCXB | 0.0142 | hist rev SBOT | 0.0369 |
| CLDN6 | 0.0153 | HCFC1R1_1 | 0.0666 | MYOHD1 | 0.1036 | SEC22C 3 | 0.1071 | surg_outcome | 0.0132 |
| CREB5 2 | 0.0067 | HDAC7_2 | 0.0093 | NFATC3_5 | 0.0218 | SELENBP1 | 0.1474 | | |
| CRYBA1 | 0.0575 | HDLBP _ 3 | 0.099 | NFATC4 | 0.0352 | SERPINB2 _ 2 | 0.0165 | | |
| CXCL13 | 0.0588 | HIC1 | 0.0033 | NLRP9 | 0.1773 | SERPINBS | 0.1785 | | |
| CYB5R3_2 | 0.1811 | HPRT1_1 | 0.1305 | NOVA2 | 0.0688 | SFN | 0.017 | | |

Table 55

| | | | | | | | | | |
|---|---|---|---|---|---|---|---|---|---|
| ABCC9_3 | 0.0363 | CYB5R3_2 | 0.1898 | HPRT1_1 | 0.1236 | NLRP9 | 0.1733 | SERPINB5 | 0.1786 |
| ABHD3 | 0.2308 | CYP1A2 | 0.071 | HPS4_1 | 0.0602 | NOVA2 | 0.0654 | SFN | 0.0076 |
| ADAM17_2 | 0.1354 | DBNDD2 | 0.1155 | HR_1 | 0.03 | NP | 0.0701 | SFRS4 | 0.0701 |
| ADAMTS1 | 0.1016 | DNAH11 | 0.0315 | HSD11B1_1 | 0.0849 | NR6A1_2 | 0.1285 | SHC1_3 | 0.0709 |
| ADAMTS2_1 | 0.0919 | DNMT3L_2 | 0.0195 | ICAM2 | 0.0189 | NRXN3_3 | 0.1626 | SLC23A1_2 | 0.1308 |
| ALS2CL_3 | 0.0595 | DOCK7_1 | 0.1142 | ICAM4_1 | 0.2914 | NT5DC1_2 | 0.1734 | SLC25A34 | 0.1157 |
| ANO7_3 | 0.03 | DSC3_1 | 0.0334 | IL1RAP_2 | 0.0755 | NTRK2_3 | 0.0138 | SLC4A5_3 | 0.0848 |
| ANTXR1_4 | 0.0244 | DUT_3 | 0.1178 | IQCA1_2 | 0.0234 | NUP155_1 | 0.0235 | SLC9A10 | 0.0604 |
| ARL6IP1_1 | 0.0574 | EEF1E1_1 | 0.1312 | KCNIP3_1 | 0.094 | NYX | 0.0955 | SNORD93 | 0.1387 |
| ARMCX3_2 | 0.1944 | ELN_2 | 0.1075 | KCNQ2_1 | 0.0971 | ODF2_3 | 0.0219 | SOX2_1 | 0.0749 |
| ATXN10_1 | 0.1342 | EMP1 | 0.2007 | KIF3C | 0.1745 | ORC1L | 0.0319 | STC1 | 0.0091 |
| AXL_1 | 0.0759 | ENO1 | 0.1647 | KRT80_2 | 0.1065 | OTUD7A_3 | 0.0385 | STC2 | 0.1176 |
| BAI1_3 | 0.05 | ENPEP_2 | 0.0593 | KRTAP10.10_2 | 0.0262 | PANK4 | 0.0535 | STYX_2 | 0.0175 |
| BCAS1_1 | 0.3006 | EPHB1 | 0.0529 | L3MBTL2_3 | 0.0598 | PDLIM2_2 | 0.2298 | SYTL3 | 0.024 |
| BDNF_2 | 0.1243 | EPYC | 0.0509 | LBH_2 | 0.0794 | PDZRN4_2 | 0.2008 | TAF15_1 | 0.0479 |
| BMPR1A | 0.1071 | ERI2_2 | 0.2695 | LENEP | 0.2337 | PHYH_1 | 0.0124 | TCEAL8_1 | 0.0069 |
| BTF3_3 | 0.0955 | ESPNL | 0.0572 | LGI3 | 0.087 | PIGA_1 | 0.1008 | THBS3 | 0.0818 |
| C10orf116 | 0.0595 | EZH2_1 | 0.0272 | LOC340508 | 0.021 | PITX2_1 | 0.2061 | THY1 | 0.0363 |
| C11orf24 | 0.1965 | FAM13AOS | 0.0728 | LOC492303 | 0.0229 | PKN1_3 | 0.0009 | TM2D3_2 | 0.1158 |
| C11orf49_3 | 0.108 | FAM187B_2 | 0.0049 | LRRC14B | 0.0771 | PLEKHG5_5 | 0.2748 | TMEM52 | 0.0037 |
| C14orf102_2 | 0.0998 | FAM70A_1 | 0.0742 | LRRC37A4_2 | 0.0118 | PLSCR4 | 0.0266 | TMEM62 | 0.0154 |
| C14orf109_2 | 0.1233 | FBXO48_2 | 0.2335 | LRRTM4 | 0.1777 | PMEPA1_4 | 0.1197 | TNFRSF18_1 | 0.209 |
| C17orf106 | 0.1689 | FKBP10 | 0.0731 | MACC1 | 0.1721 | PNMA5 | 0.1628 | TNNT2_1 | 0.0064 |
| C17orf58_2 | 0.0138 | FLJ33360 | 0.026 | MANSC1_1 | 0.1226 | PPAPDC1A | 0.1228 | TOMM20L | 0.0065 |
| C17orf58_3 | 0.0176 | FLJ43752 | 0.2477 | MCAM | 0.0209 | PRAMEF5 | 0.0044 | TPM2_2 | 0.1722 |
| C18orf56 | 0.0039 | FMNL3_2 | 0.0087 | MCART6_1 | 0.1277 | PRKAA2 | 0.1083 | TRIM58 | 0.1096 |

| | | | | | | | | | |
|---|---|---|---|---|---|---|---|---|---|
| Clorf168 | 0.0342 | FOSB | 0.2167 | MFRP | 0.231 | PSMC6_1 | 0.0355 | UBR7_1 | 0.0847 |
| Clorf64 | 0.1156 | FOSL2 | 0.0267 | MIDN | 0.0025 | RAD54B_2 | 0.1763 | UBR7_2 | 0.1296 |
| C8orf79_1 | 0.013 | FOXN1 | 0.2584 | MIR1914 | 0.0507 | RAP1A_1 | 0.2003 | WARS_2 | 0.1734 |
| CASP8AP2 | 0.1491 | GAD1_2 | 0.0243 | MIR212 | 0.0909 | RARA_3 | 0.1036 | XBP1_2 | 0.1254 |
| CCL13 | 0.1171 | GBE1 | 0.049 | MIR571 | 0.0065 | RARG | 0.0831 | XRN2_1 | 0.0348 |
| CCR2_3 | 0.1276 | GBP7 | 0.1241 | MIR576 | 0.1209 | RNASEK | 0.0789 | YARS2 | 0.022 |
| CD34_1 | 0.0281 | GJA5_1 | 0.062 | MIR654 | 0.0433 | RNF7_1 | 0.0396 | ZNF75D_2 | 0.1156 |
| CDC42BPA_2 | 0.0118 | GMNN | 0.1054 | MIR942 | 0.0953 | ROD1_1 | 0.1976 | ZSWIM4_2 | 0.1692 |
| CDC42SE2_2 | 0.0229 | GSR_2 | 0.0152 | MMP12_1 | 0.1149 | SATB2 | 0.0343 | figo_numeric | 0.0155 |
| CIDEC_1 | 0.1068 | HBA2 | 0.196 | MYCN_2 | 0.1309 | SBSN | 0.0729 | hist rev SBOT | 0.048 |
| CLDN6 | 0.0049 | HCFC1R1_1 | 0.06 | MYL9_2 | 0.1119 | SCXB | 0.0149 | surg outcome | 0.0067 |
| CREB5_2 | 0.01 | HDAC7_2 | 0.0029 | MYOHD1 | 0.0195 | SEC22C_3 | 0.1034 | | |
| CRYBA1 | 0.0522 | HDLBP_3 | 0.0906 | NFATC3_5 | 0.0451 | SELENBP1 | 0.1459 | | |
| CXCL 13 | 0.0598 | HIC1 | 0.0135 | NFATC4 | 0.0617 | SERPINB2_2 | 0.0047 | | |

EP 3 874 274 B1

115

Table 56

| | | | | | | | | |
|---|---|---|---|---|---|---|---|---|
| ABCC9_3 | 0.0551 | CYP1A2 | 0.0757 | HPS4_1 | 0.0621 | NOVA2 | 0.0704 | SFN | 0.0181 |
| ABHD3 | 0.2421 | DBNDD2 | 0.1318 | HR_1 | 0.021 | NP | 0.0733 | SFRS4 | 0.0693 |
| ADAM17_2 | 0.1462 | DNAH11 | 0.043 | HSD11B1_1 | 0.088 | NR6A1_2 | 0.1291 | SHC1_3 | 0.0685 |
| ADAMTS1 | 0.114 | DNMT3L_2 | 0.0208 | ICAM2 | 0.019 | NRXN3_3 | 0.169 | SLC23A1_2 | 0.1277 |
| ADAMTS2_1 | 0.1025 | DOCK7_1 | 0.1131 | ICAM4_1 | 0.2947 | NT5DC1_2 | 0.1829 | SLC25A34 | 0.105 |
| ALS2CL_3 | 0.0551 | DSC3_1 | 0.0415 | IL1RAP_2 | 0.0794 | NTRK2_3 | 0.0051 | SLC4A5_3 | 0.0881 |
| ANO7_3 | 0.0368 | DUT_3 | 0.1213 | IQCA1_2 | 0.0196 | NUP155_1 | 0.025 | SLC9A10 | 0.0657 |
| ARL6IP1_1 | 0.001 | EEF1E1_1 | 0.1344 | KCNIP3_1 | 0.0934 | NYX | 0.1094 | SNORD93 | 0.1246 |
| ARMCX3_2 | 0.0618 | ELN_2 | 0.1216 | KCNQ2_1 | 0.1022 | ODF2_3 | 0.0155 | SOX2_1 | 0.0791 |
| ATXN10_1 | 0.2041 | EMP1 | 0.2013 | KIF3C | 0.1799 | ORC1L | 0.0372 | STC1 | 0.003 |
| AXL_1 | 0.0781 | ENO1 | 0.1563 | KRT80_2 | 0.0974 | OTUD7A_3 | 0.0442 | STC2 | 0.1131 |
| BAI1_3 | 0.0391 | ENPEP_2 | 0.0804 | KRTAP10.10_2 | 0.0279 | PANK4 | 0.0437 | STYX_2 | 0.0137 |
| BCAS1_1 | 0.3072 | EPHB1 | 0.0428 | L3MBTL2_3 | 0.0415 | PDLIM2_2 | 0.2169 | SYTL3 | 0.027 |
| BDNF_2 | 0.1215 | EPYC | 0.0341 | LBH_2 | 0.0725 | PDZRN4_2 | 0.2047 | TAF15_1 | 0.0207 |
| BMPR1A | 0.1145 | ERI2_2 | 0.2708 | LENEP | 0.2404 | PHYH_1 | 0.0101 | TCEAL8_1 | 0.0124 |
| BTF3_3 | 0.108 | ESPNL | 0.0577 | LGI3 | 0.0883 | PIGA_1 | 0.0908 | THBS3 | 0.0997 |
| C10orf116 | 0.0775 | EZH2_1 | 0.0393 | LOC340508 | 0.0255 | PITX2_1 | 0.2007 | TIMP2_2 | 0.0391 |
| C11orf24 | 0.1816 | FAM13AOS | 0.0689 | LOC492303 | 0.0222 | PKN1_3 | 0.0052 | TM2D3_2 | 0.0923 |
| C11orf49_3 | 0.1111 | FAM187B_2 | 0.0034 | LRRC14B | 0.0768 | PLEKHG5_5 | 0.2567 | TMEM52 | 0.0006 |
| C14orf102_2 | 0.0994 | FAM70A_1 | 0.0822 | LRRC37A4_2 | 0.0204 | PLSCR4 | 0.0187 | TMEM62 | 0.0672 |
| C14orf109_2 | 0.1148 | FBXO48_2 | 0.2239 | LRRTM4 | 0.1667 | PMEPA1_4 | 0.1358 | TNFRSF18_1 | 0.222 |
| C17orf106 | 0.1615 | FKBP10 | 0.066 | MACC1 | 0.161 | PNMA5 | 0.1706 | TNNT2_1 | 0.0095 |
| C17orf58_2 | 0.019 | FLJ33360 | 0.0157 | MANSC1_1 | 0.1117 | PPAPDC1A | 0.1242 | TOMM20L | 0.0003 |
| C17orf58_3 | 0.0153 | FLJ43752 | 0.2403 | MCAM | 0.017 | PRAMEF5 | 0.0092 | TPM2_2 | 0.178 |
| C18orf56 | 0.0018 | FMNL3_2 | 0.0157 | MCART6_1 | 0.1234 | PRKAA2 | 0.1234 | TRIM58 | 0.115 |
| C1orf168 | 0.0368 | FOSB | 0.2176 | MFRP | 0.2237 | PSMC6_1 | 0.0397 | UBR7_1 | 0.0826 |

(continued)

| C1orf64 | 0.1171 | FOSL2 | 0.0301 | MIDN | 0.0021 | RAD54B_2 | 0.1761 | UBR7_2 | 0.1381 |
|---|---|---|---|---|---|---|---|---|---|
| C8orf79_1 | 0.006 | FOXN1 | 0.2623 | MIR1914 | 0.0537 | RAP1A_1 | 0.1946 | WARS_2 | 0.184 |
| CASP8AP2 | 0.1405 | GAD1_2 | 0.0161 | MIR212 | 0.0981 | RARA_3 | 0.0955 | XBP1_2 | 0.146 |
| CCL13 | 0.123 | GBE1 | 0.0553 | MIR571 | 0 | RARG | 0.0821 | XRN2_1 | 0.044 |
| CCR2_3 | 0.1285 | GBP7 | 0.1383 | MIR576 | 0.1099 | RNASEK | 0.0783 | YARS2 | 0.0299 |
| CD34_1 | 0.0266 | GJA5_1 | 0.0684 | MIR654 | 0.0423 | RNF7_1 | 0.0237 | ZNF75D_2 | 0.1344 |
| CDC42BPA_2 | 0.0051 | GMNN | 0.1055 | MIR942 | 0.0976 | ROD1_1 | 0.2044 | ZSWIM4_2 | 0.1743 |
| CDC42SE2_2 | 0.0186 | GSR_2 | 0.0228 | MMP12_1 | 0.12 | SATB2 | 0.0369 | figo_numeric | 0.0227 |
| CIDEC_1 | 0.1018 | HBA2 | 0.205 | MYCN_2 | 0.1331 | SBSN | 0.0734 | hist_rev_SBOT | 0.0382 |
| CLDN6 | 0.0127 | HCFC1R1_1 | 0.0649 | MYL9_2 | 0.1035 | SCXB | 0.0138 | surg_outcome | 0.0106 |
| CREB5_2 | 0.015 | HDAC7_2 | 0.007 | MYOHD1 | 0.0219 | SEC22C_3 | 0.1017 | | |
| CRYBA1 | 0.0605 | HDLBP_3 | 0.0942 | NFATC3_5 | 0.0381 | SELENBP1 | 0.147 | | |
| CXCL13 | 0.0588 | HIC1 | 0.0017 | NFATC4 | 0.0694 | SERPINB2_2 | 0.0097 | | |
| CYB5R3_2 | 0.184 | HPRT1_1 | 0.1355 | NLRP9 | 0.1732 | SERPINB5 | 0.1745 | | |

Table 57

| Gene | Value | Gene | Value | Gene | Value | Gene | Value | Gene | Value |
|---|---|---|---|---|---|---|---|---|---|
| ABHD3 | 0.0642 | DNAH11 | 0.0501 | HPRT1_1 | 0.1484 | NP | 0.0786 | SFN | 0.0154 |
| ADAM17_2 | 0.2339 | DNMT3L_2 | 0.02 | HPS4_1 | 0.0713 | NR6A1_2 | 0.1481 | SFRS4 | 0.0376 |
| ADAMTS1 | 0.1728 | DOCK7_1 | 0.127 | HR_1 | 0.0435 | NRXN3_3 | 0.0994 | SHC1_3 | 0.0715 |
| ALS2CL_3 | 0.1139 | DSC3_1 | 0.0611 | HSD11B1_1 | 0.1011 | NT5DC1_2 | 0.1985 | SLC23A1_2 | 0.1364 |
| ANO7_3 | 0.0798 | DUT_3 | 0.1237 | ICAM2 | 0.0497 | NTRK2_3 | 0.0061 | SLC25A34 | 0.1695 |
| ARL6IP1_1 | 0.032 | EEF1E1_1 | 0.1023 | ICAM4_1 | 0.2803 | NUP155_1 | 0.0626 | SLC4A5_3 | 0.081 |
| ARMCX3_2 | 0.0865 | EIF4ENIF1 | 0.1116 | IL1RAP_2 | 0.0686 | NYX | 0.1753 | SLC9A10 | 0.0879 |
| ATXN10_1 | 0.2036 | EMP1 | 0.1674 | IQCA1_2 | 0.0231 | ODF2_3 | 0.0161 | SNORD93 | 0.1688 |
| AXL_1 | 0.1146 | ENO1 | 0.1366 | KCNIP3_1 | 0.1037 | ORC1L | 0.0257 | SOX2_1 | 0.0728 |
| BAI1_3 | 0.0421 | ENPEP_2 | 0.0131 | KCNQ2_1 | 0.1262 | OTUD7A_3 | 0.0323 | STC1 | 0.0127 |
| BCAS1_1 | 0.3262 | EPHB1 | 0.0313 | KIF3C | 0.1913 | PANK4 | 0.0572 | STC2 | 0.135 |
| BDNF_2 | 0.124 | EPYC | 0.0352 | KRT80_2 | 0.1143 | PDLIM2_2 | 0.2354 | STYX_2 | 0.0462 |
| BMPR1A | 0.104 | ERI2_2 | 0.305 | KRTAP10.10_2 | 0.023 | PHYH_1 | 0.1976 | SYTL3 | 0.0117 |
| BTF3_3 | 0.1055 | ESPNL | 0.0421 | L3MBTL2_3 | 0.0312 | PIGA_1 | 0.0094 | TAF15_1 | 0.0117 |
| C10orf116 | 0.0282 | EZH2_1 | 0.0741 | LBH_2 | 0.0936 | PITX2_1 | 0.0919 | TCEAL8_1 | 0.0445 |
| C11orf24 | 0.1814 | FAM13AOS | 0.0355 | LENEP | 0.2283 | PKN1_3 | 0.017 | THBS3 | 0.1055 |
| C11orf49_3 | 0.1315 | FAM187B_2 | 0.0113 | LGI3 | 0.1313 | PLAC9 | 0.2381 | THY1 | 0.0613 |
| C14orf102_2 | 0.1313 | FAM70A_1 | 0.0699 | LOC492303 | 0.0382 | PLEKHG5_5 | 0.0243 | TIMP2_2 | 0.0807 |
| C14orf109_2 | 0.0748 | FBXO48_2 | 0.2634 | LRRC14B | 0.0225 | PLSCR4 | 0.1715 | TM2D3_2 | 0.0101 |
| C17orf106 | 0.2458 | FGF5_1 | 0.0715 | LRRC37A4_2 | 0.0591 | PMEPA1_4 | 0.1272 | TMEM52 | 0.0357 |
| C17orf58_2 | 0.0334 | FKBP10 | 0.0412 | LRRTM4 | 0.1778 | PNMA5 | 0.121 | TMEM62 | 0.0698 |
| C17orf58_3 | 0.0243 | FLJ33360 | 0.2035 | MACC1 | 0.1325 | PPAPDC1A | 0.1269 | TNFRSF18_1 | 0.2592 |
| C18orf56 | 0.0448 | FLJ43752 | 0.0711 | MANSC1_1 | 0.1414 | PRAMEF5 | 0.011 | TNNT2_1 | 0.0071 |
| C1orf168 | 0.0354 | FMNL3_2 | 0.0407 | MCAM | 0.0258 | PRKAA2 | 0.1396 | TOMM20L | 0.0412 |
| C1orf64 | 0.1116 | FMOD | 0.1931 | MCART6_1 | 0.1484 | PSMC6_1 | 0.0134 | TPM2_2 | 0.1777 |
| C8orf79_1 | 0.0063 | FOSB | 0.0261 | MFRP | 0.2179 | RAD54B_2 | 0.184 | TRIM58 | 0.1106 |

| | | | | | | | | | |
|---|---|---|---|---|---|---|---|---|---|
| CASP8AP2 | 0.1353 | FOSL2 | 0.2651 | MIDN | 0.044 | RAP1A_1 | 0.2177 | UBR7_1 | 0.0689 |
| CCL13 | 0.1464 | FOXN1 | 0.033 | MIR1914 | 0.0668 | RARA_3 | 0.0861 | UBR7_2 | 0.1189 |
| CCR2_3 | 0.0935 | GAD1_2 | 0.0208 | MIR212 | 0.1071 | RARG | 0.0469 | WARS_2 | 0.153 |
| CD34_1 | 0.0084 | GBE1 | 0.0481 | MIR571 | 0.035 | RNASEK | 0.0707 | XBP1_2 | 0.1393 |
| CDC42BPA_2 | 0.0185 | GBP7 | 0.13 | MIR576 | 0.0983 | RNF7_1 | 0.0183 | XRN2_1 | 0.0533 |
| CDC42SE2_2 | 0.0265 | GJA5_1 | 0.0509 | MIR654 | 0.0624 | ROD1_1 | 0.2173 | YARS2 | 0.0008 |
| CLDN6 | 0.1037 | GMNN | 0.0929 | MIR942 | 0.1443 | SATB2 | 0.0599 | ZNF75D_2 | 0.1617 |
| CREB5_2 | 0.0145 | GSR_2 | 0.0473 | MMP12_1 | 0.126 | SBSN | 0.0498 | ZSWIM4_2 | 0.1597 |
| CRYBA1 | 0.0178 | HBA2 | 0.2102 | MYCN_2 | 0.1402 | SCXB | 0.009 | figo_numeric | 0.0171 |
| CXCL13 | 0.0782 | HCFC1R1_1 | 0.0587 | NFATC3_5 | 0.1015 | SEC22C_3 | 0.116 | hist_rev_SBOT | 0.0582 |
| CYB5R3_2 | 0.1846 | HDAC7_2 | 0.0045 | NFATC4 | 0.0053 | SELENBP1 | 0.1894 | surg_outcome | 0.002 |
| CYP1A2 | 0.0522 | HDLBP_3 | 0.1011 | NLRP9 | 0.054 | SERPINB2_2 | 0.0164 | | |
| DBNDD2 | 0.1019 | HIC1 | 0.038 | NOVA2 | 0.12 | SERPINBS | 0.2094 | | |

Table 58

| | | | | | | | | | |
|---|---|---|---|---|---|---|---|---|---|
| ABHD3 | 0.0552 | DNAH11 | 0.0398 | HPS4_1 | 0.0808 | NR6A1_2 | 0.1497 | SHC1_3 | 0.0768 |
| ADAM17_2 | 0.2207 | DNMT3L_2 | 0.0242 | HR_1 | 0.0534 | NRXN3_3 | 0.0907 | SLC23A1_2 | 0.1304 |
| ADAMTS1 | 0.1613 | DOCK7_1 | 0.1054 | HSD11B1_1 | 0.0889 | NT5DC1_2 | 0.1789 | SLC25A34 | 0.1714 |
| ALS2CL_3 | 0.1019 | DSC3_1 | 0.0675 | ICAM2 | 0.074 | NTRK2_3 | 0.0085 | SLC4A5_3 | 0.0737 |
| ANO7_3 | 0.0683 | DUT_3 | 0.1206 | ICAM4_1 | 0.2733 | NUP155_1 | 0.052 | SLC9A10 | 0.0721 |
| ANTXR1_4 | 0.0226 | EEF1E1_1 | 0.1002 | IL1RAP_2 | 0.0561 | NYX | 0.1468 | SNORD93 | 0.1695 |
| ARL6IP1_1 | 0.0916 | EIF4ENIF1 | 0.1119 | IQCA1_2 | 0.0292 | ODF2_3 | 0.0051 | SOX2_1 | 0.0682 |
| ARMCX3_2 | 0.1859 | EMP1 | 0.1608 | KCNIP3_1 | 0.0983 | ORC1L | 0.0197 | STC1 | 0.0075 |
| ATXN10_1 | 0.1744 | ENO1 | 0.1399 | KCNQ2_1 | 0.1237 | OTUD7A_3 | 0.0222 | STC2 | 0.1235 |
| AXL_1 | 0.1084 | ENPEP_2 | 0.0156 | KIF3C | 0.1983 | PANK4 | 0.0714 | STYX_2 | 0.0465 |
| BAI1_3 | 0.0478 | EPHB1 | 0.0301 | KRT80_2 | 0.1125 | PDLIM2_2 | 0.2393 | SYTL3 | 0.0017 |
| BCAS1_1 | 0.3244 | EPYC | 0.048 | KRTAP10.10_2 | 0.0197 | PHYH_1 | 0.1915 | TAF15_1 | 0.0289 |
| BDNF_2 | 0.1137 | ERI2_2 | 0.294 | L3MBTL2_3 | 0.0379 | PIGA_1 | 0.0132 | TCEAL8_1 | 0.0274 |
| BMPR1A | 0.0975 | ESPNL | 0.0416 | LBH_2 | 0.1024 | PITX2_1 | 0.0995 | THBS3 | 0.0867 |
| BTF3_3 | 0.0978 | EZH2_1 | 0.0526 | LENEP | 0.217 | PKN1_3 | 0.0029 | THY1 | 0.0608 |
| C10orf116 | 0.0139 | FAM13AOS | 0.0436 | LGI3 | 0.1299 | PLAC9 | 0.2558 | TM2D3_2 | 0.105 |
| C11orf24 | 0.2032 | FAM187B_2 | 0.0219 | LOC492303 | 0.0227 | PLEKHG5_5 | 0.0321 | TlvfEbf52 | 0.0192 |
| C11orf49_3 | 0.1212 | FAM70A_1 | 0.0574 | LRRC14B | 0.0231 | PLSCR4 | 0.1527 | TMEM62 | 0.0212 |
| C14orf102_2 | 0.1265 | FBXO48_2 | 0.2748 | LRRC37A4_2 | 0.0695 | PMEPA1_4 | 0.1445 | TNFRSF18_1 | 0.2602 |
| C14orf109_2 | 0.077 | FGF5_1 | 0.0745 | LRRTM4 | 0.1848 | PNMA5 | 0.1015 | TNNT2_1 | 0.0012 |
| C17orf106 | 0.2308 | FKBP10 | 0.0583 | MACC1 | 0.1529 | PPAPDC1A | 0.1397 | TOMM20L | 0.0429 |
| C17orf58_2 | 0.0538 | FLJ33360 | 0.2091 | MANSC1_1 | 0.1436 | PRAMEF5 | 0.0006 | TPM2_2 | 0.1662 |
| C17orf58_3 | 0.0243 | FLJ43752 | 0.0662 | MCAM | 0.0259 | PRKAA2 | 0.1222 | TRIM58 | 0.0973 |
| C18orf56 | 0.0471 | FMNL3_2 | 0.0515 | MCART6_1 | 0.1532 | PSMC6_1 | 0.016 | UBR7_1 | 0.0728 |
| C1orf168 | 0.0387 | FMOD | 0.1923 | MFRP | 0.2209 | RAD54B_2 | 0.1742 | UBR7_2 | 0.107 |
| C1orf64 | 0.115 | FOSB | 0.0188 | MIDN | 0.0516 | RAP1A_1 | 0.2178 | WARS_2 | 0.1502 |

| C8orf79_1 | 0.0134 | FOSL2 | 0.2826 | MIR1914 | 0.0664 | RARA_3 | 0.0956 | XBP1_2 | 0.1143 |
|---|---|---|---|---|---|---|---|---|---|
| CASP8AP2 | 0.1576 | FOXN1 | 0.033 | MIR212 | 0.0976 | RARG | 0.048 | XRN2_1 | 0.0323 |
| CCL13 | 0.1309 | GAD1_2 | 0.0245 | MIR571 | 0.0285 | RNASEK | 0.0568 | YARS2 | 0.002 |
| CCR2_3 | 0.0953 | GBE1 | 0.0383 | MIR576 | 0.1141 | RNF7_1 | 0.0152 | ZNF75D_2 | 0.1377 |
| CD34_1 | 0.0008 | GBP7 | 0.1213 | MIR654 | 0.0578 | ROD1_1 | 0.2201 | ZSWIM4_2 | 0.1552 |
| CDC42BPA_2 | 0.0051 | GJA5_1 | 0.0562 | MIR942 | 0.1333 | SATB2 | 0.0641 | figo_numeric | 0.0113 |
| CDC42SE2_2 | 0.0384 | GMNN | 0.1037 | MMP12_1 | 0.1239 | SBSN | 0.0558 | hist_rev_SBOT | 0.0568 |
| CLDN6 | 0.1048 | GSR_2 | 0.0385 | MYCN_2 | 0.1592 | SCXB | 0.0109 | surg_outcome | 0.0124 |
| CREB5_2 | 0.0332 | HBA2 | 0.204 | MYL9_2 | 0.1096 | SEC22C_3 | 0.1123 | | |
| CRYBA1 | 0.024 | HCFC1R1_1 | 0.0443 | NFATC3_5 | 0.0169 | SELENBP1 | 0.1824 | | |
| CXCL13 | 0.0799 | HDAC7_2 | 0.0003 | NFATC4 | 0.0583 | SERPINB2_2 | 0.0044 | | |
| CYB5R3_2 | 0.1856 | HDLBP_3 | 0.0761 | NLRP9 | 0.0595 | SERPINBS | 0.1929 | | |
| CYP1A2 | 0.0556 | HIC1 | 0.0559 | NOVA2 | 0.1183 | SFN | 0.0033 | | |
| DBNDD2 | 0.0925 | HPRT1_1 | 0.1294 | NP | 0.0793 | SFRS4 | 0.0215 | | |

Table 59

| ABHD3 | 0.0671 | DNAH11 | 0.0513 | HPRT1_1 | 0.151 | NOVA2 | 0.125 | SERPINBS | 0.204 |
|---|---|---|---|---|---|---|---|---|---|
| ADAM17_2 | 0.2292 | DNMT3L_2 | 0.022 | HPS4_1 | 0.0687 | NP | 0.0783 | SFN | 0.0179 |
| ADAMTS1 | 0.1692 | DOCK7_1 | 0.1366 | HR_1 | 0.0415 | NR6A1_2 | 0.1526 | SFRS4 | 0.0369 |
| ALS2CL_3 | 0.1138 | DSC3_1 | 0.071 | HSD11B1_1 | 0.1011 | NRXN3_3 | 0.1 | SHC1_3 | 0.0687 |
| ANO7_3 | 0.0731 | DUT_3 | 0.1208 | ICAM2 | 0.065 | NT5DC1_2 | 0.1983 | SLC23A1_2 | 0.1368 |
| ARL6IP1_1 | 0.0241 | EEF1E1_1 | 0.1047 | ICAM4_1 | 0.2749 | NTRK2_3 | 0.0012 | SLC25A34 | 0.1721 |
| ARMCX3_2 | 0.0864 | EIF4ENIF1 | 0.1221 | IL1RAP_2 | 0.067 | NUP155_1 | 0.0634 | SLC4A5_3 | 0.0834 |
| ATXN10_1 | 0.2052 | EMP1 | 0.1668 | IQCA1_2 | 0.0244 | NYX | 0.1807 | SLC9A10 | 0.0815 |
| AXL_1 | 0.116 | ENO1 | 0.1329 | KCNIP3_1 | 0.1062 | ODF2_3 | 0.0127 | SNORD93 | 0.1628 |
| BAI1_3 | 0.0354 | ENPEP_2 | 0.016 | KCNQ2_1 | 0.1353 | ORC1L | 0.0228 | SOX2_1 | 0.0745 |
| BCAS1_1 | 0.3268 | EPHB1 | 0.0251 | KIF3C | 0.1922 | OTUD7A_3 | 0.0361 | STC1 | 0.0131 |
| BDNF_2 | 0.1221 | EPYC | 0.03 | KRT80_2 | 0.1104 | PANK4 | 0.0586 | STC2 | 0.1329 |
| BMPR1A | 0.1083 | ERI2_2 | 0.3053 | KRTAP10.10_2 | 0.0235 | PDLIM2_2 | 0.2387 | STYX_2 | 0.0475 |
| BTF3_3 | 0.105 | ESPNL | 0.041 | L3MBTL2_3 | 0.0295 | PHYH_1 | 0.1982 | SYTL3 | 0.0072 |
| C10orf116 | 0.0337 | EZH2_1 | 0.0705 | LBH_2 | 0.0915 | PIGA_1 | 0.0033 | TAF15_1 | 0.0023 |
| C11orf24 | 0.1795 | FAM13AOS | 0.0361 | LENEP | 0.2311 | PITX2_1 | 0.0891 | TCEAL8_1 | 0.0422 |
| C11orf49_3 | 0.1271 | FAM187B_2 | 0.0083 | LGI3 | 0.1219 | PKN1_3 | 0.0161 | THBS3 | 0.106 |
| C14orf102_2 | 0.1271 | FAM70A_1 | 0.0752 | LOC492303 | 0.0315 | PLAC9 | 0.2381 | TIMP2_2 | 0.0656 |
| C14orf109_2 | 0.0735 | FBXO48_2 | 0.2561 | LRRC14B | 0.0189 | PLEKHG5_5 | 0.0151 | TM2D3_2 | 0.0735 |
| C17orf106 | 0.2415 | FGF5_1 | 0.0735 | LRRC37A4_2 | 0.0641 | PLSCR4 | 0.167 | TMEM52 | 0.0094 |
| C17orf58_2 | 0.0464 | FKBP10 | 0.0448 | LRRTM4 | 0.1761 | PMEPA1_4 | 0.1285 | TMEM62 | 0.066 |
| C17orf58_3 | 0.0237 | FLJ33360 | 0.2023 | MACC1 | 0.1346 | PNMA5 | 0.1162 | TNFRSF18_1 | 0.2722 |
| C18orf56 | 0.0465 | FLJ43752 | 0.0722 | MANSC1_1 | 0.1377 | PPAPDC1A | 0.1306 | TNNT2_1 | 0.0012 |
| C1orf168 | 0.0392 | FMNL3_2 | 0.0414 | MCAM | 0.0211 | PRAMEF5 | 0.0005 | TOMM20L | 0.0411 |
| C1orf64 | 0.1124 | FMOD | 0.2024 | MCART6_1 | 0.1461 | PRKAA2 | 0.1411 | TPM2_2 | 0.1754 |
| C8orf79_1 | 0.0158 | FOSB | 0.0221 | MFRP | 0.2228 | PSMC6_1 | 0.0065 | TRIM58 | 0.1096 |

| | | | | | | | |
|---|---|---|---|---|---|---|---|
| CASP8AP2 | 0.1323 | FOSL2 | 0.2764 | MIDN | 0.0404 | RAD54B_2 | 0.1805 | UBR7_1 | 0.0721 |
| CCL13 | 0.1413 | FOXN1 | 0.0242 | MIR1914 | 0.0611 | RAP1A_1 | 0.2107 | UBR7_2 | 0.1192 |
| CCR2_3 | 0.0938 | GAD1_2 | 0.0147 | MIR212 | 0.1082 | RARA_3 | 0.0828 | WARS_2 | 0.1469 |
| CD34_1 | 0.001 | GBE1 | 0.0497 | MIR571 | 0.0377 | RARG | 0.0461 | XBP1_2 | 0.1332 |
| CDC42BPA_2 | 0.0178 | GBP7 | 0.1283 | MIR576 | 0.1018 | RNASEK | 0.0717 | XRN2_1 | 0.0532 |
| CDC42SE2_2 | 0.0288 | GJA5_1 | 0.0489 | MIR654 | 0.0564 | RNF7_1 | 0.0208 | YARS2 | 0.0016 |
| CLDN6 | 0.1018 | GMNN | 0.0972 | MIR942 | 0.1348 | ROD1_1 | 0.2224 | ZNF75D_2 | 0.1609 |
| CREB5_2 | 0.0178 | GSR_2 | 0.0458 | MMP12_1 | 0.1289 | SATB2 | 0.0615 | ZSWIM4_2 | 0.1604 |
| CRYBA1 | 0.0274 | HBA2 | 0.2029 | MYCN_2 | 0.1459 | SBSN | 0.051 | figo_numeric | 0.0142 |
| CXCL13 | 0.0787 | HCFC1R1_1 | 0.0588 | MYL9_2 | 0.1003 | SCXB | 0.0101 | hist_rev_SBOT | 0.0611 |
| CYB5R3_2 | 0.1839 | HDAC7_2 | 0.0054 | NFATC3_5 | 0.0044 | SEC22C_3 | 0.1062 | surg_outcome | 0.0021 |
| CYP1A2 | 0.0569 | HDLBP_3 | 0.1015 | NFATC4 | 0.055 | SELENBP1 | 0.1861 | | |
| DBNDD2 | 0.107 | HIC1 | 0.0352 | NLRP9 | 0.0689 | SERPINB2_2 | 0.0072 | | |

Table 60

| | | | | | | | | | |
|---|---|---|---|---|---|---|---|---|---|
| ABHD3 | 0.0166 | DBNDD2 | 0.0985 | HSD11B1_1 | 0.1211 | NRXN3_3 | 0.1004 | SHC1_3 | 0.0643 |
| ADAM17_2 | 0.2184 | DNAH11 | 0.0484 | ICAM2 | 0.0257 | NT5DC1_2 | 0.1871 | SLC23A1_2 | 0.0795 |
| ADAMTS1 | 0.1541 | DNMT3L_2 | 0.0068 | ICAM4_1 | 0.2568 | NTRK2_3 | 0.0063 | SLC25A34 | 0.1679 |
| ALS2CL_3 | 0.0861 | DOCK7_1 | 0.0862 | IL1RAP_2 | 0.0475 | NUP155_1 | 0.0334 | SLC4A5_3 | 0.0537 |
| ANO7_3 | 0.0199 | DSC3_1 | 0.0803 | IQCA1_2 | 0.0619 | NYX | 0.1428 | SLC9A10 | 0.072 |
| ARL6IP1_1 | 0.05 | DUT_3 | 0.1208 | KCNIP3_1 | 0.1159 | ODF2_3 | 0.0248 | SNORD93 | 0.1594 |
| ARMCX3_2 | 0.1112 | EEF1E1_1 | 0.1172 | KCNQ2_1 | 0.142 | ORC1L | 0.0191 | SOX2_1 | 0.0624 |
| ATXN10_1 | 0.2216 | EMP1 | 0.0986 | KIF3C | 0.1898 | OTUD7A_3 | 0.0018 | STC1 | 0.0161 |
| AURKA_1 | 0.1001 | ENO1 | 0.2005 | KRT80_2 | 0.1454 | PANK4 | 0.0478 | STC2 | 0.1199 |
| AXL_1 | 0.1 | ENPEP_2 | 0.1348 | KRTAP10.10_2 | 0.002 | PDLIM2_2 | 0.2087 | STYX_2 | 0.046 |
| BAI1_3 | 0.2844 | EPHB1 | 0.0508 | L3MBTL2_3 | 0.0268 | PHYH_1 | 0.1765 | SYTL3 | 0.0329 |
| BCAS1_1 | 0.1883 | EPYC | 0.0409 | LBH_2 | 0.1113 | PIGA_1 | 0.0169 | TAF15_1 | 0.0232 |
| BDNF_2 | 0.1269 | ERI2_2 | 0.2472 | LENEP | 0.1991 | PITX2_1 | 0.1426 | TCEAL8_1 | 0.0653 |
| BMPR1A | 0.0692 | ESPNL | 0.0142 | LGI3 | 0.149 | PKN1_3 | 0.0452 | THBS3 | 0.0517 |
| BTF3_3 | 0.079 | FAM13AOS | 0.057 | LOC492303 | 0.0476 | PLAC9 | 0.1953 | THY1 | 0.0583 |
| C10orf116 | 0.0448 | FAM187B_2 | 0.0043 | LRRC14B | 0.0303 | PLEKHG5_5 | 0.0013 | TIMP2_2 | 0.0906 |
| C11orf24 | 0.1449 | FAM70A_1 | 0.0234 | LRRC37A4_2 | 0.0563 | PLSCR4 | 0.2019 | TM2D3_2 | 0.0318 |
| C11orf49_3 | 0.1129 | FBXO48_2 | 0.2855 | LRRTM4 | 0.1923 | PMEPA1_4 | 0.1591 | TMEM52 | 0.039 |
| C14orf102_2 | 0.0742 | FKBP10 | 0.0479 | MACC1 | 0.0885 | PNMA5 | 0.1413 | TMEM62 | 0.0421 |
| C14orf109_2 | 0.0939 | FLJ33360 | 0.0516 | MANSC1_1 | 0.107 | PPAPDC1A | 0.1376 | TNFRSF18_1 | 0.2005 |
| C17orf106 | 0.218 | FLJ43752 | 0.1867 | MCAM | 0.0052 | PRAMEF5 | 0.0107 | TNNT2_1 | 0.003 |
| C17orf58_2 | 0.0564 | FMNL3_2 | 0.0112 | MCART6_1 | 0.1421 | PRKAA2 | 0.0698 | TOMM20L | 0.0199 |
| C17orf58_3 | 0.0299 | FOSB | 0.1898 | MFRP | 0.2159 | PSMC6_1 | 0.0067 | TPM2_2 | 0.1777 |
| C18orf56 | 0.0054 | FOSL2 | 0.0578 | MIDN | 0.0265 | RAD54B_2 | 0.1857 | TRIM58 | 0.0964 |
| C1orf168 | 0.0376 | FOXN1 | 0.2188 | MIR1914 | 0.0817 | RAP1A_1 | 0.1932 | UBR7_1 | 0.051 |
| C1orf64 | 0.1066 | GAD1_2 | 0.0242 | MIR212 | 0.0836 | RARA_3 | 0.0872 | UBR7_2 | 0.0982 |

| | | | | | | | |
|---|---|---|---|---|---|---|---|
| C8orf79_1 | 0.0136 | GBE1 | 0.0438 | MIR571 | 0.0287 | RARG | 0.0506 | WARS_2 | 0.1452 |
| CASP8AP2 | 0.1435 | GBP7 | 0.098 | MIR576 | 0.1125 | RNASEK | 0.0743 | WDR76 | 0.1101 |
| CCL13 | 0.1199 | GJA5_1 | 0.0433 | MIR654 | 0.0204 | RNF7_1 | 0.0694 | XBP1_2 | 0.0458 |
| CCR2_3 | 0.0409 | GMNN | 0.0788 | MIR942 | 0.1756 | ROD1_1 | 0.1608 | XRN2_1 | 0.0278 |
| CD34_1 | 0.0011 | GSR_2 | 0.0005 | MMP12_1 | 0.0881 | SATB2 | 0.0437 | YARS2 | 0.2501 |
| CDC42BPA_2 | 0.0136 | HBA2 | 0.1497 | MYCN_2 | 0.0687 | SBSN | 0.01 | ZNF75D_2 | 0.1344 |
| CDC42SE2_2 | 0.0308 | HCFC1R1_1 | 0.0365 | MYOHD1 | 0.0827 | SCXB | 0.0204 | ZSWIM4_2 | 0.1448 |
| CLDN6 | 0.118 | HDAC7_2 | 0.0183 | NFATC3_5 | 0.014 | SEC22C_3 | 0.1159 | figo_numeric | 0.021 |
| CREB5_2 | 0.0002 | HDLBP_3 | 0.1032 | NFATC4 | 0.0691 | SELENBP1 | 0.1537 | hist_rev_SBOT | 0.047 |
| CRYBA1 | 0.0273 | HIC1 | 0.0324 | NLRP9 | 0.1646 | SERPINB2_2 | 0.0366 | surg_outcome | 0.0123 |
| CXCL13 | 0.11 | HPRT1_1 | 0.0847 | NOVA2 | 0.0813 | SERPINBS | 0.1726 | | |
| CYB5R3_2 | 0.1351 | HPS4_1 | 0.0753 | NP | 0.0971 | SFN | 0.0182 | | |
| CYP1A2 | 0.0707 | HR_1 | 0.0263 | NR6A1_2 | 0.1233 | SFRS4 | 0.0373 | | |

EP 3 874 274 B1

Table 61

| | | | | | | | | | |
|---|---|---|---|---|---|---|---|---|---|
| ABHD3 | 0.0019 | CYP1A2 | 0.0811 | HR_1 | 0.0354 | NP | 0.0841 | SFN | 0.0197 |
| ADAM17_2 | 0.21 | DBNDD2 | 0.0943 | HSD11B1_1 | 0.1037 | NR6A1_2 | 0.134 | SFRS4 | 0.0417 |
| ADAMTS1 | 0.1502 | DNAH11 | 0.0423 | ICAM2 | 0.0493 | NRXN3_3 | 0.095 | SHC1_3 | 0.0654 |
| ALS2CL_3 | 0.0705 | DNMT3L2 | 0.0153 | ICAM4_1 | 0.2507 | NT5DC1_2 | 0.1783 | SLC23A1_2 | 0.0641 |
| ANO7_3 | 0.0243 | DOCK7_1 | 0.0719 | IL1RAP_2 | 0.0403 | NTRK2_3 | 0.0015 | SLC25A34 | 0.1718 |
| ANTXR1_4 | 0.0354 | DSC3_1 | 0.0821 | IQCA1_2 | 0.0654 | NUP155_1 | 0.0228 | SLC4A5_3 | 0.049 |
| ARL6IP1_1 | 0.1207 | DUT_3 | 0.1249 | KCNIP3_1 | 0.1142 | NYX | 0.116 | SLC9A10 | 0.0574 |
| ARMCX3_2 | 0.2073 | EEF1E1_1 | 0.1162 | KCNQ2_1 | 0.1373 | ODF2_3 | 0.0384 | SNORD93 | 0.1661 |
| ATXN10_1 | 0.1486 | EMP1 | 0.0972 | KIF3C | 0.1919 | ORC1L | 0.0208 | SOX2_1 | 0.071 |
| AURKA_1 | 0.0958 | ENO1 | 0.189 | KRT80_2 | 0.134 | OTUD7A_3 | 0.0025 | STC1 | 0.0345 |
| AXL_1 | 0.0891 | ENPEP_2 | 0.1375 | KRTAP10.10_2 | 0.0076 | PANK4 | 0.0489 | STC2 | 0.1081 |
| BAI1_3 | 0.278 | EPHB1 | 0.051 | L3MBTL2_3 | 0.0274 | PDLIM2_2 | 0.2133 | STYX_2 | 0.0504 |
| BCAS1_1 | 0.1917 | EPYC | 0.0483 | LBH_2 | 0.1174 | PHYH_1 | 0.1736 | SYTL3 | 0.0159 |
| BDNF_2 | 0.1205 | ERI2_2 | 0.2492 | LENEP | 0.1867 | PIGA_1 | 0.0214 | TAF15_1 | 0.0054 |
| BMPR1A | 0.0673 | ESPNL | 0.0136 | LGI3 | 0.1499 | PITX2_1 | 0.148 | TCEAL8_1 | 0.0537 |
| BTF3_3 | 0.0601 | FAM13AOS | 0.0489 | LOC492303 | 0.0439 | PKN1_3 | 0.0458 | THBS3 | 0.0349 |
| C10orf116 | 0.0284 | FAM187B_2 | 0.0017 | LRRC14B | 0.0361 | PLAC9 | 0.1978 | THY1 | 0.0577 |
| C11orf24 | 0.1598 | FAM70A_1 | 0.0127 | LRRC37A4_2 | 0.0698 | PLEKHG5_5 | 0.0069 | TM2D3_2 | 0.117 |
| C11orf49_3 | 0.1189 | FBXO48 2 | 0.2818 | LRRTM4 | 0.197 | PLSCR4 | 0.191 | TMEM52 | 0.0352 |
| C14orf102_2 | 0.0818 | FKBP10 | 0.0494 | MACC1 | 0.0998 | PMEPA1_4 | 0.1677 | TMEM62 | 0.017 |
| C14orf109_2 | 0.1017 | FLJ33360 | 0.0529 | MANSC1_1 | 0.1074 | PNMA5 | 0.1276 | TNFRSF18_1 | 0.1971 |
| C17orf106 | 0.208 | FLJ43752 | 0.1844 | MCAM | 0.0015 | PPAPDC1A | 0.1399 | TNNT2_1 | 0.0075 |
| C17orf58_2 | 0.0783 | FMNL3_2 | 0.0046 | MCART6_1 | 0.1464 | PRAMEF5 | 0.0059 | TOMM20L | 0.0123 |
| C17orf58_3 | 0.0303 | FOSB | 0.1927 | MFRP | 0.2112 | PRKAA2 | 0.0535 | TPM2_2 | 0.1708 |
| C18orf56 | 0.0029 | FOSL2 | 0.0505 | MIDN | 0.0338 | PSMC6_1 | 0.0074 | TRIM58 | 0.0796 |
| C1orf168 | 0.0345 | FOXN1 | 0.2285 | MIR1914 | 0.0838 | RAD54B_2 | 0.1884 | UBR7_1 | 0.063 |

| | | | | | | | | | |
|---|---|---|---|---|---|---|---|---|---|
| C1orf64 | 0.1047 | GAD1_2 | 0.0395 | MIR212 | 0.0678 | RAP1A_1 | 0.1965 | UBR7_2 | 0.0959 |
| C8orf79_1 | 0.0105 | GBE1 | 0.0372 | MIR571 | 0.0254 | RARA_3 | 0.0943 | WARS_2 | 0.1386 |
| CASP8AP2 | 0.1559 | GBP7 | 0.0889 | MIR576 | 0.1261 | RARG | 0.0654 | WDR76 | 0.0986 |
| CCL13 | 0.1015 | GJA5_1 | 0.0431 | MIR654 | 0.0265 | RNASEK | 0.0618 | XBP1_2 | 0.042 |
| CCR2_3 | 0.033 | GMNN | 0.0813 | MIR942 | 0.1625 | RNF7_1 | 0.0415 | XRN2_1 | 0.0299 |
| CD34_1 | 0.0017 | GSR_2 | 0.0019 | MMP12_1 | 0.0955 | ROD1_1 | 0.1632 | YARS2 | 0.2416 |
| CDC42BPA_2 | 0.0244 | HBA2 | 0.1452 | MYCN_2 | 0.0921 | SATB2 | 0.0509 | ZNF75D_2 | 0.1199 |
| CDC42SE2_2 | 0.0446 | HCFC1R1_1 | 0.0271 | MYL9_2 | 0.0846 | SBSN | 0.0127 | ZSWIM4_2 | 0.1456 |
| CLDN6 | 0.1185 | HDAC7_2 | 0.014 | MYOHD1 | 0.0203 | SCXB | 0.0194 | figo_numeric | 0.0052 |
| CREB5_2 | 0.0133 | HDLBP_3 | 0.0809 | NFATC3_5 | 0.0681 | SEC22C_3 | 0.0991 | hist_rev_SBOT | 0.0335 |
| CRYBA1 | 0.0219 | HIC1 | 0.0224 | NFATC4 | 0.0821 | SELENBP1 | 0.1396 | surg_outcome | 0.0306 |
| CXCL13 | 0.1102 | HPRT1_1 | 0.0729 | NLRP9 | 0.1625 | SERPINB2_2 | 0.0221 | | |
| CYB5R3_2 | 0.1396 | HPS4_1 | 0.0911 | NOVA2 | 0.082 | SERPINB5 | 0.158 | | |

Table 62

| | | | | | | | | |
|---|---|---|---|---|---|---|---|---|
| ABHD3 | 0.017 | DBNDD2 | 0.1069 | HSD11B1_1 | 0.1165 | NR6A1_2 | 0.1271 | SFRS4 | 0.0366 |
| ADAM17_2 | 0.2176 | DNAH11 | 0.0487 | ICAM2 | 0.0344 | NRXN3_3 | 0.1027 | SHC1_3 | 0.0575 |
| ADAMTS1 | 0.1527 | DNMT3L_2 | 0.0094 | ICAM4_1 | 0.2471 | NT5DC1_2 | 0.1957 | SLC23A1_2 | 0.0786 |
| ALS2CL_3 | 0.0878 | DOCK7_1 | 0.0986 | IL1RAP_2 | 0.0433 | NTRK2_3 | 0.0049 | SLC25A34 | 0.1716 |
| ANO7_3 | 0.0094 | DSC3_1 | 0.0875 | IQCA1_2 | 0.0582 | NUP155_1 | 0.0236 | SLC4A5_3 | 0.0558 |
| ARL6IP1_1 | 0.0333 | DUT_3 | 0.1196 | KCNIP3_1 | 0.1157 | NYX | 0.152 | SLC9A10 | 0.0634 |
| ARMCX3_2 | 0.1124 | EEF1E1_1 | 0.1126 | KCNQ2_1 | 0.1461 | ODF2_3 | 0.0297 | SNORD93 | 0.1581 |
| ATXN10_1 | 0.2223 | EMP1 | 0.1068 | KIF3C | 0.1849 | ORC1L | 0.0228 | SOX2_1 | 0.0701 |
| AURKA_1 | 0.105 | ENO1 | 0.2018 | KRT80_2 | 0.1425 | OTUD7A_3 | 0.0029 | STC1 | 0.0163 |
| AXL_1 | 0.0966 | ENPEP_2 | 0.1337 | KRTAP10.10_2 | 0.0006 | PANK4 | 0.0488 | STC2 | 0.1143 |
| BAI1_3 | 0.2815 | EPHB1 | 0.038 | L3MBTL2_3 | 0.0242 | PDLIM2_2 | 0.2142 | STYX_2 | 0.046 |
| BCAS1_1 | 0.1865 | EPYC | 0.0354 | LBH_2 | 0.1077 | PHYH_1 | 0.1809 | SYTL3 | 0.0239 |
| BDNF_2 | 0.1256 | ERI2_2 | 0.2532 | LENEP | 0.2008 | PIGA_1 | 0.0139 | TAF15_1 | 0.0431 |
| BMPR1A | 0.0725 | ESPNL | 0.0135 | LGI3 | 0.1389 | PITX2_1 | 0.1438 | TCEAL8_1 | 0.0643 |
| BTF3_3 | 0.0713 | FAM13AOS | 0.0501 | LOC492303 | 0.0514 | PKN1_3 | 0.0425 | THBS3 | 0.0545 |
| C10orf116 | 0.0468 | FAM187B_2 | 0.0027 | LRRC14B | 0.0342 | PLAC9 | 0.195 | TIMP2_2 | 0.0629 |
| C11orf24 | 0.139 | FAM70A_1 | 0.023 | LRRC37A4_2 | 0.0647 | PLEKHG5_5 | 0.0082 | TM2D3_2 | 0.0819 |
| C11orf49_3 | 0.1106 | FBXO48_2 | 0.283 | LRRTM4 | 0.1939 | PLSCR4 | 0.2028 | TMEM52 | 0.0349 |
| C14orf102_2 | 0.0663 | FKBP10 | 0.0465 | MACC1 | 0.0857 | PMEPA1_4 | 0.1561 | TMEM62 | 0.0479 |
| C14orf109_2 | 0.0883 | FLJ33360 | 0.0527 | MANSC1_1 | 0.0982 | PNMA5 | 0.139 | TNFRSF18_1 | 0.2089 |
| C17orf106 | 0.219 | FLJ43752 | 0.1766 | MCAM | 0.0097 | PPAPDC1A | 0.1385 | TNNT2_1 | 0.0031 |
| C17orf58_2 | 0.066 | FMNL3_2 | 0.0111 | MCART6_1 | 0.1422 | PRAMEF5 | 0.0036 | TOMM20L | 0.0204 |
| C17orf58_3 | 0.0267 | FOSB | 0.1968 | MFRP | 0.2177 | PRKAA2 | 0.0733 | TPM2_2 | 0.1781 |
| C18orf56 | 0.0012 | FOSL2 | 0.0615 | MIDN | 0.0153 | PSMC6_1 | 0.0134 | TRIM58 | 0.0987 |
| C1orf168 | 0.0394 | FOXN1 | 0.2269 | MIR1914 | 0.0808 | RAD54B_2 | 0.1888 | UBR7_1 | 0.0557 |
| C1orf64 | 0.1035 | GAD1_2 | 0.0281 | MIR212 | 0.0853 | RAP1A_1 | 0.1863 | UBR7_2 | 0.0978 |

| | | | | | | | |
|---|---|---|---|---|---|---|---|
| C8orf79_1 | 0.01 | GBE1 | 0.0417 | MIR571 | 0.0334 | RARA_3 | 0.0858 | WARS_2 | 0.1332 |
| CASP8AP2 | 0.1377 | GBP7 | 0.099 | MIR576 | 0.1152 | RARG | 0.0523 | WDR76 | 0.1104 |
| CCL13 | 0.1143 | GJA5_1 | 0.0371 | MIR654 | 0.0177 | RNASEK | 0.0758 | XBP1_2 | 0.0486 |
| CCR2_3 | 0.0434 | GMNN | 0.0809 | MIR942 | 0.164 | RNF7_1 | 0.0728 | XRN2_1 | 0.0238 |
| CD34_1 | 0.0097 | GSR_2 | 0.0039 | MMP121 | 0.0916 | ROD1_1 | 0.161 | YARS2 | 0.2485 |
| CDC42BPA_2 | 0.0176 | HBA2 | 0.1363 | MYCN_2 | 0.0695 | SATB2 | 0.0481 | ZNF75D_2 | 0.1364 |
| CDC42SE2_2 | 0.0329 | HCFC1R1_1 | 0.0394 | MYL92 | 0.0799 | SBSN | 0.0085 | ZSWIM4_2 | 0.1491 |
| CLDN6 | 0.1121 | HDAC7_2 | 0.0284 | MYOHD1 | 0.0117 | SCXB | 0.0173 | figo_numeric | 0.0153 |
| CREB5_2 | 0.0093 | HDLBP_3 | 0.1026 | NFATC3_5 | 0.0671 | SEC22C_3 | 0.1026 | hist_rev_SBOT | 0.0486 |
| CRYBA1 | 0.0359 | HIC1 | 0.0311 | NFATC4 | 0.0823 | SELENBP1 | 0.1471 | surg_outcome | 0.0178 |
| CXCL13 | 0.1118 | HPRT1_1 | 0.089 | NLRP9 | 0.1661 | SERPINB2_2 | 0.0274 | | |
| CYB5R3_2 | 0.1345 | HPS4_1 | 0.0776 | NOVA2 | 0.0826 | SERPINB5 | 0.1756 | | |
| CYP1A2 | 0.0768 | HR_1 | 0.0218 | NP | 0.1029 | SFN | 0.0273 | | |

Table 63

| | | | | | | | | | |
|---|---|---|---|---|---|---|---|---|---|
| ABHD3 | 0.0521 | DBNDD2 | 0.097 | HR_1 | 0.0561 | NR6A1_2 | 0.1466 | SFRS4 | 0.0136 |
| ADAM17_2 | 0.2213 | DNAH11 | 0.0381 | HSD11B1_1 | 0.0839 | NRXN3_3 | 0.0945 | SHC1_3 | 0.0791 |
| ADAMTS1 | 0.1658 | DNMT3L_2 | 0.0235 | ICAM2 | 0.0668 | NT5DC1_2 | 0.1696 | SLC23A1_2 | 0.1301 |
| ALS2CL_3 | 0.0907 | DOCK7_1 | 0.107 | ICAM4_1 | 0.2766 | NTRK2_3 | 0.0102 | SLC25A34 | 0.1559 |
| ANO7_3 | 0.0587 | DSC3_1 | 0.0715 | IL1RAP_2 | 0.0508 | NUP155_1 | 0.0427 | SLC4A5_3 | 0.0704 |
| ANTXR1_4 | 0.0342 | DUT_3 | 0.1158 | IQCA1_2 | 0.035 | NYX | 0.1433 | SLC9A10 | 0.0729 |
| ARL6IP1_1 | 0.0856 | EEF1E1_1 | 0.0878 | KCNIP3_1 | 0.0981 | ODF2_3 | 0.0085 | SNORD93 | 0.168 |
| ARMCX3_2 | 0.1902 | EMP1 | 0.1131 | KCNQ2_1 | 0.1202 | ORC1L | 0.0203 | SOX2_1 | 0.075 |
| ATXN10_1 | 0.169 | ENO1 | 0.176 | KIF3C | 0.1849 | OTUD7A_3 | 0.0279 | STC1 | 0.0108 |
| AXL_1 | 0.1015 | ENPEP_2 | 0.135 | KRT80_2 | 0.1107 | PANK4 | 0.0644 | STC2 | 0.1222 |
| BAI1_3 | 0.0418 | EPHB1 | 0.049 | KRTAP10.10_2 | 0.0184 | PDLIM2_2 | 0.2384 | STYX_2 | 0.0447 |
| BCAS1_1 | 0.3217 | EPYC | 0.0465 | L3MBTL2_3 | 0.0377 | PHYH_1 | 0.195 | SYTL3 | 0.0052 |
| BDNF_2 | 0.1077 | ERI2_2 | 0.2842 | LBH_2 | 0.1068 | PIGA_1 | 0.0055 | TAF15_1 | 0.0316 |
| BMPR1A | 0.1048 | ESPNL | 0.0387 | LENEP | 0.2203 | PITX2_1 | 0.1038 | TCEAL8_1 | 0.0254 |
| BTF3_3 | 0.0958 | EZH2_1 | 0.0596 | LGI3 | 0.1224 | PKN1_3 | 0.0155 | THBS3 | 0.087 |
| C10orf116 | 0.018 | FAM13AOS | 0.0447 | LOC492303 | 0.016 | PLAC9 | 0.2659 | THY1 | 0.0544 |
| C11orf24 | 0.2043 | FAM187B_2 | 0.0197 | LRRC14B | 0.0183 | PLEKHG5_5 | 0.0393 | TM2D3_2 | 0.1096 |
| C11orf49_3 | 0.1259 | FAM70A_1 | 0.0648 | LRRC37A4_2 | 0.0651 | PLSCR4 | 0.1544 | TMEM52 | 0.0147 |
| C14orf102_2 | 0.1233 | FBXO48_2 | 0.2762 | LRRTM4 | 0.1744 | PMEPA1_4 | 0.1409 | TMEM62 | 0.0156 |
| C14orf109_2 | 0.0707 | FKBP10 | 0.0741 | MACC1 | 0.1333 | PNMA5 | 0.1132 | TNFRSF18_1 | 0.2511 |
| C17orf106 | 0.2223 | FLJ33360 | 0.0615 | MANSC1_1 | 0.1395 | PPAPDC1A | 0.1394 | TNNT2_1 | 0.0045 |
| C17orf58_2 | 0.0469 | FLJ43752 | 0.2033 | MCAM | 0.0204 | PRAMEF5 | 0.0069 | TOMM20L | 0.0468 |
| C17orf58_3 | 0.0282 | FMNL3_2 | 0.0514 | MCART6_1 | 0.1343 | PRKAA2 | 0.114 | TPM2_2 | 0.1701 |
| C18orf56 | 0.0395 | FOSB | 0.1914 | MFRP | 0.2165 | PSMC6_1 | 0.0056 | TRIM58 | 0.1021 |
| C1orf168 | 0.0333 | FOSL2 | 0.019 | MIDN | 0.0501 | RAD54B_2 | 0.177 | UBR7_1 | 0.0619 |
| C1orf64 | 0.1125 | FOXN1 | 0.2729 | MIR1914 | 0.0644 | RAP1A_1 | 0.2181 | UBR7_2 | 0.124 |

| | | | | | | | | | |
|---|---|---|---|---|---|---|---|---|---|
| C8orf79_1 | 0.0242 | GAD1_2 | 0.0204 | MIR212 | 0.0935 | RARA_3 | 0.0911 | WARS_2 | 0.1597 |
| CASP8AP2 | 0.1624 | GBE1 | 0.039 | MIR571 | 0.0218 | RARG | 0.048 | XBP1_2 | 0.1142 |
| CCL13 | 0.1381 | GBP7 | 0.1183 | MIR576 | 0.1186 | RNASEK | 0.0568 | XRN2_1 | 0.0237 |
| CCR2_3 | 0.0827 | GJA5_1 | 0.0613 | MIR654 | 0.0517 | RNF7_1 | 0.0075 | YARS2 | 0.0143 |
| CD34_1 | 0.0188 | GMNN | 0.1067 | MIR942 | 0.1342 | ROD1_1 | 0.2206 | ZNF75D_2 | 0.1286 |
| CDC42BPA_2 | 0.0152 | GSR_2 | 0.0344 | MMP12_1 | 0.1318 | SATB2 | 0.0553 | ZSWIM4_2 | 0.1584 |
| CDC42SE2_2 | 0.0308 | HBA2 | 0.2027 | MYCN_2 | 0.1544 | SBSN | 0.0583 | figo_numeric | 0.0119 |
| CLDN6 | 0.1201 | HCFC1R1_1 | 0.0491 | MYOHD1 | 0.1013 | SCXB | 0.0096 | hist_rev_SBOT | 0.0486 |
| CREB5_2 | 0.0291 | HDAC7_2 | 0.0076 | NFATC3_5 | 0.02 | SEC22C_3 | 0.1209 | surg_outcome | 0.0033 |
| CRYBA1 | 0.0182 | HDLBP_3 | 0.0949 | NFATC4 | 0.0566 | SELENBP1 | 0.1867 | | |
| CXCL13 | 0.0753 | HIC1 | 0.0549 | NLRP9 | 0.1726 | SERPINB2_2 | 0.002 | | |
| CYB5R3_2 | 0.1815 | HPRT1_1 | 0.1298 | NOVA2 | 0.1196 | SERPINB5 | 0.1796 | | |
| CYP1A2 | 0.0613 | HPS4_1 | 0.0745 | NP | 0.0854 | SFN | 0.0009 | | |

Table 64

| Gene | Value | Gene | Value | Gene | Value | Gene | Value | Gene | Value | Gene | Value |
|---|---|---|---|---|---|---|---|---|---|---|---|
| ABHD3 | 0.0518 | DBNDD2 | 0.1013 | HR_1 | 0.0529 | NP | 0.0871 | SFN | 0.0004 | | |
| ADAM17_2 | 0.2189 | DNAH11 | 0.0384 | HSD11B1_1 | 0.0836 | NR6A1_2 | 0.1488 | SFRS4 | 0.0137 | | |
| ADAMTS1 | 0.1627 | DNMT3L_2 | 0.0252 | ICAM2 | 0.0729 | NRXN3_3 | 0.0968 | SHC1_3 | 0.0765 | | |
| ALS2CL_3 | 0.0917 | DOCK7_1 | 0.1162 | ICAM4_1 | 0.2734 | NT5DC1_2 | 0.1741 | SLC23A1_2 | 0.1317 | | |
| ANO7_3 | 0.0549 | DSC3_1 | 0.0776 | IL1RAP_2 | 0.0497 | NTRK2_3 | 0.0075 | SLC25A34 | 0.1593 | | |
| ANTXR1_4 | 0.0264 | DUT_3 | 0.1168 | IQCA1_2 | 0.0329 | NUP155_1 | 0.0426 | SLC4A5_3 | 0.0728 | | |
| ARL6IP1_1 | 0.0851 | EEF1E1_1 | 0.0889 | KCNIP3_1 | 0.0986 | NYX | 0.1473 | SLC9A10 | 0.0689 | | |
| ARMCX3_2 | 0.1895 | EMP1 | 0.1167 | KCNQ2_1 | 0.1228 | ODF2_3 | 0.0072 | SNORD93 | 0.1656 | | |
| ATXN10_1 | 0.1694 | ENO1 | 0.1741 | KIF3C | 0.1861 | ORC1L | 0.0217 | SOX2_1 | 0.076 | | |
| AXL_1 | 0.0998 | ENPEP_2 | 0.1352 | KRT80_2 | 0.109 | OTUD7A_3 | 0.0268 | STC1 | 0.0071 | | |
| BAI1_3 | 0.0398 | EPHB1 | 0.0453 | KRTAP10.10_2 | 0.0175 | PANK4 | 0.0671 | STC2 | 0.121 | | |
| BCAS1_1 | 0.321 | EPYC | 0.0446 | L3MBTL2_3 | 0.038 | PDLIM2_2 | 0.2424 | STYX_2 | 0.047 | | |
| BDNF_2 | 0.1038 | ERI2_2 | 0.2847 | LBH_2 | 0.1054 | PHYH_1 | 0.1974 | SYTL3 | 0.0062 | | |
| BMPR1A | 0.1059 | ESPNL | 0.0365 | LENEP | 0.2222 | PIGA_1 | 0.0054 | TAF15_1 | 0.0216 | | |
| BTF3_3 | 0.0957 | EZH2_1 | 0.0564 | LGI3 | 0.1125 | PITX2_1 | 0.1021 | TCEAL8_1 | 0.0226 | | |
| C10orf116 | 0.0167 | FAM13AOS | 0.047 | LOC492303 | 0.0128 | PKN1_3 | 0.0122 | THBS3 | 0.0857 | | |
| C11orf24 | 0.2026 | FAM187B_2 | 0.0205 | LRRC14B | 0.0167 | PLAC9 | 0.2658 | TM2D3_2 | 0.0566 | | |
| C11orf49_3 | 0.1251 | FAM70A_1 | 0.0644 | LRRC37A4_2 | 0.0674 | PLEKHG5_5 | 0.0358 | TMEM52 | 0.1043 | | |
| C14orf102_2 | 0.1184 | FBXO48_2 | 0.2709 | LRRTM4 | 0.1748 | PLSCR4 | 0.1513 | TMEM62 | 0.016 | | |
| C14orf1092 | 0.0692 | FKBP10 | 0.0741 | MACC1 | 0.1373 | PMEPA1_4 | 0.1402 | TNFRSF18_1 | 0.2581 | | |
| C17orf106 | 0.222 | FLJ33360 | 0.0643 | MANSC1_1 | 0.1381 | PNMA5 | 0.109 | TNNT2_1 | 0.0055 | | |
| C17orf58_2 | 0.0519 | FLJ43752 | 0.1985 | MCAM | 0.0174 | PPAPDC1A | 0.143 | TOMM20L | 0.0454 | | |
| C17orf58_3 | 0.0265 | FMNL3_2 | 0.0507 | MCART6_1 | 0.1343 | PRAMEF5 | 0.0032 | TPM2_2 | 0.1698 | | |
| C18orf56 | 0.0411 | FOSB | 0.1971 | MFRP | 0.2201 | PRKAA2 | 0.1167 | TRIM58 | 0.1002 | | |
| C1orf168 | 0.0355 | FOSL2 | 0.0196 | MIDN | 0.0447 | PSMC6_1 | 0.0032 | UBR7_1 | 0.0613 | | |
| C1orf64 | 0.1107 | FOXN1 | 0.2786 | MIR1914 | 0.0616 | RAD54B_2 | 0.176 | UBR7_2 | 0.1191 | | |

(continued)

| | | | | | | | | |
|---|---|---|---|---|---|---|---|---|
| C8orf79_1 | 0.0309 | GAD1_2 | 0.0218 | MIR212 | 0.0947 | RAP1A_1 | 0.2136 | WARS_2 | 0.1558 |
| CASP8AP2 | 0.1629 | GBE1 | 0.0391 | MIR571 | 0.0221 | RARA_3 | 0.0892 | XBP1_2 | 0.1152 |
| CCL13 | 0.1306 | GBP7 | 0.1191 | MIR576 | 0.1206 | RARG | 0.0474 | XRN2_1 | 0.0266 |
| CCR2_3 | 0.084 | GJA5_1 | 0.0582 | MIR654 | 0.0489 | RNASEK | 0.0544 | YARS2 | 0.0116 |
| CD34_1 | 0.0134 | GMNN | 0.1094 | MIR942 | 0.1246 | RNF7_1 | 0.009 | ZNF75D_2 | 0.1286 |
| CDC42BPA_2 | 0.0136 | GSR_2 | 0.0327 | MMP12_1 | 0.1311 | ROD1_1 | 0.2245 | ZSWIM42 | 0.1584 |
| CDC42SE2_2 | 0.0336 | HBA2 | 0.1975 | MYCN_2 | 0.1546 | SATB2 | 0.0589 | figo_numeric | 0.0112 |
| CLDN6 | 0.1165 | HCFC1R1_1 | 0.0469 | MYL9_2 | 0.1005 | SBSN | 0.0593 | hist_rev_SBOT | 0.048 |
| CREB5_2 | 0.0321 | HDAC7_2 | 0.0034 | MYOHD1 | 0.0188 | SCXB | 0.0082 | surg_outcome | 0.0076 |
| CRYBA1 | 0.0272 | HDLBP_3 | 0.0921 | NFATC3_5 | 0.0576 | SEC22C_3 | 0.1152 | | |
| CXCL13 | 0.0753 | HIC1 | 0.0553 | NFATC4 | 0.0597 | SELENBP1 | 0.1838 | | |
| CYB5R3_2 | 0.1815 | HPRT1_1 | 0.1329 | NLRP9 | 0.1731 | SERPINB22 | 0.0038 | | |
| CYP1A2 | 0.0617 | HPS4_1 | 0.0734 | NOVA2 | 0.1204 | SERPINBS | 0.1773 | | |

Table 65

| | | | | | | | | | |
|---|---|---|---|---|---|---|---|---|---|
| ABHD3 | 0.0753 | DNAH11 | 0.0499 | HPRT1_1 | 0.149 | NP | 0.0838 | SERPINB5 | 0.0096 |
| ADAM17_2 | 0.2396 | DNMT3L_2 | 0.0189 | HPS4_1 | 0.071 | NR6A1_2 | 0.1477 | SFN | 0.0559 |
| ADAMTS1 | 0.1705 | DOCK7_1 | 0.1343 | HR_1 | 0.0428 | NRXN3_3 | 0.0935 | SFRS4 | 0.0362 |
| ALS2CL_3 | 0.1143 | DSC3_1 | 0.07 | HSD11B1_1 | 0.1035 | NT5DC1_2 | 0.2034 | SHC1_3 | 0.0638 |
| ANO7_3 | 0.0691 | DUT_3 | 0.1147 | ICAM2 | 0.0492 | NTRK2_3 | 0.0026 | SLC23A1_2 | 0.1368 |
| ARL6IP1_1 | 0.0309 | EEF1E1_1 | 0.0886 | ICAM4_1 | 0.2806 | NUP155_1 | 0.0708 | SLC25A34 | 0.1838 |
| ARMCX3_2 | 0.0889 | EIF4ENIF1 | 0.1286 | IL1RAP_2 | 0.0593 | NYX | 0.1845 | SLC4A5_3 | 0.0834 |
| ATXN10_1 | 0.1967 | EMP1 | 0.1811 | IQCA1_2 | 0.019 | ODF2_3 | 0.0228 | SLC9A10 | 0.0815 |
| AXL_1 | 0.121 | ENO1 | 0.1365 | KCNIP3_1 | 0.1084 | ORC1L | 0.0184 | SNORD93 | 0.166 |
| BAI1_3 | 0.0386 | ENPEP_2 | 0.0192 | KCNQ2_1 | 0.1307 | OTUD7A_3 | 0.0362 | SOX2_1 | 0.0836 |
| BCAS1_1 | 0.3353 | EPHB1 | 0.0149 | KIF3C | 0.1841 | PANK4 | 0.0621 | STC1 | 0.0138 |
| BDNF_2 | 0.1212 | EPYC | 0.038 | KRT80_2 | 0.1226 | PDLIM2_2 | 0.2458 | STC2 | 0.1258 |
| BMPR1A | 0.1149 | ERI2_2 | 0.3036 | KRTAP10.10_2 | 0.0244 | PHYH_1 | 0.1966 | STYX_2 | 0.0528 |
| BTF3_3 | 0.1092 | ESPNL | 0.04 | L3MBTL2_3 | 0.0279 | PIGA_1 | 0.0049 | SYTL3 | 0.0215 |
| C10orf116 | 0.0388 | EZH2_1 | 0.0764 | LBH_2 | 0.0923 | PITX2_1 | 0.0986 | TAF15_1 | 0.0031 |
| C11orf24 | 0.1998 | FAM13AOS | 0.0466 | LENEP | 0.2273 | PKN1_3 | 0.0131 | TCEAL8_1 | 0.0381 |
| C11orf49_3 | 0.1186 | FAM187B_2 | 0.0017 | LGI3 | 0.1388 | PLAC9 | 0.2609 | THBS3 | 0.0936 |
| C14orf102_2 | 0.1322 | FAM70A_1 | 0.0953 | LOC492303 | 0.0409 | PLEKHG5_5 | 0.0169 | TM2D3_2 | 0.0623 |
| C14orf109_2 | 0.0672 | FBXO48_2 | 0.2665 | LRRC14B | 0.0252 | PLSCR4 | 0.1507 | TMEM52 | 0.0849 |
| C17orf106 | 0.2476 | FGF5_1 | 0.0676 | LRRC37A4_2 | 0.0573 | PMEPA1_4 | 0.1306 | TMEM62 | 0.0072 |
| C17orf58_2 | 0.0327 | FKBP10 | 0.0396 | LRRTM4 | 0.1777 | PNMA5 | 0.1068 | TNFRSF18_1 | 0.2664 |
| C17orf58_3 | 0.0286 | FLJ33360 | 0.2129 | MACC1 | 0.1394 | PPAPDC1A | 0.1249 | TNNT2_1 | 0.0068 |
| C18orf56 | 0.0457 | FLJ43752 | 0.0758 | MANSC1_1 | 0.1346 | PRAMEF5 | 0.0124 | TOMM20L | 0.0409 |
| C1orf168 | 0.0373 | FMNL3_2 | 0.0516 | MCAM | 0.0132 | PRKAA2 | 0.1392 | TPM2_2 | 0.1741 |
| C8orf79_1 | 0.1182 | FMOD | 0.2045 | MCART6_1 | 0.1464 | PSMC6_1 | 0.0212 | TRIM58 | 0.1153 |
| CALD1_2 | 0.0273 | FOSB | 0.0182 | MFRP | 0.2275 | RAD54B_2 | 0.1797 | UBR7_1 | 0.0683 |

| | | | | | | | |
|---|---|---|---|---|---|---|---|
| CASP8AP2 | 0.1379 | FOSL2 | 0.2805 | MIDN | 0.0484 | RAP1A_1 | 0.2124 | UBR7_2 | 0.1266 |
| CCL13 | 0.0946 | FOXN1 | 0.0323 | MIR1914 | 0.0643 | RARA_3 | 0.0871 | WARS_2 | 0.1377 |
| CCR2_3 | 0.0303 | GAD1_2 | 0.0022 | MIR212 | 0.1025 | RARG | 0.045 | XBP1_2 | 0.1186 |
| CD34_1 | 0.0016 | GBE1 | 0.0459 | MIR571 | 0.0364 | RNASEK | 0.071 | XRN2_1 | 0.0488 |
| CDC42BPA_2 | 0.0235 | GBP7 | 0.1193 | MIR576 | 0.0969 | RNF7_1 | 0.0109 | YARS2 | 0.0002 |
| CDC42SE2_2 | 0.0312 | GJA5_1 | 0.0518 | MIR654 | 0.057 | ROD1_1 | 0.2195 | ZNF75D_2 | 0.1579 |
| CLDN6 | 0.0946 | GMNN | 0.0993 | MIR942 | 0.1471 | SATB2 | 0.0557 | ZSWIM4_2 | 0.1639 |
| CREB5_2 | 0.0268 | GSR_2 | 0.0493 | MMP12_1 | 0.1336 | SBSN | 0.0468 | figo_numeric | 0.0091 |
| CRYBA1 | 0.0296 | HBA2 | 0.2062 | MYCN_2 | 0.1438 | SCXB | 0.0131 | hist_rev_SBOT | 0.0715 |
| CXCL13 | 0.0857 | HCFC1R1_1 | 0.0488 | NFATC3_5 | 0.1006 | SEC22C_3 | 0.1123 | surg_outcome | 0.0105 |
| CYB5R3_2 | 0.1914 | HDAC7_2 | 0.0028 | NFATC4 | 0.0092 | SELENBP1 | 0.1921 | | |
| CYP1A2 | 0.0552 | HDLBP_3 | 0.0961 | NLRP9 | 0.0491 | SERPINA12 | 0.0305 | | |
| DBNDD2 | 0.1041 | HIC1 | 0.0421 | NOVA2 | 0.1101 | SERPINB2_2 | 0.2064 | | |

EP 3 874 274 B1

Table 66

| | | | | | | | | | |
|---|---|---|---|---|---|---|---|---|---|
| ABHD3 | 0.0813 | DNAH11 | 0.0463 | HSD11B1_1 | 0.078 | NRXN3_3 | 0.093 | SFRS4 | 0.0519 |
| ADAM17_2 | 0.2417 | DNMT3L_2 | 0.0272 | ICAM2 | 0.0414 | NT5DC1_2 | 0.1888 | SHC1_3 | 0.0782 |
| ADAMTS1 | 0.168 | DOCK7_1 | 0.1553 | ICAM4_1 | 0.2728 | NTRK2_3 | 0.0016 | SLC23A1_2 | 0.1363 |
| ALS2CL_3 | 0.0825 | DSC3_1 | 0.0949 | IL1RAP_2 | 0.0598 | NUP155_1 | 0.0488 | SLC25A34 | 0.1694 |
| ANO7_3 | 0.036 | DUT_3 | 0.1324 | IQCA1_2 | 0.0368 | NYX | 0.1773 | SLC4A5_3 | 0.0799 |
| ARL6IP1_1 | 0.0313 | EEF1E11 | 0.0895 | KCNIP3_1 | 0.1115 | ODF2_3 | 0.0107 | SLC9A10 | 0.0781 |
| ARMCX3_2 | 0.0864 | EMP1 | 0.1266 | KCNQ2_1 | 0.1224 | ORC1L | 0.0338 | SNORD93 | 0.1573 |
| ATXN10_1 | 0.1628 | ENO1 | 0.2039 | KIF3C | 0.1817 | OTUD7A_3 | 0.0255 | SOX2_1 | 0.0598 |
| AXL_1 | 0.0992 | ENPEP_2 | 0.1438 | KRT80_2 | 0.1172 | PANK4 | 0.0548 | STC1 | 0.012 |
| BAI1_3 | 0.0221 | EPHB1 | 0.0327 | KRTAP10.10_2 | 0.0261 | PDLIM2_2 | 0.2515 | STC2 | 0.1203 |
| BCAS1_2 | 0.3397 | EPYC | 0.0302 | L3MBTL2_3 | 0.0233 | PHYH_1 | 0.222 | STYX_2 | 0.0493 |
| BDNF_2 | 0.0781 | ERI2_2 | 0.3129 | LBH_2 | 0.1123 | PIGA_1 | 0.0063 | SYTL3 | 0.0566 |
| BMPR1A | 0.1331 | ESPNL | 0.0357 | LENEP | 0.2331 | PITX2_1 | 0.1173 | TAF15_1 | 0.0065 |
| BTF3_3 | 0.136 | EZH2_1 | 0.0926 | LGI3 | 0.105 | PKN1_3 | 0.0283 | TCEAL8_1 | 0.0263 |
| C10orf116 | 0.0124 | FAM13AOS | 0.063 | LOC492303 | 0.0406 | PLAC9 | 0.265 | THBS3 | 0.0942 |
| C11orf24 | 0.2051 | FAM187B_2 | 0.0004 | LRRC14B | 0.0007 | PLEKHG5_5 | 0.0183 | TM2D3_2 | 0.0543 |
| C11orf49_3 | 0.1131 | FAM70A_1 | 0.0949 | LRRC37A4_2 | 0.0693 | PLSCR4 | 0.1345 | TMEM52 | 0.0817 |
| C14orf102_2 | 0.1066 | FBXO48_2 | 0.2386 | LRRTM4 | 0.1472 | PMEPA1_4 | 0.1282 | TMEM62 | 0.0063 |
| C14orf109_2 | 0.0758 | FKBP10 | 0.069 | MACC1 | 0.1316 | PNMA5 | 0.1223 | TNFRSF18_1 | 0.2525 |
| C17orf106 | 0.2221 | FLJ33360 | 0.0282 | MANSC1_1 | 0.1065 | PPAPDC1A | 0.1156 | TNNT2_1 | 0.0017 |
| C17orf58__2 | 0.0306 | FLJ43752 | 0.1748 | MCAM | 0.0085 | PRAMEF5 | 0.017 | TOMM20L | 0.0423 |
| C17orf58_3 | 0.0163 | FMNL3_2 | 0.0607 | MCART6_1 | 0.1497 | PRKAA2 | 0.135 | TPM2_2 | 0.1761 |
| C18orf56 | 0.0649 | FOSB | 0.1996 | MFRP | 0.2506 | PSMC6_1 | 0.0037 | TRIM58 | 0.0982 |
| C1orf168 | 0.0484 | FOSL2 | 0.0233 | MIDN | 0.0414 | RAD54B_2 | 0.171 | UBR7_1 | 0.08 |
| C8orf79_1 | 0.1138 | FOXN1 | 0.2601 | MIR1914 | 0.0747 | RAP1A_1 | 0.2305 | UBR7_2 | 0.1363 |
| CALD1_2 | 0.0301 | GAD1_2 | 0.0046 | MIR212 | 0.1086 | RARA_3 | 0.0855 | WARS_2 | 0.1761 |

| | | | | | | | | |
|---|---|---|---|---|---|---|---|---|
| CASP8AP2 | 0.1358 | GBE1 | 0.0512 | MIR571 | 0.01 | RARG | 0.0603 | XBP1_2 | 0.1363 |
| CCL13 | 0.0983 | GBP7 | 0.1278 | MIR576 | 0.1146 | RNASEK | 0.0682 | XRN2_1 | 0.0457 |
| CCR2_3 | 0.0515 | GJA5_1 | 0.0642 | MIR654 | 0.0528 | RNF7_1 | 0.0087 | YARS2 | 0.0061 |
| CD34_1 | 0.0251 | GMNN | 0.0978 | MIR942 | 0.1236 | ROD1_1 | 0.2205 | ZNF75D_2 | 0.1561 |
| CDC42BPA_2 | 0.0376 | GSR_2 | 0.0424 | MMP12_1 | 0.1376 | SATB2 | 0.0456 | ZSWIM4_2 | 0.1787 |
| CDC42SE2_2 | 0.0385 | HBA2 | 0.1909 | MYCN_2 | 0.1554 | SBSN | 0.0511 | figo_numeric | 0.0268 |
| CLDN6 | 0.1119 | HCFC1R1_1 | 0.0432 | MYOHD1 | 0.089 | SCXB | 0.008 | hist_rev_SBOT | 0.0578 |
| CREB5_2 | 0.0019 | HDAC7_2 | 0.0172 | NFATC3_5 | 0.0166 | SEC22C_3 | 0.119 | surg_outcome | 0.0025 |
| CRYBA1 | 0.0221 | HDLBP_3 | 0.073 5 | NFATC4 | 0.0421 | SELENBP1 | 0.1894 | | |
| CXCL13 | 0.0917 | HIC1 | 0.0085 | NLRP9 | 0.1783 | SERPINA12 | 0.0405 | | |
| CYB5R3_2 | 0.1818 | HPRT1_1 | 0.1391 | NOVA2 | 0.1139 | SERPINB2_2 | 0.2056 | | |
| CYP1A2 | 0.0482 | HPS4_1 | 0.0659 | NP | 0.1069 | SERPINB5 | 0.0027 | | |
| DBNDD2 | 0.0995 | HR_1 | 0.0647 | NR6A1_2 | 0.134 | SFN | 0.0615 | | |

Table 67

| | | | | | | | | | |
|---|---|---|---|---|---|---|---|---|---|
| ABHD3 | 0.092 | DBNDD2 | 0.0823 | HPS4_1 | 0.0446 | NOVA2 | 0.0969 | SERPINB5 | 0.2086 |
| ADAM17_2 | 0.231 | DFFB_2 | 0.0518 | HR_1 | 0.0522 | NP | 0.0897 | SFN | 0.0129 |
| ADAMTS1 | 0.1781 | DNAH11 | 0.034 | HSD11B1_1 | 0.0925 | NR6A1_2 | 0.1351 | SFRS4 | 0.0448 |
| ALS2CL_3 | 0.1139 | DNMT3L2 | 0.11 | ICAM2 | 0.0495 | NRXN3_3 | 0.0753 | SHC1_3 | 0.1023 |
| ANO7_3 | 0.0426 | DOCK7_1 | 0.0187 | ICAM4_1 | 0.2756 | NT5DC1_2 | 0.2076 | SLC23A1_2 | 0.0999 |
| ARL6IP1_1 | 0.0235 | DSC3_1 | 0.0559 | IL1RAP_2 | 0.0619 | NTRK2_3 | 0.0093 | SLC25A34 | 0.1057 |
| ARMCX3_2 | 0.0869 | DUT_3 | 0.1371 | IQCA1_2 | 0.0244 | NUP155 1 | 0.0376 | SLC4A5_3 | 0.0804 |
| ATXN10_1 | 0.1669 | EEF1E1_1 | 0.0555 | KCNIP3_1 | 0.0919 | NYX | 0.1149 | SLC9A10 | 0.0886 |
| AXL_1 | 0.0917 | EMP1 | 0.1035 | KCNQ2_1 | 0.1481 | ODF2_3 | 0.0222 | SNORD93 | 0.1509 |
| BAI1_3 | 0.0549 | ENO1 | 0.1519 | KIF3C | 0.1888 | ORCIL | 0.0674 | SOX2_1 | 0.062 |
| BCAS1_1 | 0.3084 | ENPEP_2 | 0.123 | KRT80_2 | 0.0763 | OTUD7A_3 | 0.0279 | STC1 | 0.011 |
| BDNF_2 | 0.097 | EPHB1 | 0.039 | KRTAP10.10_2 | 0.0074 | PANK4 | 0.0527 | STC2 | 0.0917 |
| BMPR1A | 0.1162 | EPYC | 0.022 | L3MBTL2_3 | 0.0295 | PDLIM2_2 | 0.2283 | STYX_2 | 0.0541 |
| BTF3_3 | 0.1203 | ERI2_2 | 0.2891 | LBH_2 | 0.104 | PHYH_1 | 0.2252 | SYTL3 | 0.0019 |
| C10orf116 | 0.0551 | ESPNL | 0.0825 | LENEP | 0.2161 | PIGA_1 | 0.0103 | TAF15_1 | 0.0193 |
| C11orf24 | 0.1302 | EZH2_1 | 0.0708 | LGI3 | 0.1333 | PITX2_1 | 0.09 | TCEAL8_1 | 0.0543 |
| C11orf49_3 | 0.1285 | FAM13AOS | 0.0307 | LOC492303 | 0.0501 | PKN13 | 0.0565 | THBS3 | 0.0886 |
| C14orf102_2 | 0.095 | FAM187B_2 | 0.0247 | LRRC14B | 0.0258 | PLAC9 | 0.2524 | TM2D3_2 | 0.0481 |
| C14orf109_2 | 0.0665 | FAM70A_1 | 0.1057 | LRRC37A4_2 | 0.0699 | PLEKHG5_5 | 0.0184 | TN19SF4 | 0.0564 |
| C17orf106 | 0.2147 | FBXO48_2 | 0.2173 | LRRTM4 | 0.1677 | PLSCR4 | 0.1682 | TMEM52 | 0.0012 |
| C17orf58_2 | 0.0276 | FKBP10 | 0.0998 | MACC1 | 0.1239 | PMEPA1_4 | 0.1253 | TMEM62 | 0.2507 |
| C17orf58_3 | 0.0332 | FLJ33360 | 0.0357 | MANSC1_1 | 0.1271 | PNMA5 | 0.1472 | TNFRSF18_1 | 0.0635 |
| C18orf56 | 0.0455 | FLJ43752 | 0.1808 | MAPK3_1 | 0.0573 | PPAPDC1A | 0.1119 | TNNT2_1 | 0.0045 |
| C1orf168 | 0.0363 | FMNL3_2 | 0.0142 | MCAM | 0.0936 | PRAMEF5 | 0.0337 | TOMM20L | 0.0402 |
| C1orf64 | 0.1077 | FOSB | 0.1906 | MCART6_1 | 0.2165 | PRKAA2 | 0.1159 | TPM2_2 | 0.1653 |
| C8orf79_1 | 0.0746 | FOSL2 | 0.0218 | MFRP | 0.0326 | PSMC6_1 | 0.008 | TRIM58 | 0.1041 |

| | | | | | | | | | |
|---|---|---|---|---|---|---|---|---|---|
| CALD1_2 | 0.1468 | FOXN1 | 0.2726 | MIDN | 0.0529 | RAD54B_2 | 0.1972 | UBR7_1 | 0.0374 |
| CASP8AP2 | 0.1247 | GAD1_2 | 0.0031 | MIR1914 | 0.0672 | RAP1A_1 | 0.2178 | UBR7_2 | 0.1358 |
| CCL13 | 0.1081 | GBE1 | 0.0632 | MIR212 | 0.0983 | RARA_3 | 0.0843 | WARS_2 | 0.1819 |
| CCR2_3 | 0.05 | GBP7 | 0.1057 | MIR571 | 0.0031 | RARG | 0.0129 | XBP1_2 | 0.1673 |
| CD34_1 | 0.0404 | GJA5_1 | 0.0456 | MIR576 | 0.0994 | RNASEK | 0.0588 | XRN2_1 | 0.0194 |
| CDC42BPA_2 | 0.0286 | GMNN | 0.0921 | MIR654 | 0.0058 | RNF7_1 | 0.0207 | YARS2 | 0.002 |
| CDC42SE2_2 | 0.0053 | GSR_2 | 0.0269 | MIR942 | 0.1102 | ROD1_1 | 0.2203 | ZNF75D_2 | 0.1469 |
| CLDN6 | 0.1173 | GUSBL2 | 0.1963 | MMP12_1 | 0.1328 | SATB2 | 0.0515 | ZSWIM4_2 | 0.1592 |
| CREB5_2 | 0.0098 | HBA2 | 0.0603 | MYCN_2 | 0.158 | SBSN | 0.055 | figo_numeric | 0.0419 |
| CRYBA1 | 0.0357 | HDAC7_2 | 0.0411 | MYOHD1 | 0.0799 | SCXB | 0.0067 | hist_rev_SBOT | 0.0451 |
| CXCL13 | 0.0825 | HDLBP_3 | 0.2042 | NFATC3_5 | 0.0219 | SEC22C_3 | 0.1065 | surg_outcome | 0.017 |
| CYB5R3_2 | 0.1634 | HIC1 | 0.0782 | NFATC4 | 0.0494 | SELENBP1 | 0.1878 | | |
| CYP1A2 | 0.0648 | HPRT1_1 | 0.1527 | NLRP9 | 0.1568 | SERPINB2_2 | 0.0114 | | |

Table 68

| | | | | | | | |
|---|---|---|---|---|---|---|---|
| ABHD3 | 0.0643 | DNAH11 | 0.0426 | HSD11B1_1 | 0.0867 | NR6A1_2 | 0.1526 | SFRS4 | 0.0288 |
| ADAM17_2 | 0.2333 | DNMT3L_2 | 0.0252 | ICAM2 | 0.0554 | NRXN3_3 | 0.0987 | SHC1_3 | 0.0719 |
| ADAMTS1 | 0.1738 | DOCK7_1 | 0.1382 | ICAM4_1 | 0.2771 | NT5DC1_2 | 0.1812 | SLC23A1_2 | 0.14 |
| ALS2CL_3 | 0.1042 | DSC3_1 | 0.0691 | IL1RAP_2 | 0.0553 | NTRK23 | 0.001 | SLC25A34 | 0.1602 |
| ANO7_3 | 0.0661 | DUT_3 | 0.1237 | IQCA1_2 | 0.0313 | NUP155_1 | 0.0463 | SLC4A5_3 | 0.084 |
| ARL6IP1_1 | 0.0312 | EEF1E1_1 | 0.0875 | KCNIP3_1 | 0.1019 | NYX | 0.171 | SLC9A10 | 0.0844 |
| ARMCX3_2 | 0.0817 | EMP1 | 0.1139 | KCNQ2_1 | 0.128 | ODF2_3 | 0.0045 | SNORD93 | 0.1626 |
| ATXN10_1 | 0.2039 | ENO1 | 0.1828 | KIF3C | 0.1851 | ORC1L | 0.033 | SOX2_1 | 0.0747 |
| AXL_1 | 0.1044 | ENPEP_2 | 0.1387 | KRT80_2 | 0.1075 | OTUD7A_3 | 0.0278 | STC1 | 0.0014 |
| BAI1_3 | 0.0254 | EPHB1 | 0.0428 | KRTAP10.10_2 | 0.0196 | PANK4 | 0.063 | STC2 | 0.1297 |
| BCAS1_1 | 0.3278 | EPYC | 0.0377 | L3MBTL2_3 | 0.0353 | PDLIM2_2 | 0.2405 | STYX_2 | 0.0473 |
| BDNF_2 | 0.1062 | ERI2_2 | 0.2923 | LBH2 | 0.0987 | PHYH_1 | 0.1978 | SYTL3 | 0.0084 |
| BMPR1A | 0.1109 | ESPNL | 0.0366 | LENEP | 0.228 | PIGA_1 | 0.0045 | TAF15_1 | 0.0097 |
| BTF3_3 | 0.1034 | EZH2_1 | 0.0721 | LGI3 | 0.1153 | PITX2_1 | 0.0862 | TCEAL8_1 | 0.0403 |
| C10orf116 | 0.0285 | FAM13AOS | 0.0541 | LOC492303 | 0.0278 | PKN1_3 | 0.0166 | THBS3 | 0.0982 |
| C11orF24 | 0.1719 | FAM187B_2 | 0.0161 | LRRC14B | 0.0144 | PLAC9 | 0.2593 | THY1 | 0.056 |
| C11orf49_3 | 0.1344 | FAM70A_1 | 0.0771 | LRRC37A4_2 | 0.0612 | PLEKHG5_5 | 0.0354 | TM2D3_2 | 0.083 |
| C14orf102_2 | 0.1273 | FBXO48_2 | 0.2613 | LRRTM4 | 0.1651 | PLSCR4 | 0.1759 | TMEM52 | 0.0074 |
| C14orf109_2 | 0.0723 | FKBP10 | 0.0654 | MACC1 | 0.1255 | PMEPA1_4 | 0.1183 | TMEM62 | 0.0205 |
| C17orf106 | 0.236 | FLJ33360 | 0.0503 | MANSC1_1 | 0.1413 | PNMA5 | 0.1235 | TNFRSF18_1 | 0.2618 |
| C17orf58__2 | 0.039 | FLJ43752 | 0.1879 | MCAM | 0.0155 | PPAPDC1A | 0.13 | TNNT2_1 | 0.0032 |
| C17orf58_3 | 0.0258 | FMNL3_2 | 0.0375 | MCART6_1 | 0.1327 | PRAMEF5 | 0.0112 | TOMM20L | 0.0376 |
| C18orf56 | 0.0357 | FOSB | 0.1977 | MFRP | 0.2201 | PRKAA2 | 0.1334 | TPM2_2 | 0.1788 |
| C1orf168 | 0.029 | FOSL2 | 0.0275 | MIDN | 0.0466 | PSMC6_1 | 0.0051 | TRIM58 | 0.1098 |
| C1orf64 | 0.1061 | FOXN1 | 0.2655 | MIR1914 | 0.0738 | RAD54B_2 | 0.1858 | UBR7_1 | 0.0567 |
| C8orf79_1 | 0.0282 | GAD1_2 | 0.0265 | MIR212 | 0.1083 | RAP1A_1 | 0.2178 | UBR7_2 | 0.1156 |

| | | | | | | | |
|---|---|---|---|---|---|---|---|
| CASP8AP2 | 0.1462 | GBE1 | 0.0413 | MIR571 | 0.034 | RARA_3 | 0.0893 | WARS_2 | 0.1603 |
| CCL13 | 0.129 | GBP7 | 0.1329 | MIR576 | 0.1089 | RARG | 0.0478 | XBP1_2 | 0.1325 |
| CCR2_3 | 0.0868 | GJA5_1 | 0.0497 | MIR654 | 0.0541 | RNASEK | 0.0584 | XRN2_1 | 0.0516 |
| CD34_1 | 0.015 | GMNN | 0.0972 | MIR942 | 0.1201 | RNF7_1 | 0.0139 | YARS2 | 0.0011 |
| CDC42BPA_2 | 0.0287 | GSR_2 | 0.0357 | MMP12_1 | 0.1355 | ROD1_1 | 0.2167 | ZNF75D_2 | 0.1494 |
| CDC42SE2_2 | 0.0189 | HBA2 | 0.2004 | MYCN_2 | 0.1427 | SATB2 | 0.0611 | ZSWIM42 | 0.1602 |
| CLDN6 | 0.1121 | HCFC1R1_1 | 0.0523 | MYL9_2 | 0.0941 | SBSN | 0.0707 | figo_numeric | 0.0217 |
| CREB5_2 | 0.0152 | HDAC7_2 | 0.0141 | MYOHD1 | 0.0068 | SCXB | 0.004 | hist_rev_SBOT | 0.0535 |
| CRYBA1 | 0.0211 | HDLBP_3 | 0.1047 | NFATC3_5 | 0.0528 | SEC22C_3 | 0.1185 | surg_outcome | 0.007 |
| CXCL13 | 0.0763 | HIC1 | 0.0469 | NFATC4 | 0.0555 | SELENBP1 | 0.1939 | | |
| CYB5R3_2 | 0.1894 | HPRT1_1 | 0.1578 | NLRP9 | 0.1795 | SERPINB2_2 | 0.0093 | | |
| CYP1A2 | 0.0571 | HPS4_1 | 0.0647 | NOVA2 | 0.1188 | SERPINB5 | 0.1987 | | |
| DBNDD2 | 0.1074 | HR_1 | 0.0449 | NP | 0.0934 | SFN | 0.0093 | | |

**Claims**

1. Bevacizumab for use in treating a patient suffering from ovarian cancer following removal of a tumor, wherein the patient has been predicted to benefit from the administration of bevacizumab by a method comprising:
determining whether the patient is predicted to benefit from the administration of bevacizumab, wherein such determination comprises:

   determining the patient's gene expression level of microfibril associated protein 2 (MFAP2); and
   determining the patient's gene expression level of vascular endothelial growth factor A (VEGFA); and
   determining the patient's predicted progression-free survival time with the administration of a platinum-based chemotherapy without bevacizumab, wherein such determination comprises:

      determining the patient's gene expression level of microfibril associated protein 2 (MFAP2);
      determining the patient's gene expression level of vascular endothelial growth factor A (VEGFA);
      determining the patient's International Federation of Gynecology and Obstetrics (FIGO) stage;
      determining the patient's Eastern Cooperative Oncology Group (ECOG) performance status; and
      determining the size of the tumor tissue remaining post-removal of the tumor, wherein:

         a gene expression level of MFAP greater than a threshold gene expression level indicates a decreased likelihood of benefit from platinum-based chemotherapy, wherein the threshold gene expression level is selected based on a clinical outcome;
         a gene expression level of VEGFA greater than a threshold gene expression level indicates an increased likelihood of benefit from the administration of platinum-based chemotherapy, wherein the threshold gene expression level is selected based on a clinical outcome;
         a FIGO stage greater than 1 indicates a decreased likelihood of benefit from platinum-based chemotherapy,
         an ECOG performance status greater than 0 indicates a decreased likelihood of benefit from platinum-based chemotherapy, and
         a tumor size smaller than 1 cm indicates an increased likelihood of benefit from platinum-based chemotherapy.

2. Bevacizumab for use according to claim 1, wherein determining whether the patient is predicted to benefit from the administration of bevacizumab comprises determining whether the patient is predicted to benefit from the administration of bevacizumab in addition to the administration of platinum-based chemotherapy.

3. Bevacizumab for use according to claim 1, wherein the clinical outcome comprises increased time of progression-free survival.

4. Bevacizumab for use according to claim 3, wherein the patient's predicted increase in progression-free survival is at least 3 months, at least 4 months, at least 5 months, at least 6 months, at least 7 months, at least 8 months, at least 9 months, or at least 10 months.

5. Bevacizumab for use according to any one of the preceding claims, wherein determining whether the patient is predicted to benefit from the administration of bevacizumab further comprises determining if the patient's predicted progression-free survival time with the administration of a platinum-based chemotherapy and bevacizumab is greater than the patient's predicted progression-free survival time with the administration of a platinum-based chemotherapy without bevacizumab.

6. Bevacizumab for use according to claim 5, wherein the patient is predicted to benefit from the administration of bevacizumab if the patient's predicted increase in progression-free survival is clinically meaningful.

7. Bevacizumab for use according to claim 6, wherein the patient is predicted to benefit from the administration of bevacizumab if the patient's predicted increase in progression-free survival is at least 3 months, at least 4 months, at least 5 months, at least 6 months, at least 7 months, at least 8 months, at least 9 months, or at least 10 months.

8. Bevacizumab for use according to any one of the preceding claims, wherein determining whether the patient is predicted to benefit from the administration of bevacizumab comprises defining a benefit threshold.

9. Bevacizumab for use according to any one of the preceding claims, wherein determining whether the patient is predicted to benefit from the administration of bevacizumab comprises applying a Cox model.

10. Bevacizumab for use according to any one of the preceding claims, wherein the method comprises administering platinum-based chemotherapy.

11. Bevacizumab for use according to any one of the preceding claims, wherein the tumor comprises a primary tumor or a secondary tumor.

12. Bevacizumab for use according to any one of the preceding claims, wherein the method further comprises:

receiving an identified set of biomarkers determined based on a set of predetermined data comprising clinical data, gene expression data, or both, wherein the identified set of biomarkers comprises at least MFAP2 and VEGFA;
identifying other sets of biomarkers based on the identified set of biomarkers and remaining data comprising the set of predetermined data excluding the identified set of biomarkers; and
generating a signature for each set of biomarkers to predict an outcome for a patient having ovarian cancer, wherein determining whether the patient is predicted to benefit from the administration of bevacizumab is based on an ensemble prediction using a plurality of signatures and patient test data comprising clinical data, gene expression data, or both.


**Patentansprüche**

1. Bevacizumab zur Verwendung bei der Behandlung eines Patienten, der nach der Entfernung eines Tumors an Eierstockkrebs leidet, wobei vorhergesagt wurde, dass der Patient von der Verabreichung von Bevacizumab durch ein Verfahren profitieren wird, umfassend:

Bestimmen, ob der Patient voraussichtlich von der Verabreichung von Bevacizumab profitieren wird, wobei dieses Bestimmen Folgendes umfasst:

Bestimmen der Genexpression des Mikrofibrillenassoziierten Proteins 2 (MFAP2) bei dem Patienten; und
Bestimmen des Genexpressionsniveaus des vaskulären endothelialen Wachstumsfaktors A (VEGFA) des Patienten; und
Bestimmen der vorhergesagten progressionsfreien Überlebenszeit des Patienten mit der Verabreichung einer platinbasierten Chemotherapie ohne Bevacizumab, wobei dieses Bestimmen Folgendes umfasst:

Bestimmen der Genexpression des Mikrofibrillenassoziierten Proteins 2 (MFAP2) bei dem Patienten;
Bestimmen des Genexpressionsniveaus des vaskulären endothelialen Wachstumsfaktors A (VEGFA) des Patienten;
Bestimmen des Stadiums der International Federation of Gynecology and Obstetrics (FIGO) des Patienten;
Bestimmen des Leistungsstatus des Patienten nach der Eastern Cooperative Oncology Group (ECOG); und
Bestimmen der Größe des Tumorgewebes, das nach der Entfernung des Tumors verbleibt, wobei:

ein Genexpressionsniveau von MFAP, das größer als ein Schwellenwertexpressionsniveau ist, eine verringerte Wahrscheinlichkeit des Nutzens einer platinbasierten Chemotherapie anzeigt, wobei das Schwellenwertexpressionsniveau basierend auf einem klinischen Ergebnis ausgewählt wird;
ein Genexpressionsniveau von VEGFA, das größer als ein Schwellenwertexpressionsniveau ist, eine erhöhte Wahrscheinlichkeit eines Nutzens der Verabreichung einer platinbasierten Chemotherapie anzeigt, wobei das Schwellenwertexpressionsniveau auf der Grundlage eines klinischen Ergebnisses ausgewählt wird;
ein FIGO-Stadium größer als 1 weist auf eine verringerte Wahrscheinlichkeit hin, von einer platinbasierten Chemotherapie zu profitieren,
ein ECOG-Leistungsstatus größer als 0 weist auf eine verringerte Wahrscheinlichkeit hin, von einer platinbasierten Chemotherapie zu profitieren, und

eine Tumorgröße von weniger als 1 cm auf eine erhöhte Wahrscheinlichkeit hinweist, von einer platinbasierten Chemotherapie zu profitieren.

2. Bevacizumab zur Verwendung nach Anspruch 1, wobei das Bestimmen, ob der Patient voraussichtlich von der Verabreichung von Bevacizumab profitiert, das Bestimmen umfasst, ob der Patient voraussichtlich von der Verabreichung von Bevacizumab zusätzlich zur Verabreichung einer platinbasierten Chemotherapie profitiert.

3. Bevacizumab zur Verwendung nach Anspruch 1, wobei das klinische Ergebnis eine verlängerte Zeit des progressionsfreien Überlebens umfasst.

4. Bevacizumab zur Verwendung nach Anspruch 3, wobei die vorhergesagte Verlängerung des progressionsfreien Überlebens des Patienten mindestens 3 Monate, mindestens 4 Monate, mindestens 5 Monate, mindestens 6 Monate, mindestens 7 Monate, mindestens 8 Monate, mindestens 9 Monate oder mindestens 10 Monate beträgt.

5. Bevacizumab zur Verwendung nach einem der vorhergehenden Ansprüche, wobei das Bestimmen, ob der Patient voraussichtlich von der Verabreichung von Bevacizumab profitiert, weiter das Bestimmen umfasst, ob die vorhergesagte progressionsfreie Überlebenszeit des Patienten bei Verabreichung einer platinbasierten Chemotherapie und Bevacizumab größer ist als die vorhergesagte progressionsfreie Überlebenszeit des Patienten bei Verabreichung einer platinbasierten Chemotherapie ohne Bevacizumab.

6. Bevacizumab zur Verwendung nach Anspruch 5, wobei vorhergesagt wird, dass der Patient von der Verabreichung von Bevacizumab profitiert, wenn die vorhergesagte Verlängerung des progressionsfreien Überlebens des Patienten klinisch bedeutsam ist.

7. Bevacizumab zur Verwendung nach Anspruch 6, wobei vorhergesagt wird, dass der Patient von der Verabreichung von Bevacizumab profitiert, wenn die vorhergesagte Verlängerung des progressionsfreien Überlebens des Patienten mindestens 3 Monate, mindestens 4 Monate, mindestens 5 Monate, mindestens 6 Monate, mindestens 7 Monate, mindestens 8 Monate, mindestens 9 Monate oder mindestens 10 Monate beträgt.

8. Bevacizumab zur Verwendung nach einem der vorhergehenden Ansprüche, wobei das Bestimmen, ob der Patient voraussichtlich von der Verabreichung von Bevacizumab profitiert, das Definieren einer Nutzenschwelle umfasst.

9. Bevacizumab zur Verwendung nach einem der vorhergehenden Ansprüche, wobei das Bestimmen, ob der Patient voraussichtlich von der Verabreichung von Bevacizumab profitieren wird, das Anwenden eines Cox-Modells umfasst.

10. Bevacizumab zur Verwendung nach einem der vorhergehenden Ansprüche, wobei das Verfahren die Verabreichung einer platinbasierten Chemotherapie umfasst.

11. Bevacizumab zur Verwendung nach einem der vorangehenden Ansprüche, wobei der Tumor einen Primärtumor oder einen Sekundärtumor umfasst.

12. Bevacizumab zur Verwendung nach einem der vorangehenden Ansprüche, wobei das Verfahren weiter Folgendes umfasst:

Empfangen eines identifizierten Satzes von Biomarkern, bestimmt basierend auf einem Satz von vorbestimmten Daten, umfassend klinische Daten, Genexpressionsdaten oder beides, wobei der identifizierte Satz von Biomarkern mindestens MFAP2 und VEGFA umfasst;
Identifizieren anderer Sätze von Biomarkern basierend auf dem identifizierten Satz von Biomarkern und verbleibenden Daten, die den Satz von vorbestimmten Daten umfassen, die den identifizierten Satz von Biomarkern ausschließen; und
Erzeugen einer Signatur für jeden Satz von Biomarkern, um ein Ergebnis für einen Patienten, der Eierstockkrebs aufweist, vorherzusagen,
wobei das Bestimmen, ob der Patient voraussichtlich von der Verabreichung von Bevacizumab profitiert, auf einer Ensemble-Vorhersage unter Verwendung einer Vielzahl von Signaturen und Patiententestdaten, die klinische Daten, Genexpressionsdaten oder beides umfassen, bestimmt wird.

**Revendications**

1. Bévacizumab destiné à être utilisé dans le traitement d'une patiente atteinte d'un cancer des ovaires après l'ablation d'une tumeur, dans lequel il a été prévu que la patiente bénéficierait de l'administration de bévacizumab par un procédé comprenant :

   le fait de déterminer si la patiente devrait bénéficier de l'administration de bévacizumab, une telle détermination comprenant :

      le fait de déterminer le niveau d'expression du gène pour la protéine 2 associée aux microfibrilles (MFAP2) de la patiente ; et
      le fait de déterminer le niveau d'expression du gène du facteur de croissance endothélial vasculaire A (VEGFA) de la patiente ; et
      le fait de déterminer la durée de survie sans progression prévue de la patiente avec l'administration d'une chimiothérapie à base de platine sans bévacizumab, une telle détermination comprenant :

         le fait de déterminer le niveau d'expression du gène de la protéine 2 associée aux microfibrilles (MFAP2) de la patiente ;
         le fait de déterminer le niveau d'expression du gène du facteur de croissance endothélial vasculaire A (VEGFA) de la patiente ;
         le fait de déterminer le stade de la Fédération Internationale de Gynécologie et d'Obstétrique (FIGO) de la patiente ;
         le fait de déterminer l'état de performance ECOG (Eastern Cooperative Oncology Group) de la patiente ; et
         le fait de déterminer la taille du tissu tumoral restant après l'ablation de la tumeur, dans lequel :

            un niveau d'expression du gène de la MFAP supérieur à un niveau d'expression du gène seuil indique une probabilité réduite de réaction bénéfique à une chimiothérapie à base de platine, dans lequel le niveau d'expression du gène seuil est sélectionné sur la base d'un résultat clinique ;
            un niveau d'expression du gène du VEGFA supérieur à un niveau d'expression du gène seuil indique une probabilité accrue de réaction bénéfique à l'administration d'une chimiothérapie à base de platine, le niveau d'expression du gène seuil étant sélectionné sur la base d'un résultat clinique ;
            un stade FIGO supérieur à 1 indique une probabilité réduite de réaction bénéfique à une chimiothérapie à base de platine,
            un état de performance ECOG supérieur à 0 indique une probabilité réduite de réaction bénéfique à une chimiothérapie à base de platine, et
            une taille de tumeur inférieure à 1 cm indique une probabilité accrue de réaction bénéfique à une chimiothérapie à base de platine.

2. Bévacizumab destiné à être utilisé selon la revendication 1, dans lequel le fait de déterminer si la patiente devrait bénéficier de l'administration de bévacizumab comprend le fait de déterminer si la patiente devrait bénéficier de l'administration de bévacizumab en plus de l'administration d'une chimiothérapie à base de platine.

3. Bévacizumab destiné à être utilisé selon la revendication 1, dans lequel le résultat clinique comprend une durée accrue de survie sans progression.

4. Bévacizumab destiné à être utilisé selon la revendication 3, dans lequel l'augmentation prévue de la survie sans progression de la patiente est d'au moins 3 mois, au moins 4 mois, au moins 5 mois, au moins 6 mois, au moins 7 mois, au moins 8 mois, au moins 9 mois ou au moins 10 mois.

5. Bévacizumab destiné à être utilisé selon l'une quelconque des revendications précédentes, dans lequel le fait de déterminer si la patiente devrait bénéficier de l'administration de bévacizumab comprend en outre le fait de déterminer si la durée de survie sans progression prévue de la patiente avec l'administration d'une chimiothérapie à base de platine et de bévacizumab est supérieure à la durée de survie sans progression prévue de la patiente avec l'administration d'une chimiothérapie à base de platine sans bévacizumab.

6. Bévacizumab destiné à être utilisé selon la revendication 5, dans lequel il est prévu que la patiente bénéficie de l'administration de bévacizumab si l'augmentation prévue du patient dans la survie sans progression est cliniquement significative.

**7.** Bévacizumab destiné à être utilisé selon la revendication 6, dans lequel il est prévu que la patiente bénéficie de l'administration de bévacizumab si l'augmentation prévue de la survie sans progression de la patiente est d'au moins 3 mois, au moins 4 mois, au moins 5 mois, au moins 6 mois, au moins 7 mois, au moins 8 mois, au moins 9 mois ou au moins 10 mois.

**8.** Bévacizumab destiné à être utilisé selon l'une quelconque des revendications précédentes, dans lequel le fait de déterminer si la patiente devrait bénéficier de l'administration de bévacizumab comprend la définition d'un seuil bénéfique.

**9.** Bévacizumab destiné à être utilisé selon l'une quelconque des revendications précédentes, dans lequel le fait de déterminer si la patiente devrait bénéficier de l'administration de bévacizumab comprend l'application d'un modèle de Cox.

**10.** Bévacizumab destiné à être utilisé selon l'une quelconque des revendications précédentes, dans lequel le procédé comprend l'administration d'une chimiothérapie à base de platine.

**11.** Bévacizumab destiné à être utilisé selon l'une quelconque des revendications précédentes, dans lequel la tumeur comprend une tumeur primaire ou une tumeur secondaire.

**12.** Bévacizumab destiné à être utilisé selon l'une quelconque des revendications précédentes, dans lequel le procédé comprend en outre :

la réception d'un ensemble identifié de biomarqueurs déterminé sur la base d'un ensemble de données prédéterminées comprenant des données cliniques, des données d'expression de gène, ou les deux, dans lequel l'ensemble identifié de biomarqueurs comprend au moins MFAP2 et VEGFA ;
l'identification d'autres ensembles de biomarqueurs sur la base de l'ensemble identifié de biomarqueurs et de données restantes comprenant l'ensemble de données prédéterminées à l'exclusion de l'ensemble identifié de biomarqueurs ; et
la génération d'une signature pour chaque ensemble de biomarqueurs afin de prédire un résultat pour une patiente atteinte d'un cancer des ovaires,
dans lequel le fait de déterminer si la patiente devrait bénéficier de l'administration de bévacizumab est basé sur une prédiction d'ensemble utilisant une pluralité de signatures et de données de test de patient comprenant des données cliniques, des données d'expression de gène, ou les deux.

FIG. 1

FIG. 2

FIG. 3

EP 3 874 274 B1

FIG. 4A

FIG. 4B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- TR 002494
- CA 077598
- WO 2015118353 A
- US 20170253933 A
- US 20100292303 A
- US 8725426 B
- WO 2015109234 A
- US 8805761 B
- US 8655821 B

### Non-patent literature cited in the description

- **MOK et al.** *Cancer Cell,* 2009, vol. 16 (6), 521-532
- **SPIVEY et al.** *Cell Adhesion and Migration,* 2010, vol. 4 (2), 169-171
- **KOMMOSS et al.** *Clin Cancer Res Off JAm Assoc Cancer Res,* 2017, vol. 23 (14), 3794-801
- **PERREN et al.** *N Engl J Med,* 2011, vol. 365 (26), 2484-96
- **TOTHILL et al.** *Clin Cancer Res Off JAm Assoc Cancer Res,* 2008, vol. 14 (16), 5198-208
- **KONECNY et al.** *J Natl Cancer Inst,* 2014, vol. 106 (10), dju249
- **WINTERHOFF et al.** *Gynecol Oncol,* 2016, vol. 141 (1), 95-100
- **KONECNY et al.** *J Natl Cancer Inst.,* 2014, vol. 106 (10), dju249
- **OKEN et al.** *Am J Clin Oncol,* 1982, vol. 5, 649-655
- **STATNIKOV ; ALIFERIS.** *PLoS Computational Biology,* 2010, vol. 6 (5), e1000790
- **ALIFERIS et al.** *Journal of Machine Learning Research,* January 2010, vol. 11, 171-234
- **STATNIKOV et al.** *Journal of Machine Learning Research,* February 2013, vol. 14, 499-566
- **STATNIKOV.** A gentle introduction to support vector machines in biomedicine: Theory and methods. World Scientific Pub. Co, 2011, vol. 1
- A Gentle Introduction to Support Vector Machines in Biomedicine. **STATNIKOV et al.** Case Studies and Benchmarks. World Scientific Pub. Co, 2013, vol. 2
- **PERREN et al.** *N Engl J Med.,* 2011, vol. 365 (26), 2484-96
- **KOMMOSS et al.** *Clin Cancer Res Off JAm Assoc Cancer Res.,* 2017, vol. 23 (14), 3794-801
- **VAPNIK V.** The Nature of Statistical Learning Theory. Springer-Verlag, 2000
- A Training Algorithm for Optimal Margin Classifiers. **BOSER et al.** Proceedings of the Fifth Annual Workshop on Computational Learning Theory. New York, NY, USA. ACM, 1992, 144-152
- **ALIFERIS et al.** *J Mach Learn Res.,* 2010, vol. 11, 171-234
- **ALIFERIS et al.** *J Mach Learn Res.,* 2010, vol. 11, 235-284
- **VERHAAK et al.** *J Clin Invest.,* 2013, vol. 123 (1), 517-25
- **EFRON.** *J Am Stat Assoc.,* 1977, vol. 72 (359), 557-65
- **CLARK et al.** *Br J Cancer,* 2003, vol. 89 (2), 232-8
- **STATNIKOV et al.** *Journal of Machine Learning Research,* 2013, vol. 14, 499-566